# EUROPEAN PATENT SPECIFICATION

(11) **EP 2 330 124 B1**
(45) Date of publication and mention of the grant of the patent: **25.02.2015**
(21) Application number: 10012149.0
(22) Date of filing: 11.08.2006
(51) Int. Cl.: C07K 14/575, A61K 38/00

(54) **Hybrid polypeptides with selectable properties**
Hybridpolypeptide mit auswählbaren Eigenschaften
Polypeptides hybrides ayant des propriétés sélectionnables

(30) Priority: 11.08.2005 US 201664; 17.08.2005 US 206903; 12.12.2005 US 301744
(43) Date of publication of application: 08.06.2011
(62) Divisional of application: 06801467.9
(73) Proprietor: Amylin Pharmaceuticals, LLC, San Diego, CA 92121 (US); AstraZeneca Pharmaceuticals LP, Wilmington, DE (US)
(72) Inventor: Levy, Odile Esther, San Diego, CA 92121 (US); Hanley, Michael R., San Diego, CA 92121 (US); Jodka, Carolyn M., San Diego, CA 92121 (US); Lewis, Diana, San Diego, CA 92121 (US); Soares, Christopher J., San Diego, CA 92121 (US); Ghosh, Soumitra S., San Diego, CA 92121 (US); D'Souza, Lawrence, San Diego, CA 92121 (US); Parkes, David G., San Diego, CA 92121 (US); Mack, Christine M., San Diego, CA 92121 (US); Forood, Behrouz Bruce, San Diego, CA 92121 (US)
(74) Representative: Teall, Charlotte

(56) References cited:
- WO-A-00/73331
- WO-A-96/40196
- WO-A-03/059934
- WO-A-2005/077072
- WO-A1-98/05351

## Description

### FIELD OF THE INVENTION

The present invention relates to peptide chemistry, and more particularly to hybrid polypeptides with selectable properties.

### BACKGROUND OF THE INVENTION

Central to many metabolic diseases and disorders is the regulation of insulin levels and blood glucose levels. Insulin secretion is modulated in part by secretagogue hormones, termed as incretins, which are produced by enteroendocrine cells. ' The incretin hormone, glucagon-like peptide-1 ("GLP-1") is a peptide hormone secreted by intestinal cells that has been shown in multiple studies to produce an enhancing effect on insulin secretion. GLP-1 is processed from proglucagon in the gut and enhances nutrient-induced insulin release (Krcymann B., et al., Lancet, 2:1300-1303 (1987)). Various truncated forms of GLP-1, are known to stimulate insulin secretion (insulinotropic action) and cAMP formation [see, *e.g*., Mojsov, S., Int. J. Pep. Pro. Res., 40:333-343 (1992)]. A relationship between various *in vitro* laboratory experiments and mammalian, especially human, insulinotropic responses to exogenous administration of GLP-1, GLP-1(7-36) amide (SEQ ID NO: 61), and GLP-1(7-37) acid (SEQ ID NO: 204) has been established (see, *e.g.*, Nauck, M. A., et al., Diabetologia, 36:741-744 (1993); Gutniak, M., et al., New Eng. J. of Med., 326(20):1316-1322 (1992); Nauck, M. A., et al., J. Clin. Invest., 91:301-307 (1993); and Thorens, B., et al., Diabetes, 42:1219-1225 (1993)).

GLP-1(7-36) amide (SEQ ID NO: 61) exerts a pronounced antidiabetogenic effect in insulin-dependent diabetics by stimulating insulin sensitivity and by enhancing glucose-induced insulin release at physiological concentrations (Gutniak M., et al., New Eng. J. Med., 326:1316-1322 (1992)). When administered to non-insulin dependent diabetics, GLP-1(7-36) amide (SEQ ID NO: 61) stimulates insulin release, lowers glucagon secretion, inhibits gastric emptying and enhances glucose utilization (Nauck, 1993; Gutniak, 1992; Nauck, 1993). However, the use of GLP-1 type molecules for prolonged therapy of diabetes has been complicated because the serum half-life of such peptides is quite short.

More particularly, GLP-1 is a 30-amino acid peptide derived from proglucagon, a 160-amino acid prohormone. Actions of different prohormone convertases in the pancreas and intestine result in the production of glucagon and other ill-defined peptides, whereas cleavage of proglucagon results in the production of GLP-1 and GLP-2 as well as two other peptides. The amino acid sequence of GLP-1 is 100% homologous in all mammals studied so far, implying a critical physiological role. GLP-1 (7-37) acid is C-terminally truncated and amidated to form GLP-1 (7-36) NH₂ (SEQ ID NO: 61). The biological effects and metabolic turnover of the free acid GLP-1 (7-37) OH (SEQ ID NO: 204), and the amide, GLP-1 (7-36) NH₂ (SEQ ID NO: 61), are indistinguishable. By convention, the numbering of the amino acids is based on the processed GLP-1 (1-37) OH (SEQ ID NO: 59) from proglucagon. The biologically active GLP-1 is the result of further processing: GLP-1 (7-36) NH₂ (SEQ ID NO: 61). Thus the first amino acid of GLP-1 (7-37) OH (SEQ ID NO: 204) or GLP-1 (7-36)NH₂ (SEQ ID NO: 61) is ⁷His.

In the gastrointestinal tract, GLP-1 is produced by L-cells of intestinal, colonic and rectal mucosa, in response to stimulation by intraluminal glucose. The plasma half-life of active GLP-1 is <5 minutes, and its metabolic clearance rate is around 12-13 minutes (Holst, Gastroenterology 107(6):1848-55 (1994)). The major protease involved in the metabolism of GLP-1 is dipeptidyl peptidase (DPP) IV (CD26) which cleaves the N-terminal His-Ala dipeptide, thus producing metabolites, GLP-1 (9-37) OH (SEQ ID NO: 205) or GLP-1 (9-36) NH₂ (SEQ ID NO: 206) which are variously described as inactive, weak agonist or antagonists of GLP-1 receptor. The GLP-1 receptor (GLP-1R) is a G protein coupled receptor of 463 amino acid and is localized in pancreatic beta cells, in the lungs, and to a lesser extent in the brain, adipose tissue and kidneys. The stimulation of GLP-1R by GLP-1 (7-37) OH (SEQ ID NO: 204) or GLP-1 (7-36)NH₂ (SEQ ID NO: 61) results in adenylate cyclase activation, cAMP synthesis, membrane depolarization, rise in intracellular calcium and increase in glucose-induced insulin secretion (Holz et al., J. Biol. Chem. 270(30):17749-57 (1995)).

GLP-1 is a potent insulin secretagogue that is secreted from the intestinal mucosa in response to food intake. The profound incretin effect of GLP-1 is underscored by the fact that GLP-1R knockout mice are glucose-intolerant. The incretin response of i.v. infused GLP-1 is preserved in diabetic subjects, though the incretin response to oral glucose in these patients is compromised. GLP-1 administration by infusion or sc injections controls fasting glucose levels in diabetic patients, and maintains the glucose threshold for insulin secretion (Gutniak et al., N. Engl. J. Med. 326:1316-22 (1992); Nauck et al., Diabet. Med. 13:(9 Suppl 5):S39-S43 (1996); Nauck et al., J. Clin. Endocrinol. Metab. 76:912-917 (1993)). GLP-1 has shown tremendous potential as a therapeutic agent capable of augmenting insulin secretion in a physiological manner, while avoiding hypoglycemia associated with sulfonylurea drugs.

Other important effects of GLP-1 on glucose homeostasis are suppression of glucagon secretion and inhibition of gastric motility. GLP-1 inhibitory actions on pancreatic alpha cell secretion of glucagon leads to decreases in hepatic glucose production *via* reduction in gluconeogenesis and glycogenolysis. This antiglucagon effect of GLP-1 is preserved in diabetic patients.

The so-called ileal brake effect of GLP-1, in which gastric motility and gastric secretion are inhibited, is effected via vagal efferent receptors or by direct action on intestinal smooth muscle. Reduction of gastric acid secretion by GLP-1 contributes to a lag phase in nutrient availability, thus obviating the need for rapid insulin response. In summary, the gastrointestinal effects of GLP-1 contribute significantly to delayed glucose and fatty acid absorption and modulate insulin secretion and glucose homeostasis.

GLP-1 has also been shown to induce beta cell specific genes, such as GLUT-1 transporter, insulin (via the interaction of PDX-1 with insulin gene promoter), and hexokinase-1. Thus GLP-1 could potentially reverse glucose intolerance normally associated with aging, as demonstrated by rodent experiments. In addition, GLP-1 may contribute to beta cell neogenesis and increase beta cell mass, in addition to restoring beta cell function during states of beta cell insufficiency.

Central effects of GLP-1 include increases in satiety coupled with decreases in food intake, effected via the action of hypothalamic GLP-1R. A 48 hour continuous SC infusion of GLP-1 in type II diabetic subjects, decreased hunger and food intake and increased satiety. These anorectic effects were absent in GLP-1R knock out mice.

Exendins are another family of peptides implicated in insulin secretion. Exendins are found in the saliva of the Gila-monster, a lizard endogenous to Arizona, and the Mexican Beaded Lizard. Exendin-3 is present in the saliva of Heloderma horridum, and exendin-4 is present in the saliva of Heloderma suspectum (Eng, J., et al., J. Biol. Chem., 265:20259-62, 1990; Eng., J., et al., J. Biol. Chem., 267:7402-05 (1992)). The exendins have some sequence similarity to several members of the glucagon-like peptide family, with the highest identity, 53%, being to GLP-1 (Goke, et al., J. Biol. Chem., 268:19650-55 (1993)).

Exendin-4 binds the GLP-1 receptors on insulin-secreting TC1 cells, at dispersed acinar cells from guinea pig pancreas, and at parietal cells from stomach; the peptide also stimulates somatostatin release and inhibits gastrin release in isolated stomachs (Goke, et al., J. Biol. Chem., 268:19650-55 (1993); Schepp, et al., Eur. J. Pharmacol., 69:183-91 (1994); Eissele, et al., Life Sci., 55:629-34 (1994)). Exendin-3 and exendin-4 were found to bind the GLP-1 receptors on, to stimulating cAMP production in, and amylase release from, pancreatic acinar cells (Malhotra, R., et al., Relulatory Peptides, 41:149-56 (1992); Raufman, et al., J. Biol. Chem., 267:21432-37 (1992); Singh, et al., Regul. Pept., 53:47-59 (1994)). The use of the insulinotropic activities of exendin-3 and exendin-4 for the treatment of diabetes mellitus and the prevention of hyperglycemia has been proposed (Eng, U.S. Pat. No. 5,424,286).

Truncated exendin peptides such as exendin[9-39], a carboxyamidated molecule, and fragments 3-39 through 9-39 have been reported to be potent and selective antagonists of GLP-1 (Goke, et al., J. Biol. Chem., 268:19650-55 (1993); Raufman, J. P., et al., J. Biol. Chem., 266:2897-902 (1991); Schepp, W., et al., Eur. J. Pharm., 269:183-91 (1994); Montrose-Rafizadeh, et al., Diabetes, 45(Suppl. 2):152A (1996)). Exendin[9-39] (SEQ ID NO: 207) blocks endogenous GLP-1 *in vivo*, resulting in reduced insulin secretion (Wang, et al., J. Clin. Invest., 95:417-21 (1995); D'Alessio, et al., J. Clin. Invest., 97:133-38 (1996)). The receptor apparently responsible for the insulinotropic effect of GLP-1 has been cloned from rat pancreatic islet cells (Thorens, B., Proc. Natl. Acad. Sci. USA 89:8641-8645 (1992)). Exendins and exendin[9-39] bind to the cloned GLP-1 receptor (rat pancreatic -cell GLP-1 receptor: Fehmann HC, et al., Peptides, 15 (3): 453-6 (1994); human GLP-1 receptor: Thorens B, et al., Diabetes, 42 (11): 1678-82 (1993)). In cells transfected with the cloned GLP-1 receptor, exendin-4 is an agonist, *i.e.*, it increases cAMP, while exendin[9-39] (SEQ ID NO: 207) is an antagonist, *i.e*., it blocks the stimulatory actions of exendin-4 and GLP-1. *Id*.

More particularly, exendin-4 is a 39 amino acid C-terminal amidated peptide found in the saliva of the Gila Monster (Heloderma suspectum), with a 53% amino acid sequence identity to the GLP-1 peptide sequence. *See, e.g.*, Eng, J., et al. "Isolation and Characterization ofExendin-4, and Exendin-3 Analogue from Heloderma suspectum Venom," J. Bio. Chem., 267:11, p. 7402-7405 (1992), Young, A. A., et al., "Glucose-Lowering and Insulin-Sensitizing Actions of Exendin-4," Diabetes, Vol. 48, p. 1026-1034, May, 1999. In terms of its activity, exendin-4 is a highly specific agonist for the GLP-1 receptor, and, like GLP-1, is able to stimulate insulin secretion. Therefore, like GLP-1, exendin-4 is regarded as an insulinotropic peptide.

However, unlike GLP-1, exendin-4 has a relatively long half-life in humans, because of its resistance to the dipeptidyl peptidase IV which rapidly degrades the GLP-1 sequence in vivo. Furthermore, it has been shown that, as compared to GLP-1, exendin-4 has a stronger capability to stimulate insulin secretion, and that a lower concentration of exendin-4 may be used to obtain such stimulating activity. See, *e.g.*, U.S. Pat. No. 5,424,286, herein incorporated by reference. Therefore exendin-4 peptides or derivatives thereof (for examples of such derivatives, *see, e.g.*, U.S. Pat. No. 6,528,486, herein incorporated by reference, and its corresponding international application WO 01/04156) have a greater potential utility for the treatment of conditions involving the dysregulation of insulin levels (*e.g.*, conditions such as diabetes) than either insulin or GLP-1.

Another family of peptide hormones implicated in metabolic diseases and disorders is the amylin family of peptide hormones, including amylin, calcitonin, calcitonin gene related peptide, adrenomedullin, and intermedin (also known as "AFP-6"). Amylin is a 37-amino acid peptide hormone. It was isolated, purified and chemically characterized as the major component of amyloid deposits in the islets of pancreases of human Type 2 diabetics (Cooper et al., Proc. Natl. Acad. Sci., USA, 84:8628-8632 (1987)). The amylin molecule has two post-translational modifications: the C-terminus is amidated, and the cysteines in positions 2 and 7 are cross-linked to form an N-terminal loop. The sequence of the open reading frame of the human amylin gene shows the presence of the Lys-Arg dibasic amino acid proteolytic cleavage signal, prior to the N-terminal codon for Lys, and the Gly prior to the Lys-Arg proteolytic signal at the CLAIMS-terminal position, a typical sequence for amidation by protein amidating enzyme, PAM (Cooper et al., Biochem. Biophys. Acta, 1014:247-258 (1989)).

Amylin is believed to regulate gastric emptying, and suppress glucagon secretion and food intake, thus regulating the rate of glucose appearance in the circulation. It appears to complement the actions of insulin, which regulates the rate of glucose disappearance from the circulation and its uptake by peripheral tissues. These actions are supported by experimental findings in rodents and humans, which indicate that amylin complements the effects of insulin in postprandial glucose control by at least three independent mechanisms, all of which affect the rate of glucose appearance. First, amylin suppresses postprandial glucagon secretion. Compared to healthy adults, patients with type 1 diabetes have no circulating amylin and patients with type 2 diabetes have diminished postprandial amylin concentrations. Furthermore, infusion of an amylin specific monoclonal antibody, which bound circulating amylin, again resulted in greatly elevated glucagon concentrations relative to controls. Both of these results point to a physiological role of endogenous amylin in the regulation of postprandial glucagon secretion. Second, amylin slows gastrointestinal motility and gastric emptying. Finally, intrahypothalamic injections of rat amylin were shown to reduce feeding in rats and alter neurotransmitter metabolism in the hypothalamus. In certain studies, food intake was significantly reduced for up to eight hours following the intrahypothalamic injection of rat amylin and rat CGRP. In human trials, an amylin analog, pramlintide, has been shown to reduce weight or weight gain. Amylin may be beneficial in treating metabolic conditions such as diabetes and obesity. Amylin may also be used to treat pain, bone disorders, gastritis, to modulate lipids, in particular triglycerides, or to affect body composition such as the preferential loss of fat and sparing of lean tissue.

The hormone calcitonin (CT) was named for its secretion in response to induced hypercalcemia and its rapid hypocalcemic effect. It is produced in and secreted from neuroendocrine cells in the thyroid that have since been termed C cells. The best-studied action of CT(1-32) (SEQ ID NO: 48) is its effect on the osteoclast. *In vitro* effects of CT include the rapid loss of ruffled borders and decreased release of lysosomal enzymes. Ultimately, the inhibition of osteoclast functions by CT results in a decrease in bone resorption. However, neither a chronic reduction of serum CT in the case of thyroidectomy nor the increased serum CT found in medullary thyroid cancer appears to be associated with changes in serum calcium or bone mass. It is thus most likely that a major function of CT(1-32) (SEQ ID NO: 48) is to combat acute hypercalcemia in emergency situations and/or protect the skeleton during periods of "calcium stress" such as growth, pregnancy, and lactation. (Reviewed in Becker, JCEM, 89(4): 1512-1525 (2004) and Sexton, Current Medicinal Chemistry 6: 1067-1093 (1999)). Consistent with this is recent data from the calcitonin gene knockout mouse, which removes both the calcitonin and the CGRP-I peptides, that revealed that the mouse had normal levels of basal calcium-related values, but an increased calcemic response (Kurihara H, et al., Hypertens Res. 2003 Feb; 26 Suppl:S105-8).CT has an effect on plasma calcium levels and inhibits osteoclast function and is widely used for the treatment of osteoporosis. Therapeutically, salmon CT (sCT) appears to increase bone density and decrease fracture rates with minimal adverse effects. CT has also been successfully used over the past 25 years as a therapy for Paget's disease of bone, which is a chronic skeletal disorder that may result in enlarged or deformed bones in one or more regions of the skeleton. CT is also widely used for its analgesic effect on bone pain experienced during osteoporosis, although the mechanism for this effect is not clearly understood.

Calcitonin gene related peptide (CGRP) is a neuropeptide whose receptors are widely distributed in the body, including the nervous system and the cardiovascular system. This peptide seems to modulate sensory neurotransmission and is one of the most potent endogenous vasodilatory peptide discovered to date. Reported biological effects for CGRP include: modulation of substance P in inflammation, nicotinic receptor activity at the neuromuscular junction, stimulation of pancreatic enzyme secretion, a reduction of gastric acid secretion, peripheral vasodilation, cardiac acceleration, neuro-modulation, regulation of calcium metabolism, osteogenic stimulation, insulin secretion, an increase in body temperature and a decrease in food intake. (Wimalawansa, Amylin, calcitonin gene-related peptide, calcitonin and ADM: a peptide superfamily. Crit Rev Neurobiol. 1997; 11(2-3):167-239). An important role of CGRP is to control blood flow to various organs by its potent vasodilatory actions, as evidenced by a decrease of mean arterial pressure following intravenous administration of α-CGRP. The vasodilatory actions are also supported by recent analysis of homozygous knockout CGRP mice, which demonstrated elevated peripheral vascular resistance and high blood pressure caused by increased peripheral sympathetic activity (Kurihara H, et al., Targeted disruption of ADM and αCGRP genes reveals their distinct biological roles. Hypertens Res. 2003 Feb; 26 Suppl:S105-8). Thus, CGRP appears to elicit vasodilatory effects, hypotensive effects and an increase in heart rate among other actions.

Prolonged infusion of CGRP into patients with congestive cardiac failure has shown a sustained beneficial effect on hemodynamic functions without adverse effects, suggesting a use in heart failure. Other indications of CGRP use include renal failure, acute and chronic coronary artery ischemia, treatment of cardiac arrhythmia, other peripheral vascular disease such as Raynaud's phenomenon, subarachnoid hemorrhage, hypertension, and pulmonary hypertension. Preeclamptic toxemia of pregnancy and preterm labor are also potentially treatable. (Wimalawansa, 1997). Recent therapeutic uses include the use of CGRP antagonists for the treatment of migraine headaches.

Adrenomedullin (ADM) is almost ubiquitously expressed with many more tissues containing the peptide than not. A published review of ADM, (Hinson, J.P. et al., Endocrine Reviews (2000) 21(2): 138-167) details its effects on the cardiovascular system, cellular growth, the central nervous system and the endocrine system, with a range of biological actions including vasodilation, cell growth, regulation of hormone secretion, and natriuresis. Studies in rat, cat, sheep, and man confirm that intravenous infusion of ADM results in potent and sustained hypotension, and is comparable to that of CGRP. However, the hypotensive effect of ADM on mean arterial pressure in the anesthetized rat is not inhibited by the CGRP antagonist CGRP₈₋₃₇ suggesting that this effect is not mediated via CGRP receptors. Acute or chronic administration of human ADM in rats, anesthetized, conscious or hypertensive, results in a significant decrease in total peripheral resistance accompanied by a fall in blood pressure, with a concomitant rise in heart rate, cardiac output and stroke volume.

ADM has also been proposed as an important factor in embryogenesis and differentiation and as an apoptosis survival factor for rat endothelial cells. This is supported by recent mouse ADM knockout studies, in which mice homozygous for loss of the ADM gene demonstrated defective vascular formation during embryogenesis and thus died mid-gestation. It was reported that ADM +/- heterozygous mice had high blood pressure along with susceptibility to tissue injury (Kurihara H, et al., Hypertens Res. 2003 Feb; 26 Suppl:S105-8).

ADM affects such endocrine organs as the pituitary, the adrenal gland, reproductive organs and the pancreas. The peptide appears to have a role in inhibiting ACTH release from the pituitary. In the adrenal gland, it appears to affect the secretory activity of the adrenal cortex in both rat and human and it increases adrenal blood flow, acting as a vasodilator in the adrenal vascular bed in intact rats. ADM has been shown to be present throughout the female reproductive tract and plasma levels are elevated in normal pregnancy. Studies in a rat model of preeclampsia show that ADM can reverse hypertension and decrease pup mortality when given to rats during late gestation. Because it did not have a similar effect in animals in early gestation or non-pregnant rats in the preeclampsia model, this suggests that ADM may play an important regulatory role in the utero-placental cardiovascular system. In the pancreas, ADM most likely plays an inhibitory role since it attenuated and delayed insulin response to an oral glucose challenge, resulting in initial elevated glucose levels. ADM can also affect renal function. A bolus administered peripherally can significantly lower mean arterial pressure and raise renal blood flow, glomerular filtration rate and urine flow. In some cases, there is also an increase in Na+ excretion.

ADM also has other peripheral effects on bone and on the lung. For bone, studies have supported a role beyond the cardiovascular system and fluid homeostasis and have demonstrated that ADM acts on fetal and adult rodent osteoblasts to increase cell growth comparable to those of known osteoblast growth factors such as transforming growth factor-β. This is important clinically as one of the major challenges in osteoporosis research is to develop a therapy that increases bone mass via osteoblastic stimulation. In the lung, ADM not only causes pulmonary vasodilation, but also inhibits bronchoconstriction induced by histamine or acetylcholine. Recent studies using aerosolized ADM to treat pulmonary hypertension in a rat model indicate that inhalation treatment of this condition is effective, as evidenced by the fact that mean pulmonary arterial pressure and total pulmonary resistance were markedly lower in rats treated with ADM than in those given saline. This result was achieved without an alteration in systemic arterial pressure or heart rate (Nagaya N et al., Am J Physiol Heart Circ Physiol. 2003;285:H2125-31).

In healthy volunteers, i.v. infusion of ADM has been shown to reduce arterial pressure and to stimulate heart rate, cardiac output, plasma levels of cAMP, prolactin, norepinephrine and rennin. In these patients, there was little or no increase in urine volume or sodium excretion observed. In patients with heart failure or chronic renal failure, i.v. ADM had similar effects to those seen in normal subjects, and also induced diuresis and natriuresis, depending on the dose administered (Nicholls, MG et al. Peptides. 2001; 22:1745-1752) Experimental ADM treatment has also been shown to be beneficial in arterial and pulmonary hypertension, septic shock and ischemia/reperfusion injury (Beltowski J., Pol J Pharmacol. 2004;56:5-27). Other indications for ADM treatment include: peripheral vascular disease, subarachnoid hemorrhage, hypertension, preeclamptic toxemia of pregnancy and preterm labor, and osteoporosis.

Expression of AFP-6 (*i.e.*, intermedin) is primarily in the pituitary and gastrointestinal tract. A specific receptor for AFP-6 has not been reported; however, binding studies indicate that AFP-6 binds to all the known receptors of the Amylin Family. AFP-6 has been shown to increase cAMP production in SK-N-MC and L6 cells expressing endogenous CGRP receptors and competes with labeled CGRP for binding to its receptors in these cells. In published *in vivo* studies, AFP-6 administration led to blood pressure reduction in both normal and spontaneously hypertensive rats, most likely via interactions with the CRLR/RAMP receptors. *In vivo* administration in mice led to a suppression of gastric emptying and food intake. (Roh et al. J Biol Chem. 2004 Feb 20;279(8):7264-74.)

It has been reported that the biological actions of amylin family peptide hormones are generally mediated via binding to two closely related type II G protein-coupled receptors (GPCRs), the calcitonin receptor (CTR) and the calcitonin receptor like receptor (CRLR). Cloning and functional studies have shown that CGRP, ADM, and amylin interact with different combinations of CTR or the CRLR and the receptor activity modifying protein (RAMP). Many cells express multiple RAMPs. It is believed that co-expression of RAMPs and either the CTR or CRLR is required to generate functional receptors for calcitonin, CGRP, ADM, and amylin. The RAMP family comprises three members (RAMP1, -2, and -3), which share less then 30% sequence identity, but have a common topological organization. Co-expression of CRLR and RAMP1 leads to the formation of a receptor for CGRP. Co-expression of CRLR and RAMP2 leads to the formation of a receptor for ADM. Co-expression of CRLR and RAMP3 leads to the formation of a receptor for ADM and CGRP. Co-expression of hCTR2 and RAMP1 leads to the formation of a receptor for amylin and CGRP. Co-expression of hCTR2 and RAMP3 leads to the formation of a receptor for amylin.

Yet another peptide hormone family implicated in metabolic diseases and disorders is the leptin family. The mature form of circulating leptin is a 146-amino acid protein that is normally excluded from the CNS by the blood-brain barrier (BBB) and the blood-CSF barrier. See, *e.g.*, Weigle et al., 1995. J Clin Invest 96 : 2065-2070. Leptin is the afferent signal in a negative feedback loop regulating food intake and body weight. The leptin receptor is a member of the cytokine receptor family. Leptin's anorexigenic effect is dependent on binding to homodimer of the Ob-Rb isoform of this receptor which encodes a long intra-cytoplasmic domain that includes several motifs for protein-protein interaction. Ob-Rb is highly expressed in the hypothalamus suggesting that this brain region is an important site of leptin action. Mutation of the mouse *ob* gene has been demonstrated to result in a syndrome that exhibits-pathophysiology that includes: obesity, increased body fat deposition, hyperglycemia, hyperinsulinemia, hypothermia, and impaired thyroid and reproductive functions in both male and female homozygous ob/ob obese mice (see *e.g.*, Ingalis, et al., 1950. J Hered 41 : 317-318. Therapeutic uses for leptin or leptin receptor include (i) diabetes (see, *e.g.*, PCT Patent Applications WO 98/55139, WO 98/12224, and WO 97/02004); (ii) hematopoiesis (see, *e.g.*, PCT Patent Applications WO 97/27286 and WO 98/18486); (iii) infertility (see, *e.g.*, PCT Patent Applications WO 97/15322 and WO 98/36763); and (iv) tumor suppression (see, *e.g.*, PCT Patent Applications WO 98/48831).

The leptin receptor (OB-R) gene has been cloned (GenBank Accession No. AF098792) and mapped to the db locus (see, *e.g.*, Tartaglia, et al., 1995. Cell 83: 1263-1271). Several transcripts of the OB-R, resulting from alternative splicing, have also been identified. Defects in OB-R produce a syndrome in the mutant diabetic ob/ob mouse that is phenotypically identical to the ob/ob mouse (see, *e.g.*, Ghilardi, et al., 1996. Proc. Natl. Acad. Sci. USA 93: 6231-6235). In contrast to ob/ob mice, however, administration of recombinant leptin to C57BLKS/J-m ob/ob mice does not result in reduced food intake and body weight (see, *e.g.*, Roberts and Greengerg, 1996. Nutrition Rev. 54: 41-49).

Most leptin-related studies able to report weight loss activity from administration of recombinant leptin, leptin fragments and/or leptin receptor variants have administered said constructs directly into the ventricles of the brain. See *e.g.*, Weigle, et al., 1995. J Clin Invest 96: 2065-2070; Barash, et al., 1996. Endocrinology 137: 3144-3147.

Other studies have shown significant weight loss activity due to administration of leptin peptides through intraperitoneally (i.p.) administration to test subjects. See, Grasso et al., 1997. Endocrinology 138: 1413-1418. Further, leptin fragments, and most particularly an 18 amino acid fragment comprising residues taken from full length human leptin, have been reported to function in weight loss, but only upon direct administration through an implanted cannula to the lateral brain ventricle of rats. See, *e.g.*, PCT Patent Applications WO 97/46585.

Another peptide hormone implicated in metabolic diseases and disorders is cholecystokinin (CCK). CCK was reportedly identified in 1928 from preparations of intestinal extracts by its ability to stimulate gallbladder contraction. Other biological actions of CCK have since been reported, including stimulation of pancreatic secretion, delayed gastric emptying, stimulation of intestinal motility and stimulation of insulin secretion. See Lieverse et al., Ann. N.Y. Acad Sci. 713: 268-272 (1994). The actions of CCK, also reportedly include effects on cardiovascular function, respiratory function, neurotoxicity and seizures, cancer cell proliferation, analgesia, sleep, sexual and reproductive behaviors, memory, anxiety and dopamine-mediated behaviors. Crawley and Corwin, Peptides 15: 731-755 (1994). Other reported effects of CCK include stimulation of pancreatic growth, stimulation of gallbladder contraction, inhibition of gastric acid secretion, pancreatic polypeptide release and a contractile component of peristalsis. Additional reported effects of CCK include vasodilation. Walsh, "Gastrointestinal Hormones," In Physiology of the Gastrointestinal Tract (3d ed. 1994; Raven Press, New York).

It has been reported that injections of combinations of glucagon, CCK and bombesin potentiated the inhibition of intake of condensed milk test meals in nondeprived rats over the inhibitions observed with individual compounds. Hinton et al., Brain Res. Bull. 17:615-619 (1986). It has also been reported that glucagon and CCK synergistically inhibit sham feeding in rats. LeSauter and Geary, Am. J. Physiol. 253:R217-225 (1987); Smith and Gibbs, Annals N.Y. Acad. Sci. 713:236-241 (1994). It has also been suggested that estradiol and CCK can have a synergistic effect on satiety. Dulawa et al., Peptides 15:913-918 (1994); Smith and Gibbs, *supra.* It has also been proposed that signals arising from the small intestine in response to nutrients therein may interact synergistically with CCK to reduce food intake. Cox, Behav. Brain Res. 38:35-44 (1990). Additionally, it has been reported that CCK induces satiety in several species. For example, it has been reported that feeding depression was caused by CCK injected intraperitoneally in rats, intraarterially in pigs, intravenously in cats and pigs, into the cerebral ventricles in monkeys, rats, dogs and sheep, and intravenously in obese and non-obese humans. See Lieverse *et al., supra.* Studies from several laboratories have reportedly confirmed the behavioral specificity of low doses of CCK on inhibition in feeding, by comparing responding for food to responding for nonfood reinforcers in both monkeys and rats and by showing that CCK elicits the sequence of behaviors normally observed after meal ingestion (*i.e*., the postprandial satiety sequence). Additionally, comparison of behavior after CCK to behavior after food ingestion, alone or in combination with CCK has reportedly revealed behavioral similarities between CCK and food ingestion. Crawley and Corwin, *supra.* It has also been reported that CCK in physiological plasma concentrations inhibits food intake and increases satiety in both lean and obese humans. See Lieverse *et al., supra.*

CCK was characterized in 1966 as a 33-amino acid peptide. Crawley and Corwin, supra. Species-specific molecular variants of the amino acid sequence of CCK have been identified. The 33-amino acid sequence and a truncated peptide, its 8-amino acid C-terminal sequence (CCK-8) have been reportedly identified in pig, rat, chicken, chinchilla, dog and humans. A 39-amino acid sequence was reportedly found in pig, dog and guinea pig. A 58-amino acid sequence was reported to have been found in cat, dog and humans. Frog and turtle reportedly show 47-amino acid sequences homologous to both CCK and gastrin. Very fresh human intestine has been reported to contain small amounts of an even larger molecule, termed CCK-83. In the rat, a principal intermediate form has been reportedly identified, and is termed CCK-22. Walsh, "Gastrointestinal Hormones," In Physiology of the Gastrointestinal Tract (3d ed. 1994; Raven Press, New York). A non-sulfated CCK-8 and a tetrapeptide (termed CCK-4 (CCK(30-33)) have been reported in rat brain. The C-terminal pentapeptide (termed CCK-4 (CCK(29-33)) conserves the structural homology of CCK, and also homology with the neuropeptide, gastrin. The C-terminal sulfated octapeptide sequence, CCK-8, is reportedly relatively conserved across species. Cloning and sequence analysis of a cDNA encoding preprocholecystokinin from rat thyroid carcinoma, porcine brain, and porcine intestine reportedly revealed 345 nucleotides coding for a precursor to CCK, which is 115 amino acids and contains all of the CCK sequences previously reported to have been isolated. Crawley and Corwin, *supra.*

CCK was characterized in 1966 as a 33-amino acid peptide. Crawley and Corwin, supra. Species-specific molecular variants of the amino acid sequence of CCK have been identified. The 33-amino acid sequence and a truncated peptide, its 8-amino acid C-terminal sequence (CCK-8) have been reportedly identified in pig, rat, chicken, chinchilla, dog and humans. A 39-amino acid sequence was reportedly found in pig, dog and guinea pig. A 58-amino acid sequence was reported to have been found in cat, dog and humans. Frog and turtle reportedly show 47-amino acid sequences homologous to both CCK and gastrin. Very fresh human intestine has been reported to contain small amounts of an even larger molecule, termed CCK-83. In the rat, a principal intermediate form has been reportedly identified, and is termed CCK-22. Walsh, "Gastrointestinal Hormones," In Physiology of the Gastrointestinal Tract (3d ed. 1994; Raven Press, New York). A non-sulfated CCK-8 and a tetrapeptide (termed CCK-4 (CCK(30-33); SEQ ID NO: 208) have been reported in rat brain. The C-terminal pentapeptide (termed CCK-4 (CCK(29-33); SEQ ID NO: 209) conserves the structural homology of CCK, and also homology with the neuropeptide, gastrin. The C-terminal sulfated octapeptide sequence, CCK-8, is reportedly relatively conserved across species. Cloning and sequence analysis of a cDNA encoding preprocholecystokinin from rat thyroid carcinoma, porcine brain, and porcine intestine reportedly revealed 345 nucleotides coding for a precursor to CCK, which is 115 amino acids and contains all of the CCK sequences previously reported to have been isolated. Crawley and Corwin, *supra.*

CCK is said to be distributed throughout the central nervous system and in endocrine cells and enteric nerves of the upper small intestine. CCK agonists include CCK itself (also referred to as CCK-33), CCK-8 (CCK(26-33); SEQ ID NO: 55), non-sulfated CCK-8, pentagastrin (CCK-5 or CCK(29-33); SEQ ID NO: 209), and the tetrapeptide, CCK-4 (CCK(30-33); SEQ ID NO: 208). At the pancreatic CCK receptor, CCK-8 reportedly displaced binding with a 1000-5000 greater potency than unsulfated CCK-8 or CCK-4, and CCK-8 has been reported to be approximately 1000-fold more potent than unsulfated CCK-8 or CCK-4 in stimulating pancreatic amylase secretion. Crawley and Corwin, supra. In homogenates from the cerebral cortex, CCK receptor binding was said to be displaced by unsulfated CCK-8 and by CCK-4 at concentrations that were equimolar, 10-fold or 100-fold greater than sulfated CCK-8. Id.

Yet another family of peptide hormones implicated in metabolic diseases and disorders is the pancreatic polypeptide family ("PPF"). Pancreatic polypeptide ("PP") was discovered as a contaminant of insulin extracts and was named by its organ of origin rather than functional importance (Kimmel et al., Endocrinology 83: 1323-30 (1968)). PP is a 36-amino acid peptide containing distinctive structural motifs. A related peptide was subsequently discovered in extracts of intestine and named Peptide YY ("PYY") because of the N- and C-terminal tyrosines (Tatemoto, Proc. Natl. Acad. Sci. USA 79: 2514-8 (1982)). A third related peptide was later found in extracts of brain and named Neuropeptide Y ("NPY") (Tatemoto, Proc. Natl. Acad. Sci. USA 79: 5485-9 (1982); Tatemoto et al., Nature 296: 659-60 (1982)).

These three related peptides have been reported to exert various biological effects. Effects of PP include inhibition of pancreatic secretion and relaxation of the gallbladder. Centrally administered PP produces modest increases in feeding that may be mediated by receptors localized to the hypothalamus and brainstem (reviewed in Gehlert, Proc. Soc. Exp. Biol. Med. 218: 7-22 (1998)).

Release of PYY occurs following a meal. An alternate molecular form of PYY is PYY(3-36) (SEQ ID NO: 58) (Eberlein et al., Peptides 10: 797-803 (1989); Grandt et al., Regul. Pept. 51: 151-9 (1994)). This fragment constitutes approximately 40% of total PYY-like immunoreactivity in human and canine intestinal extracts and about 36% of total plasma PYY immunoreactivity in a fasting state to slightly over 50% following a meal. It is apparently a dipeptidyl peptidase-IV (DPP4) cleavage product of PYY. PYY(3-36) (SEQ ID NO: 58) is reportedly a selective ligand at the Y2 and Y5 receptors, which appear pharmacologically unique in preferring N-terminally truncated (*i.e.*, C-terminal fragments of) NPY analogs. Peripheral administration of PYY reportedly reduces gastric acid secretion, gastric motility, exocrine pancreatic secretion (Yoshinaga et al., Am. J. Physiol. 263: G695-701 (1992); Guan et al., Endocrinology 128: 911-6 (1991); Pappas et al., Gastroenterology 91: 1386-9 (1986)), gallbladder contraction and intestinal motility (Savage et al., Gut 28: 166-70 (1987)). The effects of central injection of PYY on gastric emptying, gastric motility and gastric acid secretion, as seen after direct injection in or around the hindbrain/brainstem (Chen and Rogers, Am. J. Physiol. 269: R787-92 (1995); Chen et al., Regul. Pept. 61: 95-98 (1996); Yang and Tache, Am. J. Physiol. 268: G943-8 (1995); Chen et al., Neurogastroenterol. Motil. 9: 109-16 (1997)), may differ from those effects observed after peripheral injection. For example, centrally administered PYY had some effects opposite to those described herein for peripherally injected PYY(3-36) (SEQ ID NO: 58) in that gastric acid secretion was stimulated, not inhibited. Gastric motility was suppressed only in conjunction with TRH stimulation, but not when administered alone, and was indeed stimulatory at higher doses through presumed interaction with PP receptors. PYY has been shown to stimulate food and water intake after central administration (Morley et al., Brain Res. 341: 200-3 (1985); Corp et al., Am. J. Physiol. 259: R317-23 (1990)).

Metabolic diseases and disorders take on many forms, including obesity, diabetes, dyslipidemia, insulin resistance, cellular apoptosis, *etc.* Obesity and its associated disorders are common and very serious public health problems in the United States and throughout the world. Upper body obesity is the strongest risk factor known for type 2 diabetes mellitus, and is a strong risk factor for cardiovascular disease. Obesity is a recognized risk factor for hypertension, atherosclerosis, congestive heart failure, stroke, gallbladder disease, osteoarthritis, sleep apnea, reproductive disorders such as polycystic ovarian syndrome, cancers of the breast, prostate, and colon, and increased incidence of complications of general anesthesia (*see, e.g.*, Kopelman, Nature 404: 635-43 (2000)). It reduces life-span and carries a serious risk of co-morbidities above, as well disorders such as infections, varicose veins, acanthosis nigricans, eczema, exercise intolerance, insulin resistance, hypertension hypercholesterolemia, cholelithiasis, orthopedic injury, and thromboembolic disease (Rissanen et al., Br. Med. J. 301: 835-7 (1990)). Obesity is also a risk factor for the group of conditions called insulin resistance syndrome, or "Syndrome X." Recent estimate for the medical cost of obesity and associated disorders is $150 billion worldwide. The pathogenesis of obesity is believed to be multifactorial but the basic problem is that in obese subjects nutrient availability and energy expenditure do not come into balance until there is excess adipose tissue. Obesity is currently a poorly treatable, chronic, essentially intractable metabolic disorder. A therapeutic drug useful in weight reduction of obese persons could have a profound beneficial effect on their health.

Diabetes is a disorder of carbohydrate metabolism characterized by hyperglycemia and glucosuria resulting from insufficient production or utilization of insulin. Diabetes severely affects the quality of life of large parts of the populations in developed countries. Insufficient production of insulin is characterized as type 1 diabetes and insufficient utilization of insulin is type 2 diabetes. However, it is now widely recognized that there are many distinct diabetes related diseases which have their onset long before patients are diagnosed as having overt diabetes. Also, the effects from the suboptimal control of glucose metabolism in diabetes gives rise to a wide spectrum of related lipid and cardiovascular disorders.

Dyslipidemia, or abnormal levels of lipoproteins in blood plasma, is a frequent occurrence among diabetics. Dyslipidemia is typically characterized by elevated plasma triglycerides, low HDL (High Density Lipoprotein) cholesterol, normal to elevated levels of LDL (Low Density Lipoprotein) cholesterol and increased levels of small dense, LDL (Low Density Lipoprotein) particles in the blood. Dyslipidemia is one of the main contributors to the increased incidence of coronary events and deaths among diabetic subjects. Epidemiological studies have confirmed this by showing a several-fold increase in coronary deaths among diabetic subjects when compared with non-diabetic subjects. Several lipoprotein abnormalities have been described among diabetic subjects.

Insulin resistance is the diminished ability of insulin to exert its biologically action across a broad range of concentrations. In insulin resistance, the body secretes abnormally high amounts of insulin to compensate for this defect and a state of impaired glucose tolerance develops. Failing to compensate for the defective insulin action, the plasma glucose concentration inevitable rises, resulting in the clinical state of diabetes. It is being recognized that insulin resistance and relative hyperinsulinemia have a contributory role in obesity, hypertension, atherosclerosis and type 2 diabetes. The association of insulin resistance with obesity, hypertension and angina has been described as a syndrome, Syndrome X, having insulin resistance as the common pathogenic link.

Apoptosis is an active process of cellular self-destruction that is regulated by extrinsic and intrinsic signals occurring during normal development. It is well documented that apoptosis plays a key role in regulation of pancreatic endocrine beta cells. There is increasing evidence that in adult mammals the beta-cell mass is subject to dynamic changes to adapt insulin production for maintaining euglycemia in particular conditions, such as pregnancy and obesity. The control of beta cell mass depends on a subtle balance between cell proliferation, growth and programmed cell death (apoptosis). A disturbance of this balance may lead to impairment of glucose homeostasis. For example, it is noteworthy that glucose intolerance develops with aging when beta cell replication rates are reduced and human autopsy studies repeatedly showed a 40-60% reduction of beta cell mass in patients with non-insulin-dependent-diabetes mellitus compared with nondiabetic subjects. It is generally agreed that insulin resistance is an invariable accompaniment of obesity but that normoglycemia is maintained by compensatory hyperinsulinemia until the beta cells become unable to meet the increased demand for insulin, at which point type 2 diabetes begins.

Attempts to treat the multiple abnormalities associated with diabetes have prompted for the administration of several anti-diabetic medicaments in order to address these abnormalities in the different patients. Examples of anti-diabetic medicaments are proteins such as insulin and insulin analogues, and small molecules such as insulin sensitizers, insulin secretagogues and appetite regulating compounds.

There remains a need to develop polypeptides useful in the above described metabolic diseases, conditions, and disorders. Accordingly, it is an object of the present invention to provide hybrid polypeptides and methods for producing and using them. The compounds of the invention find use in the metabolic diseases, conditions, and disorders described above and herein.

All documents referred to herein are incorporated by reference into the present application as though fully set forth herein.

### SUMMARY OF THE INVENTION

The present invention relates as defined in the claims to novel, selectable hybrid polypeptides useful as agents for the treatment and prevention of metabolic diseases and disorders which can be alleviated by control of plasma glucose levels, insulin levels, and/or insulin secretion, such as diabetes and diabetes-related conditions. Such conditions and disorders include, but are not limited to, hypertension, dyslipidemia, cardiovascular disease, eating disorders, insulin-resistance, obesity, and diabetes mellitus of any kind, including type 1, type 2, and gestational diabetes.

In one aspect of the disclosure, hybrid polypeptides exhibiting at least one hormonal activity are provided. The hybrid polypeptides of the disclosure comprise at least two bio-active peptide hormone modules covalently linked together, wherein at least one of the bio-active peptide hormone modules exhibits at least one hormonal activity of a component peptide hormone. The bio-active peptide hormone modules are independently selected from: component peptide hormones, fragments of component peptide hormones that exhibit at least one hormonal activity of the component peptide hormones, analogs and derivatives of component peptide hormones that exhibit at least one hormonal activity of the component peptide hormones, fragments of analogs and derivatives of component peptide hormones that exhibit at least one hormonal activity of the component peptide hormones, and peptidic enhancers.

In one aspect of the disclosure a hybrid polypeptide is exhibiting at least one hormonal activity, the hybrid polypeptide containing at least a first bio-active peptide hormone module covalently linked to at least one additional bio-active peptide hormone module; wherein the bio-active peptide hormone modules are independently selected from the group consisting of: component peptide hormones; fragments of component peptide hormones that exhibit at least one hormonal activity of the component peptide hormones; analogs and derivatives of component peptide hormones that exhibit at least one hormonal activity of the component peptide hormones; fragments of analogs and derivatives of component peptide hormones that exhibit at least one hormonal activity of the component peptide hormones; and peptidic enhancers. The component peptide hormones are typically independently selected from at least two of the group consisting of amylin, adrenomedullin (ADM), calcitonin (CT), calcitonin gene related peptide (CGRP), intermedin, cholecystokinin ("CCK"), leptin, peptide YY (PYY), glucagon-like peptide-1 (GLP-1), glucagon-like peptide 2 (GLP-2), oxyntomodulin (OXM), a natriuretic peptide, and exendin-4. Typically peptidic enhancers are independently selected from the group consisting of structural motifs of component peptide hormones that impart a desired chemical stability, conformational stability, metabolic stability, bioavailability, organ/tissue targeting, receptor interaction, protease inhibition, plasma protein binding, or other pharmacokinetic characteristic to the hybrid polypeptide, and structural motifs of analogs or derivatives of component peptide hormones that impart a desired chemical stability, conformational stability, metabolic stability, bioavailability, organ/tissue targeting, receptor interaction, protease inhibition, plasma protein binding, or other pharmacokinetic characteristic to the hybrid polypeptide. In yet a further embodiment at least one of the bio-active peptide hormone modules exhibits at least one hormonal activity of a component peptide hormone. In yet further alternative embodiments when the at least one bio-active peptide hormone module that exhibits at least one hormonal activity of a component peptide hormone is amylin, a fragment of amylin that exhibits at least one hormonal activity, an analog or derivative of amylin that exhibits at least one hormonal activity, or a fragment of an analog or derivative of amylin that exhibits at least one hormonal activity, and the at least one other bio-active peptide hormone module is CCK, a fragment of CCK that exhibits at least one hormonal activity, an analog or derivative of CCK that exhibits at least one hormonal activity, a fragment of an analog or derivative of CCK that exhibits at least one hormonal activity, CT, a fragment of CT that exhibits at least one hormonal activity, an analog or derivative of CT that exhibits at least one hormonal activity, or a fragment of an analog or derivative of CT that exhibits at least one hormonal activity, then the hybrid polypeptide can further contain at least three bio-active peptide hormone modules selected from at least three different component peptide hormones. In yet a further alternative embodiment, when the at least one bio-active peptide hormone module that exhibits at least one hormonal activity of a component peptide hormone is GLP-1, a fragment of GLP-1 that exhibits at least one hormonal activity, an analog or derivative of GLP-1 that exhibits at least one hormonal activity, or a fragment of an analog or derivative of GLP-1 that exhibits at least one hormonal activity, and the at least one other bio-active peptide hormone module is a peptidic enhancer comprising an exendin fragment, then the hybrid polypeptide can further contain at least three bio-active peptide hormone modules.

Component peptide hormones of the disclosure include: amylin, adrenomedullin (ADM), calcitonin (CT), calcitonin gene related peptide (CGRP), intermedin, cholecystokinin ("CCK"), leptin, peptide YY (PYY), glucagon-like peptide-1 (GLP-1), glucagon-like peptide 2 (GLP-2), oxyntomodulin (OXM), natriuretic peptides, and exendin-4;

Peptidic enhancers of the disclosure include: structural motifs of component peptide hormones that impart a desired chemical stability, conformational stability, metabolic stability, bioavailability, organ/tissue targeting, receptor interaction, protease inhibition, plasma protein binding, or other pharmacokinetic characteristic to the hybrid polypeptide, and structural motifs of analogs or derivatives of component peptide hormones that impart a desired chemical stability, conformational stability, metabolic stability, bioavailability, organ/tissue targeting, receptor interaction, protease inhibition, plama protein binding, or other pharmacokinetic characteristic to the hybrid polypeptide.

In another aspect of the disclosure, methods for treating or preventing obesity are provided, wherein the method comprises administering a therapeutically or prophylactically effective amount of a hybrid polypeptide of the invention to a subject in need thereof. In a preferred embodiment, the subject is an obese or overweight subject. While "obesity" is generally defined as a body mass index over 30, for purposes of this disclosure, any subject, including those with a body mass index of less than 30, who needs or wishes to reduce body weight is included in the scope of "obese." Subjects who are insulin resistant, glucose intolerant, or have any form of diabetes mellitus (*e.g.*, type 1, 2 or gestational diabetes) can benefit from this method.

In yet another aspect of the disclosure, methods of reducing food intake, reducing nutrient availability, causing weight loss, treating diabetes mellitus or diabetes-associated conditions, and improving lipid profile (including reducing LDL cholesterol and triglyceride levels and/or changing HDL cholesterol levels) are provided, wherein the methods comprise administering to a subject an effective amount of a hybrid polypeptide of the disclosure. In a preferred embodiment, the methods of the disclosure are used to treat or prevent conditions or disorders which can be alleviated by reducing nutrient availability in a subject in need thereof, comprising administering to said subject a therapeutically or prophylactically effective amount of a hybrid polypeptide of the disclosure. In another embodiment, the methods of the disclosure are used to treat or prevent conditions or disorders which can be alleviated by control plasma glucose levels, insulin levels, and/or insulin secretion. In yet another embodiment, the methods of the disclosure are used to treat diabetes and/or diabetes-related conditions. Such conditions and disorders include, but are not limited to, hypertension, dyslipidemia, cardiovascular disease, eating disorders, insulin-resistance, obesity, and diabetes mellitus of any kind, including Type I, Type II, and gestational diabetes, diabetes complications (neuropathy (based on, *e.g.*, neurotrophic actions of exendin-4), neuropathic pain (based on, *e.g.*, amylin action), retinopathy, nephropathy, conditions of insufficient pancreatic beta cell mass (based on, *e.g*., islet neogenesis actions of exendin-4 and GLP-1).

The present disclosure also relates to pharmaceutical compositions comprising a therapeutically or prophylactically effective amount of at least one hybrid polypeptide of the invention, or a pharmaceutically acceptable salt thereof, together with pharmaceutically acceptable diluents, preservatives, solubilizers, emulsifiers, adjuvants and/or carriers useful in the delivery of the hybrid polypeptides.

These and other aspects of the invention will be more clearly understood with reference to the following preferred embodiments and detailed description.

### BRIEF DESCRIPTION OF THE DRAWINGS

Figure 1 demonstrates the effect of exemplary compounds of the invention in DIO mouse assay.
Figure 2 demonstrates the effect of exemplary compounds of the invention in DIO mouse assay.
Figures 3A-3C demonstrate the effect of exemplary compounds of the invention in DIO mouse assay.
Figures 4A-4B demonstrate the effects of exemplary compounds of the invention in food intake assay, compared to parent peptide compounds.
Figures 5A-5B demonstrate the effects of exemplary compounds of the invention in blood glucose lowering assay and food intake assay, respectively.

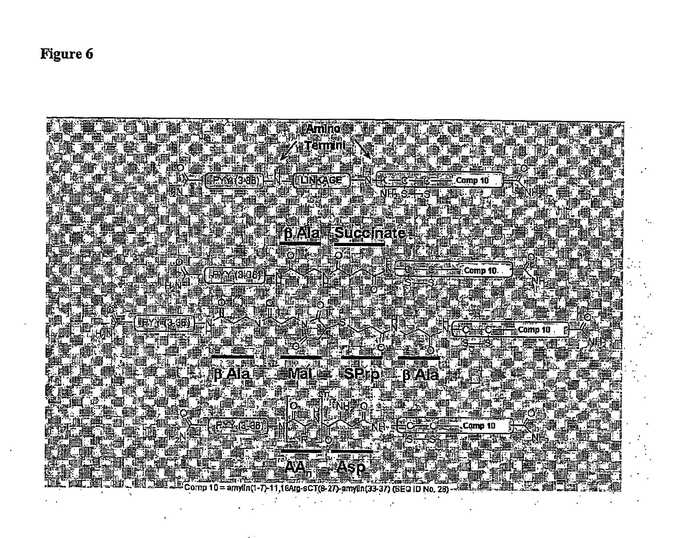

Figure 6 depicts the structures of various exemplary linkers used herein.

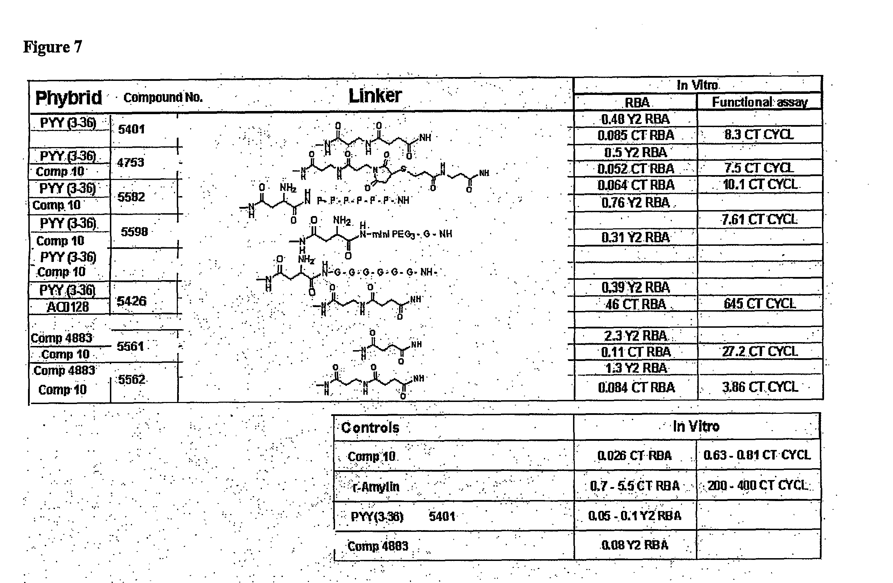

Figure 7 depicts the structures of various exemplary linkers as well as the sequence and in vitro activity of exemplary PYY and amylin family hybrids.

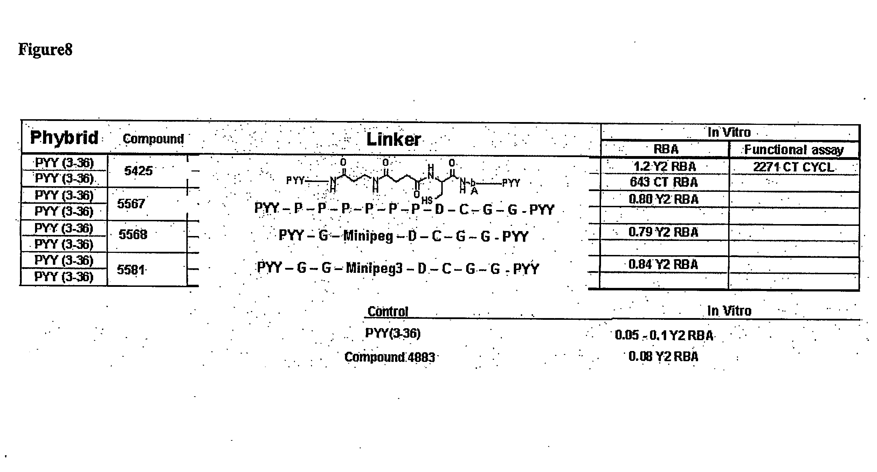

Figure 8 depicts the structures of various exemplary linkers as well as the sequence and in vitro activity of further exemplary PYY/PYY family hybrids.
Figure 9 depicts reduction of body weight by exemplary PYY and amylin family hybrids.
Figure 10 depicts a graph of calcium mobilization by CCK8 family hybrids in cell lines having a CCK receptor.
Figures 11A, B and C depict food inhibition by amylin, e.g. amylin-sCT-amylin, and PYY (e.g. PYY-NPY chimera) family hybrids.
Figure 12A depicts reduction of total food intake and Figure 12B depicts reduction of body weight by amylin-sCT-amylin/PYY family hybrids. The "*" indicates that P<0.05 compared to vehicle.
Figure 13 depicts inhibition of food intake by PYY/PYY family hybrids. Doses were 25 nmol/kg. A difference of ~15-20% is statistically significant.
Figures 14A and 14B depict inhibition of food intake activity of CCK family hybrids with either an amylin or a PYY family component. Doses were 25 nmol/kg. A difference of ~15-20% is statistically significant.
Figures 15A and 15B depict inhibition of food intake activity of MSH family hybrids with either an amylin or a PYY family component. Doses were 25 nmol/kg. A difference of ~15-20% is statistically significant.
Figure 16A depicts reduction of total food intake and Figure 16B depicts reduction of body weight by hybrids with an MSH family component and an amylin, e.g. amylin-sCT-amylin, family component. The "*" indicates that P<0.05 compared to vehicle.
Figures 17A-D depict inhibition of food intake by hybrids containing an FN-38 family component and the indicated second family component. Figure 17A-FN38 with an amylin family component, amylin-sCT-amylin chimera Compound 10. Figure 17B--FN38 with a PYY family component, PYY-3-36. Figure 17C--FN38 with a PYY family component. PYY-NPY chimera. Figure 17D-FN38 with a CCK8 family component, CCK8. Doses were 25 nmol/kg. A difference of ~15-20% is statistically significant.
Figures 18A and 18B depict inhibition of food intake by hybrids containing exendin family and amylin family components. Unless otherwise indicated doses were 25 nmol/kg. A difference of ~15-20% is statistically significant.
Figures 19A and 19B depict food intake inhibition during dark-cycle feeding and longterm inhibition of total food intake by exendin/amylin family hybrids.
Figures 20A and 20B depict chronic inhibition of food intake and lean-sparing weight loss and fat loss activity of an exemplary exendin/amylin-sCT-amylin family hybrid compared to parent molecules. "*" indicates that P<0.05 compare to vehicle.
Figures 21A, B and C demonstrate hybrid effects on metabolic parameters following 14-day treatment in rats
Figures 22-29 depict the ability of PPF polypeptide chimeras, for example, PYY-NPY chimera Compounds 4883 and 5705, to reduce cumulative food intake in the food intake assays described herein.

### DETAILED DESCRIPTION OF THE INVENTION

The present invention as defined in the claims relates generally to novel, selectable hybrid polypeptides useful as agents for the treatment and prevention of metabolic diseases and disorders which can be alleviated by control ot plasma glucose levels, insulin levels, and/or insulin secretion, such as diabetes and diabetes-related conditions. Such conditions and disorders include, but are not limited to, hypertension, dyslipidemia, cardiovascular disease, eating disorders, insulin-resistance, obesity, and diabetes mellitus of any kind, including type 1, type 2, and gestational diabetes.

In one aspect, the invention involves the modular assembly of physiologically, metabolically, and/or pharmacokinetically active peptidic modules that may be selectable based on "bio-activities", *e.g*., therapeutic efficacy, scope of function, duration of action, physicochemical properties, and/or other pharmacokinetic properties.

Without intending to be limited by theory, the present invention relates at least in part to a "toolbox" approach, wherein bio-active peptide hormone modules are linked in binary, tertiary or higher order combinations to create novel, efficacious therapeutic agents with selectable properties. The "bio-active peptide hormone modules" may be peptide hormones, peptide fragments with hormonal activity, or structural motifs of peptide hormones that impart chemical, metabolic, and/or other pharmacokinetic stability. The peptide hormones can include native peptide hormones, as well as peptide hormone analogs and derivatives, as known in the art and described herein.

In one aspect of the invention, it has been found that the combination of certain physicochemical characteristics of two or more peptide hormones into a single modality can facilitate intervention at several points in a dysfunctional metabolic circuit. As such, in one aspect of the invention, rationally-designed hybrid polypeptides are provided that integrate selectable bio-activities into a single polypeptide agent. In one embodiment, the selectable hybrid polypeptides of the invention involve the use of chemically stable linkers as defined in the claims to covalently attach the bio-active modules. In another disclosure, the selectable hybrid polypeptides of the disclosure may involve the use of cleavable linkers, which themselves may be or form part of a bio-active module.

Again, without intending to be limited by theory, design of the hybrid polypeptides of the present disclosure may generally involve: (1) the identification, selection and pairing of bio-active peptide hormone modules for desired efficacy and therapeutic use, and (2) the covalent linking of the bio-active modules (*e.g.* native peptide hormones, peptide hormone analogs or derivatives with hormonal activity, peptide hormone fragments with hormonal activity, stabilizing motifs, *etc.)* either directly or *via* a linker without loss of bio-activity of the component modules. In certain embodiments, module selection criteria may include, but not be limited to: (a) desired *in vivo* efficacy for desired therapeutic or prophylactic indication, such as an additive or a synergistic effect; (b) optional synergism or dual action of the linked modules for multiple therapeutic or prophylactic indications; and/or (c) a desired chemical stability, conformational stability, metabolic stability, bioavailability, organ/tissue targeting, receptor interaction, protease inhibition, plasma protein binding, and/or other pharmacokinetic characteristic.

The section headings are used herein for organizational purposes only, and are not to be construed as in any way limiting the subject matter described.

### Hybrid Polypeptides of the Invention

As mentioned above, the present invention as defined in the claims relates in part to hybrid polypeptides comprising at least two bio-active peptide hormone modules selectable from component peptide hormones described herem. The hybrid polypeptides of the present invention will generally be useful in the treatment and prevention of metabolic conditions and disorders. The hybrid polypeptides of the invention will exhibit at least one hormonal activity of a component peptide hormone, and may include at least one additional bio-activity of a second component peptide hormone.

In one embodiment, the hybrid polypeptides of the invention comprise at least two bio-active peptide hormone modules, wherein each of said at least two bio-active peptide hormone modules exhibits at least one hormonal activity of a component peptide hormone. In another disclosure, the hybrid polypeptides of the disclosure may comprise at least two bio-active peptide hormone modules, wherein at least one of said bio-active peptide hormone modules exhibits at least one hormonal activity of a component peptide hormone and at least one of said bio-active peptide hormone modules imparts a desired chemical stability, conformational stability, metabolic stability, bioavailability, organ/tissue targeting, receptor interaction, protease inhibition, plasma protein binding, and/or other pharmacokinetic characteristic to the hybrid polypeptide.

In a preferred embodiment, the hybrid polypeptides of the invention may have comparable or higher potency in the treatment and/or prevention of metabolic conditions and disorders, as compared to the component peptide hormones. In another embodiment, the hybrid polypeptides of the invention may have comparable or higher potency in the treatment and/or prevention of diabetes and/or diabetes-related disorders, as compared to the component peptide hormones. Alternatively, preferred hybrid polypeptides of the invention may exhibit improved ease of manufacture, stability, and/or ease of formulation, as compared to the component peptide hormones.

More particularly, the hybrid polypeptides of the present invention will comprise a first bio-active peptide hormone module covalently linked to at least one additional bio-active peptide hormone module. In the disclosure, the bio-active peptide hormone modules may be covalently linked together in any manner known in the art, including but not limited to direct amide bonds or chemical linker groups, as described in further detail herein. In one disclosure, chemical linker groups may include peptide mimetics which induce or stabilize polypeptide conformation.

The first bio-active peptide hormone module may be selected from a first component peptide hormone, and may be a peptide hormone (including native peptide hormones as well as analogs and derivatives thereof), a peptide fragment with hormonal activity (including fragments of native peptides hormones as well as analogs and derivatives thereof), or a structural motif of a peptide hormone (including native peptide hormones as well as analogs and derivatives thereof) that imparts a desired chemical stability, conformational stability, metabolic stability, bioavailability, organ/tissue targeting, receptor interaction, protease inhibition, plasma protein binding, and/or other pharmacokinetic characteristic to the hybrid polypeptide. Likewise, the additional bio-active peptide module(s) may be selected from component peptide hormones, and may be a peptide hormone (including native peptide hormones as well as analogs and derivatives thereof), a peptide fragment with hormonal activity (including fragments of native peptides hormones as well as analogs and derivatives thereof), or a structural motif of a hormone peptide (including native peptide hormones as well as analogs and derivatives thereof) that imparts a desired chemical stability, conformational stability, metabolic stability, bioavailability, organ/tissue targeting, receptor interaction, protease inhibition, plasma protein binding, and/or other pharmacokinetic characteristic to the hybrid polypeptide. The first peptide hormone and the additional peptide hormone may be the same peptide hormone, may be from the same family of peptide hormones, or may be different peptide hormones, depending on the desired characteristics of the bio-active peptide hormone modules.

As used herein, the term "bio-active" refers to (1) biological activity in at least one *in vivo* hormonal pathway, or (2) modulation of the therapeutic efficacy, scope of function, duration of action, physicochemical properties, and/or other pharmacokinetic properties of such biological activity. Biological activity may be evaluated through target hormone receptor binding assays, or through metabolic studies that monitor a physiological indication, as known in the art and described herein. Modulation of the therapeutic efficacy, scope of function, duration of action, physicochemical properties, and/or other pharmacokinetic properties of such biological activity may be modified through changed in, *e.g.,* chemical stability, conformational stability, metabolic stability, bioavailability, organ/tissue targeting, receptor interaction, protease inhibition, plasma protein binding, and/or other pharmacokinetic characteristics.

In one embodiment, the hybrid polypeptides of the invention retain at least about 25%, preferably about 30%, 40%, 50%, 60%, 70%, 80%, 90%, 95%, 98%, or 99% percent of the biological activity of a component peptide hormone. Preferred hybrid polypeptides are those having a potency in one of the metabolic-related assays known in the art or described herein (*e.g.,* receptor binding, food intake, gastric emptying, pancreatic secretion, insulin secretion, blood glucose lowering, weight reduction, *etc.*) which is equal to or greater than the potency of component peptide hormone in that same assay. Alternatively, preferred hybrid polypeptides of the invention may exhibit improved ease of manufacture, stability, and/or ease of formulation, as compared to component peptide hormones.

In another embodiment, the hybrid polypeptides of the invention retain at least about 25%, preferably about 30%, 40%, 50%, 60%, 70%, 80%, 90%, 95%, 98%, or 99% percent of the biological activity of a native component peptide hormone with regard to the reduction of nutrient availability, the reduction of food intake, the effect of body weight gain, and/or the treatment and prevention of metabolic conditions and disorders. In yet another embodiment, the hybrid polypeptides of the invention exhibit at least about 110%, 125%, 130%, 140%, 150%, 200%, or more of the biological activity of a native peptide hormone with regard to the reduction of nutrient availability the reduction of food intake, the effect of body weight gain, and/or the treatment and prevention of metabolic conditions and disorders. In another embodiment, the hybrid polypeptides of the invention exhibit improved component peptide hormone receptor agonist activity.

### Component Peptides Hormones, Analogs and Derivatives

Component peptide hormones generally include peptide hormones useful in the treatment or prevention of metabolic diseases and disorders including: (a) the amylin family, including amylin, adrenomedullin ("ADM"), calcitonin ("CT"), calcitonin gene related peptide ("CGRP"), intermedin (also known as "AFP-6") and related peptides; (b) cholecystokinin ("CCK"); (c) the leptin family, including leptin and leptin-like peptides; (d) the pancreatic polypeptide family, including pancreatic polypeptide ("PP") and peptide YY ("PYY"); (e) incretins and incretin mimetics, including: peptide hormones derived from the proglucagon gene such as: glucagon, glucagon-like peptide-1 ("GLP-1"), glucagon-like peptide 2 ("GLP-2"), and oxyntomodulin ("OXM"); and exendins such as: exendin-3, and exendin-4; and (f) natriuretic peptides including ANP, BNP, CNP, and urodilatin, their precursor forms and peptides derived therefrom (g) urocortin family and the (h) neuromedin family, and analogs, derivatives and fragments thereof. As discussed herein component peptide hormones of the invention also include analogs and derivatives as defined in the claims that retain hormonal activity of these native peptide hormones. In one embodiment, such analogs and derivatives are agonists of the target hormone receptor.

By "amylin" is meant the human peptide hormone referred to as amylin and secreted from the beta cells of the pancreas, and species variations thereof, as described in U.S. Pat. No. 5,234,906, issued Aug. 10, 1993, for "Hyperglycemic Compositions."

More particularly, amylin is a 37-amino acid polypeptide hormone normally co-secreted with insulin by pancreatic beta cells in response to nutrient intake (*see, e.g.,* Koda et al., Lancet 339:1179-1180, 1992). In this sense, "amylin," "wild-type amylin," and "native amylin," *i.e.,* unmodified amylin, are used interchangeably.

By "adrenomedullin" or "ADM" is meant the human peptide hormone and species variants thereof. More particularly, ADM is generated from a 185 amino acid preprohormone through consecutive enzymatic cleavage and amidation. This process culminates in the liberation of a 52 amino acid bioactive peptide.

By "calcitonin" or "CT" is meant the human peptide hormone and species variants thereof, including salmon calcitonin ("sCT"). More particularly, CT is a 32 amino acid peptide cleaved from a larger prohormone. It contains a single disulfide bond, which causes the amino terminus to assume the shape of a ring. Alternative splicing of the calcitonin pre-mRNA can yield a mRNA encoding calcitonin gene-related peptide; that peptide appears to function in the nervous and vascular systems. The calcitonin receptor has been cloned and shown to be a member of the seven-transmembrane, G protein-coupled receptor family.

By "calcitonin gene related peptide" or "CGRP" is meant the human peptide hormone and species variants thereof, in any physiological form.

By "intermedin" or "AFP-6" is meant the human peptide hormone and species variants thereof, in any physiological form.

By "cholecystokinin" or "CCK" is meant the human peptide hormone and species variants thereof. More particularly, CCK is a 33-amino acid sequence first identified in humans, and includes a 8-amino acid *in vivo* C-terminal fragment ("CCK-8") that has been reportedly demonstrated in pig, rat, chicken, chinchilla, dog and humans. Thus, the term CCK-33 will generally refer to human CCK(1-33), while CCK-8 (CCK(26-33); SEQ ID NO: 55) will refer to the C-terminal octapeptide generically in both the sulfated and unsulfated unless otherwise specified. Further, pentagastrin or CCK-5 will refer to the C-terminal peptide CCK(29-33) (SEQ ID NO: 209), and the CCK-4 will refer to the C-terminal tetrapeptide CCK(30-33) (SEQ ID NO: 208). However, as used herein, CCK will generally refer to all naturally occurring variations of the hormone, including CCK-33, CCK-8, CCK-5, and CCK-4, in the sulfated and unsulfated form unless otherwise specified.

By "leptin" is meant the naturally occurring leptin from any species, as well as biologically active D-isoforms, or fragments of naturally occurring leptin and variants thereof, and combinations of the preceding. Leptin is the polypeptide product of the ob gene as described in the International Patent Publication No. WO 96/05309.

Putative analogs and fragments of leptin are reported in US Patent 5,521,283, U. S. Patent 5,532,336, PCT/US96/22308 and PCT/US96/01471.

By "PP" is meant human pancreatic peptide polypeptide or species variants thereof, in any physiological form. Thus, the term "PP" includes both the human full length, 36 amino acid peptide as set forth in (SEQ ID NO: 290), and species variations of PP, including, e.g., murine, hamster, chicken, bovine, rat, and dog PP. In this sense, "PP," "wild-type PP," and "native PP," i.e., unmodified PP, are used interchangeably..

By "PYY" is meant human peptide YY polypeptide or species variants thereof, in any physiological form. Thus, the term "PYY" includes both the human full length, 36 amino acid peptide, and species variations of PYY, including *e.g.,* murine, hamster, chicken, bovine, rat, and dog PYY. In this sense, "PYY" and "wild-type PYY" and "native PYY," *i.e.,* unmodified PYY, are used interchangeably. In the context of the present invention, all modifications discussed with reference to the PYY analog polypeptides of the present invention are based on the 36 amino acid sequence of native human PYY.

By "GLP-1" is meant human glucagon like peptide-1 or species variants thereof, in any physiological form. The term "GLP-1" includes human GLP-1(1-37) (SEQ ID NO: 59), GLP-1(7-37) (SEQ ID NO: 204), and GLP-1(7-36)amide (SEQ ID NO: 61), with reference to the full length human GLP-1(1-37) (SEQ ID NO: 59), and species variations of GLP-1, including, *e.g.,* murine, hamster, chicken, bovine, rat, and dog PP. In this sense, "GLP-1," "wild-type GLP-1," and "native GLP-1," *i.e.,* unmodified GLP-1, are used interchangeably.

By "GLP-2" is meant human glucagon like peptide-2 or species variants thereof, in any physiological form. More particularly, GLP-2 is a 33 amino acid peptide, co-secreted along with GLP-1 from intestinal endocrine cells in the small and large intestine.

By "OXM" is meant human oxyntomodulin or species variants thereof in any physiological form. More particularly, OXM is a 37 amino acid peptide that contains the 29 amino acid sequence of glucagon followed by an 8 amino acid carboxyterminal extension.

By "exendin" is meant a peptide hormone found in the saliva of the Gila-monster, a lizard endogenous to Arizona, and the Mexican Beaded Lizard, as well as species variants thereof. More particularly, Exendin-3 is present in the saliva of Heloderma horridum, and exendin-4 is present in the saliva of Heloderma suspectum (Eng, J., et al., J. Biol. Chem., 265:20259-62, 1990; Eng., J., et al., J. Biol. Chem., 267:7402-05 (1992)). The exendins have some sequence similarity to several members of the glucagon-like peptide family, with the highest identity, 53%, being to GLP-1 (Goke, et al., J. Biol. Chem., 268:19650-55 (1993)). In this sense, "exendin," "wild-type exendin," and "native exendin," *i.e.,* unmodified exendin, are used interchangeably.

By "urocortin" is meant a human urocortin petide hormone or species variants thereof in any physiological form. More particularly, there are three human urocortins: Ucn-1, Ucn-2 and Ucn-3. For example, human urocortin 1 has the formula: Asp-Asn-Pro-Ser-Leu-Ser-Ile-Asp-Leu-Thr-Phe-His-Leu-Leu-Arg-Thr-Leu-Leu-Glu-Leu-Ala-Arg-Thr-Gln-Ser-Gln-Arg-Glu-Arg-Ala-Glu-Gln-Asn-Arg-Ile-Ile-Phe-Asp-Ser-Val-NH2 (SEQ ID NO: 294). Rat-derived urocortin is identical but for 2 substitutions: Asp2 for Asn2 and Pro4 for Ser4. Human Ucn-2 has the sequence Ile Val Leu Ser Leu Asp Val Pro Ile Gly Leu Leu Gln Ile Leu Leu Glu Gln Ala Arg Ala Arg Ala Ala Arg Glu Gln Ala Thr Thr Asn Ala Arg Ile Leu Ala Arg Val Gly His Cys (SEQ ID NO: 399). Human Ucn-3 has the sequence Phe Thr Leu Ser Leu Asp Val Pro Thr Asn Ile Met Asn Leu Leu Phe Asn Ile Ala Lys Ala Lys Asn Leu Arg Ala Gln Ala Ala Ala Asn Ala His Leu Met Ala Gln Ile (SEQ ID NO: 299). Ucn-3 is preferably in amide form. Further urocortins and analogs are described in the literature, for example in U.S. Patent 6214797. Urocortins Ucn-2 and Ucn-3, which retain the food-intake suppression and antihypertensive/cardioprotective/inotropic properties, find particular use in the hybrids of the invention. Stresscopin (Ucn-3) and Stresscopin-related peptide (Ucn 2), named for their ability to suppress the chronic HPA activation following a stressful stimulus such as dieting/fasting, are specific for the CRF type 2 receptor and do not activate CRF-R1 which mediates ACTH release. Hybrids comprising a urocortin, e.g., Ucn-2 or Ucn-3, are particularly useful for vasodilation and thus for cardiovascular uses as described herein, e.g CHF. Urocortin containing hybrids of the invention find particular use in treating or preventing conditions associated with stimulating ACTH release, hypertension due to vasodilatory effects, inflammation mediated via other than ACTH elevation, hyperthermia, appetite disorder, congestive heart failure, stress, anxiety, and psoriasis. Such compounds are also useful for an antiproliferative effect, such as for treating or preventing cancers or tumor growth. Of particular interest are urocortin peptide hormone module combined with a natriuretic peptide module, amylin family, an exendin family, or a GLP1 family module to provide an enhanced cardiovascular benefit, e.g. treating CHF, as by providing a beneficial vasodilation effect.

By "neuromedin" is meant the neuromedin family of peptides including neuromedin U and S peptides, more particularly their active hormone sequences. For example, the native active human neuromedin U peptide hormone is neuromedin-U25: Phe Arg Val Asp Glu Glu Phe Gln Ser Pro Phe Ala Ser Gln Ser Arg Gly Tyr Phe Leu Phe Arg Pro Arg Asn (SEQ ID NO: 308), particularly in the amide form. Pig U25 has the sequence: FKVDEEFQGPIVSQNRRYFLFRPRN (SEQ ID NO: 314), particularly its amide form. Other neuromedin U family members include the following listed as their SWISS-PROT designations and entry numbers: NEUU_CANFA (P34962), NEUU_CAVPO (P34966), NEUU_CHICK (P34963), NEUU_HUMAN (P48645), NEUU_LITCE (P81872), NEUU_MOUSE (Q9QXK8), NEUU_PIG (P34964), NEUU_RABIT (P34965), NEUU_RANTE (P20056), and NEUU_RAT (P12760). Of particular interest are their processed active peptide hormones and analogs, derivatives and fragments thereof. Included in the neuromedin U family are various truncated or splice variants, e.g., FLFHYSKTQKLGKSNVVEELQSPFASQSRGYFLFRPRN (SEQ ID NO: 300). Exemplary of the neuromedin S family is human neuromedin S with the sequence ILQRGSGTAAVDFTKKDHTATWGRPFFLFRPRN (SEQ ID NO: 315), particularly its amide form. Hybrids of the invention having neuromedin module will an anorexigenic effect, and thus have beneficial value in treating obesity, diabetes, reducing food intake, and other related conditions and disorders as described herein. Of particular interest are neuromedin modules combined with an amylin family peptide, an exendin peptide family or a GLP1 peptide family module.

As used herein, an "analog" refers to a peptide whose sequence was derived from that of a base reference peptide (*e.g.,* PP, PYY, amylin, GLP-1, exendin, *etc*.), including insertions, substitutions, extensions, and/or deletions of the reference amino acid sequence, preferably having at least 50 or 55% amino acid sequence identity with the base peptide, more preferably having at least 70%, 80%, 90%, or 95% amino acid sequence identity with the base peptide. In one embodiment, such analogs may comprise conservative or non-conservative amino acid substitutions (including non-natural amino acids and L and D forms).

A "derivative" is defined as a molecule having the amino acid sequence of a native reference peptide or analog, but additionally having chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the desamino, N-lower alkyl, N-di-lower alkyl, constrained alkyls (e.g. branched, cyclic, fused, adamantyl) and N-acyl modifications. Modifications of the terminal carboxy group include, without limitation, the amide, lower alkyl amide, constrained alkyls (e.g. branched, cyclic, fused, adamantyl) alkyl, dialkyl amide, and lower alkyl ester modifications. Lower alkyl is C1-C4 alkyl. Furthermore, one or more side groups, or terminal groups, may be protected by protective groups known to the ordinarily-skilled peptide chemist The α-carbon of an amino acid may be mono- or dimethylated.

By "agonist" is meant a compound which elicits a biological activity of native human reference peptide, preferably having a potency better than the reference peptide, or within five orders of magnitude (plus or minus) of potency compared to the reference peptide, more preferably 4, 3, 2, or 1 order of magnitude, when evaluated by art-known measures such as receptor binding/competition studies. In one embodiment, the terms refer to a compound which elicits a biological effect similar to that of native human reference peptide, for example a compound (1) having activity in the food intake, gastric emptying, pancreatic secretion, or weight loss assays similar to native human reference peptide, or (2) which binds specifically in a reference receptor assay or in a competitive binding assay with labeled reference peptide. Preferably, the agonists will bind in such assays with an affinity of greater than 1 µM, and more preferably with an affinity of greater than 1-5 nM. In another embodiment, the terms refer to a compound which elicits a biological effect in the treatment of diabetes or a diabetes related condition or disorder. Such agonists may comprise a polypeptide comprising an active fragment of a reference peptide or a small chemical molecule.

By "amino acid" and "amino acid residue" is meant natural amino acids, unnatural amino acids, and modified amino acid. Unless stated to the contrary, any reference to an amino acid, generally or specifically by name, includes reference to both the D and the L stereoisomers if their structure allow such stereoisomeric forms. Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), Lysine (Lys), methionine (Met), phenylalanine (Phe), proline (Pro), serine (Ser), threonine (Thr), tryptophan (Trp), tyrosine (Tyr) and valine (Val). Unnatural amino acids include, but are not limited to homo-lysine, homo-arginine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2aminoisobutyric acid, 3-aminoisbutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, pentylglycine, pipecolic acid and thioproline. Additional unnatural amino acids include modified amino acid residues which are chemically blocked, reversibly or irreversibly, or chemically modified on their N-terminal amino group or their side chain groups, as for example, N-methylated D and L amino acids or residues wherein the side chain functional groups are chemically modified to another functional group. For example, modified amino acids include methionine sulfoxide; methionine sulfone; aspartic acid-(beta-methyl ester), a modified amino acid of aspartic acid; N-ethylglycine, a modified amino acid of glycine; or alanine carboxamide, a modified amino acid of alanine. Additional residues that can be incorporated are described in Sandberg et al., J. Med. Chem. 41: 2481-91, 1998.

As used herein: "5 Apa" means 5 amino-pentanoyl, "12 Ado" means 12-amino dodecanoyl, "PEG(8)" mean 3,6,-dioxyoctanoyl, and "PEG(13)" means 1-amino-4,7,10-trioxa-13-tridecanamine succinimoyl.

As discussed herein native component peptide hormones are known in the art, as are their analogs and derivatives. For reference, the sequences of several native component peptide hormones are provided in Table 1.

**Table 1: Exemplary Component Peptide Hormones**

| *Seq ID* | *Description* | *Sequence* |
|---|---|---|
| 44 | Rat Amylin | KCNTATCATQRLANFLVRSSNNLGPVLPPTNVGSNTY |
| 45 | h-Amylin | KCNTATCATQRLANFLVHSSNNFGAILSSTNVGSNTY |
| 46 | h-ADM | |
| 47 | s-CT | CSNLSTCVLGKLSQELHKLQTYPRTNTGSGTP |
| 48 | h-CT | CGNLSTCMLGTYTQDFNKFHTFPQTAIGVGAP |
| 49 | h-CGRP α | ACDTATCVTHRLAGLLSRSGGVVKNNFVPTNVGSKAF |
| 50 | H-CGRP β | ACNTATCVTHRLAGLLSRSGGMVKSNFVPTNVGSKAF |
| 51 | h-AFP-6 (1-47) | |
| 52 | h-AFP-6 (8-47) | VGCVLGTCQVQNLSHRLWQLMGPAGRQDSAPVDPSSPHSY |
| 53 | Mouse AFP-6(1-47) | |
| 54 | Mouse AFP-6 (8-47) | VGCVLGTCQVQNLSHRLWQLVRPAGRRDSAPVDPSSPHSY |
| 55 | CCK-8-sulfated | DY(SO₃)MGWMDF |
| 56 | h-Leptin | |
| 57 | h-PYY | YPIKPEAPGEDASPEELNRYYASLRHYLNLVTRQRY |
| 58 | h-PYY (3-36) | IKPEAPGEDASPEELNRYYASLRHYLNLVTRQRY |
| 59 | hGLP-1 (1-37) | HDEFERHAEGTFTSDVSSTLEGQAALEFIAWLVKGRG |
| 60 | Frog GLP-1 | HAEGTYTNDVTEYLEEKAAKEFIEWLIKGKPKKIRYS-OH; |
| 188 | Frog GLP-1 | HAEGTFTSDVTQQLDEKAAKEFIDWLINGGPSKEIIS-OH |
| 61 | h-GLP-1 (7-36) | HAEGTFTSDVSSYLEGQAALEFIAWLVKGR |
| 62 | h-GLP-2 | HADGSFSDEMNTILDNLAARFINWLIETKITD |
| 63 | Frog GLP-2 | HAEGTFTNDMTNYLEEKAAKEFVGWLIKGRP-OH |
| 64 | OXM | HSQGTFTSDYSKYLDSRRAQDFVQWLMNTKRNRNNIA |
| 65 | Exendin-3 | HSDGTFTSDLSKQMEEEAVRLFIEWLKNGGPSSGAPPPS |
| 66 | Exendin-4 | HGEGTFTSDLSKQMEEEAVRLFIEWLKNGGPSSGAPPPS |
| 291 | Urocortin II (Mouse) | VILSLDVPIGLLRILLEQARYKAARNQAATNAQILAHV-NH2 |
| 292 | WP-24 (Urocortin) | WSPGARNQGGGARALLLLLAERFP-OH |
| 293 | TV-18 (Urocortin) | TQSQRERAEQNRIIFDSV-NH2 |
| 294 | Human Urocortin | DNPSLSIDLTFHLLRTLLELARTQSQRERAEQNRIIFDSV-NH2 |
| 295 | SE-20 (Urocortin-111/Stressco pin) | SFHYLRSRDASSGEEEEGKE-OH |
| 296 | AI-13 (Urocortin-III/Stresscopi n) | AQAAANAHLMAQI-OH |
| 297 | DA-21 (Urocortin) | DNPSLSIDLTFHLLRTLLELA-OH |
| 298 | TL-26 (Urocortin-III/ Stresscopin) | TKFTLSLDVPTNIMNLLFNIAKAKNL-OH |
| 299 | Human Urocortin III | FTLSLDVPTNIMNLLFNIAKAKNLRAQAAANAHLMAQI-NH2 |
| 300 | FN-38 (SLM14) | FLFHYSKTQKLGKSNVVEELQSPFASQSRGYFLFRPRN-NH2 |
| 301 | alpha-Atrial Natriuretic Polypeptide (1-28) human, porcine, bovive | SLRRSSCFGGRMDRIGAQSGLGCNSFRY-OH |
| 302 | Brain natriuretic peptide, Rat; BNP, Rat | c(NSKMAHSSSCFGQKIDRIGAVSRLGCDGLRLF)-OH |
| 303 | Brain Natriuretic Peptide (BNP) (human) | SPKMYQGSGCFGRKMDRISSSSGLGCKVLRRH-OH |
| 304 | C-TYPE Natriuretic peptide, Porcine; Cnp, Porcine | GLSKGCFGLKLDRIGSMSGLGC-OH |
| 305 | Neuromedin U-8 (porcine) | YFLFRPRN-NH2 |
| 306 | Neuromedin U (rat) | YKVNEYQGPVAPSGGFFLFRPRN-NH2 |
| 307 | Neuromedin U-9 | GYFLFRPRN-NH2 |
| 308 | Neuromedin (U25), human | FRVDEEFQSPFASQSRGYFLFRPRN-NH2 |

These peptides are generally C-terminally amidated when expressed physiologically, but need not be for the purposes of the instant invention. In other words, the C-terminus of these peptides, as well as the hybrid polypeptides of the present invention, may have a free -OH or-NH₂ group. These peptides may also have other post-translational modifications. One skilled in the art will appreciate that the hybrid polypeptides of the present invention may also be constructed with an N-terminal methionine residue.

Exemplary peptide modules for use in the disclosure further include, N-terminally extendable peptide modules (and their analogs and fragments) including Apelin, which exists in 2 forms, Apelin 36 and 13, both active at the AJP receptor (LVQPRGSRNGPGPWQGGRRKFRRQRPRLSHKGPMPF-OH (SEQ ID NO: 316) and pERPRLSHKGPMPF-OH (SEQ ID NO: 317)); Prolactin Releasing peptide, which exists in 2 forms, PRP31 and PRP20, equally active at GPR10 (SRTHRHSMEIRTPDINPAWYASRGIRPVGRF-NH2 (SEQ ID NO: 318) and TPDINPAWYASRGIRPVGRF-NH2 (SEQ ID NO: 319)); Gastrin, which exists as big gastrin and mini-gastrin, the bulk of activity however residing in resides in pentagastrin (QLGPQGPPHLVADPSKKQGPWLEEEEEAYGWMDF-NH2 (SEQ ID NO: 320); pEGPWLEEEEEAYGWMDF-NH2 (SEQ ID NO: 321); beta-AWMDF-NH2 (SEQ ID NO: 322)); CCK, which exists as CCK33 or CCK8 (central vs. peripheral; KAPSGRMSIVKNLQNLDPSHRISDRDYMGWMDF-NH2 (SEQ ID NO: 323); DYMGWMDF-NH2) (SEQ ID NO: 55); Cortistatin, which exists as cortistatin 17 or 29 (QEGAPPQQSARRDRMPCRNFFWKTFSSCK-OH (SEQ ID NO: 324) and DRMPCRNFFWKTFSSCK-OH (SEQ ID NO: 325)); somatostatin, which exists as somatostatin 14 or 28 (SANSNPAMAPRERKAGCKNFFWKTFTSC-OH (SEQ ID NO: 326); AGCKNFFWKTFTSC-OH (SEQ ID NO: 327)); GRP for which a C-terminal 10 amino acid sequence possesses most of the activity (VPLPAGGGTVLTKMYPRGNHWAVGHLM-NH2 (SEQ ID NO: 328); GNHWAVGHLM-NH2 (SEQ ID NO: 329)); Neuromedin B for which a C-terminal 10 amino acid region possesses most of the activity (LSWDLPEPRSRASKIRVHSRGNLWATGHFM-NH2 (SEQ ID NO: 330); GNLWATGHFM-NH2 (SEQ ID NO: 331)); Neuromedin S for which a C-terminal 9 amino acid region possesses most of the activity (ILQRGSGTAAVDFTKKDHTATWGRPFFLFRPRN-NH2 (SEQ ID NO: 315); PFFLFRPRN-NH2 (SEQ ID NO: 332)); Neuromedin U for which a C-terminal 9 amino acid region possesses most of the activity (FRVDEEFQSPFASQSRGYFLFRPRN-NH2 (SEQ ID NO: 308); GYFLFRPRN-NH2 (SEQ ID NO: 307)); Neurotensin, which exists as long and short forms (KIPYILKRQLYENKPRRPYIL-OH (SEQ ID NO: 333); QLYENKPRRPYIL-OH) (SEQ ID NO: 334); Kiss-1 whose activity lies mainly in its C-terminus (GTSLSPPPESSGSPQQPGLSAPHSRQIPAPQGAVLVQREKDLPNYNWNSFGLRF-NH2 (SEQ ID NO: 335); EKDLPNYNWNSFGLRF-NH2 (SEQ ID NO: 336)); RF-amide-3, whose C-terminal fragments possess activity (SAGATANLPLRSGRNMEVSLVRRVPNLPQRF-NH2 (SEQ ID NO: 337); VPNLPQRF-NH2 (SEQ ID NO: 338)); Dynorphin, which exists as big dynorphin (A) of dynorphin B (rimorphin) (YGGFLRRIRPKLKWDNQKRYGGFLRRQFKVVT-OH (SEQ ID NO: 339) and YGGFLRRQFKVVT-OH (SEQ ID NO: 340)); PYY whose C-terminal fragments are active at Y2 receptor (YPIKPEAPGEDASPEELNRYYASLRHYLNLVTRQRY-NH2 (SEQ ID NO: 57); SLRHYLNLVTRQRY-NH2 (SEQ ID NO: 341)); AFP-6 whose 7-47 region retains activity (TQAQLLRVGCVLGTCQVQNLSHRLWQLMGPAGRQDSAPVDPSSPHSY-NH2 (SEQ ID NO: 51); VGCVLGTCQVQNLSHRLWQLMGPAGRQDSAPVDPSSPHSY-NH2 (SEQ ID NO: 52)); the amylin family including adrenomdullin, calcitonin and CGRP; Oxytocin whose C-terminal amide is generally needed for activity and can tolerate N-terminal extensions.

Exemplary peptide modules for use in the disclosure further include, C-Terminally extendable peptide modules including, Endothelin I, II and III: ETI (CSCSSLMDKECVYFCHLDIIWVNTPEHVVPYGLGSPRS-OH (SEQ ID NO: 342); CSCSSLMDKECVYFCHLDIIW-OH (SEQ ID NO: 343)), ETII (CSCSSWLDKECVYFCHLDIIWVNTPEQTAPYGLGNPP-OH (SEQ ID NO: 344); CSCSSWLDKECVYFCHLDIIW-OH (SEQ ID NO: 345)) and ETIII (CTCFTYKDKECVYYCHLDIIWINTPEQTVPYGLSNYRGSFR-NH2 (SEQ ID NO: 346); CTCFTYKDKECVYYCHLDIIW-OH (SEQ ID NO: 347)); ghrelin whose activity lies mainly in its first 10 residues (GSSFLSPEHQRVQQRKESKKPPAKLQP-OH (SEQ ID NO: 348); GSSFLSPEHQ-OH (SEQ ID NO: 349)); glucagons, including oxyntomodulin which is a C-terminally extended glucagon with glucagons-like activity (HSQGTFTSDYSKYLDSRRAQDFVQWLMNTKRNRNNIA-OH (SEQ ID NO: 350); HSQGTFTSDYSKYLDSRRAQDFVQWLMNT-OH (SEQ ID NO: 351)); GLP-1/GLP-2 whose activities are retained with or without a C-terminal amide; GIP, which circulates in 2 forms, GIP1-42 and GIP1-30, both fully active at GIP Receptor (YAEGTFISDYSIAMDKIHQQDFVNWLLAQKGKKNDWKHNITQ-OH (SEQ ID NO: 352); YAEGTFISDYSLAAMKIHQQDFVNWLLAQK-NH2 (SEQ ID NO: 353)); neuropeptide W, which exists as NPW23 and NPW30, equally active at GPR7 and 8 (WYKHVASPRYHTVGRAAGLLMGLRRSPYLW-OH (SEQ ID NO: 354); WYKHVASPRYHTVGRAAGLLMGL-OH (SEQ ID NO: 355)); PACAP which exists in 2 forms, PACAP27 and 38 (HSDGIFTDSYSRYRKQMAVKKYLAAVLGKRYKQRVKNK-NH2 (SEQ ID NO: 356); HSDGIFTDSYSRYRKQMAVKKYLAAVL-NH2 (SEQ ID NO: 357)); PHI and PHV (HADGVFTSDFSKLLGQLSAKKYLESLMGKRVSSNISEDPVPV-OH (SEQ ID NO: 358); HADGVFTSDFSKLLGQLSAKKYLESLM-NH2 (SEQ ID NO: 359)); GRF, which exists in 2 forms GRF29 and GRF40 (YADAIFTNSYRKVLGQLSARKLLQDIMSRQQGESNQERGARARL-NH2 (SEQ ID NO: 360); YADAIFTNSYRKVLGQLSARKLLQDIMS-OH (SEQ ID NO: 361)); PTH 1-34 and 1-37 forms which possess activity of full length PTH 1-84 (SVSEIQLMHNLGKHLNSMERVEWLRKKLQDVHNFVALGAPLAPRDAGSQRPRKKEDN VLVESHEKSLGEADKADVNVLTKAKSQ (SEQ ID NO: 362); SVSEIQLMHNLGKHLNSMERVEWLRKKLQDVHNFVAL-OH (SEQ ID NO: 363); SVSEIQLMHNLGKHLNSMERVEWLRKKLQDVHNF-OH (SEQ ID NO: 364)) PTH-RP for which 1-36 possesses activity of full length 1-86 (AVSEHQLLHDKGKSIQDLRRRFFLHHLIAEIHTAEIRATSEVSPNSKPSPNTKNHPVRFGS DDEGRYLTQETNKVETYKEQPLKTPGKKKKGKP-NH2 (SEQ ID NO: 365); AVSEHQLLHDKGKSIQDLRRRFFLHHLIAEIHTAEI-OH (SEQ ID NO: 366)) gamma-MSH for which the shorter gamma-MSH1 and the longer gamma-MSH3 have similar activities (YVMGHFRWDRFGRRNSSSSGSSGAGQ-OH (SEQ ID NO: 367); YVMGHFRWDRF-NH2 (SEQ ID NO: 368)); MSH for which alpha-MSH is an active portion of ACTH (SYSMEHFRWGKPVGKKRRPVKVYPNGAEDESAEAFPLEF-OH (SEQ ID NO: 369); SYSMEHFRWGKPV-NH2 (SEQ ID NO: 370)); and endorphins for which the A, delta, and γ endorphin are active subpeptides of the larger β endorphin (YGGFMTSEKSQTPLVTLFKNAIIKNAYKKGE-OH (SEQ ID NO: 371); YGGFMTSEKSQTPLVTLFKNAIIKNAY-OH (SEQ ID NO: 372); YGGFMTSEKSQTPLVTL-OH (SEQ ID NO: 373); YGGFMTSEKSQTPLVT-OH (SEQ ID NO: 374)).

For example, the melanocortins are peptides from the pro-opiomelanocortin gene, including alpha-melanocyte-stimulating hormone (alpha-MSH) and adrenocorticotrophic hormone (ACTH), and five melanocortin receptors are known, MC1-5R. MC4R appears to play a role in energy balance and obesity. See, for example, Anderson et al., Expert Opin. Ther. Patents 11:1583-1592 (2001), Speake et al., Expert Opin. Ther. Patents 12:1631-1638 (2002), Bednarek et al., Expert Opin. Ther. Patents 14:327-336 (2004).

The analogs of the above component peptide hormones are known in the art, but generally include modifications such as substitutions, deletions, and insertions to the amino acid sequence of such component peptide hormones, and any combination thereof. The substitutions, insertions and deletions may be at the N-terminal or C-terminal end, or may be at internal portions of the component peptide hormone. In a preferred aspect, analogs of the component peptide hormones of the invention include one or more modifications of a "non-essential" amino acid residue. In the context of the invention, a "non-essential" amino acid residue is a residue that can be altered, *i.e.,* deleted or substituted, in the native human amino acid sequence of the fragment, *e.g.,* the component peptide hormone fragment, without abolishing or substantially reducing the component peptide hormone receptor agonist activity of the resulting analog.

Preferred substitutions include conserved amino acid substitutions. A "conservative amino acid substitution" is one in which the amino acid residue is replaced with an amino acid residue having a similar side chain, or physicochemical characteristics (*e.g.,* electrostatic, hydrogen bonding, isosteric, hydrophobic features). Families of amino acid residues having similar side chains are known in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, methionine, cysteine), nonpolar side chains (*e.g.,* alanine, valine, leucine, isoleucine, proline, phenylalanine, tryptophan), β-branched side chains (*e.g.,* threonine, valine, isoleucine) and aromatic side chains (*e.g.,* tyrosine, phenylalanine, tryptophan, histidine).

The present invention also relates to derivatives of the component peptide hormones. Such derivatives include component peptide hormones and analogs thereof conjugated to one or more water soluble polymer molecules, such as polyethylene glycol ("PEG") or fatty acid chains of various lengths (*e.g.,* stearyl, palmitoyl, octanoyl, etc.), or by the addition of polyamino acids, such as poly-his, poly-arg, poly-lys, and poly-ala. Modifications to the component peptide hormones or analogs thereof can also include small molecule substituents, such as short alkyls and constrained alkyls (*e.g.,* branched, cyclic, fused, adamantyl), and aromatic groups. The water soluble polymer molecules will preferably have a molecular weight ranging from about 500 to about 20,000 Daltons.

Such polymer-conjugations and small molecule substituent modifications may occur singularly at the N- or C-terminus or at the side chains of amino acid residues within the sequence of the hybrid polypeptides. Alternatively, there may be multiple sites of derivatization along the hybrid polypeptide. Substitution of one or more amino acids with lysine, aspartic acid, glutamic acid, or cysteine may provide additional sites for derivatization. *See, e.g.,* U.S. Patent Nos. 5,824,784 and 5,824,778. Preferably, the hybrid polypeptides may be conjugated to one, two, or three polymer molecules.

The water soluble polymer molecules are preferably lined to an amino, carboxyl, or thiol group, and may be linked by N or C terminus, or at the side chains of lysine, aspartic acid, glutamic acid, or cysteine. Alternatively, the water soluble polymer molecules may be linked with diamine and dicarboxylic groups. In a preferred embodiment, the hybrid polypeptides of the invention are conjugated to one, two, or three PEG molecules through an epsilon amino group on a lysine amino acid.

Derivatives of the invention also include component peptide hormones or analogs with chemical alterations to one or more amino acid residues. Such chemical alterations include amidation, glycosylation, acylation, sulfation, phosphorylation, acetylation, and cyclization. The chemical alterations may occur singularly at the N- or C-terminus or at the side chains of amino acid residues within the sequence of the PPF hybrid polypeptides. In one embodiment, the C-terminus of these peptides may have a free -OH or -NH₂ group. In another embodiment, the N-terminal end may be capped with an isobutyloxycarbonyl group, an isopropyloxycarbonyl group, an n-butyloxycarbonyl group, an ethoxycarbonyl group, an isocaproyl group (isocap), an octanyl group, an octyl glycine group (G(Oct)), or an 8-aminooctanic acid group. In a preferred embodiment, cyclization can be through the formation of disulfide bridges. Alternatively; there may be multiple sites of chemical alteration along the hybrid polypeptide.

### The Amylin Family

As discussed herein component peptide hormones useful in the present invention as defined in the claims include amylin family peptide hormones including amylin, adrenomedullin ("ADM"), calcitonin ("CT"), calcitonin gene related peptide ("CGRP"), intermedin (also known as "AFP-6") and related peptides. Native amylin family peptide hormones are known in art, as are functional peptide analogs and derivatives. Certain preferred native peptides, peptide analogs and derivatives are described herein, however it should be recognized that any known amylin family peptides that exhibit hormonal activity known in the art may be used in conjunction with the present invention.

Any amylin analog or derivative known in the art may be used in conjunction with the present invention. The amylin analogs and derivatives have at least one hormonal activity of native amylin. In certain embodiments, the amylin analogs are agonists of a receptor which native amylin is capable of specifically binding. Preferred amylin analogs and derivatives include those described in US 2003/0026812 A1.

Exemplary amylin analogs include:

| SEQ ID: | |
|---|---|
| 67 | ^{25,28,29}Pro-h-amylin (pramlintide) |
| 68 | des-¹Lys-h-amylin |
| 69 | ²⁵Pro,²⁶Val,^{28,29}Pro-h-amylin |
| 70 | ¹⁸Arg,^{25,28}Pro-h-amylin |
| 71 | des-¹Lys,¹⁸Arg,^{25,28}Pro-h-amylin |
| 72 | ¹⁸Arg,^{25,28,29}Pro-h-amylin |
| 73 | des-¹Lys,¹⁸Arg,^{25,28,29}Pro-h-amylin |
| 74 | des-¹,Lys^{25,28,29}Pro-h-amylin |
| 75 | ²⁵Pro,²⁶Val,^{28,29}Pro-h-amylin |
| 76 | ²⁸Pro-h-amylin, 2,7-Cyclo-[²Asp,⁷Lys]-h-amylin |
| 77 | ²⁻³⁷h-amylin |
| 78 | ¹Ala-h-amylin |
| 79 | ²Ala-h-amylin |
| 80 | ^{2,7}Ala-h-amylin |
| 81 | ¹Ser-h-amylin |
| 82 | ²⁹Pro-h-amylin |
| 83 | ^{25,28}Pro-h-amylin |
| 84 | des-¹Lys,^{25,28}Pro-h-amylin |
| 85 | ²³Leu,²⁵Pro,²⁶Val,^{28,29}Pro-h-amylin |
| 86 | ²³Leu²⁵Pro²⁶Val²⁸Pro-h-amylin |
| 87 | des-¹Lys,²³Leu,²⁵Pro,²⁶Val,²⁸Pro-h-amylin |
| 88 | ¹⁸Arg,²³Leu, ²⁵Pro,²⁶Val,²⁸Pro-h-amylin |
| 89 | ¹⁸Arg,²³Leu,^{25,28,29}Pro-h-amylin |
| 90 | ¹⁸Arg²³Leu,^{25,28}Pro-h-amylin |
| 91 | ¹⁷Ile,²³Leu,^{25,28,29}Pro-h-amylin |
| 92 | ¹⁷Ile,^{25,28,29}Pro-h-amylin |
| 93 | des-¹Lys,¹⁷Ile,²³Leu,^{25,28,29}Pro-h-amylin |
| 94 | ¹⁷Ile,¹⁸Arg,²³Leu-h-amylin |
| 95 | ¹⁷Ile,¹⁸Arg,²³Leu,²⁶Val,²⁹Pro-h-amylin |
| 96 | ¹⁷Ile,¹⁸Arg,²³Leu,²⁵Pro,²⁶Val,^{28,29}Pro-h-amylin, |
| 97 | ¹³Thr,²¹His,²³Leu,²⁶Ala,²⁸Leu,²⁹Pro,³¹Asp-h-amylin |
| 98 | ¹³Thr,²¹His,²³Leu,²⁶Ala,²⁹Pro,³¹Asp-h-amylin |
| 99 | des-¹Lys,¹³Thr,²¹His,²³Leu,²⁶Ala,²⁸Pro,³¹Asp-h-amylin |
| 100 | ¹³Thr,¹⁸Arg,²¹His,²³Leu,²⁶Ala,²⁹Pro,³¹Asp-h-amylin |
| 101 | ¹³Thr,¹⁸Arg,²¹His,²³Leu,^{28,29}Pro,³¹Asp-h-amylin |
| 102 | ¹³Thr,¹⁸Arg,²¹His,²³Leu,²⁵Pro,²⁶Ala,^{28,29}Pro,³¹Asp-h-amylin |

As known in the art, such amylin analogs are preferably amidated, but within the context of the present invention, may optionally be in the acid form unless otherwise specified.

Any ADM analog or derivative known in the art may be used in conjunction with the present disclosure. In one embodiment, the ADM analogs and derivatives have at least one hormonal activity of native ADM. In certain embodiments, the ADM analogs are agonists of a receptor which native ADM is capable of specifically binding.

Any CT analog or derivative known in the art may be used in conjunction with the present disclosure. In one embodiment, the CT analogs and derivatives have at least one hormonal activity of native CT. In certain embodiments, the CT analogs are agonists of a receptor which native CT is capable of specifically binding. Preferred CT analogs and derivatives include those described in U.S. Patent Nos. 4,652,627; 4,606,856; 4,604,238; 4,597,900; 4,537,716; 4,497,731; 4,495,097; 4,444,981; 4,414,149; 4,401,593; and 4,397,780.

Exemplary CT analogs include:

| SEQ ID: | |
|---|---|
| 103 | ⁸Gly-CT |
| 104 | ²²Leu-CT |
| 105 | ²Gly,³Ser,⁸Gly,²²des-Tyr-CT |
| 106 | ¹⁴Glu-sCT, |
| 107 | ¹⁸Arg-sCT, |
| 108 | ^{11,18}Arg-sCT, |
| 109 | ¹⁴Glu,¹⁸Arg-sCT, |
| 110 | ¹⁴Glu,^{11,18}Arg-sCT |

As known in the art, such CT analogs are preferably amidated, but within the context of the present : disclosure, may optionally be in the acid form unless otherwise specified.

Any CGRP analog or derivative known in the art may be used in conjunction with the present disclosure. In one embodiment, the CGRP analogs and derivatives have at least one hormonal activity of native CGRP. In certain embodiments, the CGRP analogs are agonists of a receptor which native CGRP is capable of specifically binding. Preferred CGRP analogs and derivatives include those described in U.S. Patent Nos. 4,697,002; and 4,687,839.

Exemplary CGRP analogs include:

| SEQ ID: | |
|---|---|
| 111 | ³⁶D-Ser-CGRP |
| 112 | ³⁶-Thr-CGRP |
| 113 | ³⁶D-Asp-CGRP |
| 114 | ³⁶D-Asn-CGRP |
| 115 | ³⁶Ser-CGRP |
| 116 | ³⁶Hse-CGRP |
| 117 | ³⁰Asp-CGRP |
| 118 | ³⁶Thr-CGRP |
| 119 | ³⁶Asn-CGRP |

Any AFP-6 analog or derivative known in the art may be used in conjunction with the present disclosure. In one embodiment, the AFP-6 analogs and derivatives have at least one hormonal activity of native AFP-6. In certain embodiments, the AFP-6 analogs are agonists of a receptor which native AFP-6 is capable of specifically binding. Preferred AFP-6 analogs and derivatives include those described in WO 2003/022304.

Exemplary AFP-6 analogs include:

| SEQ ID: | |
|---|---|
| 120 | TQAQLLRVGCGNLSTCQVQNLSHRLWQLMGPAGRQDSAPVDPSSPHSY |
| 121 | TQAQLLRVGCDTATCQVQNLSHRLWQLMGPAGRQDSAPVDPSSPHSY |
| 122 | TQAQLLRVGMVLGTMQVQNLSHRLWQLMGPAGRQDSAPVDPSSPHSY |
| 123 | TQAQLLRVGCVLGTCQVQNLSHRLWQLMGPAGRQDSAPVEPSSPHSY |
| 124 | TQAQLLRVGCVLGTCQVQNLSHRLWQLMGPAGRQESAPVEPSSPHSY |
| 125 | TQAQLLRVGCVLGTCQVQNLSHRLWQL----RQDSAPVDPSSPHSY |
| 126 | TQAQLLRVGCVLGTCQVQNLSHRLWQL----DSAPVDPSSPHSY |
| 127 | RVGCVLGTCQVQNLSHRLWQLMGPAGRQDSAPVDPSSPHSY |
| 128 | VGCVLGTCQVQNLSHRLWQLMGPAGRQDSAPVEPSSPHSY |
| 129 | VGCVLGTCQVQNLSHRLWQL----RQDSAPVEPSSHSY |
| 130 | GCVLGTCQVQNLSHRLWQLMGPAGRQDSAPVDPSSPHSY |
| 131 | GCNTATCQVQNLSHRLWQL----RQDSAPVDPSSPHSY |
| 132 | GCNTATCQVQNLSHRLWQL----RQDSAPVEPSSPHSY |
| 133 | GCSNLSTCQVQNLSHRLWQL----RQDSAPVEPSSPHSY |
| 134 | GCGNLSTCQVQNLSHRLWQL----RQDSAPVEPSSPHSY |
| 135 | GCVLGTCQVQNLSHRLWQL----RQESAPVEPSSPHSY |
| 136 | CVLGTCQVQNLSHRLWQLMGPAGRQDSAPVDPSSPHSY |
| 137 | QVQNLSHRLWQLMGPAGRQDSAPVDPSSPHSY |
| 138 | VQNLSHRLWQLMGPAGRQDSAPVDPSSPHSY |
| 139 | VQNLSHRL----QLMGPAGRQDSAPVDPSSPHSY |
| 140 | GTMQVQNLSHRLWQL---RQDSAPVEPSSPHSY |

As known in the art, such AFP-6 analogs are preferably amidated, but within the context of the present disclosure, may optionally be in the acid form unless otherwise specified.

### The CCK Family

CCKs, including hCCK and species variants, and various analogs thereof are known in the art. Generally, CCK has a 33-amino acid sequence first identified in humans, and includes a 8-amino acid *in vivo* C-terminal fragment ("CCK-8") that has been reportedly demonstrated in pig, rat, chicken, chinchilla, dog and humans. Other species variants include a 39-amino acid sequence found in pig, dog and guinea pig, and a 58-amino acid found in cat, dog and humans, and a 47-amino acid sequences homologous to both CCK and gastrin. The C-terminal tyrosine-sulfated octapeptide sequence (CCK-8) is relatively conserved across species, and may be the minimum sequence for biological activity in the periphery of rodents. Thus, the term CCK-33 will generally refer to human CCK(1-33), while CCK-8 (CCK(26-33); SEQ ID NO: 55) will refer to the C-terminal octapeptide generically in both the sulfated and unsulfated unless otherwise specified. Further, pentagastrin or CCK-5 will refer to the C-terminal peptide CCK(29-33) (SEQ ID NO: 209), and the CCK-4 will refer to the C-terminal tetrapeptide CCK(30-33) (SEQ ID NO: 208).

The type A receptor subtype (CCK_{A}) has been reported to be selective for the sulfated octapeptide. The Type B receptor subtype (CCK_{B}) has been identified throughout the brain and in the stomach, and reportedly does not require sulfation or all eight amino acids.

Various *in vivo* and *in vitro* screening methods for CCK analogs are known in the art. Examples include *in vivo* assays involving the contraction of the dog or guinea pig gallbladder after rapid intravenous injection of the compound to be tested for CCK-like activity, and *in vitro* assays using strips of rabbit gallbladder. *See* Walsh, "Gastrointestinal Hormones", In Physiology of the Gastrointestinal Tract (3d ed. 1994; Raven Press, New York).

Certain exemplary CCKs and CCK analogs with CCK activity include:

| SEQ ID: | |
|---|---|
| 141 | DY(SO₃H)MGWMDF |
| 142 | DYMGWMDF |
| 143 | MGWMDF |
| 144 | GWMDF |
| 145 | WMDF |
| 146 | KDY(SO₃H)MGWMDF |
| 147 | KDYMGWMDF |
| 148 | KMGWMDF |
| 149 | KGWMDF |
| 150 | KWMDF |

As known in the art, such CCK peptides are preferably amidated, but within the context of the present disclosure, may optionally be in the acid form unless otherwise specified.

### The Leptin Family

Component peptide hormones useful in the present disclosure also include leptin family peptide hormones. Native leptin family peptide hormones are known in art, as are functional peptide analogs and derivatives. Certain preferred native peptides, peptide analogs and derivatives are described herein, however it should be recognized that any known amylin family peptides that exhibit hormonal activity known in the art may be used in conjunction with the present disclosure.

Any leptin analog or derivative known in the art may be used in conjunction with the present disclosure. In one embodiment, the leptin analogs and derivatives have at least one hormonal activity of native leptin. In certain embodiments, the leptin analogs are agonists of a receptor which native leptin is capable of specifically binding. Preferred leptin analogs and derivatives include those described in, e.g., WO 2004/039832, WO 98/55139, WO 98/12224, and WO 97/02004.

In one embodiment leptin peptides include MVPIQK, VQDDTK, TLIK, TIVTR, INDISHTQSVSSK, VTGLDFIPGLHPILTLSK, NVIQISNDLENLR, DLLHVLAFSK, SCHLPWASGLETLDSLGGVLEASGYSTEWALSR and LQGSLQDMLWQLDLSPGC as disclosed in WO97046585.

In one embodiment a leptin peptide may have an amino acid sequence Xaan-Ser-Cys-Xaa1-Leu-Pro-Xaa2-Xaa3-Xaan, wherein Xaan may be zero residues in length, or may be a contiguous stretch of peptide residues derived from full length human or mouse leptin sequences, a stretch of between 1 and 7 at either the C-terminus or N-terminus, or wherein the leptin peptide is a total of 15 amino acids or less in length. In another embodiment, Xaa1, Xaa2 or Xaa3 may be any amino acid substitution. In yet another embodiment, Xaa1, Xaa2 or Xaa3 may be any conservative amino acid substitution of the respective residues in full length mouse or human leptin. In a further embodiment, Xaa1 may be selected from the group consisting of His or Ser, and Xaa2 or Xaa3 is any amino acid substitution. In another embodiment, Xaa2 may be selected from the group consisting of Trp or Gln, and Xaa1 or Xaa3 is any amino acid substitution. In yet another embodiment, Xaa3 may be selected from the group consisting of Ala or Thr, and Xaa1 or Xaa2 is any amino acid substitution. In another embodiment Xaa1 is selected from the group consisting of His or Ser, Xaa2 is selected from the group consisting of Trp or Gln, and Xaa3 is selected from the group consisting of Ala or Thr. See WO04039832.

In one embodiment leptin peptide comprises the. C-terminal amino acid residues 116-122 of native full length human or mouse leptin (corresponding to positions 95-101 of their mature forms) and D-isoforms, fragments, derivatives, analogs and homologs thereof, which possess the ability to modulate body mass homeostasis in test animals upon i.p. (intraperitoneal) administration. Specific mouse D-substituted peptides of sequence SCSLPQT include [D- Ser-1]-, [D-Cys-2]-, [D-Ser-3]-, [D-Leu-4]-, [D-Pro-5]-, [D-Gln-6]-,[D-Thr-7]-SCSLPQT and all [D] SCSLPQT. Specific human D-substituted peptides of SCHLPWA include [D-Ser-1]-, [D-Cys-2]-, [D-His-3]-, [D-Leu-4]-, [D- Pro-5]-, [D-Trp-6]-, [D-Ala-7]-SCHLPWA and all [D]-SCHLPWA. In addition the SCHLPWA and SCSLPQT peptides may contain D-substituted amino acids for any two, three, four, five or six positions. Also disclosed are leptin related peptides comprising N-terminal amino acids 21-35, 31-45, 41-55 and 51-65 of native leptin and fragments, derivatives, analogs and homologs thereof. Additional leptin peptides of the invention comprise amino acids sequences 61-75, 71-85, 81-95, 91-105, 106-120, 116-130, 126-140, 136-150, 146-160, and 156-167 of mouse and/or human full length leptin. See WO04039832.

In one embodiment the leptin is of the sequence Ser Cys His Leu Pro Xaa Ala Ser Gly Leu Glu Thr Leu Asp Ser Leu Gly Gly Val Leu Glu Ala Ser Gly Tyr Ser Thr Glu Val Val Ala Leu Ser Arg Leu Xaa Gly Ser Leu Xaa Asp Xaa Leu Xaa Xaa Leu Asp Leu Ser Pro Gly Cys wherein: Xaa at position 6 is Trp or Gln; Xaa at position 36 is Gln or Glu; Xaa at position 40 is Gln or Glu; Xaa at position 42 is Ile, Leu, Met or methionine sulfoxide; Xaa at position 44 is Trp or Gln; and Xaa at position 45 is Gln or Glu. In another embodiment are leptin of the above are wher Xaa at position 6 is Trp; Xaa at position 36 is Gln; Xaa at position 40 is Gln; Xaa at position 42 is Met; Xaa at position 44 is Trp; and Xaa at position 45 is Gln. See U.S. patent 5521283.

In one embodiment leptins are native sequences, including
Murine leptin: Val Pro Ile Gln Lys Val Gln Asp Asp Thr Lys Thr Leu Ile Lys Thr Ile Val Thr Arg Ile Asn Asp Ile Ser His Thr Xaa Ser Val Ser Ser Lys Gln Lys Val Thr Gly Leu Asp Phe Ile Pro Gly Leu His Pro Ile Leu Thr Leu Ser Lys Met Asp Gln Thr Leu Ala Val Tyr Gln Gln Ile Leu Thr Ser Met Pro Ser Arg Asn Val Ile Gln Ile Ser Asn Asp Leu Glu Asn Leu Arg Asp Leu Leu His Val Leu Ala Phe Ser Lys Ser Cys His Leu Pro Gln Ala Ser Gly Leu Glu Thr Leu Glu Ser Leu Gly Gly Val Leu Glu Ala Ser Gly Tyr Ser Thr Glu Val Val Ala Leu Ser Arg Leu Gln Gly Ser Leu Gln Asp Met Leu Gln Gln Leu Asp Leu Ser Pro Gly Cys, wherein: Xaa at position 28 is Gln or absent;
Porcine leptin: Val Pro Ile Trp Arg Val Gln Asp Asp Thr Lys Thr Leu Ile Lys Thr Ile Val Thr Arg Ile Ser Asp Ile Ser His Met Gln Ser Val Ser Ser Lys Gln Arg Val Thr Gly Leu Asp Phe Ile Pro Gly Leu His Pro Val Leu Ser Leu Ser Lys Met Asp Gln Thr Leu Ala Ile Tyr Gln Gln Ile Leu Thr Ser Leu Pro Ser Arg Asn Val Ile Gln Ile Ser Asn Asp Leu Glu Asn Leu Arg Asp Leu Leu His Leu Leu Ala Ser Ser Lys Ser Cys Pro Leu Pro Gln Ala Arg Ala Leu Glu Thr Leu Glu Ser Leu Gly Gly Val Leu Glu Ala Ser Leu Tyr Ser Thr Glu Val Val Ala Leu Ser Arg Leu Gln Gly Ala Leu Gln Asp Met Leu Arg Gln Leu Asp Leu Ser Pro Gly Cys;
Bovine leptin: Val Pro Ile Cys Lys Val Gln Asp Asp Thr Lys Thr Leu Ile Lys Thr Ile Val Thr Arg Ile Asn Asp Ile Ser His Thr Xaa Ser Val Ser Ser Lys Gln Arg Val Thr Gly Leu Asp Phe Ile Pro Gly Leu His Pro Leu Leu Ser Leu Ser Lys Met Asp Gln Thr Leu Ala Ile Tyr Gln Gln Ile Leu Thr Ser Leu Pro Ser Arg Asn Val Val Gln Ile Ser Asn Asp Leu Glu Asn Leu Arg Asp Leu Leu His Leu Leu Ala Ala Ser Lys Ser Cys Pro Leu Pro Gln Val Arg Ala Leu Glu Ser Leu Glu Ser Leu Gly Val Val Leu Glu Ala Ser Leu Tyr Ser Thr Glu Val Val Ala Leu Ser Arg Leu Gln Gly Ser Leu Gln Asp Met Leu Arg Gln Leu Asp Leu Ser Pro Gly Cys wherein Xaa at position 28 is Gln or absent;
Human leptin: Val Pro Ile Gln Lys Val Gln Asp Asp Thr Lys Thr Leu Ile Lys Thr Ile Val Thr Arg Ile Asn Asp Ile Ser His Xaa Xaa Ser Val Ser Ser Lys Gln Lys Val Thr Gly Leu Asp Phe Ile Pro Gly Leu His Pro Ile Leu Thr Leu Ser Lys Met Asp Gln Thr Leu Ala Val Tyr Gln Gln Ile Leu Thr Ser Met Pro Ser Arg Asn Val Ile Gln Ile Ser Asn Asp Leu Glu Asn Leu Arg Asp Leu Leu His Val Leu Ala Phe Ser Lys Ser Cys His Leu Pro Trp Ala Ser Gly Leu Glu Thr Leu Asp Ser Leu Gly Gly Val Leu Glu Ala Ser Gly Tyr Ser Thr Glu Val Val Ala Leu Ser Arg Leu Gln Gly Ser Leu Gln Asp Met Leu Trp Gln Leu Asp Leu Ser Pro Gly Cys wherein: Xaa at position 27 is Thr or Ala; and Xaa at position 28 is Gln or absent; Rhesus Leptin: Val Pro Ile Gln Lys Val Gln Ser Asp Thr Lys Thr Leu Ile Lys Thr Ile Val Thr Arg Ile Asn Asp Ile Ser His Thr Gln Ser Val Ser Ser Lys Gln Arg Val Thr Gly Leu Asp Phe Ile Pro Gly Leu His Pro Val Leu Thr Leu Ser Gln Met Asp Gln Thr Leu Ala Ile Tyr Gln Gln Ile Leu Ile Asn Leu Pro Ser Arg Asn Val Ile Gln Ile Ser Asn Asp Leu Glu Asn Leu Arg Asp Leu Leu His Leu Leu Ala Phe Ser Lys Ser Cys His Leu Pro Leu Ala Ser Gly Leu Glu Thr Leu Glu Ser Leu Gly Asp Val Leu Glu Ala Ser Leu Tyr Ser Thr Glu Val Val Ala Leu Ser Arg Leu Gln Gly Ser Leu Gln Asp Met Leu Trp Gln Leu Asp Leu Ser Pro Gly Cys; and
Rat leptin: Val Pro Ile His Lys Val Gln Asp Asp Thr Lys Thr Leu Ile Lys Thr Ile Val Thr Arg Ile Asn Asp Ile Ser His Thr Gln Ser Val Ser Ala Arg Gln Arg Val Thr Gly Leu Asp Phe Ile Pro Gly Leu His Pro Ile Leu Ser Leu Ser Lys Met Asp Gln Thr Leu Ala Val Tyr Gln Gln Ile Leu Thr Ser Leu Pro Ser Gln Asn Val Leu Gln Ile Ala His Asp Leu Glu Asn Leu Arg Asp Leu Leu His Leu Leu Ala Phe Ser Lys Ser Cys Ser Leu Pro Gln Thr Arg Gly Leu Gln Lys Pro Glu Ser Leu Asp Gly Val Leu Glu Ala Ser Leu Tyr Ser Thr Glu Val Val Ala Leu Ser Arg Leu Gln Gly Ser Leu Gln Asp Ile Leu Gln Gln Leu Asp Leu Ser Pro Glu Cys.

In another embodiment leptin peptides are of the sequence:
Val Pro Ile GIn Lys Val Gln Asp Asp Thr Lys Thr Leu Ile Lys Thr Ile Val Thr Arg Ile Xaa Asp Ile Ser His Xaa Xaa Ser Val Ser Ser Lys Gln Lys Val Thr Gly Leu Asp Phe Ile Pro Gly Leu His Pro Ile Leu Thr Leu Ser Lys Xaa Asp Gln Thr Leu Ala Val Tyr Gln Gln Ile Leu Thr Ser Xaa Pro Ser Arg Xaa Val Ile Gln Ile Xaa Asn Asp Leu Glu Asn Leu Arg Asp Leu Leu His Val Leu Ala Phe Ser Lys Ser Cys His Leu Pro Trp Ala Ser Gly Leu Glu Thr Leu Asp Ser Leu Gly Gly Val Leu Glu Ala Ser Xaa Tyr Ser Thr Glu Val Val Ala Leu Ser Arg Leu Gln Gly Ser Leu Gln Asp Met Leu Trp Gln Leu Asp Leu Ser Pro Gly Cys, wherein: Xaa at position 22 is Asn, Asp or Glu; Xaa at position 27 is Thr or Ala; Xaa at position 28 is Gln, Glu, or absent; Xaa at position 54 s Met or Ala; Xaa at position 68 s Met or Leu; Xaa at position 72 Asn, Asp or Glu; Xaa at position 77 is Ser or Ala; Xaa at position 118 is Gly or Leu; said protein having at least one substitution selected from the group consisting of: His at position 97 is replaced with Ser or Pro; Trp at position 100 is replaced with Gln, Ala or Leu; Ala at position 101 is replaced with Thr or Val; Ser at position 102 is replaced with Arg; Gly at position 103 is replaced with Ala; Glu at position 105 is replaced with Gln; Thr at position 106 is replaced with Lys or Ser; Leu at position 107 is replaced with Pro; Asp at position 108 is replaced with Glu; or Gly at position 111 is replaced with Asp.

In another embodiment leptin are of the sequence: Val Pro Ile Gln Lys Val Gln Asp Asp Thr Lys Thr Leu Ile Lys Thr Ile Val Thr Arg Ile Asn Asp Ile Ser His Xaa Gln Ser Val Ser Ser Lys Gln Lys Val Thr Gly Leu Asp Phe Ile Pro Gly Leu His Pro Ile Leu Thr Leu Ser Lys Met Asp Gln Thr Leu Ala Val Tyr Gln Gln Ile Leu Thr Ser Met Pro Ser Arg Asn Val Ile Gln Ile Xaa Asn Asp Leu Glu Asn Leu Arg Asp Leu Leu His Val Leu Ala Phe Ser Lys Ser Cys His Leu Pro Trp Ala Ser Gly Leu Glu Thr Leu Asp Ser Leu Gly Gly Val Leu Glu Ala Ser Xaa Tyr Ser Thr Glu Val Val Ala Leu Ser Arg Leu Gln Gly Ser Leu Gln Asp Met Leu Trp Gln Leu Asp Leu Ser Pro Gly Cys, wherein: Xaa at position 27 is Thr or Ala; Xaa at position 77 is Ser or Ala; Xaa at position 118 is Gly or Leu; said protein having at least one substitution, preferably having one to five substitutions and, most preferably, one to two substitutions selected from the group consisting of: His at position 97 is replaced with Ser; Trp at position 100 is replaced with Gln; Ala at position 101 is replaced with Thr; Glu at position 105 is replaced with Gln; Thr at position 106 is replaced with Lys; Leu at position 107 is replaced with Pro; Asp at position 108 is replaced with Glu; or Gly at position 111 is replaced with Asp. In further embodiments of the above sequence Xaa at position 27 is Thr; Xaa at position 77 is Ser; Xaa at position 118 is Gly; and the amino acid residues at positions 97, 100, 101,105,106,107,108, and 111 are as in the following table:

| | 97 | 100 | 101 | 105 | 106 | 107 | 108 | 111 |
|---|---|---|---|---|---|---|---|---|
| native human | His | Trp | Ala | Glu | Thr | Leu | Asp | Gly |
| | Ser | Trp | Ala | Glu | Thr | Leu | Asp | Gly |
| | His | Gln | Ala | Glu | Thr | Leu | Asp | Gly |
| | His | Trp | Thr | Glu | Thr | Leu | Asp | Gly |
| | His | Trp | Ala | Gln | Thr | Leu | Asp | Gly |
| | His | Trp | Ala | Glu | Lys | Leu | Asp | Gly |
| | His | Trp | Ala | Glu | Thr | Pro | Asp | Gly |
| | His | Trp | Ala | Glu | Thr | Leu | Glu | Gly |
| | His | Trp | Ala | Glu | Thr | Leu | Asp | Asp |
| | Ser | Gln | Ala | Glu | Thr | Leu | Asp | Gly |
| | Ser | Trp | Thr | Glu | Thr | Leu | Asp | Gly |
| | Ser | Trp | Ala | Gln | Thr | Leu | Asp | Gly |
| | Ser | Trp | Ala | Glu | Lys | Leu | Asp | Gly |
| | Ser | Trp | Ala | Glu | Thr | Pro | Asp | Gly |
| | Ser | Trp | Ala | Glu | Thr | Leu | Glu | Gly |
| | Ser | Trp | Ala | Glu | Thr | Leu | Asp | Asp |
| | His | Gln | Thr | Glu | Thr | Leu | Asp | Gly |
| | His | Gln | Ala | Gin | Thr | Leu | Asp | Gly |
| | His | Gln | Ala | Glu | Lys | Leu | Asp | Gly |
| | His | Gln | Ala | Glu | Thr | Pro | Asp | Gly |
| | His | Gln | Ala | Glu | Thr | Leu | Glu | Gly |
| | His | Gln | Ala | Glu | Thr | Leu | Asp | Asp |
| | His | Trp | Thr | Gln | Thr | Leu | Asp | Gly |
| | His | Trp | Thr | Glu | Lys | Leu | Asp | Gly |
| | His | Trp | Thr | Glu | Thr | Pro | Asp | Gly |
| | His | Trp | Thr | Glu | Thr | Leu | Glu | Gly |
| | His | Trp | Thr | Glu | Thr | Leu | Asp | Asp |
| | His | Trp | Ala | Gln | Lys | Leu | Asp | Gly |
| | His | Trp | Ala | Gln | Thr | Pro | Asp | Gly |
| | His | Trp | Ala | Gln | Thr | Leu | Glu | Gly |
| | His | Trp | Ala | Gln | Thr | Leu | Asp | Asp |
| | His | Trp | Ala | Glu | Lys | Pro | Asp | Gly |
| | His | Trp | Ala | Glu | Lys | Leu | Glu | Gly |
| | His | Trp | Ala | Glu | Lys | Leu | Asp | Asp |
| | His | Trp | Ala | Glu | Thr | Pro | Glu | Gly |
| | His | Trp | Ala | Glu | Thr | Pro | Asp | Asp |
| | His | Trp | Ala | Glu | Thr | Leu | Glu | Asp |
| | Ser | Gln | Thr | Glu | Thr | Leu | Asp | Gly |
| | Ser | Gln | Ala | Gln | Thr | Leu | Asp | Gly |
| | Ser | Gln | Ala | Glu | Lys | Leu | Asp | Gly |
| | Ser | Gln | Ala | Glu | Thr | Pro | Asp | Gly |
| | Ser | Gln | Ala | Glu | Thr | Leu | Glu | Gly |
| | Ser | Gln | Ala | Glu | Thr | Leu | Asp | Asp |
| | Ser | Trp | Thr | Gln | Thr | Leu | Asp | Gly |
| | Ser | Trp | Thr | Glu | Lys | Leu | Asp | Gly |
| | Ser | Trp | Thr | Glu | Thr | Pro | Asp | Gly |
| | Ser | Trp | Thr | Glu | Thr | Leu | Glu | Gly |
| | Ser | Trp | Thr | Glu | Thr | Leu | Asp | Asp |
| | Ser | Trp | Ala | Gln | Lys | Leu | Asp | Gly |
| | Ser | Trp | Ala | Gln | Thr | Pro | Asp | Gly |
| | Ser | Trp | Ala | Gln | Thr | Leu | Glu | Gly |
| | Ser | Trp | Ala | Gln | Thr | Leu | Asp | Asp |
| | Ser | Trp | Ala | Glu | Lys | Pro | Asp | Gly |
| | Ser | Trp | Ala | Glu | Lys | Leu | Glu | Gly |
| | Ser | Trp | Ala | Glu | Lys | Leu | Asp | Asp |
| | Ser | Trp | Ala | Glu | Thr | Pro | Glu | Gly |
| | Ser | Trp | Ala | Glu | Thr | Pro | Asp | Asp |
| | Ser | Trp | Ala | Glu | Thr | Leu | Glu | Asp |
| | His | Gln | Thr | Gln | Thr | Leu | Asp | Gly |
| | His | Gln | Thr | Glu | Lys | Leu | Asp | Gly |
| | His | Gln | Thr | Glu | Thr | Pro | Asp | Gly |
| | His | Gln | Thr | Glu | Thr | Leu | Glu | Gly |
| | His | Gln | Thr | Glu | Thr | Leu | Asp | Asp |
| | His | Gln | Ala | Gln | Lys | Leu | Asp | Gly |
| | His | Gln | Ala | Gln | Thr | Pro | Asp | Gly |
| | His | Gln | Ala | Gln | Thr | Leu | Glu | Gly |
| | His | Gln | Ala | Gln | Thr | Leu | Asp | Asp |
| | His | Gln | Ala | Glu | Lys | Pro | Asp | Gly |
| | His | Gln | Ala | Glu | Lys | Leu | Glu | Gly |
| | His | Gln | Ala | Glu | Lys | Leu | Asp | Asp |
| | His | Gln | Ala | Glu | Thr | Pro | Glu | Gly |
| | His | Gln | Ala | Glu | Thr | Pro | Asp | Asp |
| | His | Gln | Ala | Glu | Thr | Leu | Glu | Asp |
| | His | Trp | Thr | Gln | Lys | Leu | Asp | Gly |
| | His | Trp | Thr | Gln | Thr | Pro | Asp | Gly |
| | His | Trp | Thr | Gin | Thr | Leu | Glu | Gly |
| | His | Trp | Thr | Gln | Thr | Leu | Asp | Asp |
| | His | Trp | Thr | Glu | Lys | Pro | Asp | Gly |
| | His | Trp | Thr | Glu | Lys | Leu | Glu | Gly |
| | His | Trp | Thr | Glu | Lys | Leu | Asp | Asp |
| | His | Trp | Thr | Glu | Thr | Pro | Glu | Gly |
| | His | Trp | Thr | Glu | Thr | Pro | Asp | Asp |
| | His | Trp | Thr | Glu | Thr | Leu | Glu | Asp |
| | His | Trp | Ala | Gln | Lys | Pro | Asp | Gly |
| | His | Trp | Ala | Gln | Lys | Leu | Glu | Gly |
| | His | Trp | Ala | Gln | Lys | Leu | Asp | Asp |
| | His | Trp | Ala | Gln | Thr | Pro | Glu | Gly |
| | His | Trp | Ala | Gln | Thr | Pro | Asp | Asp |
| | His | Trp | Ala | Gln | Thr | Leu | Glu | Asp |
| | His | Trp | Ala | Glu | Lys | Pro | Glu | Gly |
| | His | Trp | Ala | Glu | Lys | Pro | Asp | Asp |
| | His | Trp | Ala | Glu | Lys | Leu | Glu | Asp |
| | His | Trp | Ala | Glu | Thr | Pro | Glu | Asp |
| | Ser | Gln | Thr | Gln | Thr | Leu | Asp | Gly |
| | Ser | Gln | Thr | Glu | Lys | Leu | Asp | Gly |
| | Ser | Gln | Thr | Glu | Thr | Pro | Asp | Gly |
| | Ser | Gln | Thr | Glu | Thr | Leu | Glu | Gly |
| | Ser | Gln | Thr | Glu | Thr | Leu | Asp | Asp |
| | Ser | Gln | Ala | Gln | Lys | Leu | Asp | Gly |
| | Ser | Gln | Ala | Gln | Thr | Pro | Asp | Gly |
| | Ser | Gln | Ala | Gln | Thr | Leu | Glu | Gly |
| | Ser | Gln | Ala | Gln | Thr | Leu | Asp | Asp |
| | Ser | Gln | Ala | Glu | Lys | Pro | Asp | Gly |
| | Ser | Gln | Ala | Glu | Lys | Leu | Glu | Gly |
| | Ser | Gln | Ala | Glu | Lys | Leu | Asp | Asp |
| | Ser | Gln | Ala | Glu | Thr | Pro | Glu | Gly |
| | Ser | Gln | Ala | Glu | Thr | Pro | Asp | Asp |
| | Ser | Gln | Ala | Glu | Thr | Leu | Glu | Asp |
| | Ser | Trp | Thr | Gln | Lys | Leu | Asp | Gly |
| | Ser | Trp | Thr | Gln | Thr | Pro | Asp | Gly |
| | Ser | Trp | Thr | Gln | Thr | Leu | Glu | Gly |
| | Ser | Trp | Thr | Gln | Thr | Leu | Asp | Asp |
| | Ser | Trp | Thr | Glu | Lys | Pro | Asp | Gly |
| | Ser | Trp | Thr | Glu | Lys | Leu | Glu | Gly |
| | Ser | Trp | Thr | Glu | Lys | Leu | Asp | Asp |
| | Ser | Trp | Thr | Glu | Thr | Pro | Glu | Gly |
| | Ser | Trp | Thr | Glu | Thr | Pro | Asp | Asp |
| | Ser | Trp | Thr | Glu | Thr | Leu | Glu | Asp |
| | Ser | Trp | Ala | Gln | Lys | Pro | Asp | Gly |
| | Ser | Trp | Ala | Gln | Lys | Leu | Glu | Gly |
| | Ser | Trp | Ala | Gln | Lys | Leu | Asp | Asp |
| | Ser | Trp | Ala | Gln | Thr | Pro | Glu | Gly |
| | Ser | Trp | Ala | Gln | Thr | Pro | Asp | Asp |
| | Ser | Trp | Ala | Gln | Thr | Leu | Glu | Asp |
| | Ser | Trp | Ala | Glu | Lys | Pro | Glu | Gly |
| | Ser | Trp | Ala | Glu | Lys | Pro | Asp | Asp |
| | Ser | Trp | Ala | Glu | Lys | Leu | Glu | Asp |
| | Ser | Trp | Ala | Glu | Thr | Pro | Glu | Asp |
| | His | Gln | Thr | Gln | Lys | Leu | Asp | Gly |
| | His | Gln | Thr | Gln | Thr | Pro | Asp | Gly |
| | His | Gln | Thr | Gln | Thr | Leu | Glu | Gly |
| | His | Gln | Thr | Gln | Thr | Leu | Asp | Asp |
| | His | Gln | Thr | Glu | Lys | Pro | Asp | Gly |
| | His | Gln | Thr | Glu | Lys | Leu | Glu | Gly |
| | His | Gln | Thr | Glu | Lys | Leu | Asp | Asp |
| | His | Gln | Thr | Glu | Thr | Pro | Glu | Gly |
| | His | Gln | Thr | Glu | Thr | Pro | Asp | Asp |
| | His | Gln | Thr | Glu | Thr | Leu | Glu | Asp |
| | His | Gln | Ala | Gln | Lys | Pro | Asp | Gly |
| | His | Gln | Ala | Gln | Lys | Leu | Glu | Gly |
| | His | Gln | Ala | Gln | Lys | Leu | Asp | Asp |
| | His | Gln | Ala | Gln | Thr | Pro | Glu | Gly |
| | His | Gln | Ala | Gln | Thr | Pro | Asp | Asp |
| | His | Gln | Ala | Gln | Thr | Leu | Glu | Asp |

In one embodiment the leptin is of the sequence: Ile Pro Gly Leu His Pro Ile Leu Thr Leu Ser Lys Xaa Asp Xaa Thr Leu Ala Val Tyr Xaa Xaa Ile Leu Thr Ser Xaa Pro Ser Arg Xaa Val Ile Xaa Ile Ser Xaa Asp Leu Glu Xaa Leu Arg Asp Leu Leu His Val Leu Ala Phe Ser Lys Ser Cys His Leu Pro Xaa Ala Ser Gly Leu Glu Thr Leu Asp Ser Leu Gly Gly Val Leu Glu Ala Ser Gly Tyr Ser Thr Glu Val Val Ala Leu Ser Arg Leu Xaa Gly Ser Leu Xaa Asp Xaa Leu Xaa Xaa Leu Asp Leu Ser Pro Gly Cys, wherein: Xaa at position 13 is Ile, Leu, Met or methionine sulfoxide; Xaa at position 15 is Gln or Glu; Xaa at position 21 is Gln or Glu; Xaa at position 22 is Gln or Glu; Xaa at position 27 is Ile, Leu, Met or methionine sulfoxide; Xaa at position 31 is Asn, Asp or Gln; Xaa at position 34 is Gln or Glu; Xaa at position 37 is Asn, Asp or Gln; Xaa at position 41 is Asn, Asp or Gln; Xaa at position 59 is Trp or Gln; Xaa at position 89 is Gln or Glu; Xaa at position 93 is Gln or Glu; Xaa at position 95 is Ile, Leu, Met or methionine sulfoxide; Xaa at position 97 is Trp or Gln; and Xaa at position 98 is Gln or Glu. In another embodiment the leptin of the above formula has Xaa at position 13 is Met; Xaa at position 15 is Gln; Xaa at position 21 is Gln; Xaa at position 22 is Gln; Xaa at position 27 is Met; Xaa at position 31 is Asn; Xaa at position 34 is Gln; Xaa at position 37 is Asn; Xaa at position 41 is Asn; Xaa at position 59 is Trp; Xaa at position 89 is Gln; Xaa at position 93 is Gln; Xaa at position 95 is Met; Xaa at position 97 is Trp; and Xaa at position 98 is Gln. See U.S. patent 5532336.

Exemplary leptin analogs include those where the amino acid at position 43 is substituted with Asp or Glu; position 48 is substituted Ala; position 49 is substituted with Glu, or absent; position 75 is substituted with Ala; position 89 is substituted with Leu; position 93 is substituted with Asp or Glu; position 98 is substituted with Ala; position 117 is substituted with Ser, position 139 is substituted with Leu, position 167 is substituted with Ser, and any combination thereof.

Certain exemplary leptin and leptin analogs with leptin activity include:

| SEQ ID: | |
|---|---|
| 151 | ⁴³Asp-leptin |
| 152 | ⁴³Glu-leptin |
| 153 | ⁴⁸Ala-leptin |
| 154 | ⁴⁹Glu-leptin |
| 155 | ⁴⁹Des-AA-leptin |
| 156 | ⁷⁵Ala-leptin |
| 157 | ⁸⁹Leu-leptin |
| 158 | ⁹³Asp-leptin |
| 159 | ⁹³Glu-leptin |
| 160 | ⁹⁸Ala-leptin |
| 161 | ¹¹⁷Ser-leptin |
| 162 | ¹³⁹Leu-leptin |
| 163 | ¹⁶⁷Ser-leptin |
| 164 | ⁴³Asp, ⁴⁹Glu-leptin |
| 165 | ⁴³Asp, ⁷⁵Ala-leptin |
| 166 | ⁸⁹Leu, ¹¹⁷Ser-leptin |
| 167 | ⁹³Glu, ¹⁶⁷Ser-leptin |

### The PPF Family

Component peptide hormones useful in the present disclosure also include PPF peptide hormones, including PP and PYY. Native PPF peptide hormones are known in art, as are functional peptide analogs and derivatives. Certain preferred native peptides, peptide analogs and derivatives are described herein, however it should be recognized that any known amylin family peptides that exhibit hormonal activity known in the art may be used in conjunction with the present disclosure.

Any PPF analog or derivative known in the art may be used in conjunction with the present disclosure. In one embodiment, the PPF analogs and derivatives have at least one hormonal activity of a native PPF polypeptide. In certain embodiments, the PPF analogs are agonists of a receptor which native PPF polypeptide is capable of specifically binding. Preferred PPF analogs and derivatives include those described in WO 03/026591 and WO 03/057235.

In one embodiment, preferred PPF analogs and derivatives that exhibit at least one PPF hormonal activity generally comprise at least two PYY motifs including a polyproline motif and C-terminal tail motif. Such analogs are generally described in U.S. Provisional Application No. 60/543,406 filed February 11, 2004, published as US 2006/013547A1 on June 22, 2006.

Other preferred PPF analogs are disclosed in PCT/US2005/004351, entitled "Pancreatic Polypeptide Family Motifs and Polypeptides Comprising the Same", published as WO2005/077094 on August 25, 2005.

Other preferred PPF analogs are disclosed in PCT/US2005/045471, entitled "Pancreatic Polypeptide Family Motifs, Polypeptides and Methods Comprising the Same", published as WO2006/066024 on June 22, 2006.

By way of background, research has suggested that the differences in Y receptor binding affinities are correlated with secondary and tertiary structural differences. *See, e.g.,* Keire et al., Biochemistry 2000, 39, 9935-9942. Native porcine PYY has been characterized as including two C-terminal helical segments from residues 17 to 22 and 25 to 33 separated by a kink at residues 23, 24, and 25, a turn centered around residues 12-14, and the N-terminus folded near residues 30 and 31. Further, full-length porcine PYY has been characterized as including the PP fold, stabilized by hydrophobic interactions among residues in the N- and C- termini. *See id.*

A "PYY motif' is generally a structural component, primary, secondary, or tertiary, of a native PP family polypeptide that is critical to biological activity, *i.e.,* biological activity is substantially decreased in the absence or disturbance of the motif. Preferred PYY motifs include the N-terminal polyproline type II motif of a native PP family polypeptide, the type II β-turn motif of native PP family polypeptide, the α-helical motif at the C-terminal end of native PP family polypeptide, and the C-terminal tail motif of native PP family polypeptide. More particularly, in the N-terminal polyproline region, amino acids corresponding to residues 5 and 8 of a native PP family polypeptide are generally conserved as a proline. The type II β-turn motif will generally include amino acids corresponding to residues 12-14 of a native PP family polypeptide. The α-helical motif can generally extend from amino acids corresponding to approximately residue 14 of a native PP family polypeptide to any point up to and including the C-terminal end, so long as the α-helical motif includes a sufficient number of amino acid residues such that an α-helical turn is formed in solution. The α-helical motif can also include amino acid substitutions, insertions and deletions to the native PP family sequence, so long as the α-helical turn is still formed in solution. The C-terminal tail motif generally includes amino acids corresponding to approximately the last 10 residues of a native PP family polypeptide, more preferably the last 7, 6, or 5 residues of a native PP family polypeptide, and more preferably amino acid residues 32-35.

Preferred PYY analogs include those with internal deletions, insertions, and substitutions in areas of the PYY molecule not corresponding to the polyproline motif and/or the C-terminal tail motif. For instance, internal deletions at positions 4, 6, 7, 9, or 10 are envisioned.

In another embodiment of particular interest, the component hormone is a PPF polypeptide containing at least two PPF motifs including at least the N-terminal polyproline PPF motif and the C-terminal tail PPF motif. As used herein, "motif" refers to an amino acid sequence that is characteristic of a specific biochemical function or defines an independently folded domain. Additional PPF motifs can correspond to a motif of any of the PP family polypeptides, including PP, PYY and NPY, for example the type II β-turn region motif of PYY, or the α-helical motif at the C-terminal end of PYY.

In yet another embodiment the PPF family component module is a PPF chimeric polypeptide comprising a fragment of a PP, PYY or NPY polypeptide covalently linked to at least one additional fragment of a second PP, PYY or NPY polypeptide, wherein each PP, PYY or NPY fragment includes a PPF motif. Alternatively, the PPF chimeric can comprise a fragment of a PP family polypeptide linked to one, two, three, or four polypeptides segments, wherein at least one of the linked polypeptide segments is a fragment of a second PP family polypeptide. In certain embodiments, PPF polypeptides do not include an N-terminal PP fragment with a C-terminal NPY fragment. PPF chimeric polypeptide component module will exhibit at least 50% sequence identity to a native PYY(3-36) over the entire length of the PYY(3-36). In some embodiments, such PPF chimeric polypeptide can exhibit at least 60%, at least 70%, at least 80%, at least 90%, at least 92%, at least 94% or at least 97% sequence identity to a native PYY(3-36) over the entire length of the PYY(3-36). Such PPF chimeric polypeptides can exhibit at least 50%, at least 60%, at least 70%, at least 80%, at least 90%, at least 92%, at least 94% or at least 97% sequence identity to a native PP. In yet another embodiment, such PPF chimeric polypeptide can exhibit at least 50%, at least 60%, at least 70%, at least 80%, at least 90%, at least 92%, at least 94% or at least 97% sequence identity to a native NPY. In some embodiments, the PPF chimeric polypeptides can include at least the N-terminal polyproline PPF motif and the C-terminal tail PPF motif. These PPF chimeras as well as other PYY and PP analogs, are described in US 2006/013547A1 published June 22, 2006. In one embodiment, any of the PPF family peptides, if not contained as a hybrid component, can be provided as a second agent, e.g. as a second anti-obesity agent, with a hybrid as described herein.

Again; the PPF chimeric polypeptide will generally retain, at least in part, a biological activity of native human PP, PYY, or NPY. In some embodiments, the PPF chimeric polypeptide exhibit biological activity in the treatment and prevention of metabolic conditions and disorders.

The polypeptide fragments of the PPF chimeras can be covalently linked together in any manner known in the art, including but not limited to direct amide bonds or chemical linker groups. Chemical linker groups may include peptide mimetics which induce or stabilize polypeptide conformation. PPF chimeric polypeptides include PYY-PP, PYY-NPY, PP-PYY, PP-NPY, NPY-PP, or NPY-PYY chimeras.

The PPF chimera can be at least 21, 22, 23, 24, 25, 26, 27, 28, 29, 30, 31, 32, 33, or 34 amino acids in length. In some embodiments, the PYY analog polypeptides include only natural L amino acid residues and/or modified natural L amino acid residues. In some embodiments, the PYY analog polypeptides do not include unnatural amino acid residues.

In some embodiments, the PPF chimeric polypeptides include: hPP(1-7)-pNPY, hPP(1-17)-pNPY, hPP(19-23)-pNPY, hPP(19-23)-Pro³⁴pNPY, hPP(19-23)-His³⁴pNPY, rPP(19-23)-pNPY, rPP(19-23)-Pro³⁴pNPY, rPP(19-23)-His³⁴pNPY, hPP(1-17)-His³⁴pNPY, NPY(1-7)-hPP, pNPY(1-7, 19-23)-hPP, cPP(1-7)-pNPY(19-23)-hPP, cPP(1-7)-NPY(19-23)-His³⁴hPP, hPP(1-17)-His³⁴pNPY, hPP(19-23)-pNPY, hPP(19-23)-Pro³⁴pNPY, pNPY(1-7)-hPP, pNPY(19-23)-hPP, pNPY(19-23)-Gln³⁴hPP, pNPY(19-23)-His³⁴hPP, pNPY(19-23)-Phe⁶Gln³⁴hPP, pNPY(19-23)-Phe⁶His³⁴hPP, pNPY(1-7,19-23)-hPP, pNPY(1-7,19-23)-Gln³⁴ hPP, cPP(20-23)-Pro³⁴-pNPY, cPP(21-23)-Pro³⁴-pNPY, cPP(22-23)-Pro³⁴-pNPY, cPP(1-7)-Pro³⁴-pNPY, cPP(20-23)-Pro³⁴-pNPY, cPP(1-7,20-23)-Pro³⁴-pNPY, cPP(1-7)-pNPY(19-23)-hPP, cPP(1-7)-pNPY(19-23)-His³⁴hPP, cPP(1-7)-gPP(19-23)-hPP, cPP(1-7)-pNPY(19-23)-Ala³¹Aib³²Gln³⁴-hPP, cPP(1-7)-pNPY(19-23)-Ala³¹Aib³²His³⁴-hPP hPP(1-7)-Ala³¹Aib³²-pNPY, hPP(1-17)-Ala³¹Aib³²-pNPY, pNPY(1-7)-Ala³¹Aib³²Gln³⁴-hPP, or pNPY(1-7, 19-23)-Ala³¹Aib³²Gln³⁴-hPP.

In some embodiments, the PPF chimeric polypeptides can comprise fragments of PP family analog polypeptides. For instance, the PPF chimeric polypeptides may comprise PPF analog polypeptides described herein, as well as PP analog polypeptides, and NPY analog polypeptides.

PYY analog polypeptide for use in the hybrids of the disclosure or as a second agent are those having a potency in one of the assays described herein (including food intake, gastric emptying, pancreatic secretion, body composition or weight reduction assays) which is equal to or greater than the potency of NPY, PYY, or PYY(3-36) in that same assay. In some embodiments, the PPY analog polypeptides may be useful in the treatment of metabolic diseases, such as, for example, obesity, insulin resistence syndrome (Syndrome X) or diabetes mellitus. In some embodiments, PYY analog polypeptides may exhibit improved ease of manufacture, stability, and/or ease of formulation, as compared to PP, NPY, PYY, or PYY(3-36).

In some embodiments, the PPF chimeric polypeptides retain at least about 25%, or from about 30%, about 40%, about 50%, about 60%, about 70%, about 80%, about 90%, about 95%, about 98%, or about 99% percent of the biological activity of native human PYY with regard to the reduction of nutrient availability, the reduction of food intake, the effect of body weight gain, and/or the treatment and prevention of metabolic conditions and disorders. In another embodiment, the PPF chimeric polypeptides exhibit improved PYY agonist activity. In some embodiments, the PPF chimeric polypeptides exhibits at least about 110%, about 125%, about 130%, about 140%, about 150%, about 200%, or more of the biological activity of native human PYY with regard to the reduction of nutrient availability the reduction of food intake, the effect of body weight gain, and/or the treatment and prevention of metabolic conditions and disorders.

More particularly, in one aspect, the PPF chimeric polypeptides comprise a fragment of PP linked to a fragment of PYY. In one embodiment, the PPF chimeric polypeptides comprise an N-terminal fragment of PP or a PP analog polypeptide linked at its C-terminal end to a C-terminal fragment of PYY or a PYY analog polypeptide. In another embodiment, the PPF chimeric polypeptides comprise an N-terminal fragment of PYY, PYY(3-36), or a PYY analog polypeptide linked at its C-terminal end to a C-terminal fragment of PP or a PP analog polypeptide.

In some embodiments, the PPF chimeric polypeptides comprise a fragment of PYY linked to a fragment of NPY. In one embodiment, the PPF chimeric polypeptides comprise an N-terminal fragment of PYY, PYY(3-36), or a PYY analog polypeptide linked at its C-terminal end to a C-terminal fragment of NPY or a NPY analog polypeptide. In another embodiment, the PPF chimeric polypeptides comprise an N-terminal fragment of NPY or a NPY analog polypeptide linked at its C-terminal end to a C-terminal fragment of PYY or a PYY analog polypeptide.

In some embodiments, the PPF chimeric polypeptides comprise a fragment of PP linked to a fragment of NPY. In one embodiment, the PPF chimeric polypeptides comprise an N-terminal fragment of PP or a PP analog polypeptide linked at its C-terminal end to a C-terminal fragment of NPY or a NPY analog polypeptide. In another embodiment, the PPF chimeric polypeptides comprise an N-terminal fragment of NPY or a NPY analog polypeptide linked at its C-terminal end to a C-terminal fragment of PP or a PP analog polypeptide.

In some embodiments, a fragment of PP, a PP analog polypeptide, PYY, PYY(3-36), a PYY analog polypeptide, NPY, or an NPY analog polypeptide is a fragment comprising anywhere from 4 to 20 amino acid residues of the PP, PP analog polypeptide, PYY, PYY(3-36), PYY analog polypeptide, NPY, or NPY analog polypeptide. In some embodiments, the length of fragment is selected so as to obtain a final PPF chimeric polypeptide of at least 34 amino acids in length.

The PPF chimeric polypeptides may also comprise further modifications including, but are not limited to, substitution, deletion, and insertion to the amino acid sequence of such PPF chimeric polypeptides and any combination thereof. In some embodiments, the PPF chimeric polypeptides include one or more modifications of a "non-essential" amino acid residue. A "non-essential" amino acid residue is a residue that can be altered, *i.e.,* deleted or substituted, in the native human amino acid sequence without abolishing or substantially reducing the activity interest.

Derivatives of the PPF chimeric polypeptides are also useful. Such derivatives include PPF chimeric polypeptides conjugated to one or more water soluble polymer molecules, such as polyethylene glycol ("PEG") or fatty acid chains of various lengths (e.g., stearyl, palmitoyl, octanoyl, oleoyl etc.), or by the addition of polyamino acids, such as poly-his, poly-arg, poly-lys, and poly-ala. Modifications to the PPF chimeric polypeptides can also include small molecule substituents, such as short alkyls and constrained alkyls (e.g., branched, cyclic, fused, adamantyl), and aromatic groups. In some embodiments, the water soluble polymer molecules will have a molecular weight ranging from about 500 to about 20,000 Daltons.

Such polymer-conjugations and small molecule substituent modifications may occur singularly at the N- or C-terminus or at the side chains of amino acid residues, which are not involved in formation of the hybrid, within the sequence of the PPF chimeric polypeptides. Alternatively, there may be multiple sites of derivatization along the PPF chimeric polypeptide. Substitution of one or more amino acids with lysine, aspartic acid, glutamic acid, or cysteine may provide additional sites for derivatization. *See, e.g.,* U.S. Patent Nos. 5,824,784 and 5,824,778. In some embodiments, the PPF chimeric polypeptides may be conjugated to one, two, or three polymer molecules.

In some embodiments, the water soluble polymer molecules are linked to an amino, carboxyl, or thiol group, and may be linked by N or C terminus, or at the side chains of lysine, aspartic acid, glutamic acid, or cysteine. Alternatively, the water soluble polymer molecules may be linked with diamine and dicarboxylic groups. In some embodiments, the PPF chimeric polypeptides are conjugated to one, two, or three PEG molecules through an epsilon amino group on a lysine amino acid.

PPF chimeric polypeptides also include PPF chimeric polypeptides with chemical alterations to one or more amino acid residues. Such chemical alterations include amidation, glycosylation, acylation, sulfation, phosphorylation, acetylation, and cyclization. The chemical alterations may occur singularly at the N- or C-terminus or at the side chains of amino acid residues within the sequence of the PPF chimeric polypeptides. In one embodiment, the C-terminus of these peptides may have a free -OH or NH₂ group. In another embodiment, the N-terminal end may be capped with an isobutyloxycarbonyl group, an isopropyloxycarbonyl group, an n-butyloxycarbonyl group, an ethoxycarbonyl group, an isocaproyl group (isocap), an octanyl group, an octyl glycine group (G(Oct)), or an 8-aminooctanic acid group. In some embodiments, cyclization can be through the formation of disulfide bridges. Alternatively, there may be multiple sites of chemical alteration along the PYY analog polypeptide.

In some embodiments, the PPF chimeric polypeptides include those having an amino acid sequence of SEQ ID NOs. 238-347 of US2006/013547A1.

Exemplary PPF chimeric polypeptides include polypeptides of the Formula (VI):
Xaa₁ Xaa₂ Xaa₃ Xaa₄ Pro Glu Xaa₇ Pro Xaa₉ Glu
Asp Xaa₁₂ Xaa₁₃ Xaa₁₄ Glu Xaa₁₆Xaa₁₇ Xaa₁₈ Xaa₁₉ Tyr
Xaa₂₁ Xaa₂₂ Xaa₂₃ Leu Xaa₂₅ Xaa₂₆ Tyr Xaa₂₈ Asn Xaa₃₀
Xaa₃₁ Thr Arg Gln Xaa₃₅ Xaa₃₆
wherein:
Xaa₁ is Tyr or absent;
Xaa₂ is Ile, Pro, or absent;
Xaa₃ is Ile, BH-modified Lys, Lys, Val, or Pro;
Xaa₄ is Lys, BH-modified Lys, Ala, Ser, or Arg;
Xaa₇ is Ala, Gly, or His;
Xaa₉ is Gly or Ala;
Xaa₁₂ is Ala or Pro;
Xaa₁₃ is Ser or Pro;
Xaa₁₄ is Pro, Ala, or Ser;
Xaa₁₆ is Glu or Asp;
Xaa₁₇ is Leu or Ile;
Xaa₁₈ is Asn or Ala;
Xaa₁₉ is Arg, Lys, BH-modified Lys, Gln, or N(Me)Ala;
Xaa₂₁ is Tyr, Ala, Phe, Lys or BH-modified Lys;
Xaa₂₂ is Ala or Ser;
Xaa₂₃ is Ser, Ala, or Asp;
Xaa₂₅ is Arg, Lys or BH-modified Lys;
Xaa₂₆ is His, Ala, or Arg;
Xaa₂₈ is Leu or Ile;
Xaa₃₀ is Leu or Met;
Xaa₃₁ is Val, Ile, or Leu;
Xaa₃₅ is Lys, BH-modified Lys, or Arg; and
Xaa₃₆ is Tyr, Trp, or Phe.

In one embodiment the PPF polypeptide of Formula VI can be a native PPF polypeptide, PYY(2-36), Val3hPYY(3-36), Lys25hPYY(3-36), Lys25Ile28hPYY(3-36), Lys25Ile31hPYY(3-36), Lys25Leu31hPYY(3-36), Lys25Phe36hPYY(3-36), Ile28hPYY(3-36), Ile31hPYY(3-36), Leu31hPYY(3-36), Phe36hPYY(3-36), Leu31Phe36hPYY(3-36), or Pro13Ala14hPYY.

As will be recognized by one of skill in the art, the polypeptides of Formula VI may be in the free acid form, or may be C-terminally amidated.

In some embodiments, the PPF polypeptide may comprise an N-terminal fragment consisting essentially of the first 17 amino acid residues of native human PYY (Tyr Pro Ile Lys Pro Glu Ala Pro Gly Glu Asp Ala Ser Pro Glu Glu Leu Asn Arg Tyr Tyr Ala Ser Leu Arg His Tyr Leu Asn Leu Val Thr Arg Gln Arg Tyr) linked to a C-terminal fragment consisting essentially of amino acid residues 18-36 of native human NPY (Tyr Pro Ser Lys Pro Asp Asn Pro Gly Glu Asp Ala Pro Ala Glu Asp Met Ala Arg Tyr Tyr Ser Ala Leu Arg His Tyr Ile Asn Leu Ile Thr Arg Gln Arg Tyr), wherein one or more amino acid residues at the N-terminus of the PYY fragment may be deleted or absent, and wherein one, two, three, four, five, six, seven, eight, nine or ten amino acid substitutions may be made in each of the PYY and NPY fragments. In some embodiments, an N-terminal fragment consisting essentially of the first 17 amino acids of the PPF polypeptide may exhibit at least 50%, at least 60%, at least 70%, at least 80%, at least 90%, at least 92%, at least 94% or at least 97% sequence identity to the first 17 amino acids of a native PYY. In some embodiments, a C-terminal fragment of the PPF polypeptide consisting essentially of amino acid residues 18-36 may exhibit at least 50%, at least 60%, at least 70%, at least 80%, at least 90%, at least 92%, at least 94% or at least 97% sequence identity to amino acids 18-36 of a native NPY. In some embodiments, amino acids in the N-terminal fragment of PYY (e.g., prolines at position 5 and 8, glutamates at positions 6, 10 and 15, or aspartate at position 11), and/or amino acids in the C-terminal fragment of NPY (e.g., tyrosines at positions 20 and 27, leucine at position 24, asparagine at position 29, threonine at position 32, arginine at position 33, or glutamine at position 34) are not substituted. In some embodiments, the PPF polypeptides include those having an amino acid sequence of SEQ ID Nos. 266, 267, 274, 282, 320, and 436 to 480 of US2006/013547A1. In some embodiments, the PPF polypeptides further comprise an N-terminal cap. Examples of these PPF polypeptides include SEQ ID NOs: 282, 320, 437, 441, 444, 445-447, 452, 454-459, 461-464, 466, 468-470 and 472-480 of US2006/013547A1.

Other PPF polypeptides include polypeptides of the Formula (VII):
Xaa₁ Xaa₂ Pro Xaa₄ Pro Xaa₆ His Pro Xaa₉ Xaa₁₀
Xaa₁₁ Xaa₁₂ Xaa₁₃ Xaa₁₄ Xaa₁₅ Xaa₁₆ Xaa₁₇ Ala Xaa₁₉ Tyr
Xaa₂₁ Xaa₂₂ Xaa₂₃ Leu Xaa₂₅ Xaa₂₆ Xaa₂₇ Xaa₂₈ Xaa₂₉ Xaa₃₀
Xaa₃₁ Thr Arg Gln Arg Tyr
wherein:
Xaa₁ is Tyr or absent;
Xaa₂ is Ile, Pro, or absent;
Xaa₄ is Lys, BH-modified Lys, Ala, Ser, or Arg;
Xaa₆ is Glu, Gln, Ala, Asn, Asp, or Val;
Xaa₉ is Gly or Ala;
Xaa₁₀ is Glu, Ala, Asp, Asn, Gln, Gly, Pro, or Aib;
Xaa₁₁ is Glu, Ala, Asp, Asn, Gln, Gly, Pro, or Aib;
Xaa₁₂ is Ala or Pro;
Xaa₁₃ is Ser or Pro;
Xaa₁₄ is Pro, Ala, or Ser;
Xaa₁₅ is Glu, Ala, Asp, Asn, Gln, Gly, Pro, or Aib;
Xaa₁₆ is Glu or Asp;
Xaa₁₇ is Leu or Ile;
Xaa₁₉ is Arg, Lys, BH-modified Lys, Gln, or N(Me)Ala;
Xaa₂₁ is Tyr, Ala, Phe, Lys, or BH-modified Lys;
Xaa₂₂ is Ala or Ser;
Xaa₂₃ is Ser, Ala, or Asp;
Xaa₂₅ is Arg, Lys or BH-modified Lys;
Xaa₂₆ is His, Ala, or Arg;
Xaa₂₇ is Tyr, or Phe;
Xaa₂₈ is Leu or Ile;
Xaa₂₉ is Asn, or Gln;
Xaa₃₀ is Leu or Met; and
Xaa₃₁ is Val, Ile, or Leu.

As will be recognized by one of skill in the art, the polypeptides of Formula VII may be in the free acid form, or may be C-terminally amidated.

In some embodiments, the PPF polypeptide may comprise an N-terminal fragment consisting essentially of the first 17 amino acid residues of native human PYY (SEQ ID NO: 2 of US2006/013547A1) linked to a C-terminal fragment consisting essentially of amino acid residues 18-36 of native human NPY (SEQ ID NO: 4 of US 2006/013547A1), wherein one or more amino acid residues at the N-terminus of the PYY fragment may be deleted or absent, and wherein one, two, three, four, five, six, seven, eight, nine or ten amino acid substitutions may be made in each of the PYY and NPY fragments. In some embodiments, an N-terminal fragment consisting essentially of the first 17 amino acids of the PPF polypeptide may exhibit at least 50%, at least 60%, at least 70%, at least 80%, at least 90%, at least 92%, at least 94% or at least 97% sequence identity to the first 17 amino acids of a native PYY. In some embodiments, a C-terminal fragment of the PPF polypeptide consisting essentially of amino acid residues 18-36 may exhibit at least 50%, at least 60%, at least 70%, at least 80%, at least 90%, at least 92%, at least 94% or at least 97% sequence identity to amino acids 18-36 of a native NPY. In some embodiments, amino acids in the N-terminal fragment of PYY (e.g., prolines at positions 3, 5 and 8, or histidine 7), and/or amino acids in the C-terminal fragment of NPY (e.g., alanine at position 18, tyrosines at positions 20 and 36, leucine at position 24, threonine at position 32, arginine at position 33, glutamine at position 34, or arginine at position 35) are not substituted. In some embodiments, the PPF polypeptides include those having an amino acid sequence of a PYY-NPY chimera such as SEQ ID Nos. 266, 437, 438, 439, 442, 462, 469, 470, 471 and 472 of US 2006/013547A1, or a compound with the amino acid sequence Ile, Lys, Pro, Glu, His, Pro, Gly, Glu, Asp, Ala, Ser, Pro, Glu, Glu, Leu, Ala, Arg, Tyr, Tyr, Ala, Ser, Leu, Arg, Ala, Tyr, Ile, Asn, Leu, Ile, Thr, Arg, Gln, Arg, Tyr-NH2. In some embodiments, the PPF polypeptides further comprise an N-terminal cap. Examples of these PPF polypeptides include SEQ ID NOs: 437, 462, 469, 470 and 472 of US 2006/013547A1. For example sequence 438 of US2006/013547A1 is Pro Lys Pro Glu His Pro Gly Glu Asp Ala Ser Pro Glu Glu Leu Ala Arg Tyr Tyr Ala Ser Leu Arg Ala Tyr Ile Asn Leu Ile Thr Arg Gln Arg Tyr. In one embodiment, a hybrid of the present disclosure includes a one component the sequence 438 of US2006/013547A1, particularly in hybrids useful to treat obesity, reduce weight, reduce or redistribute fat, and reduce caloric intake. Such a hybrid can also contain an amylinomimetic component or a leptin component or both.

The PPF polypeptides and PPF chimeras, when used alone or as a second agent or as a component of a hybrid of the disclosure, find use in methods including, altering body composition of a subject, comprising administering to the subject the compound (PPF polypeptides or PPF chimera alone, as a second agent, or as a component of a hybrid of the invention) wherein the compound alters the fat to lean ratio, thereby altering body composition. The PPF polypeptide can comprise an amino acid sequence selected from the group consisting PYY-NPY chimera designated 5705 and of the following sequences from US2006/013547A1: SEQ ID NOs: 266, 267, 274, 282, 320, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466; 467, 468, 469, 470, 471, 472, 473, 474, 475, 476, 477, 478, 479 and 480. In one embodiment body fat is reduced and lean body mass is maintained or increased. In one embodiment the body fat and lean body mass are measured as percent body fat and percent lean body mass, respectively. In one further embodiment body weight is reduced. In another embodiment body weight is maintained or increased. The compounds can be administered peripherally. The PPF polypeptide or PPF chimera or PPF-containing hybrid can be used in a method that further comprises administering to the subject at least one other agent selected from the group consisting of an amylin, amylin agonist or amylin analog agonist, salmon calcitonin, a cholecystokinin (CCK) or CCK agonist, a leptin (OB protein) or leptin agonist, an exendin or exendin analog agonist, a GLP-1, GLP-1 agonist or GLP-1 analog agonist, a CCK or CCK agonist, calcitonin, a calcitonin agonist, a small molecule cannabinoid CB1 receptor antagonist, rimonabant, an 11 beta-hydroxysteroid dehydrogenase-1 inhibitor, sibutramine, and phentermine. In one embodiment the subject is overweight or obese. In yet another embodiment the PPF polypeptides and PPF chimeras, when used alone or as a second agent or as a component of a hybrid of the invention, find use in methods including a method for preferentially lowering plasma triglyceride levels in a subject, comprising administering to the subject an amount of the compound effective to lower plasma triglyceride levels, wherein cholesterol levels are lowered to a lesser extent. In a further embodiment triglyceride levels are lowered and cholesterol levels are not lowered. In a further embodiment triglyceride levels are lowered and LDL cholesterol levels are not lowered. In a further embodiment triglyceride levels are lowered and LDL cholesterol levels are lowered to a lesser extent. In yet a further embodiment amylase levels are also lowered. In yet another embodiment the PPF polypeptides and PPF chimeras, when used alone or as a second agent or as a component of a hybrid of the invention, find use in methods including a method for reducing body fat or body fat gain in a subject while maintaining or increasing lean body mass, comprising administering to the subject an amount of the compound effective to reduce body fat or body fat gain while maintaining or increasing lean body mass. In another embodiment the PPF polypeptides and PPF chimeras, when used alone or as a second agent or as a component of a hybrid of the disclosure, find use in methods including a method of reducing visceral body fat in a subject, comprising administering to the subject an amount of the compound effective to reduce visceral body fat and preserve or increase lean body mass. In another embodiment the PPF polypeptides and PPF chimeras, when used alone or as a second agent or as a component of a hybrid of the disclosure, find use in methods including a method to alter fat distribution in the subject. In one aspect, the alteration results from an increased metabolism of visceral or ectopic fat, or both in the subject. In another embodiment the PPF polypeptides and PPF chimeras, when used alone or as a second agent or as a component of a hybrid of the invention, find use in methods including a method of increasing fatty acid β-oxidation while preserving or increasing lean body mass in a subject comprising administering to the subject an amount of the compound effective to increase fatty acid β-oxidation while preserving or increasing lean body mass. In another embodiment the PPF polypeptides and PPF chimeras, when used alone or as a second agent or as a component of a hybrid of the disclosure, find use in methods including a method of treating nonalcoholic steatohepatitis or lipodystrophy in a subject comprising administering to the subject an amount of a compound effective to treat nonalcoholic steatohepatitis or lipodystrophy. Hybrids of particular interest in the above uses ca contain a PPF chimera as described herein in combination with a component from the leptin family or the amylin family, such as an amylin-sCT-amylin hybrid, or both. A PPF-chimera/leptin hybrid or a PPF-chimera/amylin-sCT-amylin hybrid will provide an effect superior to either compound alone. In yet further embodiments a PPF-chimera/leptin hybrid is administered with an amylinomimetic such as an amylin-sCT-amylin chimera or a PPF-chimera/amylin-sCT-amylin hybrid is administered with a leptin.

When not a component module of a hybrid, the PPF polypeptide chimeras mentioned herein can be administered alone or as a second agent, preferably in combination with a hybrid of the invention. They can be provided with or without a pharmaceutically acceptable carrier or excipient, in either single or multiple doses. These pharmaceutical compounds may be formulated with pharmaceutically acceptable carriers or diluents as well as any other known adjuvants and excipients in accordance with conventional techniques such as those disclosed in Remington's Pharmaceutical Sciences by E. W. Martin. See also Wang, Y. J. and Hanson, M. A. "Parenteral Formulations of Proteins and Peptides: Stability and Stabilizers," Journal of Parenteral Science and Technology, Technical Report No. 10, Supp. 42:2S (1988), incorporated by reference. The PPF polypeptides may be provided in dosage unit form. For example, therapeutically effective amounts of the PPF polypeptide for affecting body composition will vary with many factors including the age and weight of the patient, the patient's physical condition, their use in combination with other treatments, the ultimate goal that is to be achieved, such as overall weight loss and/or maintaining or increasing lean body mass, as well as other factors. However, typical doses (when not a component of a hybrid) may contain from a lower limit of about 0.05 µg, about 0.1 µg, about 1 µg, about 5 µg, about 10 µg, about 50 µg, about 75 µg or about 100 µg, to an upper limit of about 50 µg, about 100 µg, about 500 µg, about 1 mg, about 5 mg, about 10 mg, about 15 mg, about 50 mg, about 100 mg or about 150 mg of the pharmaceutical compound per day. Also contemplated are other dose ranges such as 0.1 µg to 1 mg of the compound per dose, or at about 0.001 µg/kg to about 500 µg/kg per dose. In some embodiments, the PPF polypeptide chimera is administered peripherally at a dose of about 0.5 µg to about 5 mg per day in single or divided doses or controlled continual release, or at about 0.01 µg/kg to about 500 µg/kg per dose, or at about 0.05 µg/kg to about 250 µg/kg. In some embodiments, the PPF polypeptide chimera is administered at a dose below about 50 µg/kg. Dosages in these ranges will vary with the potency of each analog or derivative, of course, and may be readily determined by one of skill in the art. The doses per day may be delivered in discrete unit doses, provided continuously in a 24 hour period or any portion of that the 24 hours. The number of doses per day may be from 1 to about 4 per day, although it could be more. Continuous delivery can be in the form of a continuous infusion. Other contemplated exemplary doses and infusion rates include from 0.005 nmol/kg to about 20 nmol/kg per discrete dose or from about 0.01/pmol/kg/min to about 10 pmol/kg/min in a continuous infusion. These doses and infusions can be delivered by any known conventional or future-developed peripheral method, e.g., intravenous (i.v.), intradermal, intramuscular, intramammary, intraperitoneal, intrathecal, retrobulbar, intrapulmonary (e.g., term release); subcutaneous administration (s.c.), by oral, sublingual, nasal, anal, vaginal, or transdermal delivery, or by surgical implantation at a particular site. Exemplary total dose/delivery of the pharmaceutical composition given *i.v.* may be about 1 µg to about 8 mg per day, whereas total dose/delivery of the pharmaceutical composition given s.c. may be about 6 µg to about 16 mg per day.

### Incretins and Incretin Mimetics

Component peptide hormones useful in the present invention also include GLP-1 peptide hormones. Native GLP-1 peptide hormones, including GLP-1(1-37) (SEQ ID NO: 59), GLP-1(7-37) (SEQ ID NO: 204), and GLP-1(7-36)amide (SEQ ID NO: 61), are known in art, as are functional peptide analogs and derivatives. As used herein, GLP-1 refers to all native forms of GLP-1 peptide hormones. Certain preferred native peptides, peptide analogs and derivatives are described herein, however it should be recognized that any known GLP-1 peptides that exhibit hormonal activity known in the art may be used in conjunction with the present disclosure.

Any GLP-1 peptide analog or derivative known in the art may be used in conjunction with the present disclosure. In one embodiment, the GLP-1 peptide analogs and derivatives have at least one hormonal activity of a native GLP-1 peptide. In certain embodiments, the GLP-1 peptide analogs are agonists of a receptor which a native GLP-1 peptide is capable of specifically binding. Preferred GLP-1 peptide analogs and derivatives include those described in, e.g., WO 91/11457.

GLP-1 analogs known in the art include:

| SEQ ID: | |
|---|---|
| 168 | ⁹Gln-GLP-1(7-37) |
| 169 | D-⁹Gln-GLP-1(7-37) |
| 170 | ¹⁶Thr-¹⁸Lys⁻GLP-1(7-37) |
| 171 | ¹⁸Lys-GLP-1(7-37) |
| 172 | ⁸Gly-GLP-1 (7-36) |
| 173 | ⁹Gln-GLP-1 (7-37) |
| 174 | D-⁹Gln-GLP-1 (7-37) |
| 175 | acetyl-⁹Lys-GLP-1(7-37) |
| 176 | ⁹Thr-GLP-1(7-37) |
| 177 | D-⁹Thr-GLP-1 (7-37) |
| 178 | ⁹Asn-GLP-1 (7-37) |
| 179 | D-⁹Asn-GLP-1 (7-37) |
| 180 | ²²Ser²³Arg²⁴Ar²⁶Gln-GLP-1(7-37) |
| 181 | ¹⁶Thr¹⁸Lys-GLP-1(7-37) |
| 182 | ¹⁸Lys-GLP-1(7-37) |
| 183 | ²³Arg-GLP-1(7-37) |
| 184 | ²⁴Arg-GLP-1(7-37) |

As known in the art, such GLP-1 analogs may preferably be amidated, but within the context of the present disclosure, may optionally be in the acid form unless otherwise specified.

Other GLP-1 analogs and derivatives are disclosed in U.S. Pat. No. 5,545,618. A preferred group of GLP-1 analogs and derivatives include those disclosed in U.S. Patent No. 6,747,006. The use in the present invention of a molecule described in U.S. Pat. No. 5, 188, 666, is also contemplated. Another group of molecules for use in the present disclosure includes components described in U.S. Pat. No. 5,512,549. Another preferred group of GLP-1 compounds for use in the present disclosure is disclosed in WO 91/11457.

Component peptide hormones useful in the present disclosure also include GLP-2 peptide hormones. Native GLP-2 peptide hormones, e.g., rat GLP-2 and its homologous including ox GLP-2, porcine GLP-2, degu GLP-2, bovine GLP-2, guinea pig GLP-2, hamster GLP-2, human GLP-2, rainbow trout GLP-2, and chicken GLP-2, are known in art, as are functional peptide analogs and derivatives. Certain preferred native peptides, peptide analogs and derivatives are described herein, however it should be recognized that any known GLP-2 peptides that exhibit hormonal activity known in the art may be used in conjunction with the present disclosure.

Any GLP-2 peptide analog or derivative known in the art maybe used in conjunction with the present disclosure. In one embodiment, the GLP-2 peptide analogs and derivatives have at least one hormonal activity of a native GLP-2 peptide. In certain embodiments, the GLP-2 peptide analogs are agonists of a receptor which a native GLP-2 peptide is capable of specifically binding. Preferred GLP-2 peptide analogs and derivatives include those described in, e.g., U.S. Ser. No. 08/669,791 and PCT Application PCT/CA97/00252,

Specific GLP-2 analogs known in the art include: rat or human GLP-2 altered at position 2 to confer DPP-IV resistance by substituting a Gly for an Ala.

Component peptide hormones useful in the present disclosure also include oxyntomodulin (OXM) peptide hormones. Native OXM peptide hormones are known in art, as are functional peptide analogs and derivatives. Certain preferred native peptides, peptide analogs and derivatives are described herein, however it should be recognized that any known OXM peptides that exhibit hormonal activity known in the art may be used in conjunction with the present disclosure.

Any OXM peptide analog or derivative known in the art may be used in conjunction with the present disclosure. In one embodiment, the OXM peptide analogs and derivatives have at least one hormonal activity of a native OXM peptide. In certain embodiments, the OXM peptide analogs are agonists of a receptor which a native OXM peptide is capable of specifically binding.

Component peptide hormones useful in the present invention as defined in the claims also include exendin peptide hormones. Native exendin peptide hormones are known in art, as are functional peptide analogs and derivatives. Certain preferred native peptides, peptide analogs and derivatives are described herein, however it should be recognized that any known exendin peptides that exhibit hormonal activity known in the art may be used in conjunction with the present invention.

Any exendin peptide analog or derivative known in the art may be used in conjunction with the present invention. The exendin peptide analogs and derivatives have at least one hormonal activity of a native exendin peptide.

The exendin peptide analogs are agonists of a receptor which a native exendin peptide'is capable of specifically binding.

Exemplary exendin analogs include:

| SEQ ID: | |
|---|---|
| 185 | ¹⁴Leu,²⁵Phe-exendin-4 |
| 186 | ⁵Ala,¹⁴Leu,²⁵Phe-exendin-4 |
| 187 | ¹⁴Leu,²²Ala,²⁵Phe-exendin-4 |

As known in the art, such exendin analogs are preferably amidated, but within the context of the present invention, may optionally be in the acid form unless otherwise specified.

Additional exemplary exendin analogs and derivatives are described in PCT Application Serial No. PCT/US98/16387 filed Aug. 6, 1998, entitled "Novel Exendin Agonist Compounds," which claims the benefit of U.S. patent application Ser. No. 60/055,404, filed Aug. 8, 1997 Other exendin analogs and derivatives are described in PCT Application Serial No. PCT/US98/24210, filed Nov. 13, 1998, entitled "Novel Exendin Agonist Compounds," which claims the benefit of U.S. Provisional Application No. 60/065,442 filed Nov. 14, 1997.

Still other exendin analogs and derivatives are described in PCT Application Serial No. PCT/US98/24273, filed Nov. 13, 1998, entitled "Novel Exendin Agonist Compounds," which claims the benefit of U.S. Provisional Application No. 60/066,029 filed Nov. 14, 1997.

Still other exendin analogs and derivatives are described in PCT Application Serial No. PCT/US97/14199, filed Aug. 8, 1997, entitled "Methods for Regulating Gastrointestinal Activity," which is a continuation-in-part of U.S. patent application Ser. No. 08/694,954 filed Aug. 8, 1996.

Still other exendin analogs and derivatives are described in PCT Application Serial No. PCT/US98/00449, filed Jan. 7, 1998, entitled "Use of Exendins and Agonists Thereof for the Reduction of Food Intake," which claims priority to U.S. Provisional Application No. 60/034,905 filed Jan. 7, 1997, both of which are hereby incorporated by reference. Yet other exendin analogs and derivatives are described in US 2004/0209803 A1, filed December 19, 2003, entitled "Compositions for the Treatment and Prevention of Neuropathy," which is hereby incorporated by reference.

### Natriuretic Peptides.

Natriuretic peptides are a family of hormones that consist of atrial natriuretic peptide (ANP), brain natriuretic peptide (BNP), and C-type natriuretic peptide (CNP). They are synthesized and stored as 3 distinct precursor prohormones, which are the 126 amino acid ANP, 108 amino acid BNP, and 104 amino acid CNP. They are each encoded by separate genes and have distinct sites of synthesis and mechanisms of regulation. Parental natriuretic peptide sequences include:

| | | |
|---|---|---|
| SEQ ID NO: 375 | 151 amino acid human ANP preprohormone | |
| SEQ ID NO: 376 | 134 amino acid human BNP preprohormone | |
| SEQ ID NO: 377 | 126 amino acid human CNP preproCNP | |

The main site of synthesis of the ANP prohormone is the atrial myocyte where it is synthesized as a 151-amino acid preprohormone. Removal of a 25-amino acid signal peptide from its N terminal end occurs in the endoplasmic reticulum, leaving a 126-amino acid ANP prohormone (ProANP), the main storage form of ANP within the heart. The prohormone consists of 4 biologically active peptide segments: amino acids 1-30 (ProANF 1-30, also known as long acting Na stimulator), 31-67 (ProANF 31-67, also known as vessel dilator), 79-98 (ProANF 79-98, also known as potassium excreter), and 99-126 (ANF, also known as atrial natriuretic factor).

BNP was originally isolated from porcine brain but in humans it is synthesized and secreted from the left ventricle. Sequence analysis reveals that preproBNP consists of 134 residues and is cleaved to a 108-amino acid ProBNP. Cleavage of a 32-amino acid sequence from the C-terminal end of ProBNP results in human BNP (77-108), which is the physiologically active form in plasma.

CNP is the third member of the natriuretic peptide system and is primarily found in human vascular endothelial cells, kidney, and porcine brain. High concentrations of CNP are also found in human hypothalamus and midbrain. In humans, preproCNP is a 126-amino acid precursor processed into proCNP by cleavage of 23 residues from its N-terminal end. This 23-amino acid sequence serves as a signal peptide. The terminal 22 (105-126) amino acids are cleaved from proCNP to yield a biologically active form of CNP.

Urodilatin is a kidney-derived member of the natriuretic peptide family and is formed from the same ANP prohormone and consists of amino acids 95-126. Except for the 4 amino acid N terminal extension, it is identical to ANF (99-126). Urodilatin appears to be an important regulator of sodium and water handling in the kidney, as well as a mediator of sodium excretion in patients with congestive heart failure (CHF).

Natriuretic peptides exert their biologic effects by binding to high-affinity receptors on the surface of target cells. Three subtypes of NPRs-NPR-A, NPR-B, and NPRC-have been isolated. Consequently, in one embodiment is provided a method to screen hybrids for natriuretic receptor binding and/or activation. Natriuretic peptides including prohormone variants can impart numerous natriuretic peptide hormone activities to hybrids of the invention. In other embodiments of interest are natriuretic antagonist hybrids. Natriuresis is the excretion of an excessively large amount of sodium into the urine. Natriuresis is similar to diuresis (the excretion of an unusually large quantity of urine), except that in natriuresis the urine is exceptionally salty. Natriuresis occurs with some diuretics and diseases (as of the adrenal) and can lead to the salt-losing syndrome characterized by dehydration, vomiting, low blood pressure, and the risk of sudden death. Exogenous administration of the 4 independent circulating peptides of the ANP prohormone (1-30, 31-67, 79-98, and 99-126) produce in vivo vasodilation, diuresis, suppression of the renin-angiotensin-aldosterone system and enhanced natriuresis and/or kaliuresis. ProANF 1-30, ProANF 31-67 and ANF 99-126 each have natriuretic, blood pressure lowering and diuretic properties with ProANF 31-67 and ANF 99-126 having the greatest impact on blood pressure. There are varying effects of the ANP peptides on potassium homeostasis: ProANF 79-98 stimulates potassium excretion, whereas ProANF 31-67 spares potassium loss by inhibiting Na/K ATPase in the medullary collecting duct cells. Specific to ANF 99-126 is a dose-dependent inhibition of angiotensin II-mediated aldosterone secretion, whereas proANF 31-67 has the property of inducing natriuresis through generation of prostaglandin.

BNP produces similar biologic effects as ANF in normal humans. Infusions of BNP in normal men produced a 2-fold increase in sodium excretion, 50% reduction in plasma renin, angiotensin II and aldosterone secretion as well as a reduction in plasma volume.

CNP induces cardiovascular effects similar to the other natriuretic peptides but does not appear to mediate any renal effects. When CNP is infused in anesthetized dogs at equivalent doses of ANF, plasma cGMP rose with a concomitant reduction in mean arterial pressure, right atrial pressure and cardiac output, but glomerular filtration rate, renal blood flow and sodium excretion decreased.

Natriuretic peptides can provide therapeutic benefit in heart failure. Congestive heart failure (CHF) is associated with increases in vasopressin, endothelin, and with activation of the renin-angiotensin-aldosterone system, and sympathetic nervous systems, mediating vasoconstriction, sodium and water retention, and negative vascular and cardiac remodeling. These effects occur despite the elevated levels of the natriuretic peptides in patients with heart failure. In one embodiment of the invention are hybrids that provide increased or therapeutic serum levels of natriuretic peptide activity for treatment or prevention of cardiac related diseases and conditions, including CHF. Although ANF infusion in normal individuals can result in a sustained increase in sodium excretion and urine flow rates, in the heart failure patient a marked beneficial reduction in renal response can be obtained. BNP infusion markedly increases sodium excretion in patients with heart failure and exerts significant beneficial hemodynamic effects. As compared with ANP, the diuretic and natriuretic effects of BNP are significantly greater. BNP is cleared more slowly than ANP and exerts other effects including suppressing aldosterone secretion and increasing serum levels of ANP. BNP peptides can also provide a beneficial decrease in pulmonary capillary wedge pressure, systemic vascular resistance, right atrial pressure and systolic blood pressure, with an increase in cardiac index in patients hospitalized for symptomatic CHF. In patients with decompensated heart failure, natriuretic peptide hybrids can provide a beneficial decrease in pulmonary capillary wedge pressure and an improved dyspnea score. (Dyspnea is an unpleasant sensation of difficulty in breathing, typically associated with early stages of cardiac heart failure.) The hybrids containing one, two or three natriuretic hormone functions provide methods of administration of pharmaceutically active compositions that are useful for both the prophylactic and therapeutic treatment of CHF patients, preferably CHF patients that are decompensated, patients with chronic CHF, and patients with hypertension. The natriuretic portion(s) of a hybrid is sufficient to provide a therapeutically effective amount of a natriurertic peptide to such patient when administered in a therapeutically effective dose over a therapeutically effective period.

As discussed herein any of the family of therapeutically effective natriuretic peptides or their analogs can be used. Useful natriuretic peptides include, for example, atrial natriuretic peptide (ANP), brain natriuretic peptide (BNP or B-type natriuretic peptide) and C-type natriuretic peptide (CNP). Sequences of useful forms of natriuretic peptides are disclosed in U.S. Patent Publication 20010027181, which is incorporated herein by reference. Examples of ANPs include human ANP (Kangawa et al., BBRC 118:131 (1984)) or that from various species, including pig and rat ANP (Kangawa et al., BBRC 121:585 (1984)). Such ANPs comprise 28 amino acids. Such ANPs may be administered as a peptide having a ring structure of ANP (formation of a disulfide bond based on Cys), and a C- terminal portion succeeding the ring structure. An example of such a peptide is a peptide having amino acid residues at the 7-position to the 28-position of ANP is provided in U.S. Patent Application Publication No. 20010027181. Another example is frog ANP. Specific examples of BNPs that can be used in the methods of the invention include human BNP (hBNP). Human BNP comprises 32 amino acids and involves the formation of a disulfide bond (Sudoh et al., BBRC 159:1420 (1989)) and U.S. Pat. Nos. 5,114,923, 5,674,710, 5,674,710, and 5,948,761, each of which is incorporated by reference. Various BNP's of origin other than human, including as pig BNP and rat BNP, are also known, and can be used. A further example is chicken BNP. Examples of CNPs that can be used in the methods of the invention include pig CNP. Pig CNP comprises 22 amino acids and involves the formation of a disulfide bond, like the above- described ANP and BNP (Sudoh et al., BBRC 168:863 (1990)) (human and rat have the same amino acid sequence), chicken CNP (Arimura et al., BBRC 174:142 (1991)). Frog CNP (Yoshihara et al., BBRC 173:591 (1990) can also be used. As discussed herein, one skilled in the art can apply modifications, such as a deletion, substitution, addition or insertion, and/or chemical modification to amino acid residues in the amino acid sequence of a known natriuretic peptide as desired, by known methods. The resulting compound is a compound which has the activity of acting on a receptor of the starting ANP, BNP or CNP. Analogs having this activity, therefore, are included in the hybrids for use in accordance with the methods of the present disclosure.

In another embodiment, the hybrids containing one or more natriuretic functions can be used in treating hypertension. In one embodiment a natriuretic hybrid will have no deleterious effect on heart rate and is not associated with arrhythmias. In one embodiment the hybrid will comprise at least one, two or three natriuretic peptide functions, for example, both ANP and BNP activity. One or more natriuretic hormone functions can be combined with any other hormone function or peptidic enhancer, as described herein. In another embodiment the natriuretic portion(s) is a more stable analog having an extended in vivo half-life when compared with that of a native natriuretic peptide. Analogs that prevent undesirable cleavage by endogenous enzymes such as NEP are also envisioned. The natriuretic containing hybrids are also further directed to hypertension reduction, diuresis inducement, natriuresis inducement, vascular conduct dilatation or relaxation, natriuretic peptide receptors (such as NPR-A) binding, renin secretion suppression from the kidney, aldostrerone secretion suppression from the adrenal gland, treatment of cardiovascular diseases and disorders, reducing, stopping or reversing cardiac remodeling in congestive heart failure, treatment of renal diseases and disorders; treatment or prevention of ischemic stroke, and treatment of asthma. Hybrids can be administered to patients that would benefit from inducing natriuresis, diuresis and vasodilatation. Hybrids can be administered alone or in combination with one or more of the following types of compounds: ACE inhibitors, beta-blockers, diuretics, spironolactone, digoxin, anticoagulation and antiplatelet agents, and angiotensin receptor blockers. Additional diseases or conditions include renal disorders and diseases, asthma, hypertension and pulmonary hypertension. Hybrids are also useful to treat inflammatory-related diseases, erectile dysfunction and hypercholesterolemia.

### Bio-Active Peptide Hormone Modules

As discussed herein the hybrid polypeptides of the present invention as defined in the claims comprise at least two bio-active peptide hormone modules covalently linked together. The bio-active peptide hormone modules may be: (a) native component peptide hormones, (b) analogs or derivatives of native component peptide hormones that retain hormonal activity, (c) fragments of native component peptide hormones that retain hormonal activity, (d) fragments of analogs or derivatives of native component peptide hormones that retain hormonal activity, (e) structural motifs of native component peptide hormones that impart a desired chemical stability, conformational stability, metabolic stability, bioavailability, organ/tissue targeting, receptor interaction, protease inhibition, plasma protein binding, and/or other pharmacokinetic characteristic to the hybrid polypeptide; or (f) structural motifs of analogs or derivatives of native component peptide hormones that impart a desired chemical stability, conformational stability, metabolic stability, bioavailability, organ/tissue targeting, receptor interaction, protease inhibition, plasma protein binding, and/or other pharmacokinetic characteristic to the hybrid polypeptide. The structural motifs of (e) and (f) will collectively be referred to herein as "peptidic enhancers".

Preferred bio-active peptide hormone modules include native peptide hormones selected from: amylin, ADM, CT, CGRP, intermedin, CCK(1-33), CCK-8, leptin, PYY(1-36) (SEQ ID NO: 57), PYY(3-36) (SEQ ID NO: 58), GLP-1(1-37) (SEQ ID NO: 59), GLP-1(7-37) (SEQ ID NO: 204), GLP-1(7-36) (SEQ ID NO: 61), GLP-2, OXM, exendin-3, exendin-4, natriuretic peptide hormones, urocortin family peptides, e.g., Ucn-2 and Ucn-3, neuromedin family peptides, e.g. neuromedin U25 or splice variants, and ANP, BNP, CNP or urodilatin.

Other preferred bio-active peptide hormone modules include analogs and derivatives of a component peptide hormone selected from: amylin, ADM, CT, CGRP, intermedin, CCK, leptin, PYY(1-36) (SEQ ID NO: 57), PYY(3-36) (SEQ ID NO: 58), GLP-1(1-37) (SEQ ID NO: 59), GLP-1(7-37) (SEQ ID NO: 204), GLP-1(7-36) (SEQ ID NO: 61), GLP-2, OXM, exendin-3, and exendin-4, natriuretic peptide hormones, urocortin family peptides, e.g., Ucn-2 and Ucn-3, neuromedin family peptides, e.g. neuromedin U25 or splice variants, and ANP, BNP, CNP or urodilatin, wherein the analog or derivative exhibits at least one hormonal activity of the component peptide hormone. The analog may comprise one or more insertions, deletions, or substitutions of the amino acid sequence of the component peptide hormone, and the derivative may comprise one or more chemical modifications of an amino acid residue of an analog or component peptide hormone, as described more fully herein and known in the art.

More specifically, analogs and derivatives may be selected from any described above and/or known in the art. Particularly preferred analogs and derivatives that exhibit at least one hormonal activity useful as bio-active peptide hormone modules of the disclosure include the following:

| | |
|---|---|
| Amylin: | ²Ala-h-amylin (SEQ ID NO: 79), ^{2,7}Ala-h-amylin (SEQ ID NO: 80), ²⁸Pro-h-amylin (SEQ ID NO: 189), ^{25,28}Pro-h-amylin (SEQ ID NO: 83), ^{25,28,29}Pro-h-amylin (SEQ ID NO: 67), ²⁵Pro,²⁶Val,^{28,29}Pro-h-amylin (SEQ ID NO: 69), ¹⁸Arg,^{25,28}Pro-h-amylin (SEQ ID NO: 70), ¹⁸Arg,^{25,28,29}Pro-h-amylin (SEQ ID NO: 72), ²⁵Pro,²⁶Val,^{28,29}Pro-h-amylin (SEQ ID NO: 75), ¹⁸Arg,²³Leu,^{25,28,29}Pro-h-amylin (SEQ ID NO: 89), ¹⁸Arg²³Leu,^{25,28}Pro-h-amylin (SEQ ID NO: 90), and 2,7-Cyclo-[²Asp,⁷Lys]-h-amylin (SEQ ID NO: 76) |
| CT: | ¹⁴Glu-sCT (SEQ ID NO: 106), ¹⁸Arg-sCT (SEQ ID NO: 107), ^{11,18}Arg-sCT (SEQ ID NO: 108), ¹⁴Glu,¹⁸Arg-sCT (SEQ ID NO: 109), ¹⁴Glu,^{11,18}Arg-sCT (SEQ ID NO: 110) |
| CGRP: | ^{3s}D-Ser-CGRP (SEQ ID NO: 111), ³⁶D-Thr-CGRP (SEQ ID NO: 112), ³⁶D-Asp-CGRP (SEQ ID NO: 113), ³⁶D-Asn-CGRP (SEQ ID NO: 114), ³⁶Ser-CGRP (SEQ ID NO: 115), ³⁶Hse-CGRP (SEQ ID NO: 116), ³⁶Asp-CGRP (SEQ ID NO: 117), ³⁶Thr-CGRP (SEQ ID NO: 118), ³⁶Asn-CGRP (SEQ ID NO: 119) |
| AFP-6: | |
| CCK: | DY(OSO₃H)MGWMDF (SEQ ID NO: 141), DYMGWMDF (SEQ ID NO: 142), MGWMDF (SEQ ID NO: 143), GWMDF (SEQ ID NO: 144), WMDF (SEQ ID NO: 145), KDY(OSO₃MMGWMDF (SEQ ID NO: 146), KDYMGWMDF (SEQ ID NO: 147), KMGWMDF (SEQ ID NO: 148), KGWMDF (SEQ ID NO: 149), KWMDF (SEQ ID NO: 150) |
| Leptin: | ⁴³Asp-leptin (SEQ ID NO: 151), ⁴³Gln-leptin (SEQ ID NO: 152), ⁴⁸Ala-leptin (SEQ ID NO: 153), ⁴⁹Glu-leptin (SEQ ID NO: 154), ⁴⁹Des-AA-leptin (SEQ ID NO: 155), ⁷⁵Ala-leptin (SEQ ID NO: 156), ⁸⁹Leu-leptin (SEQ ID NO: 157), ⁹³Asp-leptin (SEQ ID NO: 158), ⁹³Glu-leptin (SEQ ID NO: 159), ⁹⁸Ala-leptin (SEQ ID NO: 160), ¹³⁹Leu-leptin (SEQ ID NO: 162), |
| PYY: | ³Leu-PYY (SEQ ID NO: 192), ³Val-PYY (SEQ ID NO: 193), ⁴Arg-PYY (SEQ ID NO: 194), ⁴Gln-PYY (SEQ ID NO: 195), ⁴Asn-PYY (SEQ ID NO: 196), ²⁵Lys-PYY (SEQ ID NO: 197), ³⁴Pro-PYY (SEQ ID NO: 198), ³⁴His-PYY (SEQ ID NO: 199), ^{1,36}Tyr-PYY (SEQ ID NO: 57), ¹³Pro¹⁴Ala-PYY (SEQ ID NO: 200), ³¹Leu³⁴Pro-PYY (SEQ ID NO: 201), des-AA-4-PYY (SEQ ID NO: 202) |
| GLP-1 | ⁹Gln-GLP-¹⁸(7-37) (SEQ ID NO: 168), D-⁹Gln -GLP-1(7-37) (SEQ ID NO: 169), ¹⁶Thr-¹⁸ Lys ⁻GLP-1(7-37) (SEQ ID NO: 170), ¹⁸Lys-GLP-1(7-37) (SEQ ID NO: 171), ⁸Gly-GLP-1 (7-36) (SEQ ID NO: 172), ⁹Gln-GLP-1 (7-37) (SEQ ID NO: 173), D-⁹Gln-GLP-1 (7-37) (SEQ ID NO: 174), acetyl-⁹Lys-GLP-1(7-37) (SEQ ID NO: 175), ⁹Thr-GLP-1(7-37) (SEQ ID NO: 176), D-⁹Thr-GLP-1 (7-37) (SEQ ID NO: 177), ⁹Asn-GLP-1 (7-37) (SEQ ID NO: 178), D-⁹Asn-GLP-1 (7-37) (SEQ ID NO: 179), ²²Ser²³Arg²⁴Arg²⁶Gln-GLP-1(7-37) (SEQ ID NO: 180), ¹⁶Thr¹⁸Lys-GLP-1(7-37) (SEQ ID NO: 181), ¹⁸Lys-GLP-1(7-37) (SEQ ID NO: 182), ²³Arg-GLP-1(7-37) (SEQ ID NO: 183), ²⁴Arg-GLP-1(7-37) (SEQ ID NO: 184) |
| Exendin | ¹⁴Leu,²⁵Phe-exendin-4 (SEQ ID NO: 185), ¹⁴Leu,²⁵Phe-exendin-4 (SEQ ID NO: 185), ⁵Ala,¹⁴Leu,²⁵Phe-exendin-4 (SEQ ID NO: 186), and ¹⁴Leu,²²Ala,²⁵phe-exendin-4 (SEQ ID NO: 187). |

As known in the art, such peptide compounds may preferably be amidated, but within the context of the present invention, may optionally be in the acid form unless otherwise specified.

Still other preferred bioactive peptide hormone modules include fragments of a component peptide hormone selected from: amylin, ADM, CT, CGRP, intermedin, CCK, leptin, PYY(1-36) (SEQ ID NO: 57), PYY(3-36) (SEQ ID NO: 58), GLP-1(1-37) (SEQ ID NO: 59), GLP-1(7-37) (SEQ ID NO: 204), GLP-1(7-36) (SEQ ID NO: 61), GLP-2, OXM, a natriuretic peptide, exendin-3, and exendin-4, urocortin family peptides, e.g., Ucn-2 and Ucn-3, neuromedin family peptides, e.g. neuromedin U25 or splice variants, and ANP, BNP, CNP or urodilatin, wherein the fragment exhibits at least one hormonal activity of the component peptide hormone.

Yet other preferred bioactive peptide hormone modules include fragments of analogs or derivatives of a component peptide hormone selected from: amylin, ADM, CT, CGRP, intermedin, CCK, leptin, PYY(1-36) (SEQ ID NO: 57), PYY(3-36) (SEQ ID NO: 58),GLP-1(1-37) (SEQ ID NO: 59), GLP-1(7-37) (SEQ ID NO: 204), GLP-1(7-36) (SEQ ID NO: 61), GLP-2, OXM, ANP, BNP, CNP, urodilatin, exendin-3, exendin-4, a natriuretic peptide hormones, urocortin family peptides, e.g., Ucn-2 and Ucn-3, neuromedin family peptides, e.g. neuromedin U25 or splice variants, and ANP, BNP, CNP or urodilatin, wherein the fragment exhibits at least one hormonal activity of the component peptide hormone. Again, the analog may comprise one or more insertions, deletions, or substitutions of the amino acid sequence of the component peptide hormone, and the derivative may comprise one or more chemical modifications of an amino acid residue of an analog or component peptide hormone, as described more fully herein and known in the art.

Certain preferred fragments that exhibit at least one hormonal activity include the following. However, it should be understood that combinations of the above-described analogs and derivatives taken with fragments known in the art, including the preferred fragments described below, are contemplated.

| | |
|---|---|
| Amylin: | amylin(1-36) (SEQ ID NO: 210), amylin(1-35) (SEQ ID NO: 211), amylin(1-20) (SEQ ID NO: 212), amylin(1-18) (SEQ ID NO: 213), amylin(1-17) (SEQ ID NO: 214), amylin (1-16) (SEQ ID NO: 215), amylin(1-15) (SEQ ID NO: 216), amylin(1-7) (SEQ ID NO: 217) |
| CT: | CT(8-32) (SEQ ID NO: 218), CT(8-27) (SEQ ID NO: 219), CT(8-26) (SEQ ID NO: 220), CT(8-10) (SEQ ID NO: 221), CT(18-26) (SEQ ID NO: 222), CT(18-27) (SEQ ID NO: 223) |
| AFP-6: | AFP-6(18-27) (SEQ ID NO: 224) |
| CCK: | CCK-8, CCK-5, CCK-4 |
| Leptin: | leptin (22-167) (SEQ ID NO: 225), leptin(56-73) (SEQ ID NO: 226) |
| PYY: | PYY(1-35) (SEQ ID NO: 227), PYY(1-30) (SEQ ID NO: 228), PYY(1-25) (SEQ ID NO: 229), PYY(1-15) (SEQ ID NO: 230), PYY(1-10) (SEQ ID NO: 231), PYY(2-36) (SEQ ID NO: 232), PYY(3-36) (SEQ ID NO: 58), PYY(4-36) (SEQ ID NO: 233), PYY(5-36) (SEQ ID NO: 234) |
| GLP-1 | GLP-1(7-37) (SEQ ID NO: 204), GLP-1(7-36) (SEQ ID NO: 61), GLP-1(7-35) (SEQ ID NO: 235) |
| Exendin | exendin-4(1-27) (SEQ ID NO: 236), exendin-4(1-28) (SEQ ID NO: 237), exendin-4(1-29) (SEQ ID NO: 238), exendin-4(1-30) (SEQ ID NO: 239) or longer |

Again, as known in the art, such peptide compounds may preferably be amidated, but within the context of the present invention, may optionally be in the acid form unless otherwise specified. Further, the above preferred fragments may be combined with any of the analogs or derivatives discussed herein or known in the art. For example, preferred analog fragments may include ⁵Ala,¹⁴Leu,²⁵Phe-exendin-4(1-28) (SEQ ID NO: 240), ¹⁴Leu,²⁵Phe-exendin-4(1-27) (SEQ ID NO: 241), ⁵Ala,¹⁴Leu,²⁵Phe-exendin-4(1-28) (SEQ ID NO: 240), ¹⁴Leu,²⁵Phe-exendin-4(1-27) (SEQ ID NO: 241), or any other combinations of the disclosed fragments, analogs, and derivatives.

Yet other preferred bio-active peptide modules include "peptidic enhancer", *i.e.*, structural motifs of component peptide hormones (including analogs and derivatives thereof) that impart a desired chemical stability, conformational stability, metabolic stability bioavailability, organ/tissue targeting, receptor interaction, protease inhibition, plasma protein binding, and/or other pharmacokinetic characteristic to the hybrid polypeptide. Exemplary peptidic enhancers include the following. Again, it should be understood that combinations of the above-described analogs and derivatives taken together with the following bio-active peptide modules are contemplated. For example, the last six amino acid residues of amylin family peptide hormone analogs and derivatives known in the art and/or described above are also contemplated as preferred bio-active peptide modules.

| | |
|---|---|
| Amylin Family | amylin(32-37) (SEQ ID NO: 242), amylin(33-37) (SEQ ID NO: 243), amylin(34-37) (SEQ ID NO: 244), amylin(35-37), amylin(36-37), amylin(37), ADM(47-52) (SEQ ID NO: 245), ADM(48-52) (SEQ ID NO: 246), ADM(49-52) (SEQ ID NO: 247), ADM(50-52), ADM(51-52), ADM(52), CT(27-32) (SEQ ID NO: 248), CT(27-32) (SEQ ID NO: 249), CT(28-32) (SEQ ID NO: 250), CT(29-32), CT(30-32), CT(31-32), CT(32), CGRP(32-37) (SEQ ID NO: 251), CGRP(33-37) (SEQ ID NO: 252), CGRP(34-37) (SEQ ID NO: 253), CGRP(35-37), CGRP(36-37), CGRP(37), intermedin (42-47) (SEQ ID NO: 254), intermedin (43-47) (SEQ ID NO: 255), intermedin (44-47) (SEQ ID NO: 256), intermedin (45-47), intermedin (46-47), intermedin (47) |
| PYY | PYY(25-36) (SEQ ID NO: 257), PYY(26-36) (SEQ ID NO: 258), PYY(27-36) (SEQ ID NO: 259), PYY(28-36) (SEQ ID NO: 260), PYY(29-36) (SEQ ID NO: 261), PYY(30-36) (SEQ ID NO: 262), PYY(31-36) (SEQ ID NO: 263), PYY(32-36) (SEQ ID NO: 264), PYY(25-35) (SEQ ID NO: 265), PYY(26-35) (SEQ ID NO: 266), PYY(27-35) (SEQ ID NO: 267), PYY(28-35) (SEQ ID NO: 268), PYY(29-35) (SEQ ID NO: 269), PYY(30-35) (SEQ ID NO: 270), PYY(31-35) (SEQ ID NO: 271), PYY(32-35) (SEQ ID NO: 272) |
| GLP-1 and 2 | frog GLP-1(29-37) (SEQ ID NO: 273); frog GLP-1(30-37) (SEQ ID NO: 274); frog GLP-2(24-31) (SEQ ID NO: 275), frog GLP-2(25-31) (SEQ ID NO: 276) |
| Exendin-4 | exendin-4(31-39) (SEQ ID NO: 277), exendin-4(32-39) (SEQ ID NO: 278), exendin-4(33-39) (SEQ ID NO: 279), exendin-4(34-39) (SEQ ID NO: 280), exendin-4(35-39) (SEQ ID NO: 281), exendin-4(36-39) (SEQ ID NO: 282), exendin-4(37-39), exendin-4(38-39), exendin-4(39) |

### Peptide Module Selection Considerations, Spacers, and Linking Groups

The hybrid polypeptides of the present invention as defined in the claims comprise at least two bio-active peptide hormone modules of the invention, wherein at least one of the bio-active peptide hormone modules exhibits at least one hormonal activity. The bio-active peptide hormone module that exhibits the at least one hormonal activity may be located at the N-terminal end of the hybrid polypeptide, the C-terminal end of the hybrid polypeptide, or in the event that the hybrid polypeptide comprises more than two bio-active peptide hormone modules, may be located in the internal portion of the hybrid polypeptide.

In certain embodiments, it may be preferable to locate the bio-active peptide hormone module exhibiting the at least one hormonal activity such that the C-terminal end of the bio-active peptide hormone module is amidated. Amidation of the C-terminal end of the bio-active peptide hormone module may be accomplished by locating the module at the C-terminal end of the hybrid peptide, or by configuring the module in the C-terminal-to-N-terminal direction at the N-terminal end of the hybrid polypeptide. In both configurations, the C-terminal end of the bio-active peptide hormone module is available for amidation. Specific component peptide hormones where C-terminal amidation may be preferable include amylin family peptide hormones, CCK, PYY, hGLP-1(7-36) (SEQ ID NO: 61), and hGLP-2. Specific component peptide hormones where C-terminal amidation is not necessarily preferred (stated otherwise, where elongation at the C-terminal end of the module is easily tolerated) include exendin-4, exendin-4(1-28) (SEQ ID NO: 237), GLP-1(7-37) (SEQ ID NO: 204), frog GLP-1(7-36) (SEQ ID NO: 283), and frog GLP-2. However, if these component peptide hormones are located at the C-terminal end of the hybrid polypeptide, they may still be optionally amidated, and in fact may preferably be optionally amidated.

The bio-active peptide hormone modules may be covalently linked in any manner known in the art. Stable linkages may be used, or cleavable linkage may be used. In one embodiment, the carboxy of a first module may be directly linked to the amino of a second module. In another embodiment, linking groups may be used to attached modules. Further, if desired, spacers or turn inducers known in the art may be employed to stabilize the linkage. By way of example, where amidation of the C-terminal end of the N-terminally located bio-active peptide hormone module is not desired, the module may be attached to a second module directly, or using any appropriate linking group known in the art, such as, an alkyl; PEG; amino acid, e.g., Lys, Glu, β-Ala; polyaminoacids, e.g., poly-his, poly-arg, poly-lys, poly-ala, Gly-Lys-Arg (GKR) *etc.;* bifunctional linker (see, e.g., Pierce catalog, Rockford, Il); aminocaproyl ("Aca"), β-alanyl, 8-amino-3,6-dioxaoctanoyl, or other cleavable and non-cleavable linker known in the art. Specifically described herein, as if each were explicitly drawn, are embodiments of specific hybrids in which the linker in each exemplified linker-containing hybrid is replaced by a Gly linker, particularly embodiments where the Gly linker is Gly-Gly-Gly. As an example, for exemplified species ²⁹5 Apa-Exendin(1-28)-¹des-LyS-hAmylin(I-7)-^{11,18}Arg-sCt(8-27)-hAmylin(33-37) (SEQ ID NO: 32) (see tables herein) its Gly linker species analog is also specifically intended and disclosed. This species is ²⁹GlyGlyGly-Exendin(1-28)-¹des-Lys-hAmylin(1-7)-^{11,18}Arg-sCt(8-27)-hAmylin(33-37) (SEQ ID NO: 313), where the three glycines are located after the exendin(1-28) sequence. In one embodiment a linker or spacer is 1 to 30 residues long, in another embodiment 2 to 30 residues, and in yet another 3-30 residues long, and any integer length from 2 to 30 inclusive; each integer unit is contemplated, e.g. 2, 3, 4, 5, 6, 7, etc. In one embodiment a Gly linker is used, and in a particular embodiment a three residue linker Gly-Gly-Gly.

Where amidation of the C-terminal end of N-terminally located bio-active peptide hormone module is desired, the module may again be attached to a second module using any appropriate linking group known in the art. More specifically, in the event that a bio-active peptide hormone module exhibiting at least one hormonal activity has been configured in the C-terminal-to-N-terminal orientation, resulting in an amino to amino linkage, preferred linking groups include dicarboxylic acids, alkyls, PEGs, and amino acids such as Lys, Cys, and Glu.

As mentioned above, the hybrid polypeptides may also preferably include spacer to further stablize the linkage of the bio-active peptide hormone modules. Any spacer or turn inducer known in the art may be used. By way of example, referred β-turn mimetics include mimic A and mimic B illustrated below, also Ala-Aib and Ala-Pro dipeptides. Their IUPAC names are Mimic A: N-(3S,6S,9S)-2-oxo-3-amino-1-azabicyclo[4.3.0]-nonane-9-carboxylic acid. Mimic B: *N*-(3S,6S,9R)-2-oxo-3-amino-7-thia-1-azabicyclo[4.3.0]-nonane-9-carboxylic acid.

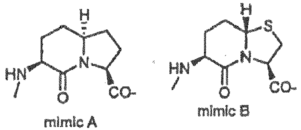

### Further Exemplary Combinations and Specific Embodiments

Exemplary combinations of bio-active peptide hormone modules to form the hybrid polypeptides of the disclosure include combinations of two or more bio-active peptide hormone modules selected from: native peptide hormones, analogs and derivatives of peptide hormones that exhibit at least one hormonal activity, fragments of native peptide hormones that exhibit at least one hormonal activity, fragments of analogs and derivatives of peptides hormones that exhibit at least one hormonal activity, and peptidic enhancers, with the proviso that at least one module exhibit at least one hormonal activity.

The hybrid polypeptides of the invention will include at least two bio-active peptide hormone modules, wherein each module is comprised from component peptide hormones. In the context of the present disclosure, the component peptide hormones of the hybrid polypeptide may be the same or different, with the proviso that at least two of the component peptide hormones are different. In a preferred disclosure, at least two of the component peptide hormones are from different peptide hormone families, *e.g*., the amylin family, CCK, the leptin family, PPF, the proglucagon family, the natriuretic peptide family, urocortin family peptides, e.g., Ucn-2 and Ucn-3, neuromedin family peptides, e.g. neuromedin U25 or splice variants, and ANP, BNP, CNP or urodilatin and the GLP-1 and exendin family.

In certain embodiments, the hybrid polypeptides of the disclosure may comprise two or more modules that exhibit at least one hormonal activity. For instance, the hybrid polypeptide may comprise a fragment of a first peptide hormone or analog that exhibits at least one hormonal activity covalently, linked to a fragment of at least one additional peptide hormone analog. The additional fragment(s) may optionally exhibit at least one hormonal activity. The first peptide hormone may be the same or different from the additional peptide hormone(s), with the proviso that at least one of the additional peptide hormones are different from the first peptide hormone, and the first hormonal activity may be the same or different from the optional additional hormonal activity.

In other embodiments, the hybrid polypeptides of the disclosure may comprise one or more more modules that exhibit at least one hormonal activity in combination with one or more peptidic enhancer modules. For instance, a fragment of a first peptide hormone that exhibits a at least one hormonal activity may be covalently linked to a peptidic enhancer, or a fragment of a first peptide hormone that exhibits at least one hormonal activity may be covalently linked to a second peptide hormone that exhibits at least one hormonal activity, which is in turn linked to a peptidic enhancer. Alternatively, a peptidic enhancer may be located between two peptide hormone modules as a stabilizing spacer. Again, the first peptide hormone may be the same or different from the second peptide hormone, and the first hormonal activity may be the same or different from the second hormonal activity.

The hybrid polypeptides of the invention may comprise two, three, four, or more bio-active peptide hormone modules. Exemplary combinations of the disclosure include a module with a hormonal activity in combination with one, two, or three peptidic enhancers; two modules with a hormonal activity in combination with one or two peptidic enhancers; three modules with a hormonal activity in combination with one peptidic enhancer, *etc.*

The component peptide hormones are preferably selected from amylin, adrenomedullin, calcitonin, calcitonin gene related peptide, intermedin, cholecystokinin, leptin peptide YY, glucagon-like peptide-1, glucagon-like peptide 2, oxyntomodulin, ANP, BNP, CNP, urodilatin, natriuretic peptide hormones, urocortin family peptides, e.g., Ucn-2 and Ucn-3, neuromedin family peptides, e.g. neuromedin U25 or splice variants, and ANP, BNP, CNP or urodilatin or exendin-4.

More particularly, preferred module combinations include those involving combinations of exendin, amylin (and/or sCT), BNP, and PYY as the component peptide hormones. Particular combinations include exendin-4/PYY and PYY/exendin-4 combinations, with and without spacers or linking groups. Other combinations include exendin/amylin and amylin/exendin combinations, with and without spacers or linking groups. Yet other combinations include amylin/PYY and PYY/amylin combinations, with and without spacers or linking groups.

In one aspect, preferred module combinations include those involving a first module comprising exendin-4, a fragment of exendin-4 that exhibits at least one hormonal activity, an exendin-4 analog or derivative that exhibits at least one hormonal activity, or a fragment of an exendin-4 analog that exhibits at least one hormonal activity in combination with at least one additional bio-active peptide hormone module. In one embodiment, the first module is linked to one, two, or three additional bio-active peptide hormone modules.

In preferred embodiments, a first module comprising an exendin-4 peptide is linked to a second bio-active peptide hormone module comprising an amylin (and/or sCT) peptide that exhibits at least one hormonal activity. In another embodiment, the second module is further linked to a third bio-active peptide hormone module comprising a calcitonin peptide that exhibits at least one hormonal activity. In yet another embodiment, the third module may be further linked to a fourth bio-active peptide hormone module comprising a peptidic enhancer selected from amylin peptides. In one embodiment, the first module may be located at the C-terminal end of the hybrid polypeptide. Alternatively, the first module may be located at the N-terminal end of the hybrid polypeptide. In certain embodiments, spacers or linkers such as βAla may be inserted if desired to link the modules.

Preferred exendin-4 peptides include: exendin-4, exendin-4(1-27) (SEQ ID NO: 236), exendin-4(1-28) (SEQ ID NO: 237), ¹⁴Leu,²⁵Phe-exendin-4(1-28) (SEQ ID NO: 284), and ⁵Ala,¹⁴Leu,²⁵Phe-exendin-4(1-28) (SEQ ID NO: 240). Also useful are exendin(7-15) and its Ser2 analog, HSEGTFTSD (SEQ ID NO. 378). Preferred amylin peptides that exhibit at least one hormonal activity include amylin, amylin fragments such as amylin(1-17) (SEQ ID NO: 214), amylin (1-16) (SEQ ID NO: 215), amylin(1-15) (SEQ ID NO: 216), and amylin(1-7) (SEQ ID NO: 217), and amylin analogs such as pramlintide, ²Ala-h-amylin (SEQ ID NO: 79), ^{2,7}Ala-h-amylin (SEQ ID NO: 80), and fragments thereof. Preferred calcitonin peptides that exhibit at least one hormonal activity sCT, sCT fragments such as sCT(8-10), sCT(8-27) (SEQ ID NO: 288), and, and calcitonin analogs such as ^{11,18}Arg-sCT (SEQ ID NO: 108), ¹⁸Arg-sCT (SEQ ID NO: 107), ¹⁴Glu,¹⁸Arg-sCT (SEQ ID NO: 109), ¹⁴Glu,^{11,18}Arg-sCT (SEQ ID NO: 110), and fragments thereof. Preferred amylin peptidic enhancers include amylin(32-37) (SEQ ID NO: 242), amylin(33-37) (SEQ ID NO: 243), and amylin(34-37) (SEQ ID NO: 244), and analogs thereof Amylin-sCT combinations useful in connection with the present invention include those disclosed in PCT/US2005/004631, Amylin Family Agonist, Attorney Docket 18528.835, which is herein incorporated by reference. An amylin-sCT chimera particularly useful for creating hybrids of the invention is Compound 10 (described herein and in PCT/US2005/004631) and analogs and derivatives thereof.

In one aspect, preferred module combinations include those involving a first module comprising exendin-4, a fragment of exendin-4 that exhibits at least one hormonal activity, an exendin-4 analog or derivative that exhibits at least one hormonal activity, or a fragment of an exendin-4 analog that exhibits at least one hormonal activity in combination with a peptidic enhancer. Preferred exendin-4 compounds include: exendin-4, exendin-4(1-27) (SEQ ID NO: 236), exendin-4(1-28) (SEQ ID NO: 237), ¹⁴Leu,⁵Phe-exendin-4(1-28) (SEQ ID NO: 284), and ⁵Ala,¹⁴Leu,²⁵Phe-exendin-4(1-28) (SEQ ID NO: 240). Preferred peptidic enhancers include: PYY(25-36) (SEQ ID NO: 257), PYY(30-36) (SEQ ID NO: 262) and PYY(31-36) (SEQ ID NO: 263). In one embodiment, the first module is located at the C-terminal end of the hybrid polypeptide and the peptidic enhancer is located at the N-terminal end of the hybrid polypeptide. Alternatively, the first module may be located at the N-terminal end of the hybrid polypeptide and the peptidic enhance may be located at the C-terminal end of the hybrid polypeptide. In certain embodiments, spacers or linkers such as βAla may be inserted if desired to attach the modules.

In another aspect, preferred module combinations include those involving a first module comprising exendin-4, a fragment of exendin-4 that exhibits at least one hormonal activity, an exendin-4 analog or derivative that exhibits at least one hormonal activity, or a fragment of an exendin-4 analog that exhibits at least one hormonal activity in combination with a second module comprising CCK, a fragment of CCK that exhibits at least one hormonal activity, a CCK analog or derivative that exhibits at least one hormonal activity, or a fragment of a CCK analog that exhibits at least one hormonal activity. Again, preferred exendin-4 compounds include: exendin-4, exendin-4(1-27) (SEQ ID NO: 236), exendin-4(1-28) (SEQ ID NO: 237), ¹⁴Leu,²⁵Phe-exendin-4(1-28) (SEQ ID NO: 284), ⁵Ala,¹⁴Leu,²⁵Phe-exendin-4(1-28) (SEQ ID NO: 240), and ¹⁴Leu-exendin-4(1-28) (SEQ ID NO: 190). Preferred CCK compounds include: CCK-8, and CCK-8(Phe(CH₂SO₃)). In one embodiment, the first module is located at the C-terminal end of the hybrid polypeptide and the second module is located at the N-terminal end of the hybrid polypeptide. Alternatively, the first module may be located at the N-terminal end of the hybrid polypeptide and the peptidic enhance may be located at the C-terminal end of the hybrid polypeptide. In certain embodiments, spacers or linkers such as βAla may be inserted if desired to attach the modules.

In another aspect, preferred module combinations include those involving a first module comprising amylin, a fragment of amylin that exhibits at least one hormonal activity, an amylin analog or derivative that exhibits at least one hormonal activity, or a fragment of an amylin analog that exhibits at least one hormonal activity in combination with a second module comprising with a peptidic enhancer, such as PYY(25-36) (SEQ ID NO: 257) or PYY(30-36) (SEQ ID NO: 262). In one embodiment, the first module is located at the C-terminal end of the hybrid polypeptide and the peptidic enhancer is located at the N-terminal end of the hybrid polypeptide. Alternatively, the first module may be located at the N-terminal end of the hybrid polypeptide and the peptidic enhance may be located at the C-terminal end of the hybrid polypeptide. In certain embodiments, spacers or linkers such as βAla may be inserted if desired to attach the modules.In another aspect, preferred module combinations include those involving a first module comprising amylin, a fragment of amylin that exhibits at least one hormonal activity, an amylin analog or derivative that exhibits at least one hormonal activity, or a fragment of an amylin analog that exhibits at least one hormonal activity in combination with a second module comprising with a peptidic enhancer, such as PYY(25-36) or PYY(30-36). In one embodiment, the first module is located at the C-terminal end of the hybrid polypeptide and the peptidic enhancer is located at the N-terminal end of the hybrid polypeptide. Alternatively, the first module may be located at the N-terminal end of the hybrid polypeptide and the peptidic enhance may be located at the C-terminal end of the hybrid polypeptide. In certain embodiments, spacers or linkers such as βAla may be inserted if desired to attach the modules.

Other preferred module combinations include those involving combinations of exendin and CCK or amylin, calcitonin, and CCK as a tertiary combination. Particular combinations include exendin/CCK and CCK/exendin, with and without spacers or linkers and linking groups. Other combinations include CCK/amylin/calcitonin and CCK/amylin/calcitonin/amylin, with and without spacers or linking groups. Each module may independently be a peptidic enhancer or may exhibit a hormonal activity, depending on the desired properties of the hybrid polypeptide. In one embodiment the amylin/calcitonin/amylin is provided as a chimera of amylin/calcitonin/amylin such as in Compound 10.

Yet other preferred module combinations include those involving combinations of exendin, amylin and calcitonin as tertiary and tetra-hybrid molecules. Exemplary combinations include exendin/amylin/calcitonin; exendin/amylin/calcitonin/amylin; amylin/calcitonin/exendin; and amylin/calcitonin/amylin/exendin combinations, with and without spacers or linking groups. Each module may independently be a peptidic enhancer or may exhibit a hormonal activity, depending on the desired properties of the hybrid polypeptide. In one embodiment the amylin/calcitonin/amylin is provided as a chimera of amylin/calcitonin/amylin such as in Compound 10.

In one embodiment, when one of the bio-active peptide hormone module(s) that exhibits at least one hormonal activity is amylin or an analog or fragment thereof, and a second bio-active peptide hormone module comprises CCK, then the hybrid polypeptide should preferably comprise a third bio-active peptide hormone module selected from a different component peptide hormone. Exemplary third bio-active peptide hormone modules include calcitonins, more preferably salmon calcitonin, analogs or fragments thereof.

In another embodiment, when one of the bio-active peptide hormone module(s) that exhibits at least one hormonal activity is amylin or an analog or fragment thereof, and a second bio-active peptide hormone module comprises CT, then the hybrid polypeptide should preferably comprise a third bio-active peptide hormone module selected from a different component peptide hormone. Exemplary third bio-active peptide hormone modules include exendin-4, analogs or fragments thereof.

In yet another embodiment, when one of the bio-active peptide hormone module(s) that exhibits at least one hormonal activity is GLP-1 or an analog or fragment thereof, and a second bio-active peptide hormone module is a peptidic enhancer comprising an exendin fragment, then the hybrid polypeptide should preferably comprise a third bio-active peptide hormone module. Exemplary third bio-active peptide hormone modules include PYY (including analogs, derivatives and fragments thereof) and CCK (including analogs, derivatives and fragments thereof).

Within each of the combinations described herein, it is understood that reference to a component peptide hormone includes reference to analogs, derivatives, fragments, as well as peptidic enhancers related thereto.

In a preferred aspect, the hybrid polypeptides include:

| SEQ ID NO: | |
|---|---|
| 1 | Exendin-4-PYY(22-36) |
| 2 | Exendin-4-PYY(25-36) |
| 3 | Exendin-4-PYY(18-36) |
| 4 | Exendin-4-βAla-βAla-PYY(22-36) |
| 5 | Exendin-4-βAla-βAla-PYY(25-36) |
| 6 | Exendin-4-βAla-βAla-PYY(31-36) |
| 7 | Exendin-4(1-28)-PYY(22-36) |
| 8 | Exendin-4(1-28)-PYY(25-36) |
| 9 | Exendin-4(1-28)-PYY(18-36) |
| 10 | Exendin-4(1-28)-βAla-βAla-PYY(22-36) |
| 11 | Exendin-4(1-28)-βAla-βAla-PYY(25-36) |
| 12 | Exendin-4(1-28)-βAla-βAla-PYY(31-36) |
| 13 | ⁵Ala, ¹⁴Leu,²⁵Phe-Exendin-4(1-28)-PYY(18-36) |
| 14 | ⁵Ala,¹⁴Leu,²⁵Phe-Exendin-4(1-28)-PYY(22-36) |
| 15 | ⁵Ala,¹⁴Leu,²⁵Phe-Exendin-4(1-28)-PYY(25-36) |
| 16 | ⁵Ala,¹⁴Leu,Phe-Exendin-4(1-17)-PYY(18-36) |
| 17 | ⁵Ala,¹⁴Leu,²⁵Phe-Exendin-4(1-28)-βAla-βAla -PYY(22-36) |
| 18 | ⁵Ala,¹⁴Leu,²⁵Phe-Exendin-4(1-28)-βAla-βAla-PYY(25-36) |
| 19 | ⁵Ala,¹⁴Leu,²⁵Phe-Exendin-4(1-28)-βAla-βAla -PYY(31-36) |
| 20 | Exendin-4-CCK-8 |
| 21 | Exendin-4(1-28)-CCK-8 |
| 22 | Exendin-4(1-28)-CCK-8(Phe(CH₂SO₃)) |
| 23 | Exendin-4(1-28)-(8-amino-3,6-dioxactoanoyl)-CCK-8 |
| 24 | Exendin-4(1-28)-(8-amino-3,6-dioxactoanoyl)-CCK-8(Phe(CH₂SO₃)) |
| 25 | Exendin-4(1-27)-hAmylin(1-7)-^{11,187}Arg-sCT(8-27)-Amylin(33-37) |
| 26 | Exendin-4(1-27)-^{2,7}Ala-hAmylin(1-7)-sCT(8-10) |
| 27 | ²⁹12 Ado-Exendin(1-28)-hAmylin(1-7)-^{11,18}Arg-sCt(8-27)-hAmylin(33-37) |
| 28 | ²⁹12 Ado-Exendin(1-28)-¹des-Lys-hAmylin(1-7)-^{11,18}Arg-sCt(8-27)-hAmylin(33-37) |
| 29 | ²⁹3,6-dioxaoctanoyl-Exendin(1-28)-hAmylin(1-7)-^{11,18}Arg-sCt(8-27)-hAmylin(33-37) |
| 30 | ²⁹3,6-dioxaoctanoyl-Exendin(1-28)-¹des-Lys-hAmylin(1-7),^{11,18}Arg-sCt(8-27)-hAmylin(33-37) |
| 31 | ²⁹5 Apa -Exendin(1-28)-hAmylin(1-7)-^{11,18}Arg-sCt(8-27)-hAmylin(33-37) |
| 32 | ²⁹5 Apa-Exendin(1-28) -¹des-Lys-hAmylin(1-7)-^{11,18}Arg-sCt(8-27)-hAmylin(33-37) |
| 33 | ²⁹βAla-βAla-Exendin(1-28),hAmylm(1-7)-^{11,18}Arg-sCt(8-27)-Amylin(33-37) |
| 34 | ²⁹βAla-βAla-Exendin(1-28)-¹des-Lys-hAmylin(1-7)-^{11,18}Arg-sCt(8-27)-hAmylin(33-37) |
| 35 | ²⁹4,7,10-trioxa-13-tridecanamine succinimidyl-Exendin(1-28)-hAmylin(1-7)^{11,18}Arg-sCt(8-27)-hAmylin(33-37) |
| 36 | ²⁹4,7,10-trioxa-13-tridecanamine succinimidyl-Exendin(1-28)-¹des-Lys-hAmylin(1-7)-^{11,18}Arg-sCt(8-27)-hAmylin(33-37) |
| 37 | CCK-8-GKR-¹⁵Glu-hAmylin(1-17)-¹⁸Arg-sCT(18-26)-Amylin(32-37) |
| 38 | Amylin(1-18)-PYY(19-36) |
| 39 | isocaproyl-STAVL-(Aib)-K(formyl)-LSQEL-(Aib)-K(formyl)-LQT-PYY(18-36) |
| 40 | isocaproyl-STAVL-(Aib)-K(formyl)-LSQEL-(Aib)-K(formyl)-L-PYY(16-36) |
| 41 | CCK-8-[Succinoyl-Cys]-PYY(3-36) |
| 42 | CCK-8-[Bis-Cys(N-Acetyl)]-PYY(3-36) |
| 43 | CCK-8-[Gly-Aminoxymethylcarbonyl]-PYY(3-36) |
| 379 | Exendin-4(1-27)-hAmylin(1-7)-¹⁴Glu,^{11,18}Arg-sCT(8-27)-Amylin(33-37) |
| 380 | ²⁹12 Ado-Exendin(1-28)-hAmylin(1-7)-¹⁴G1u,^{11,18}Arg-sCt(8-27)-hAmylin(33-37) |
| 381 | ²⁹12 Ado-Exendin(1-28)-¹des-Lys-hAmylin(1-7)-¹⁴Glu,^{11,18}Arg-sCt(8-27)-hAmylin(33-37) |
| 382 | ²⁹3,6-dioxaoctanoyl-Exendin(1-28)-hAmylin(1-7)-¹⁴Glu,^{11,18}Arg-sCt(8-27)-hAmylin(33-37) |
| 383 | ²⁹3,6-dioxaoctanoyl-Exendin(1-28)-¹des-Lys-hAmylin(1-7), ¹⁴Glu,^{11,18}Arg-sCt(8-27)-hAmylin(33-37) |
| 384 | ²⁹5 Apa -Exendin(1-28)-hAmylm(1-7)-¹⁴Glu,^{11,18}Arg-sCt(8-27)-hAmylin(33-37) |
| 385 | ²⁹5 Apa-Exendin(1-28) -¹des-Lys-hAmylin(1-7)-¹⁴Glu,^{11,18}Arg-sCt(8-27)-hAmylin(33-37) |
| 386 | ²⁹βAla-βAla-Exendin(1-28),hAmylin(1-7)-¹⁴Glu,^{11,18}Arg-sCt(8-27)-hAmylin(33-37) |
| 387 | ²⁹βAla-βAla-Exendin(1-28)-¹des-Lys-hAmylin(1-7)-¹⁴Glu,^{11,18}Arg-sCt(8-27)-hAmylin(33-37) |
| 388 | ²⁹4,7,10-trioxa-13-tridecanamine succinimidyl-Exendin(1-28)-hAmylin(1-7)-¹⁴Glu,^{11,18}Arg-sCt(8-27)-hAmylin(33-37) |
| 389 | ²⁹4,7,10-trioxa-13-tridecanamine succinimidyl-Exendin(1-28)-¹des-Lys-hAmylin(1-7)-¹⁴Glu,^{11,18}Arg-sCt(8-27)-hAmylin(33-37) |
| 390 | CCK-8-*GKR*-¹⁵Glu-hAmylin(1-17)- ¹⁸Arg-sCT(18-26)-Amylin(32-37) |

Exemplary exendin and neuromedin hybrids include
Exendin-(1-28)-beta-Ala-beta-Ala-FN-38: Exendin-(1-28)-beta-Ala-beta-Ala-Neuromedin(U25:) Exendin-(1-28)-beta-Ala-beta-Ala-Neuromedin(LT-9):
HGEGTFTSDLSKQMEEEAVRLFIEWLKN-beta-Ala-beta-Ala-GYFLFRPRN-NH2 (SEQ ID NO: 393). The beta-Ala-beta-Ala spacer is optional, and can be replaced with Gly-Gly-Gly, a mini-PEG group, or other linker known in the art, particularly those described herein.

Exemplary exendin and natriuretic peptide hybrids include exendin-hBNP peptide hybrids, including
Exendin-(1-28)-beta-Ala-beta-Ala-hBNP: and
Exendin-beta-Ala-beta-Ala-hBNP:

As in all of the hybrids of the disclosure, a beta-Ala-beta-Ala spacer is optional, and can be replaced with Gly-Gly-Gly, a mini-PEG group, or other linker known in the art, particularly those described herein.

The hybrid polypeptides of the present invention may also comprise further modifications including, but are not limited to, substitution, deletion, and insertion to the amino acid sequence of such hybrid polypeptides and any combination thereof. In a preferred aspect, the hybrid polypeptides of the invention include one or more modifications of a "non-essential" amino acid residue. In the context of the invention, a "non-essential" amino acid residue is a residue that can be altered, *i.e*., deleted or substituted, in the native human amino acid sequence of the fragment, *e.g.*, the component peptide hormone fragment, without abolishing or substantially reducing the component peptide hormone receptor agonist activity of the hybrid polypeptide.

Preferred substitutions include conserved amino acid substitutions. A "conservative amino acid substitution" is one in which the amino acid residue is replaced with an amino acid residue having a similar side chain, or physicochemical characteristics (*e.g*., electrostatic, hydrogen bonding, isosteric, hydrophobic features). Families of amino acid residues having similar side chains are known in the art. These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, methionine, cysteine), nonpolar side chains (*e.g*., alanine, valine, leucine, isoleucine, proline, phenylalanine, tryptophan), β-branched side chains (*e.g.*, threonine, valine, isoleucine) and aromatic side chains (*e.g*., tyrosine, phenylalanine, tryptophan, histidine).

The present invention also relates to derivatives of the hybrid polypeptides. Such derivatives include hybrid polypeptides conjugated to one or more water soluble polymer molecules, such as polyethylene glycol ("PEG") or fatty acid chains of various lengths (*e.g*., stearyl, palmitoyl, octanoyl, etc.), or by the addition of polyamino acids, such as poly-his, polyarg, poly-lys, and poly-ala. Modifications to the hybrid polypeptides can also include small molecule substituents, such as short alkyls and constrained alkyls (*e.g*., branched, cyclic, fused, adamantyl), and aromatic groups. The water soluble polymer molecules will preferably have a molecular weight ranging from about 500 to about 20,000 Daltons.

Such polymer-conjugations and small molecule substituent modifications may occur singularly at the N- or C-terminus or at the side chains of amino acid residues within the sequence of the hybrid polypeptides. Alternatively, there may be multiple sites of derivatization along the hybrid polypeptide. Substitution of one or more amino acids with lysine, aspartic acid, glutamic acid, or cysteine may provide additional sites for derivatization. *See, e.g.,* U.S. Patent Nos. 5,824,784 and 5,824,778. Preferably, the hybrid polypeptides may be conjugated to one, two, or three polymer molecules.

The water soluble polymer molecules are preferably linked to an amino, carboxyl, or thiol group, and may be linked by N or C terminus, or at the side chains of lysine, aspartic acid, glutamic acid, or cysteine. Alternatively, the water soluble polymer molecules may be linked with diamine and dicarboxylic groups. In a preferred embodiment, the hybrid polypeptides of the invention are conjugated to one, two, or three PEG molecules through an epsilon amino group on a lysine amino acid.

Hybrid polypeptide derivatives of the invention also include hybrid polypeptides with chemical alterations to one or more amino acid residues. Such chemical alterations include amidation, glycosylation, acylation, sulfation, phosphorylation, acetylation, and cyclization. The chemical alterations may occur singularly at the N- or C-terminus or at the side chains of amino acid residues within the sequence of the PPF hybrid polypeptide. In one embodiment, the C-terminus of these peptides may have a free -OH or NH₂ group. In another embodiment, the N-terminal end may be capped with an isobutyloxycarbonyl group, an isopropyloxycarbonyl group, an n-butyloxycarbonyl group, an ethoxycarbonyl group, an isocaproyl group (isocap), an octanyl group, an octyl glycine group (G(Oct)), or an 8-aminooctanic acid group. In a preferred embodiment, cyclization can be through the formation of disulfide bridges. Alternatively, there may be multiple sites of chemical alteration along the hybrid polypeptide.

In a further embodiment, the hybrids do not include any of the hybrids disclosed in WO2005/077072. Accordingly in one embodiment are claimed novel hybrids. In another embodiment is claimed new uses, as disclosed herein, of any of the hybrids disclosed herein, in WO2005/077072, or elsewhere. Examples of hybrid polypeptides of the present invention are provided in the Sequence Listing and further discussed in the Examples section herein.

### Use of Hybrid Polypeptides in the Treatment or Prevention of Metabolic Conditions or Disorders

Hybrids of the invention can be useful for reducing food intake, reducing appetite, reducing caloric intake, inducing satiety, reducing nutrient availability, causing weight loss, affecting body composition, altering body energy content or energy expenditure, improving lipid profile (including reducing LDL cholesterol and triglyceride levels and/or changing HDL cholesterol levels), slowing gastrointestinal motility, delay gastric emptying, moderating the postprandial blood glucose excursions, preventing or inhibiting glucagon secretion, and decreasing blood pressure. In one embodiment such hybrids contain an exendin, GLP1, amylin and/or sCT portion.

Thus, in certain embodiments, the hybrids of the invention are useful for treating or preventing conditions or disorders which can be alleviated by reducing nutrient availability comprising administering to said subject a therapeutically or prophylactically effective amount of a compound of the invention. Such conditions and disorders include, but are not limited to, eating disorders, insulin-resistance, obesity, abnormal postprandial hyperglycemia, diabetes of any kind, including Type I, Type II, and gestational diabetes, Metabolic Syndrome, Dumping Syndrome, hypertension, dyslipidemia, cardiovascular disease, hyperlipidemia, sleep apnea, cancer, pulmonary hypertension, cholecystitis, and osteoarthritis. In one embodiment such hybrids contain an exendin, GLP1, amylin and/or sCT portion.

Exemplary peptide module pairings include cardioactive/protective peptides, for example a urocortin with a GLP-1 or exendin, an ANP, BNP or CNP with a GLP-1 or exendin, and a urocortin with an ANP, BNP or CNP Such hybrids will be cardioprotective and particularly useful for the related diseases and conditions described herein, including acute or chronic CHF, ischemia reperfusion, myocardial infarction, and for vasodilator actions useful to treat or prevent antihypertensive indications and angina. Ucn 2 and 3 are particularly useful in hybrids of the invention.

Non-limiting examples of a cardiovascular condition or disease are hypertension, myocardial ischemia, and myocardial reperfusion. Compounds of the invention may also be useful in treating or preventing other conditions associated with obesity including stroke, cancer (*e.g*,. endometrial, breast, prostate, and colon cancer), gallbladder disease, sleep apnea, reduced fertility, and osteoarthritis, (see Lyznicki et al, Am. Fam. Phys. 63:2185, 2001). In other embodiments, compounds of the invention may be used to alter body composition for aesthetic reasons, to enhance one's physical capabilities, or to produce a leaner meat source. Hybrids are useful to change body composition by decreasing fat without significant decrease in muscle mass, thus producing a desirable loss of body fat while preserving lean body mass. In one embodiment such hybrids contain an exendin, GLP1, amylin and/or sCT portion.

In another general aspect, hybrids of the invention may be used to inhibit the secretion of ghrelin. Accordingly, compounds of the invention may be utilize this mechanism to treat or prevent ghrelin related disorders such as Prader-Willi syndrome, diabetes of all types and its complications, obesity, hyperphagia, hyperlipidemia, or other disorders associated with hypernutrition. In one embodiment such hybrids contain an exendin, GLP1, amylin and/or sCT portion.

In another general aspect, it is now recognized that hybrids containing amylin and/or sCT portions can be useful for treating or preventing Barrett's esophagus, Gastroesophageal Reflux Disease (GERD) and conditions associated therewith. Such conditions can include, but are not limited to, heartburn, heartburn accompanied by regurgitation of gastric/intestinal contents into the mouth or the lungs, difficulty in swallowing, coughing, intermittent wheezing and vocal cord inflammation (conditions associated with GERD), esophageal erosion, esophageal ulcer, esophageal stricture, Barrett's metaplasia (replacement of normal esophageal epithelium with abnormal epithelium), Barrett's adenocarcinoma, and pulmonary aspiration. Such hybrids have anti-secretory properties, such as inhibition of gastric acids, inhibition of bile acids, and inhibition of pancreatic enzymes. Moreover, such hybrids can have a gastroprotective effect, which renders them particularly useful in the treatment or prevention of Barrett's esophagus, and/or GERD and related or associated conditions as described herein.

In another general aspect, hybrids can be further be useful for treating or preventing pancreatitis, pancreatic carcinoma, and gastritis, particularly in the treatment and prevention of pancreatitis in patients who have undergone endoscopic retrograde cholangiopancreatography (ERCP). Amylin and/or sCT containing hybrid agonists can have a suprisingly superior therapeutic effect when combined with somatostatin. Accordingly, in certain embodiments, methods for treating or preventing pancreatitis comprise administering such hybrids and administering somatostatin and somatostatin agonists to a subject.

In another general aspect, hybrids are useful for decreasing bone resorption, decreasing plasma calcium, and inducing an analgesic effect, particularly to treat bone disorders such as osteopenia and osteoporosis. In yet other embodiments, hybrids are useful to treat pain and painful neuropathy. In one embodiment such hybrids contain an exendin, GLP1, amylin and/or sCT portion.

In another aspect of the disclosure, methods for treating or preventing obesity are provided, wherein the method comprises administering a therapeutically or prophylactically effective amount of a hybrid polypeptide to a subject in need thereof. In a preferred embodiment, the subject is an obese or overweight subject. While "obesity" is generally defined as a body mass index over 30, for purposes of this disclosure, any subject, including those with a body mass index of less than 30, who needs or wishes to reduce body weight is included in the scope of "obese." Subjects who are insulin resistant, glucose intolerant, or have any form of diabetes mellitus (*e.g*., type 1, 2 or gestational diabetes) can benefit from these hybrids. In one embodiment such hybrids contain an exendin, PYY, GLP1, amylin and/or sCT portion.

In yet another embodiment is provided a method to reduce weight in a morbidly obese subject by first reducing the subject's weight to a level below that of being morbidly obese, then administering to the subject a combination of anti-obesity agents in effective amounts to further reduce the subject's weight. Methods for reducing a subject's weight to below that of morbid obesity include reducing caloric intake, increasing physical activity, drug therapy, bariatric surgery, such as gastric bypass surgery, or any combinations of the preceeding methods. In one aspect, administering the combination of anti-obesity agents further reduces the weight of the subject. In another embodiment, methods are provided for reducing the body mass index in a subject having a body mass index of 40 or less by administering a combination of anti-obesity agents in effective amounts to further reduce the subject's weight.

By reducing weight it is meant that the subject loses a portion of his/her total body weight over the course of treatment, whether the course of treatment be days, weeks, months or years. Alternatively, reducing weight can be defined as a decrease in proportion of fat mass to lean mass (in other words, the subject has lost fat mass, but maintained or gained lean mass, without necessarily a corresponding loss in total body weight). An effective amount of the anti-obesity agents administered in combination in this embodiment is an amount effective to reduce a subject's body weight over the course of the treatment, or alternatively an amount effective to reduce the subject's percentage of fat mass over the course of the treatment. In certain embodiments, the subject's body weight is reduced, over the course of treatment, by at least about 1%, by at least about 5%, by at least about 10%, by at least about 15%, or by at least about 20%. Alternatively, the subject's percentage of fat mass is reduced, over the course of treatment, by at least 1%, at least 5%, at least 10%, at least 15%, at least 20%, or at least 25%.

In other aspects of the disclosure,methods of reducing food intake, reducing nutrient availability, causing weight loss, affecting body composition, and altering body energy content or increasing energy expenditure, treating diabetes mellitus, and improving lipid profile (including reducing LDL cholesterol and triglyceride levels and/or changing HDL cholesterol levels) are provided, wherein the methods comprise administering to a subject an effective amount of a hybrid polypeptide of the invention. In a preferred embodiment, the methods of the disclosure are used to treat or prevent conditions or disorders which can be alleviated by reducing nutrient availability in a subject in need thereof, comprising administering to said subject a therapeutically or prophylactically effective amount of a hybrid polypeptide of the invention. Such conditions and disorders include, but are not limited to, hypertension, dyslipidemia, cardiovascular disease, eating disorders, insulin-resistance, obesity, and diabetes mellitus of any kind. In one embodiment such hybrids contain an exendin, PYY, GLP1, amylin and/or sCT portion.

Without intending to be limited by theory, it is believed that the effects of peripherally-administered hybrid polypeptides of the present invention in the reduction of food intake, in the delay of gastric emptying, in the reduction of nutrient availability, and in the causation of weight loss are determined by interactions with one or more unique receptor classes in, or similar to, those in the PP family. More particularly, it appears that a receptor or receptors similar to the PYY-preferring (or Y7) receptors are involved.

Of particular interest as anti-obesity, weight reduction, food reduction, metabolic rate increasing, and body fat reduction and/or fat redistribution hybrids are those that have at least one, preferably two components, which act on the CNS. Particular areas of the forebrain (telencephalonic- and diencephalonic-derived constituents of the brain) and hindbrain or brainstem (including the midbrain, pons and medulla) have been identified as being involved in controlling energy balance. Forebrain structures or nuclei residing in the hypothalamus involved in food intake and/or body weight modulation include, for example, the arcuate nucleus (ARC), the paraventricular nucleus (PVN), the dorsomedial hypothalamus (DMH), the ventromedial nucleus (VMH), and the lateral hypothalamus nucleus (LHA). Hindbrain structures or nuclei residing in the brainstem involved in food intake and/or body weight modulation include, for example, the nucleus of the solitary tract (NST), the area postrema (AP), and the lateral parabrachial nucleus (lPBN). Brainstem nuclei that control the elements of the consummatory motor control system are likely controlled by primary or second order projections from brainstem regions like the NST, AP, and 1PBN. It is noteworthy that the AP, NST and lPBN have all been shown to (collectively and independently) possess their own integrative abilities.

A variety of CNS-directed anti-obesity agents act upon these forebrain structures residing in the hypothalamus involved in food intake and/or body weight modulation. In addition, CNS-directed anti-obesity agents act upon hindbrain structures residing in the brainstem involved in food intake and/or body weight modulation. Examples of such anti-obesity agents are described herein. See the table below for further examples of peptide family modules that can be combined to form an anti-obesity agent hybrid, and can be combined to form an anti-obesity hybrid with activity on both the forebrain and hindbrain. Such components include, for example, neuropeptide Y1 (NPY1) receptor antagonists, NPY5 receptor antagonists, leptin and leptin agonists, ciliary neurotrophic factor (CNTF) and CNTF agonists, peptide YY (PYY) and PYY agonists, exendin and exendin agonists, GLP-1 and GLP-1 agonist, ghrelin and ghrelin antagonists, cholecystokinin (CCK) and CCK agonists, and amylin and amylin agonists, including those described herein. Further peptide family components and clinical guidance can be found in applicant's co-pending patent application, PCT/US06/17529.

### Individual anti-obesity targets and location

| System | CNS Region | Food Intake Role | Anti-obesity agents |
|---|---|---|---|
| Neuropeptide Y (NPY) | Forebrain (ARC/PVN) | Increases intake | NPY1 and NPY5 receptor antagonists |
| Leptin | Forebrain (ARC) | Decreases intake | Leptin, or agonists |
| Ciliary neurotrophic factor (CNTF) | Forebrain (ARC) | Decreases intake | CNTF |
| Peptide YY (PYY) | Forebrain (ARC) | Decrease intake | PYY(3-36) agonists |
| Glucagon-like peptide-1 (GLP-1) | Forebrain (PVN) | Decrease intake | Exenatide and other GLP-1 ligands, DPP-IV inhibitors |
| Ghrelin | Forebrain (ARC) | Increase intake | Ghrelin antagonists |
| Cholecystokinin (CCK) | Hindbrain (AP) | Decrease intake | CCK agonists |
| Amylin | Hindbrain (AP) | Decrease intake | Amylin agonists, Pramlintide, amylin analogs |
| Melanocortins (MC) | Forebrain (PVN/ARC) | Agonists decrease intake | MC4 agonists |

In certain embodiments, the hybrid is an anti-obesity agent that can include one or more predominantly forebrain acting peptide family components. In other embodiments, the hybrid is an anti-obesity agent that can include one or more predominantly hindbrain acting anti-obesity agents. Exemplary peptide families and components are an NPY1 receptor antagonist, an NPY5 receptor antagonist, a leptin or a leptin agonist or analog, a CNTF, an NPY2 receptor agonist (e.g., a PYY(3-36) or a PYY(3-36) agonist), an exendin or an exendin agonist or analog, a GLP-1 or a GLP-1 agonist or analog, a ghrelin antagonist, a CCK or a CCK agonist or analog, and an amylin or an amylin agonist or analog.

In certain embodiments, the hybrid and method for it use include a first component that predominantly targets the energy balance centers of the hypothalamus, such as the ARC, PVN, VM, and LH. In one embodiment the hybrid contains one or more other peptide family component that also target the hypothalamus but at a different location or via a different mechanism of action than the first component. When the hybrid contains more than one other peptide family component and these also target the hypothalamus, the more than one other peptide family components may target the same location via the same mechanism of action as each other, or they may target different locations and/or different mechanisms of action. In another embodiment, the hybrid then contains one or more other peptide family components that provide one or more additional beneficial therapeutic effects as desired, including an anti-obesity effect via a location or mechanism of action different than the first component and each other, control of blood glucose, cardioprotection, and/or control of hypertension. In certain embodiments, the additional peptide family component is one that predominantly targets the energy balance centers of the hindbrain such as the NST, the AP and the 1PBN.

In certain embodiments, the hybrid and method for it use include a first component that predominantly targets the energy balance centers of the hindbrain such as the NST, the AP and the 1PBN. In one embodiment the hybrid contains one or more other peptide family component that also target the hypothalamus but at a different location or via a different mechanism of action than the first component and each other. In another embodiment, the hybrid then contains one or more other peptide family components that provide one or more additional beneficial therapeutic effects as desired, including an anti-obesity effect via a location or mechanism of action different than the first component and each other, control of blood glucose, cardioprotection, and/or control of hypertension. In certain embodiments, the additional peptide family component is one that predominantly targets the energy balance centers of the hypothalamus, such as the ARC, PVN, VM, and LH.

As used herein, an anti-obesity agent that "acts on a forebrain structure involved in food intake and/or body weight modulation" stimulates or suppresses activity of a particular region, e.g., particular nuclei and/or neuronal circuits, in the forebrain. This forebrain stimulation or suppression leads to a reduction in nutrient availability to the body. An anti-obesity agent that "acts on a hindbrain structure involved in food intake and/or body weight modulation" stimulates or suppresses activity of a particular region, e.g., particular nuclei and/or neuronal circuits, in the hindbrain. This hindbrain stimulation or suppression results in a reduction in nutrient availability to the body.

In another aspect, methods for reducing fat mass by increasing the metabolic rate in a subject are provided, where the methods comprise administering an anti-obesity hybrid in amounts effective to reduce fat mass by increasing the subject's metabolic rate. Fat mass can be expressed as a percentage of the total body mass. In some aspects, the fat mass is reduced by at least 1%, at least 5%, at least 10%, at least 15%, at least 20%, or at least 25% over the course of treatment. In one aspect, the subject's lean mass is not decreased over the course of the treatment. In another aspect, the subject's lean mass is maintained or increased over the course of the treatment. In another aspect, the subject is on a reduced calorie diet or restricted diet. By "reduced calorie diet" is meant that the subject is ingesting fewer calories per day than compared to the same subject's normal diet. In one instance, the subject is consuming at least 50 fewer calories per day. In other instances, the subject is consuming at least 100, 150, 200, 250, 300, 400, 500, 600, 700, 800, 900, or 1000 fewer calories per day.

In one embodiment, methods of use in altering fat distribution, reducing fat mass, or both in a subject are provided. Accordingly, subjects for whom altering body composition is of benefit can also benefit from the present methods. Altered body composition, as intended herein, includes loss or maintenance of body fat, with minimization of loss, maintenance, or gain of lean body mass. In such situations, weight may increase as well as decrease. Accordingly, subjects may be lean, overweight, or obese as these terms are generally used in the art. Methods provided may also include reducing fat in non-adipose tissue while sparing lean mass. Uses for this method include treating diseases such as nonalcoholic steatohepatitis (NASH) or lipodystrophy.

In one embodiment, a method for altering the fat distribution in a subject is provided where the method comprises administering an anti-obesity hybrid in amounts effective to alter fat distribution in the subject. In one aspect, the alteration results from an increased metabolism of visceral or ectopic fat, or both in the subject. By "fat distribution" is meant the location of fat deposits in the body. Such locations of fat deposition include, for example, subcutaneous, visceral and ectopic fat depots. By "subcutaneous fat" is meant the deposit of lipids just below the skin's surface. The amount of subcutaneous fat in a subject can be measured using any method available for the measurement of subcutaneous fat. Methods of measuring subcutaneous fat are known in the art, for example, those described in U.S. Patent No. 6,530,886.

By "ectopic fat storage" is meant lipid deposits within and around tissues and organs that constitute the lean body mass (e.g., skeletal muscle, heart, liver, pancreas, kidneys, blood vessels). Generally, ectopic fat storage is an accumulation of lipids outside classical adipose tissue depots in the body. By "visceral fat" is meant the deposit of fat as intra-abdominal adipose tissue. Visceral fat surrounds vital organs and can be metabolized by the liver to produce blood cholesterol. Visceral fat has been associated with increased risks of conditions such as polycystic ovary syndrome, metabolic syndrome and cardiovascular diseases. In some embodiments, the method involves the metabolism of visceral or ectopic fat or both at a rate of at least about 5%, 10%, 15%, 20%, 25%, 30%, 40%, or 50% greater than for subcutaneous fat. In one aspect, the methods result in a favorable fat distribution. In one embodiment, favorable fat distribution is an increased ratio of subcutaneous fat to visceral fat, ectopic fat, or both. In one aspect, the method involves an increase in lean body mass, for example, as a result of an increase in muscle cell mass.

In another embodiment, methods for reducing the amount of subcutaneous fat in a subject are provided, wherein the method comprises administering, to a subject in need thereof, an anti-obesity hybrid in amounts effective to reduce the amount of subcutaneous fat in the subject. In one instance, the amount of subcutaneous fat is reduced in a subject by at least about 5%. In other instances, the amount of subcutaneous fat is reduced by at least about 10%, 15%, 20%, 25%, 30% 40%, or 50% compared to the subject prior to administration of the anti-obesity hybrid.

The methods described herein can be used to reduce the amount of visceral fat in a subject In one instance, the visceral fat is reduced in a subject by at least about 5%. In other instances, the visceral fat is reduced in the subject by at least about 10%, 15%, 20%, 25%, 30% 40%, or 50% compared to the subject prior to administration of the anti-obesity hybrid. Visceral fat can be measured through any means available to determine the amount of visceral fat in a subject. Such methods include, for example, abdominal tomography by means of CT scanning and MRI. Other methods for determining visceral fat are described, for example, in U.S. Patent Nos. 6,864,415, 6,850,797, and 6,487,445.

In one embodiment, a method for preventing the accumulation of ectopic fat or reducing the amount of ectopic fat in a subject is provided, wherein the method comprises administering, to a subject in need thereof, an anti-obesity hybrid in amounts effective to prevent accumulation of ectopic fat or to reduce the amount of ectopic fat in the subject. In one instance, the amount of ectopic fat is reduced in a subject by at least about 5% compared to the subject prior to administration of the anti-obesity hybrid. In other instances, the amount of ectopic fat is reduced in a subject by at least about 10%, or by at least about 15%, 20%, 25%, 30% 40%, or 50%. Alternatively, the amount of ectopic fat is proportionally reduced 5%, 10%, 15%, 20%, 25%, 30%, 40%, 50%, 60%, 70%, 80%, 90%, or 100% in comparison to subcutaneous fat in a subject. Ectopic fat can be measured in a subject using any method available for measuring ectopic fat.

In another embodiment, methods are provided for producing a more favorable fat distribution in a subject, where the method comprises administering to a subject a hybrid that is effective as an anti-obesity agent in an amount effective to produce a favorable fat distribution. In one embodiment, administration of an anti-obesity hybrid reduces the amount of visceral fat or ectopic fat, or both, in a subject. In one embodiment is administered an anti-obesity hybrid that comprises at least one family module that acts upon forebrain structures involved in food intake or body weight modulation or both in combination with at least one family module that acts upon hindbrain structures involved in food intake or body weight modulation or both. In one embodiment, the methods preferentially reduce the amount of visceral or ectopic fat, or a combination of both, over the reduction in subcutaneous fat. Such methods result in a higher ratio of subcutaneous fat to visceral fat or ectopic fat. Such improved ratios may result in a reduced risk of the development of cardiovascular diseases, polycystic ovary syndrome, metabolic syndrome, or any combinations thereof. In one embodiment, ectopic or visceral fat is metabolized at a rate 5% greater than subcutaneous fat. In other embodiments, ectopic or visceral fat is metabolized at a rate at least 10% 15%, 20%, 25%, 30% 50%, 60%, 70%, 80%, 90%, or 100% greater than subcutaneous fat.

Of particular interest for anti-obesity, body weight and fat composition related treatments as discussed herein are hybrids containing an amylin (e.g. amylin-sCT-amylin chimera), leptin and/or PPF (e.g. PYY analogs or PPY/NPY chimera) family modules. For example, an amylin family module can be attached to a leptin family module and either administered alone, or in a further embodiment administered in combination (e.g. separately or mixed together) with a PPF family compound. In another embodiment, the hybrid contains a leptin-PPF combination that is administered alone or in combination with an amylin family compound. In another embodiment, the hybrid contains an amylin-PPF combination that is administered alone or in combination with a leptin family compound. In yet a further embodiment a hybrid contains all three peptide family modules. For example, an amylin-PPF hybrid can be provided in a sterile, pharmaceutically acceptable solution that is then used to dissolve a lyophilized or powdered leptin family compound, as in a dual chamber delivery system.

In still another aspect, provided is a method for the administration of a therapeutically effective amount of a hybrid effective as an anti-obesity agent administered in combination with glucocorticosteroids. Glucocorticosteroids have the adverse effect of increasing fat mass and decreasing lean mass. Accordingly, it is contemplated that the anti-obesity agent combination can be used in conjunction with glucocorticosteroids under conditions where glucocorticosteroid use is beneficial, in order to counteract the adverse effect of the glucocoriticosteroid.

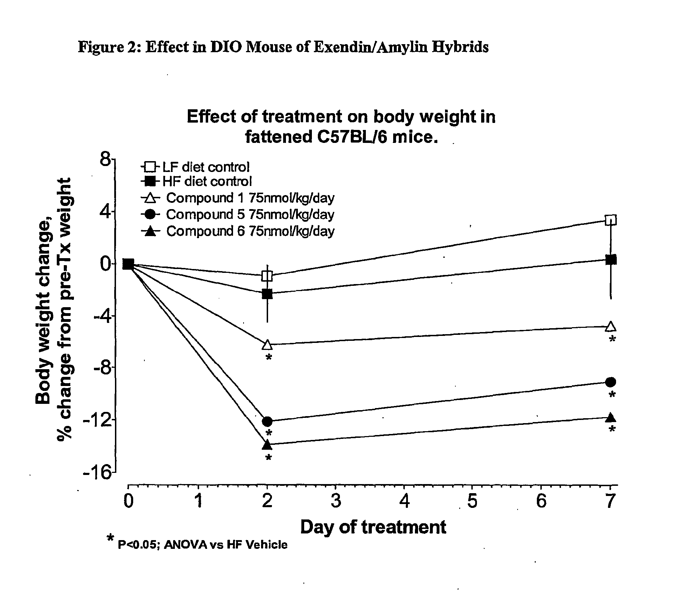

Further regarding lipid lowering, hybrids of the invention find use in lowering of blood lipid levels such as triglycerides, total cholesterol, LDL cholesterol and VLDL cholesterol, and providing a more beneficial lipid profile in subjects in need of such treatment. Accordingly, in one embodiment a method is provided for lowering blood triglycerides, total cholesterol, LDL cholesterol, VLDL cholesterol or any combination thereof comprising administering to the subject in need thereof a lipid-lowering hybrid. In a further embodiment the lipid-lowering hybrid can contain an exendin (incretin) family component, an amylin family component, a PPF or a PYY (NPY) family component or any combination thereof. In one embodiment of the method the lipid to be lowered is plasma triglyceride. In another it is total plasma cholesterol. In another it is LDL cholesterol. In another it is VLDL cholesterol. The hybrid can be effective acutely and/or chronically to maintain or reduce fasting lipid levels and/or reduce post-prandial lipid (particularly triglyceride) excursions. The patient in need of such treatment may have elevated triglyceride, elevated LDL cholesterol, elevated VLDL cholesterol or any combination thereof. Such patients can include those who may otherwise appear normal, have diabetes or pre-diabetes, have obesity, have a lipid disease or condition such as dyslipidemia, hypercholesterolemia or hypertriglyceridemia, and/or have a cardiovascular disease. This effect of hybrids is beneficial in reducing cardiac and atherosclerotic risk in patients at higher risk, e.g., those who are genetically predisposed, obese, diabetic, etc. Thus the patient may be one suffering from atherosclerosis with elevated lipid or cholesterol levels. The present method provides a method of reducing post-prandial triglyceride excursions, reducing circulating lipid levels, treating dyslipidemia, improving circulating lipid profile, treating hypertriglyceridemia, treating hypercholesterolemia, and/or reducing post-prandial triglyceride concentrations in a subject, that comprises administering an effective amount of a hybrid of the invention to the subject in need of such treatment As used herein, "lipid profile" means the balance, proportion, or actual concentration of circulating lipids, including triglyceride levels, HDL, LDL, cholesterol, etc. In a preferred aspect, the methods of the disclosure are useful for lowering triglyceride levels in a patient comprising administering an effective amount of an amylin or amylin agonist. Overall lipid or triglyceride levels can be reduced with this method, e.g., fasting levels, post-prandial peak levels, and overall post-prandial lipid/triglyceride level excursions (e.g., as measured by area under the curve (AUC) of post-prandial triglyceride increase compared to the increase in a non-amylin agonist treated state). Individual clinically relevant measures, such as fasting lipid levels (including triglyceride, cholesterol, HDL, and LDL, etc.) and post-prandial lipid (e.g., triglyceride) levels are also reduced by the methods of the invention. Patients having dyslipidemia or altered lipid levels as compared to normal can be treated by administration of amylin or amylin agonists. Diabetic and obese patients, as well as those who are genetically predisposed to dyslipidemia or cardiovascular disease, are particularly suited to treatment by the methods of the invention. As used herein, "treating elevated triglyceride levels in a patient" means either preventing an increasing in those levels or causing a reduction in those levels relative to the level prior to treatment. As used herein, "reducing post-prandial triglyceride excursions" means lowering the both the peak concentration and the total area under the triglyceride concentration curve that is seen in patients after eating a meal. This typically will mean lowering the total area under the curve for a graph such as those provided in figures 2-4 within the hour following a meal. As used herein "reducing circulating lipid levels" in a patient means lowering the measurable amount of blood lipids relative to the level before treatment. As used herein, "treating dyslipidemia" means improving or restoring to a level, ratio, profile, or balance closer to that medically defined as normal and/or healthy any or all lipid or lipoprotein parameters clinically measurable. This includes, but is not limited to, levels of triglycerides, LDL, HDL, IDL, VLDL, total cholesterol, apolipoproteins, etc. As used herein, "improving circulating lipid profile in a patient" means causing a change in concentration of one or more lipids found in blood in order to change the overall blood lipid content of the patient to a preferred state. It can also include shifting the distribution of lipids over different lipoprotein fractions, without changing the overall content/concentration of circulating lipids. As used herein, "treating hypertriglyceridemia" in a patient means causing a lowering in concentration of triglycerides found in the blood of the patient at one or more relevant times, for example while fasting or post-prandial. As used herein "reducing post-prandial circulating triglycerides" in a patient means lowering the measurable amount of triglycerides in the circulation after a meal (e.g., in the period about 4-6 hours after the meal) relative to the level of such lipids seen post-prandially in the patient before or without treatment for a similar meal.

The hybrids and PYY chimeras of the disclosure can be used with other lipid-lowering drugs. Lipid-lowering drugs includes any compound able to reduce plasma lipid levels. Exemplary lipid-lowering drugs include, but are not limited to, a statin such as atorvastatin, lovastatin, pravastatin, simvastatin, fluvastatin, cerivastatin, or rosuvastatin; a bile acid binder such as cholestyramine or colestipol; a peroxisome proliferative activated receptor (PPAR) agonists such as tesaglitazar, Vitamin E; a cholesterol ester transfer protein (CETP) inhibitor such as ezetimibe, JTT-705, and Torcetrapib.

Additional assays useful to the invention include those that can determine the effect of hybrid compounds on body composition. An exemplary assay can be one that involves utilization of a diet-induced obese (DIO) mouse model for metabolic disease. Prior to the treatment period, male C57BL/6J mice can be fed a high-fat diet (#D12331, 58% of calories from fat; Research Diets, Inc.,) for 6 weeks beginning at 4 weeks of age. During the study, the mice can continue to eat their high-fat diet Water can be provided *ad* libitum throughout the study. One group of similarly-aged non-obese mice can be fed a low-fat diet (#D12329, 11% of calories from fat) for purposes of comparing metabolic parameters to DIO groups.

DIO mice can be implanted with subcutaneous (SC) intrascapular osmotic pumps to deliver either vehicle (50% dimethylsulfoxide (DMSO) in water) or a compound of the invention. The pumps of the latter group can be set to deliver any amount, e.g., 1000 µg/kg/d of a compound of the invention for 7-28 days.

Body weights and food intake can be measured over regular intervals throughout the study periods. Respiratory quotient (RQ, defined as CO₂ production - O₂ consumption) and metabolic rate can be determined using whole-animal indirect calorimetry (Oxymax, Columbus Instruments, Columbus, OH). The mice can be euthanized by isoflurane overdose, and an index of adiposity (bilateral epididymal fat pad weight) measured. Moreover, prior to determination of epididymal weight, body composition (lean mass, fat mass) for each mouse can be analyzed using a Dual Energy X-ray Absorptiometry (DEXA) instrument per manufacturer's instructions (Lunar Piximus, GE Imaging System). In the methods of the disclosure, preferred hybrid polypeptide of the invention are those having a potency in one of the assays described herein (preferably food intake, gastric emptying, pancreatic secretion, weight reduction or body composition assays) which is greater than the potency of a component peptide hormone in that same assay.

In addition to the amelioration of hypertension in subjects in need thereof as a result of reduced food intake, weight loss, or treating obesity, compounds of the invention may be used to treat hypotension.

Compounds of the invention may also be useful for potentiating, inducing, enhancing or restoring glucose responsivity in pancreatic islets or cells. These actions may be useful for treating or preventing conditions associated with metabolic disorders such as those described above and in U.S. patent application no. US20040228846. Assays for determining such activity are known in the art. For example, in published U.S. patent application no. US20040228846, assays are described for islet isolation and culture as well as determining fetal islet maturation. In the examples of patent application US20040228846, intestine-derived hormone peptides including pancreatic polypeptide (PP), neuropeptide Y (NPY), neuropeptide K (NPK), PYY, secretin, glucagon-like peptide-1 (GLP-1) and bombesin were purchased from Sigma. Collagenase type XI was obtained from Sigma. RPMI 1640 culture medium and fetal bovine serum were obtained from Gibco. A radioimmunoassay kit containing anti-insulin antibody ([¹²⁵I]-RIA kit) was purchased from Linco, St Louis.

Post-partem rat islets were obtained from P-02 year old rats. Adult rat islets were obtained from 6-8 week old rats. Fetal rat islets were obtained as follows. Pregnant female rats were sacrificed on pregnancy day E21. Fetuses were removed from the uterus. 10-14 pancreata were dissected from each litter and washed twice in Hanks buffer. The pancreata were pooled, suspended in 6 ml 1 mg/ml collagenase (Type XI, Sigma) and incubated at 37° C for 8-10 minutes with constant shaking. The digestion was stopped by adding 10 volumes of ice-cold Hanks buffer followed by three washes with Hanks buffer. The islets were then purified by Ficoll gradient and cultured in 10% fetal bovine serum (FBS)/RPMI medium with or without addition of 1 µM IBMX. At the end of five days, 20 islets were hand picked into each tube and assayed for static insulin release. Generally, islets were first washed with KRP buffer and then incubated with 1 ml of KRP buffer containing 3 mM (low) glucose for 30 minutes at 37° C. with constant shaking. After collecting the supernatant, the islets were then incubated with 17 mM (high) glucose for one hour at 37° C. The insulin released from low or high glucose stimulation were assayed by radioimmunoassay (RIA) using the [¹²⁵I]-RIA kit. E21 fetal islets were cultured for 5 days in the presence of 200 ng/ml PYY, PP, CCK, NPK, NPY, Secretin, GLP-1 or Bombesin.

An exemplary *in vivo* assay is also provided using the Zucker Diabetic Fatty (ZDF) male rat, an inbred (>F30 Generations) rat model that spontaneously expresses diabetes in all fa/fa males fed a standard rodent diet Purina 5008. In ZDF fa-fa males, hyperglycemia begins to develop at about seven weeks of age and glucose levels (fed) typically reach 500 mg/DL by 10 to 11 weeks of age. Insulin levels (fed) are high during the development of diabetes. However, by 19 weeks of age insulin drops to about the level of lean control litter mates. Triglyceride and cholesterol levels of obese rats are normally higher than those of leans. In the assay, three groups of 7-week old ZDF rats, with 6 rats per group, received the infusion treatment by ALZA pump for 14 days: 1) vehicle control, 2) and 3), PYY with two different doses, 100 pmol/kg/hr and 500 pmol/kg/hr respectively. Four measurements were taken before the infusion and after the infusion at day 7 and day 14: 1) plasma glucose level, 2) plasma insulin level, and 3) plasma triglycerides (TG) level, as well as oral glucose tolerance (OGTT) test. Accordingly, these assays can be used with compounds of the invention to test for desired activity.

Other uses contemplated for the hybrid polypeptides include methods for reducing aluminum (Al) concentrations in the central nervous system (see U.S. Pat. 6,734,166, incorporated by reference in its entirety) for treating, preventing, or delay the onset of Alzheimer's disease. Assays for determining effects on Al are known in the art and can be found in US Pat 6,734,166 using diploid and Ts mice. These mice were individually housed in Nalgene® brand metabolism or polypropylene cages and given three days to adjust to the cages before experimentation. Mice had free access to food (LabDiet® NIH Rat and Moust/Auto 6F5K52, St. Louis, Mo.) and water during the experiment except for the 16 hours prior to euthanasia when no food was provided. Mice were given daily subcutaneous injections of either active compound or saline. Mice were sacrificed at the end of day 13 for one experiment and day 3 for another, and samples were collected. Mice brain samples were weighted in clean teflon liners and prepared for analysis by microwave digestion in low trace element grade nitric acid. Samples were then analyzed for Al content using Inductively Coupled Plasma Mass Spectrometry (Nuttall et al., Annals of Clinical and Laboratory Science 25, 3, 264-271 (1995)). All tissue handling during analysis took place in a clean room environment utilizing HEPA air filtration systems to minimize background contamination. Hybrids of the invention are useful for prevention and treatment of nephropathy, including hypertensive and diabetic nephropathy, and nephropathy associated with insulin resistance and metabolic syndrome. Hybrids achieve these ends by, among other things, improving or preventing worsening of hypertension, endothelial function, renal function, and glomerulosclerosis. In one embodiment, the invention provides a method for preventing or treating nephropathy, including hypertensive and diabetic nephropathy, or that related to insulin resistance, comprising administering a compound of the invention. Hybrids find further use for improving endothelial function in a patient having reduced vasodilatory capacity, or having glomerulosclerosis or any other reduction in glomerular flow. Such improvement in endothelial function serves both to reduce hypertension and to improve the function of the capillaries of the glomeruli. In additional embodiments, the molecules of the invention are useful to prevent progression of nephropathy to ESRD, to prevent, slow the progression of, treat or ameliorate proteinuria and/or glomerulosclerosis. Hybrids are useful for reducing the risk of suffering from, preventing, or treating cardiac arrhythmias. Hybrids can provide anti-arrhythmic effects in patients with cardiac ischemia, cardiac ischemia-reperfusion, and congestive heart failure. For example, GLP-1 has been found to reduce cardiac injury and enhance recovery in patients with these disorders. Incretins, including GLP-1, are glucose-dependent insulinotropic hormones. GLP-1 and exendin effectively enhance peripheral glucose uptake without inducing dangerous hypoglycemia. They also strongly suppress glucagon secretion, independent of its insulinotropic action, and thereby powerfully reduce plasma free fatty acid (FFA) levels substantially more than can be accomplished with insulin. High FFA levels have been implicated as a major toxic mechanism during myocardial ischemia. In another embodiment hybrids are useful for preventing and treating cardiac arrhythmias that reliably reduce injury associated with reperfusion and ischemia, and enhance patient recovery. In yet a further embodiment hybrid treatment after acute stroke or hemorrhage, preferably intravenous administration, provides a means for optimizing insulin secretion, increasing brain anabolism, enhancing insulin effectiveness by suppressing glucagon, and maintaining euglycemia or mild hypoglycemia with no risk of severe hypoglycemia or other adverse side effects. In one embodiment such hybrids contain a GLP1 or exendin portion. In a further embodiment a GLP1 or exendin family module is combined with a natriuretic family peptide, an amylin family peptide, a urocortin family peptide module to obtain enhanced treatment or prevention of cardiovascular conditons or diseases, including CHF, as described herein.

Congestive heart failure is one of the most significant causes of morbidity and mortality in developed countries. It occurs as a late manifestation in diverse cardiovascular diseases characterized by loss of contractile mass and/or by volume or pressure overload (Fortuno, Hypertension 38: 1406-1412 (2001)). Numerous studies have proposed that cardiac remodeling is a major determinant of the clinical course of CHF, irrespective of its etiology (Fedak, Cardiovascular Pathology 14:1-11 (2005)). Cardiac remodeling is thus an attractive target for the treatment of congestive heart failure. As such, agents that act to prevent or decrease cardiac remodeling are desired. Indeed, the literature has identified a need for molecules that can attenuate cardiac remodeling (Fortuno, Hypertension 38:1406-1412 (2001)). Literature reports indicate that attenuating ventricular remodeling also improves survival after myocardial insult, while treatments which worsen remodeling have been associated with poorer outcomes even when they improve systolic function (See, Somasundaram, Med. Clin. N. Am., 88: 1193-1207 (2004)).

Accordingly, provided herein are methods for treating cardiovascular disease, in one embodiment heart failure, acute or chronic, in another embodiment myocardial infarction, in another embodiment ischemic heart failure, and in yet another embodiment congestive heart failure. In one embodiment this treatment is provided by preventing or ameliorating hyperglycemia-induced damage to the cardiovascular system. In one embodiment this treatment is provided by providing a cardioprotective effect. In another embodiment this treatment is achieved by preventing, delaying the onset of, attenuating, or ameliorating cardiac remodeling. In general, cardiac remodeling refers to a restructuring and reshaping of any of the cardiac chambers of the heart. In one embodiment, cardiac remodeling refers to the restructuring and reshaping of the ventricles. As described above and without intending to be limited by theory, cardiac remodeling can be described as genomic changes following an insult to the myocardium, with subsequent molecular, cellular and interstitial changes, leading to the restructuring and reshaping of the cardiac chambers. Such restructuring and reshaping can be manifested clinically as changes in size, shape, and function of the heart. Cardiac remodeling can occur in response to any stimulus or combination of stimuli to the myocardium. In one embodiment, cardiac remodeling is the result of a myocardial insult. By way of non-limiting examples, cardiac remodeling can occur in response to myocardial insults resulting from myocardial infarction, hypertension, volume overload (e.g. from aortic regurgitation), infection, inflammation, diabetes, viral cardiomyopathy, and idiopathic cardiomyopathy.

In one aspect, cardiac remodeling is prevented, delayed, attenuated, or ameliorated by the administration of a hybrid of the invention. The hybrid can comprise the ability to ameliorate (improve) at least one of the following cardiac parameters: left ventricular diastolic function, E wave to A wave ratio, left ventricular end diastolic pressure, cardiac output, cardiac contractility, left ventricular mass, left ventricular mass to body weight ratio, left ventricular volume, left atrial volume, left ventricular end diastolic dimension or systolic dimension, infarct size, exercise capacity, exercise efficiency or any measure of cardiac systolic and/or diastolic function; or attenuate, delay, or prevent enlargement of a heart chamber or a deleterious effect on one of the above cardiac parameters. In one embodiment the hybrid contains a member of the incretin family, e.g. exendin-4, that binds to a GLP-1 or exendin receptor. In the context of the present methods, prevention or amelioration of cardiac remodeling can include a reduction of cardiac remodeling by any amount. In an embodiment, prevention or amelioration of cardiac remodeling is accompanied by a reduced risk of congestive heart failure.

In one embodiment, cardiac remodeling is ameliorated or reduced to an amount that is less than about 1%, 2%, 5%, 10%, 20%, 30%, 40%, 50%, 60%, 70%, 80%, or 90% of the amount of cardiac remodeling in the absence of administering the hybrid. In another embodiment, cardiac remodeling can be slightly reduced, moderately reduced, substantially reduced, or substantially eliminated, as compared to the occurrence of cardiac remodeling in the absence of administering the hybrid. As used herein, a slight reduction of cardiac remodeling refers to cardiac remodeling that is decreased by about 25% or less as compared with cardiac remodeling in the absence of administering the hybrid. A moderate reduction in cardiac remodeling refers to cardiac remodeling that decreased by about 50% or less as compared with cardiac remodeling in the absence of administering the hybrid. A substantial reduction in cardiac remodeling refers to cardiac remodeling that decreased by about 80% or less as compared with cardiac remodeling in the absence of administering the hybrid. A substantial elimination of cardiac remodeling refers to cardiac remodeling that is decreased by about 80% or more as compared with cardiac remodeling in the absence of administering the hybrid.

In order to assess the degree to which cardiac remodeling is prevented, ameliorated, attenuated or delayed, any means available to the skilled worker in the art can be employed. For example, cardiac remodeling can be assessed by analyses including but not limited to histological examination of the heart, LV mass, or during the life of the subject, by measurement of chamber dimensions and wall thickness and motion, for example by echocardiography or quantifying the Left Ventricle (LV) diastolic function using the peak velocity ratio of the E wave and A wave (E/A ratio).

In one embodiment, subjects that may be benefited by administration of a the hybrid to prevent, ameliorate, attenuate, or delay cardiac remodeling can be ascertained by the skilled person in light of conditions and risk factors related to the subject. In one embodiment, subjects may be in need of prevention, amelioration, attenuation, or delay of cardiac remodeling. In another embodiment, the subject may be desirous of preventing, ameliorating, attenuating or delaying cardiac remodeling. One risk factor may be a genetic predisposition for a heart to undergo cardiac remodeling. Exemplary samples and subjects of the present methods provided herein include those which have experienced, are experiencing or are at risk to experience a condition associated with cardiac remodeling. A condition associated with cardiac remodeling can be any condition or disorder in which cardiac remodeling is known to occur or thought to be a risk. Conditions associated with cardiac remodeling include, for example, myocardial infarction, inflammation, ischemia/reperfusion, oxidative stress, cor pulmonale, advanced glycation endproducts, abnormal cardiac wall tension, sympathetic stimulation, myocarditis, hypertension, viral cardiomyopathy, idiopathic cardiomyopathy, heart transplantation, and surgical procedures of the heart.

As mentioned above, the hybrid may be administered as a result of an acute event or a chronic condition. Whether it is an acute event or a chronic condition, methods provided herein include chronic treatment with the hybrid. Thus, length of chronic treatment may include the time when the event has passed and the subject is considered to have recovered from the acute event or recovered from the chronic condition.

Chronic administration of or treatment with the hybrid for the prevention, attenuation, delay, or amelioration of cardiac remodeling may be warranted where no particular transient event or transient condition associated with cardiac remodeling is identified. Chronic administration includes administration of the hybrid over a continuing, but indefinite period of time on the basis of a general predisposition to cardiac remodeling or on the basis of a predisposing condition that is non-transient (e.g., a condition that is non-transient may be unidentified or unamenable to elimination, such as diabetes). The hybrid may be administered chronically in the methods provided herein in order to prevent cardiac remodeling in a subject who exhibits congestive heart failure, regardless of etiology. Chronic administration of the hybrid for the prevention or amelioration of cardiac remodeling may also be implicated in diabetics at risk for congestive heart failure. The hybrid may also be administered on a chronic basis in order to preserve a transplanted organ in individuals who have received a heart transplant. When the hybrid is administered chronically, administration may continue for any length of time. However, chronic administration often occurs for an extended period of time. For example, in an exemplary embodiment, chronic administration continues for 6 months, 1 year, 2 years or longer.

In another embodiment, the methods disclosed herein lead to improved cardiac contractility. Improving cardiac contractility may include the ability of cardiac myocytes to contract. In order to evaluate the improvement of cardiac contractility, any mode of assessment may be used. For example, clinical observation, such as an increase in cardiac output or a decrease in cardiac rate or both, may lead to a determination of increased cardiac contractility. Alternatively, in vivo, an increased contractility of the heart may be assessed by a determination of an increased fractional shortening of the left ventricle. Fractional shortening of the left ventricle may be observed by any available means such as echocardiograph. In evaluating increased cardiac contractility, the increase in fractional shortening of the left ventricle may be an increase of any amount as compared with the fractional shortening before administration of the hybrid. For example, the increase in shortening may be about 10%, 20%, 30%, 40%, 50%, 60%, 70%, 80%, 90%, 100%, 150%, 200% or more than about 200%.

In yet another aspect, a method for reducing or preventing atrial remodeling by the administration of the hybrid is provided. Reduction or prevention of atrial remodeling may be evaluated as compared to atrial remodeling before administration of the hybrid. The therapeutic effects of such reduction or prevention of atrial remodeling includes a reduction in atrial fibrillation. In still another aspect, a method for reducing or preventing ventricular remodeling by the administration of the hybrid is provided. Reduction or prevention of ventricular remodeling may be evaluated as compared to ventricular remodeling before administration of the hybrid.

In a further aspect, prophylactic and therapeutic methods are provided. Treatment on an acute or chronic basis is contemplated. In addition, treatment on an acute basis may be extended to chronic treatment, if so indicated. Chronic treatment is contemplated as being longer than 2 weeks. In certain embodiments, chronic treatment may be longer than 1 month, 3 months, 6 months, 1 year, 2 years, 5 years, or over a life. In one aspect, provided herein is a method for the treatment or prevention of a condition associated with cardiac remodeling in a subject in need thereof. The method generally comprises administering to the subject an amount of the hybrid effective to prevent or ameliorate cardiac remodeling, wherein the condition associated with remodeling is thereby improved, prevented or delayed. As described herein, administration of the hybrid may be done in any manner, including with other agents that provide cardiovascular benefit.

In yet another embodiment, the methods provided herein further comprise the identification of a subject in need of treatment. Any effective criteria may be used to determine that a subject may benefit from administration of the hybrid. Methods for the diagnosis of heart disease and/or diabetes, for example, as well as procedures for the identification of individuals at risk for development of these conditions, are well known to those in the art. Such procedures may include clinical tests, physical examination, personal interviews and assessment of family history.

In yet a further embodiment hybrids that are capable of lowering insulin resistance or increasing insulin sensitivity are useful to treat polycystic ovary syndrome (PCOS). Administering hybrids of the invention can reduce or prevent insulin resistance in a subject suffering from PCOS. In yet another embodiment hybrids prevent the onset of type-2 diabetes in a subject suffering from PCOS. Further hybrids can restore regular inenses, ovulation, or fertility in a subject suffering from PCOS. In one embodiment such hybrids contain a GLP1 or an exendin portion for binding and activating a GLP1 receptor.

The compounds of the invention exhibit a broad range of biological activities, some related to their antisecretory and antimotility properties. The compounds may suppress gastrointestinal secretions by direct interaction with epithelial cells or, perhaps, by inhibiting secretion of hormones or neurotransmitters which stimulate intestinal secretion. Anti-secretory properties include inhibition of gastric and/or pancreatic secretions and can be useful in the treatment or prevention of diseases and disorders including gastritis, pancreatitis, Barrett's esophagus, and Gastroesophageal Reflux Disease.

Compounds of the invention are useful in the treatment of any number of gastrointestinal disorders (see *e.g.,* Harrison's Principles of Internal Medicine, McGraw-Hill Inco, New York, 12th Ed.) that are associated with excess intestinal electrolyte and water secretion as well as decreased absorption, *e.g.,* infectious diarrhea, inflammatory diarrhea, short bowel syndrome, or the diarrhea which typically occurs following surgical procedures, *e.g.,* ileostomy. Examples of infectious diarrhea include, without limitation, acute viral diarrhea, acute bacterial diarrhea (*e.g.,* salmonella, campylobacter, and clostridium or due to protozoal infections), or traveller's diarrhea (*e.g.,* Norwalk virus or rotavirus). Examples of inflammatory diarrhea include, without limitation, malabsorption syndrome, tropical sprue, chronic pancreatitis, Crohn's disease, diarrhea, and irritable bowel syndrome. It has also been discovered that the peptides of the invention can be used to treat an emergency or life-threatening situation involving a gastrointestinal disorder, *e.g.,* after surgery or due to cholera.

Compounds of the invention may also be useful for treating or preventing intestinal damage as opposed to merely treating the symptoms associated with the intestinal damage (for example, diarrhea). Such damage to the intestine may be, or a result of, ulcerative colitis, inflammatory bowel disease, bowel atrophy, loss bowel mucosa, and/or loss of bowel mucosal function (see WO 03/105763. Assays for such activity, as described in WO 03/105763, include 11 week old male HSD rats, ranging 250- 300 grams housed in a 12:12 light:dark cycle, and allowed ad libitum access to a standard rodent diet (Teklad LM 485, Madison, WI) and water. The animals were fasted for 24 hours before the experiment. A simple and reproducible rat model of chronic colonic inflammation has been previously described by Morris GP, et al., "Hapten- induced model of chronic inflammation and ulceration in the rat colon." Gastroenterology. 1989; 96:795-803. It exhibits a relatively long duration of inflammation and ulceration, affording an opportunity to study the pathophysiology of colonic inflammatory disease in a specifically controlled fashion, and to evaluate new treatments potentially applicable to inflammatory bowel disease in humans.

Rats were anesthetized with 3% isofluorane and placed on a regulated heating pad set at 37°C. A gavage needle was inserted rectally into the colon 7 cm. The hapten trinitrobenzenesulfonic acid (TNBS) dissolved in 50% ethanol (v/v) was delivered into the lumen of the colon through the gavage needle at a dose of 30 mg/kg, in a total volume of 0 0.4-0.6 mL, as described in Mazelin, et al., Juton Nerv Syst. 1998;73:38 45. Control groups received saline solution (NaCl 0.9%) intracolonically.

Four days after induction of colitis, the colon was resected from anesthetized rats, which were then euthanized by decapitation. Weights of excised colon and spleen were measured, and the colons photographed for scoring of gross morphologic damage. Inflammation was defined as regions of hyperemia and bowel wall thickening.

Hybrid polypeptides of the disclosure may also be used to treat or prevent pancreatic tumors (*e.g*., inhibit the proliferation of pancreatic tumors). Methods of the disclosure include reducing the proliferation of tumor cells. The types of benign pancreatic tumor cells which may be treated in accordance with the present disclosure include serous cyst adenomas, microcystic tumors, and solid-cystic tumors. The method is also effective in reducing the proliferation of malignant pancreatic tumor cells such as carcinomas arising from the ducts, acini, or islets of the pancreas. U.S. Pat. 5,574,010 provides exemplary assays for testing anti-proliferative properties. For example, the '010 patent provides that PANC-1 and MiaPaCa-2 are two human pancreatic adenocarcinoma cancer cell lines which are available commercially from suppliers such as American Type Culture Collection, ATCC (Rockville, Md.). The two tumor cells were grown in RPMI-1640 culture media supplemented with 10% fetal bovine serum, 29.2 mg/L of glutamine, 25 µg gentamicin, 5 ml penicillin, streptomycin, and fungizone solution (JRH Biosciences, Lenexa, Kans.) at 37 degrees Celcius in a NAPCO water jacketed 5 % CO₂ incubator. All cell lines were detached with 0.25 % trypsin (Clonetics, San Diego, Calif.) once to twice a week when a confluent monolayer of tumor cells was achieved. Cells were pelleted for 7 minutes at 500 g in a refrigerated centrifuge at 4 degrees Celcius, and resuspended in trypsin free fortified RPMI 1640 culture media. Viable cells were counted on a hemocytometer slide with trypan blue.

Ten thousand, 20,000, 40,000 and 80,000 cells of each type were added to 96 well microculture plates (Costar, Cambridge, Mass.) in a total volume of 200 ul of culture media per well. Cells were allowed to adhere for 24 hours prior to addition of the PYY or test peptide. Fresh culture media was exchanged prior to addition of peptides. In vitro incubation of pancreatic tumor cells with either PYY or test compound was continued for 6 hours and 36 hours in length. PYY was added to cells at doses of 250 pmol, 25 pmol, and 2.5 pmol per well (N =14). Test compound was added to cells cultures at doses of 400 pmol, 40 pmol, and 4 pmol per well. Control wells received 2 ul of 0.9% saline to mimic the volume and physical disturbance upon adhered tumor cells. Each 96 well plate contained 18 control wells to allow for comparison within each plate during experimentation. Ninety-six (96) well plates were repeated 6 times with varying concentrations of PYY and test compound in both the PANC-1 and MiaPaCa-2 cells.

At the end of the incubation period, 3-(4,5-dimethylthiazolyl-2-yl)-2,5-diphenyltetrazolium bromide, MTr tetrazolium bromide (Sigma, St. Louis, Mo.) was added to fresh culture media at 0.5 mg/ml. Culture media was exchanged and tumor cells were incubated for 4 hours with MTT tetrazolium bromide at 37°C. At the end of incubation, culture media was aspirated. Formazon crystal precipitates were dissolved in 200 µl of dimethyl sulfoxide (Sigma, St. Louis, Mo.). Quantitation of solubilized formazon was performed by obtaining absorption readings at 500 nm wavelength on an ELISA reader (Molecular Devices, Menlo Park, Calif.). The MTT assay measures mitochondrial NADH dependent dehydrogenase activity, and it has been among the most sensitive and reliable method to quantitative *in vitro* chemotherapy responses of tumor cells. (Alley, M. C., et al., Cancer Res., 48:589-601, 1988; Carmichael, J., et al., Cancer Res., 47:936-942, 1987; McHale, A. P., et al., Cancer Lett., 41:315-321, 1988; and Saxton, R. E., et al., J. Clin. Laser Med. and Surg., 10(5):331-336, 1992.) Analysis of absorption readings at 550 nm were analyzed by grouping wells of the same test conditions and verifying differences occurring between control and the various peptide concentration treatments by one-way ANOVA.

An exemplary *in vivo* assay is also provided. The human pancreatic ductal adenocarcinoma Mia Paca-2 was examined for in vivo growth inhibition by peptide YY and test compound. Seventy thousand to 100,000 human Mia PaCa-2 cells were orthotopically transplanted into 48 male athymic mice. After one week, the animals were treated with either PYY or test compound at 200 pmol/kg/hr via mini-osmotic pumps for four weeks. The paired cultures received saline. At sacrifice, both tumor size and mass were measured. Control mice had significant human cancer growth within the pancreas as evidenced by histologic sections. At 9 weeks, ninety percent (90%) of control mice had substantial metastatic disease. Tumor mass was decreased by 60.5 % in test treated mice and 27% in PYY treated mice.

Hybrids are also useful for the therapeutic and prophylactic treatment of neurological and nervous system disorders associated with neuronal loss or dysfunction, including, but not limited to, Parkinson's Disease, Alzheimer's Disease, Huntington's Disease, ALS, stroke, ADD, and neuropsychiatric syndromes, and to enhance or facilitate learning, memory and cognition in mammals. Particularly useful in this regard are hybrids containing an exendin or GLP1 active portion, more specifically comprising at least the N-terminal 7-15 amino acids or analog thereof, for example HSEGTFTSD (SEQ ID NO: 378).

For all indications, in preferred embodiments, the hybrid polypeptide of the invention is administered peripherally at a dose of about 0.5 µg to about 5 mg per day in single or divided doses or controlled continual release, or at about 0.01 µg/kg to about 500 µg/kg per dose, more preferably about 0.05 µg/kg to about 250 µg/kg, most preferably below about 50 µg/kg. Dosages in these ranges will vary with the potency of each analog or derivative, of course, and may be determined by one of skill in the art.

In the methods of the present disclosure, hybrid polypeptides of the invention may be administered separately or together with one or more other compounds and compositions that exhibit a long term or short-term action to reduce nutrient availability, including, but not limited to other compounds and compositions that comprise an amylin or amylin analog agonist, salmon calcitonin, a cholecystokinin (CCK) or CCK agonist, a leptin (OB protein) or leptin agonist, an exendin or exendin analog agonist, or a GLP-1 or GLP-1 analog agonist. Suitable amylin agonists include, for example, [^{25,28,29}Pro-] human amylin (SEQ ID NO: 67) (also known as "pramlintide," and described in U.S. Pat. Nos. 5,686,511 and 5,998,367). The CCK used is preferably CCK octapeptide (CCK-8), more preferably its sulfated form. Leptin is discussed in, for example, (Pelleymounter et al., Science 269: 540-3 (1995); Halaas et al., Science 269: 543-6 (1995); Campfield et al., Science 269: 546-9 (1995)). Suitable exendins include exendin-3 and exendin-4, and exendin agonist compounds include, for example, those described in PCT Publications WO 99/07404, WO 99/25727, and WO 99/25728.

As discussed herein, a hybrid of the invention can be administered separately or together with one or more other agents in order to obtain additional benefits or to enhance the effect of either the hybrid or the other agent. For example, an anti-obesity hybrid can be administered with an anti-obesity agent or a cardioprotective or anti-hypertension agent, depending on the risk factors pertinent to the subject in need of treatment and desired treatment outcome. Exemplary anti-obesity agents for administration (either separately or mixed; either prior to, concomitantly or after) with a hybrid include serotonin (5HT) transport inhibitors, including, but not limited to, paroxetine, fluoxetine, fenfluramine, fluvoxamine, sertraline, and imipramine. Anti-obesity agents also include selective serotonin reuptake inhibitors, including, but not limited to dexfenfluramine, fluoxetine, sibutramine (e.g., MERIDIA®) and those described in U.S. Pat. No. 6,365,633 and PCT Patent Application Publication Nos. WO 01/27060 and WO 01/162341. 5HT transport inhibitors and serotonin reuptake inhibitors, analogs, derivatives, preparations, formulations, pharmaceutical compositions, doses, and administration routes have previously been described.

Anti-obesity agents also include selective serotonin agonists and selective 5-HT2C receptor agonists, including, but not limited to, U.S. Pat. No. 3,914,250; and PCT Application Publication Nos. WO 02/36596, WO 02/48124, WO 02/10169, WO 01/66548, WO 02/44152; WO 02/51844, WO 02/40456, and WO 02/40457.

Such selective serotonin agonists and 5-HT2C receptor agonists, compositions containing such agonists, and administration routes appropriate for use in the methods provided are known in the art. See, for example, Halford et al. (2005) Curr. Drug Targets 6:201-213 and Weintraub et al. (1984) Arch. Intern. Med. 144:1143-1148.

Anti-obesity agents also include antagonists/inverse agonists of the central cannabinoid receptors (the CB-1 receptors), including, but not limited to, rimonabant (Sanofi Synthelabo), and SR-147778 (Sanofi Synthelabo). CB-1 antagonists/inverse agonsits, derivatives, preparations, formulations, pharmaceutical compositions, doses, and administration routes have previously been described, for example, in U.S. Pat. Nos. 6,344,474, 6,028,084, 5,747,524, 5,596,106, 5,532,237, 4,973,587, 5,013,837, 5,081,122, 5,112,820, 5,292,736, 5,624,941; European Patent Application Nos. EP-656 354 and EP-658546; and PCT Application Publication Nos. WO 96/33159, WO 98/33765, WO98/43636, WO98/43635, WO 01/09120, WO98/31227, WO98/41519, WO98/37061, WO00/10967, WO00/10968, WO97/29079, WO99/02499, WO 01/58869, and WO 02/076949.

Anti-obesity agents also include melanocortins and melanocortin agonists. The receptor MC4R appears to play a role in energy balance and obesity. See, for example, Anderson et al., Expert Opin. Ther. Patents 11:1583-1592 (2001), Speake et al,. Expert Opin. Ther. Patents 12:1631-1638 (2002), Bednarek et al., Expert Opin. Ther. Patents 14:327-336 (2004). Melanocorfin agonists, including, but not limited to, MC4R agonists, and composition containing such agonist appropriate for use in the methods provided are known in the art. MCR agonists, MC4R agonists, derivatives, preparations, formulation, pharmaceutical compositions, doses, and administration routes have previously been described, for example, in the following PCT patent applications. 03/007949, WO 02/068388, WO 02/068387, WO 02/067869, WO 03/040117, WO 03/066587, WO 03/068738, WO 03/094918, and WO 03/031410.

Anti-obesity agents also include metabotropic glutamate subtype 5 receptor (mGluR5) antagonists, including, but are not limited to, compounds such as 2-methyl-6-(phenylethynyl)-pyridine (MPEP) and (3-[(2-methyl-1,3-thiazol-4-yl)ethynyl]pyridine) (MTEP) and those compounds described in Anderson et al. J. Eur. J. Pharmacol. 473:35-40 (2003); Cosford et al. Bioorg. Med. Chem. Lett. 13(3):351-4 (2003); and Anderson et al. J. Pharmacol. Exp. Ther. 303:1044-1051 (2002).

Anti-obesity agents also include topiramate (TOPIMAX® (Ortho McNeil Pharmaceuticals), indicated as an anti-convulsant and an anti-convulsant, but also shown to increase weight loss. The agent may alos include lipase inhibitors such as orlistat.

Anti-obesity agents also include neuropeptide Y1 (NPY1) antagonists and NPY5 antagonists. NPY1 and NPY5 antagonists are known in the art. See, for example Duhault et al. (2000) Can. J Physiol. Pharm. 78:173-185, and U.S. Pat. Nos. 6,124,331, 6,214,853, and 6,340,683. NPY1 and NPY5 antagonists, derivatives, preparations, formulation, pharmaceutical compositions, doses, and administration routes have previously been described. NPY1 antagonists useful in the compositions and methods provided include: U.S. Pat. No. 6,001,836; and PCT Application Publication Nos. WO 96/14307, WO 01/23387, WO 99/51600, WO 01/85690, WO 01/85098, WO 01/85173, and WO 01/89528.

NPY5 antagonists useful in compositions and methods of use provided herein, include, but are not limited to, the compounds described in: U.S. Pat. Nos. 6,140,354, 6,191,160, 6,258,837, 6,313,298, 6,337,332, 6,329,395, 6,340,683, 6,326,375, and 6,335,345; European Patent Nos. EP-01010691, and EP-01044970; and PCT Patent Publication Nos. WO 97/19682, WO 97/20820, WO 97/20821, WO 97/20822, WO 97/20823, WO 98/27063, WO 00/64880, WO 00/68197, WO 00/69849, WO 01/09120, WO 01/85714, WO 01/85730, WO 01/07409, WO 01/02379, WO 01/02379, WO 01/23388, WO,01/23389, WO 01/44201, WO 01/62737, WO 01/62738, WO 01/09120, WO 02/22592, WO 0248152, WO 02/49648, and WO 01/14376.

Anti-obesity agents also include melanin-concentrating hormone (MCH) antagonists including melanin-concentrating hormone 1 receptor (MCH1R) antagonists, such as T-226296 (Takeda) and melanin-concentrating hormone 2 receptor (MCH2R) antagonists. MCH receptor antagonists, derivatives, preparations, formulation, pharmaceutical compositions, doses, and administration routes have previously been described, for example, in U.S. Patent Application Publication Nos. 2005/0009815, 2005/0026915, 2004/0152742, 2004/0209865; PCT Patent Application Publication Nos. WO 01/82925, WO 01/87834, WO 02/06245, WO 02/04433, and WO 02/51809; and Japanese Patent Application No. JP 13226269.

Anti-obesity agents also include opioid antagonists, including, but not limited to those described in PCT Application No. WO 00/21509. Specific opioid antagonists useful in compositions and methods of use provided herein include, but are not limited to, nalmefene (REVEX®), 3-methoxynaltrexone naloxone, naltrexone, naloxonazine, beta-funaltrexamine, deltal ([D-Ala2,Leu5,Cys6]-enkephalin (DALCE), naltrindole isothiocyanate, and norbinaltorphamine.

Anti-obesity agents also include orexin antagonists, including, but not limited to, those described in PCT Patent Application Nos. WO 01/96302, WO 01/68609, WO 02/51232, and WO 02/51838. Specific orexin antagonists useful in compositions and methods of use provided include, but are not limited to, SB-334867-A.

Anti-obesity agents also include neuropeptide Y2 (NPY2) agonists, including, but not limited to, compounds such as PYY3-36 (e.g., Batterham et al. (2003) Nature 418:650-654), NPY3-36 and other Y2 agonists such as N acetyl [Leu(28,31)] NPY 24-36 (White-Smith et al. (1999) Neuropeptides 33:526-533, TASP-V (Malis et al. (1999) Br. J. Pharmacol. 126:989-996), cyclo-(28/32)-Ac-[Lys28-Glu32]-(25-36)-pNPY (Cabrele et al. (2000) J. Pept. Sci. 6:97-122), which can be either a hybrid component as discussed or administered separately. Anti-obesity agents provided also include neuropeptide Y4 (NPY4) agonists including, but not limited to, compounds such as pancreatic peptide (PP) (e.g., Batterham et al. (2003) J. Clin. Endocrinol. Metab. 88:3989-3992) and other Y4 agonists such as 1229U91 (Raposinho et al. (2000) Neuroendocrinology 71:2-7). NPY2 agonists and NPY4 agonsits, derivatives, preparations, formulations, pharmaceutical compositions, doses, and administration routes have previously been described, for example, in U.S. Pat. Publication No. 2002/0141985 and PCT Application Publication No. WO 2005/077094.

Anti-obesity agents also include histamine 3 (H3) antagonist/inverse agonists including but not limited to, those described in PCT Application No. WO 02/15905, O-[3-(1H-imidazol-4-yl)propanol]carbamates (Kiec-Kononowicz et al. (2000) Pharmazie 55:349-355), piperidine-containing histamine H3-receptor antagonists (Lazewska et al. (2001) Pharmazie 56:927-932), benzophenone derivatives and related compounds (Sasse et al. (2001) Arch. Pharm.(Weinheim) 334:45-52), substituted N-phenylcarbamates (Reidemeister et al. (2000) Pharmazie 55:83-86), and proxifan derivatives (Sasse et al. (2000) J. Med. Chem. 43:3335-3343). Specific H3 antagonists/inverse agonists useful in compositions and methods of use provided include, but are not limited to, thioperamide, 3-(1H-imidazol-4-yl)propyl N-(4-pentenyl)carbamate, clobenpropit, iodophenpropit, imoproxifan, and GT2394 (Gliatech).

Anti-obesity agents also include cholecystokinin (CCK) and CCK agonists. Cholecystokinin-A (CCK-A) agonists of use include, but are not limited to, those described in U.S. Pat. No. 5,739,106. Specific CCK-A agonists include, but are not limited to, AR-R 15849, GI 181771, JMV-180, A-71378, A-71623 and SR146131.

Anti-obesity agents also include ghrelin antagonists such as those described in PCT Application Publication Nos. WO 01/87335 and WO 02/08250. Ghrelin antagonists are also known as GHS (growth hormone secretagogue receptor) antagonists. The compositions and methods provided therefore contemplate the use GHS antagonists in place of ghrelin antagonists.

Anti-obesity agents include obestatin and obestatin analogs and agonists. Obestatin is a peptide derived from the same precursor from which ghrelin is derived, preproghrelin. See, for example, Zhang et al. (2005) Science 310: 996-999; Nogueiras et al. (2005) Science 310: 985-986; Pan et al. (2006) Peptides 27:911-916. In contrast to the activity of ghrelin, obestatin appears to act as an anorexic hormone by decreasing food intake, gastric emptying activities, jejunal motility, and body weight gain. Obestatin peptides of use include, but are not limited to those described in Zhang et al. (2005) Science 310: 996-999.

And the amylinomimetics, e.g. pramlintide, amylin-sCT-amylin (Compound 10), incretins, e.g. exendin-4, and PYY analogs, are anti-obesity agents that also can be administered as an anti-obesity agents with a hybrid. For example, a leptin-Compound 10 hybrid may be administered with exendin-4, a PYY analog or both. In another embodiment a leptin-PYY analog hybrid may be administered with exendin-4, an amylinomimetic, or both.

In embodiments of particular interest for treating diabetes and related diseases as discussed herein, are hybrids comprising an exendin family and an alpha-MSH module, and exendin and an amylin family member. Of particular interest are hybrids where the exendin is exendin-4 or analog or derivative thereof and the amylin component is pramlintide or an amylin-sCT-amylin chimera.

In embodiments of particular interest for treating obesity and related diseases and conditions (body fat reduction) as discussed herein, are hybrids comprising an exendin family in combination with an alpha-MSH module, exendin with an amylin family member, an amylin family member with a PYY family member, an amylin family member with a CCK family member, an amylin family member with an alpha-MSH family member, an FN-38 family member, amylin family member with a PYY family member, a PYY family member with another same or different PYY family member, a PPY family member with a CCK family member, a PYY family member with an FN-38 family member, a CCK family member with an FN-38 family member. Of particular interest are hybrids where the exendin is exendin-4 or analog or derivative thereof, the amylin component is pramlintide or an amylin-sCT-amylin chimera, the FN38 family member is FN38 or an analog or derivative thereof, the PYY chimera is a PYY-NPY chimera as described herein such as SEQ ID Nos. 266, 437, 438, 439, 442, 462, 469, 470, 471 and 472 of US 2006/013547A1 and the PYY-NPY chimera 5705 for example.

### Polypeptide Production and Purification

The hybrid polypeptides described herein may be prepared using standard recombinant techniques or chemical peptide synthesis techniques known in the art, *e.g*., using an automated or semi-automated peptide synthesizer, or both.

The hybrid polypeptides of the invention can be synthesized in solution or on a solid support in accordance with conventional techniques. Various automatic synthesizers are commercially available and can be used in accordance with known protocols. *See, e.g*., Stewart and Young, Solid Phase Peptide Synthesis, 2d. ed., Pierce Chemical Co. (1984); Tam et al., J. Am. Chem. Soc. 105: 6442 (1983); Merrifield, Science 232: 341-7 (1986); and Barany and Merrifield, The Peptides, Gross and Meienhofer, eds., Academic Press, New York, 1-284 (1979). Solid phase peptide synthesis may be carried out with an automatic peptide synthesizer (e.g., Model 430A, Applied Biosystems Inc., Foster City, California) using the NMP/HOBt (Option 1) system and tBoc or Fmoc chemistry (*see,* Applied Biosystems User's Manual for the ABI 430A Peptide Synthesizer, Version 1.3B July 1, 1988, section 6, pp. 49-70, Applied Biosystems, Inc., Foster City, California) with capping. Peptides may also be assembled using an Advanced Chem Tech Synthesizer (Model MPS 350, Louisville, Kentucky). Peptides may be purified by RP-HPLC (preparative and analytical) using, *e.g.,* a Waters Delta Prep 3000 system and a C4, C8, or C18 preparative column (10 µ, 2.2x25 cm; Vydac, Hesperia, California). The active peptide can be readily synthesized and then screened in screening assays designed to identify reactive peptides.

The hybrid polypeptides of the present invention may alternatively be produced by recombinant techniques well known in the art. *See, e.g.,* Sambrook et al., Molecular Cloning: A Laboratory Manual, 2d ed., Cold Spring Harbor (1989). These hybrid polypeptides produced by recombinant technologies may be expressed from a polynucleotide. One skilled in the art will appreciate that the polynucleotides, including DNA and RNA, that encode such the various fragments of the hybrid polypeptides may be obtained from the wild-type cDNA, taking into consideration the degeneracy of codon usage, or may be engineered as desired. These polynucleotide sequences may incorporate codons facilitating transcription and translation of mRNA in microbial hosts. Such manufacturing sequences may readily be constructed according to the methods well known in the art. *See, e.g.,* WO 83/04053. The polynucleotides above may also optionally encode an N-terminal methionyl residue. Non-peptide compounds useful in the present invention may be prepared by art-known methods. For example, phosphate-containing amino acids and peptides containing such amino acids may be prepared using methods known in the art. *See, e.g.,* Bartlett and Landen, Bioorg. Chem. 14: 356-77 (1986).

A variety of expression vector/host systems may be utilized to contain and express a hybrid polypeptide coding sequence. These include but are not limited to microorganisms such as bacteria transformed with recombinant bacteriophage, plasmid or cosmid DNA expression vectors; yeast transformed with yeast expression vectors; insect cell systems infected with virus expression vectors (*e.g.,* baculovirus); plant cell systems transfected with virus expression vectors (*e.g.,* cauliflower mosaic virus, CaMV; tobacco mosaic virus, TMV) or transformed with bacterial expression vectors (*e.g.,* Ti or pBR322 plasmid); or animal cell systems. Mammalian cells that are useful in recombinant protein productions include but are not limited to VERO cells, HeLa cells, Chinese hamster ovary (CHO) cell lines, COS cells (such as COS-7), WI 38, BHK, HepG2, 3T3, RIN, MDCK, A549, PC12, K562 and 293 cells. Exemplary protocols for the recombinant expression of the protein are described herein.

As such, polynucleotide sequences provided by the invention are useful in generating new and useful viral and plasmid DNA vectors, new and useful transformed and transfected procaryotic and eucaryotic host cells (including bacterial, yeast, and mammalian cells grown in culture), and new and useful methods for cultured growth of such host cells capable of expression of the present hybrid polypeptides. The polynucleotide sequences encoding hybrid polypeptides herein may be useful for gene therapy in instances where underproduction of the component peptide hormone(s) of the chimera would be alleviated, or the need for increased levels of such would be met.

The present invention also provides for processes for recombinant DNA production of the present hybrid polypeptides. Provided is a process for producing the hybrid polypeptides from a host cell containing nucleic acids encoding such hybrid polypeptides comprising: (a) culturing said host cell containing polynucleotides encoding such hybrid polypeptides under conditions facilitating the expression of such DNA molecule; and (b) obtaining such hybrid polypeptides.

Host cells may be prokaryotic or eukaryotic and include bacteria, mammalian cells (such as Chinese Hamster Ovary (CHO) cells, monkey cells, baby hamster kidney cells, cancer cells or other cells), yeast cells, and insect cells.

Mammalian host systems for the expression of the recombinant protein also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing, which cleaves a "'prepro" form of the protein, may also be important for correct insertion, folding and/or function. Different host cells, such as CHO, HeLa, MDCK, 293, WI38, and the like, have specific cellular machinery and characteristic mechanisms for such post-translational activities, and may be chosen to ensure the correct modification and processing of the introduced foreign protein.

Alternatively, a yeast system may be employed to generate the hybrid polypeptides of the present invention. The coding region of the hybrid polypeptide cDNA is amplified by PCR. A DNA encoding the yeast pre-pro-alpha leader sequence is amplified from yeast genomic DNA in a PCR reaction using one primer containing nucleotides 1-20 of the alpha mating factor gene and another primer complementary to nucleotides 255-235 of this gene (Kurjan and Herskowitz, Cell, 30: 933-43 (1982)). The pre-pro-alpha leader coding sequence and hybrid polypeptide coding sequence fragments are ligated into a plasmid containing the yeast alcohol dehydrogenase (ADH2) promoter, such that the promoter directs expression of a fusion protein consisting of the pre-pro-alpha factor fused to the mature hybrid polypeptide. As taught by Rose and Broach, Meth. Enz. 185: 234-79, Goeddel ed., Academic Press, Inc., San Diego, California (1990), the vector further includes an ADH2 transcription terminator downstream of the cloning site, the yeast "2-micron" replication origin, the yeast leu-2d gene, the yeast REP1 and REP2 genes, the E. coli β-lactamase gene, and an E. coli origin of replication. The β-lactamase and leu-2d genes provide for selection in bacteria and yeast, respectively. The leu-2d gene also facilitates increased copy number of the plasmid in yeast to induce higher levels of expression. The REP 1 and REP2 genes encode proteins involved in regulation of the plasmid copy number.

The DNA construct described in the preceding paragraph is transformed into yeast cells using a known method, *e.g*., lithium acetate treatment (Steams et al., Meth. Enz. 185: 280-97 (1990)). The ADH2 promoter is induced upon exhaustion of glucose in the growth media (Price et al., Gene 55: 287 (1987)). The pre-pro-alpha sequence effects secretion of the fusion protein from the cells. Concomitantly, the yeast KEX2 protein cleaves the pre-pro sequence from the mature PYY analog polypeptides (Bitter et al., Proc. Natl. Acad. Sci. USA 81: 5330-4 (1984)).

Hybrid polypeptides of the invention may also be recombinantly expressed in yeast using a commercially available expression system, *e.g*., the Pichia Expression System (Invitrogen, San Diego, California), following the manufacturer's instructions. This system also relies on the pre-pro-alpha sequence to direct secretion, but transcription of the insert is driven by the alcohol oxidase (AOX1) promoter upon induction by methanol. The secreted hybrid polypeptide is purified from the yeast growth medium by, *e.g*., the methods used to purify hybrid polypeptide from bacterial and mammalian cell supernatants.

Alternatively, the cDNA encoding hybrid polypeptides may be cloned into the baculovirus expression vector pVL1393 (PharMingen, San Diego, California). This hybrid polypeptide-containing vector is then used according to the manufacturer's directions (PharMingen) to infect Spodoptera frugiperda cells in sF9 protein-free media and to produce recombinant protein. The protein is purified and concentrated from the media using a heparin-Sepharose column (Pharmacia, Piscataway, New Jersey) and sequential molecular sizing columns (Amicon, Beverly, Massachusetts), and resuspended in PBS. SDS-PAGE analysis shows a single band and confirms the size of the protein, and Edman sequencing on a Proton 2090 Peptide Sequencer confirms its N-terminal sequence.

For example, the DNA sequence encoding the hybrid polypeptide may be cloned into a plasmid containing a desired promoter and, optionally, a leader sequence (*see, e.g*., Better et al., Science 240: 1041-3 (1988)). The sequence of this construct may be confirmed by automated sequencing. The plasmid is then transformed into E. coli, strain MC1061, using standard procedures employing CaCl₂ incubation and heat shock treatment of the bacteria (Sambrook *et al*., *supra*). The transformed bacteria are grown in LB medium supplemented with carbenicillin, and production of the expressed protein is induced by growth in a suitable medium. If present, the leader sequence will affect secretion of the hybrid polypeptide and be cleaved during secretion. The secreted recombinant protein is purified from the bacterial culture media by the method described herein.

Alternatively, the hybrid polypeptides of the invention may be expressed in an insect system. Insect systems for protein expression are well known to those of skill in the art. In one such system, Autographa californica, nuclear polyhedrosis virus (AcNPV) is used as a vector to express foreign genes in Spodoptera frugiperda cells or in Trichoplusia larvae. The hybrid polypeptide coding sequence is cloned into a nonessential region of the virus, such as the polyhedrin gene, and placed under control of the polyhedrin promoter. Successful insertion of hybrid polypeptide will render the polyhedrin gene inactive and produce recombinant virus lacking coat protein coat. The recombinant viruses are then used to infect S. frugiperda cells or Trichoplusia larvae in which hybrid polypeptide is expressed (Smith et al., J. Virol. 46: 584 (1983); Engelhard et al., Proc. Natl. Acad. Sci. USA 91: 3224-7 (1994)).

In another example, the DNA sequence encoding the hybrid polypeptide may be amplified by PCR and cloned into an appropriate vector, for example, pGEX-3X (Pharmacia, Piscataway, New Jersey). The pGEX vector is designed to produce a fusion protein comprising glutathione-S-transferase (GST), encoded by the vector, and a protein encoded by a DNA fragment inserted into the vector's cloning site. The primers for the PCR may be generated to include, for example, an appropriate cleavage site. The recombinant fusion protein may then be cleaved from the GST portion of the fusion protein. The pGEX-3X/PYY analog polypeptide construct is transformed into E. coli XL-1 Blue cells (Stratagene, La Jolla, California), and individual transformants are isolated and grown at 37°C in LB medium (supplemented with carbenicillin) to an optical density at wavelength 600 nm of 0.4, followed by further incubation for 4 hours in the presence of 0.5 mM Isopropyl β-D-Thiogalactopyranoside (Sigma Chemical Co., St. Louis, Missouri). Plasmid DNA from individual transformants is purified and partially sequenced using an automated sequencer to confirm the presence of the desired PPF hybrid polypeptide-encoding gene insert in the proper orientation.

The fusion protein, expected to be produced as an insoluble inclusion body in the bacteria, may be purified as follows. Cells are harvested by centrifugation; washed in 0.15 M NaCl, 10 mM Tris, pH 8, 1 mM EDTA; and treated with 0.1 mg/mL lysozyme (Sigma Chemical Co.) for 15 min. at room temperature. The lysate is cleared by sonication, and cell debris is pelleted by centrifugation for 10 min. at 12,000xg. The fusion protein-containing pellet is resuspended in 50 mM Tris, pH 8, and 10 mM EDTA, layered over 50% glycerol, and centrifuged for 30 min. at 6000xg. The pellet is resuspended in standard phosphate buffered saline solution (PBS) free of Mg⁺⁺ and Ca⁺⁺. The fusion protein is further purified by fractionating the resuspended pellet in a denaturing SDS polyacrylamide gel (Sambrook et al., supra). The gel is soaked in 0.4 M KCl to visualize the protein, which is excised and electroeluted in gel-running buffer lacking SDS. If the GST/PYY analog polypeptide fusion protein is produced in bacteria as a soluble protein, it may be purified using the GST Purification Module (Pharmacia Biotech).

The fusion protein may be subjected to digestion to cleave the GST from the PPF hybrid polypeptide. The digestion reaction (20-40 µg fusion protein, 20-30 units human thrombin (4000 U/mg (Sigma) in 0.5 mL PBS) is incubated 16-48 hrs. at room temperature and loaded on a denaturing SDS-PAGE gel to fractionate the reaction products. The gel is soaked in 0.4 M KCl to visualize the protein bands. The identity of the protein band corresponding to the expected molecular weight of the hybrid polypeptide may be confirmed by partial amino acid sequence analysis using an automated sequencer (Applied Biosystems Model 473A, Foster City, California).

In a particularly preferred method of recombinant expression of the hybrid polypeptides of the present invention, 293 cells may be co-transfected with plasmids containing the hybrid polypeptide cDNA in the pCMV vector (5' CMV promoter, 3' HGH poly A sequence) and pSV2neo (containing the neo resistance gene) by the calcium phosphate method. Preferably, the vectors should be linearized with ScaI prior to transfection. Similarly, an alternative construct using a similar pCMV vector with the neo gene incorporated can be used. Stable cell lines are selected from single cell clones by limiting dilution in growth media containing 0.5 mg/mL G418 (neomycin-like antibiotic) for 10-14 days. Cell lines are screened for hybrid polypeptide expression by ELISA or Western blot, and high-expressing cell lines are expanded for large scale growth.

It is preferable that the transformed cells are used for long-term, high-yield protein production and as such stable expression is desirable. Once such cells are transformed with vectors that contain selectable markers along with the desired expression cassette, the cells may be allowed to grow for 1-2 days in an enriched media before they are switched to selective media. The selectable marker is designed to confer resistance to selection, and its presence allows growth and recovery of cells that successfully express the introduced sequences. Resistant clumps of stably transformed cells can be proliferated using tissue culture techniques appropriate to the cell.

A number of selection systems may be used to recover the cells that have been transformed for recombinant protein production. Such selection systems include, but are not limited to, HSV thymidine kinase, hypoxanthine-guanine phosphoribosyltransferase and adenine phosphoribosyltransferase genes, in tk-, hgprt- or aprt- cells, respectively. Also, anti-metabolite resistance can be used as the basis of selection for dhfr, that confers resistance to methotrexate; gpt, that confers resistance to mycophenolic acid; neo, that confers resistance to the aminoglycoside, G418; also, that confers resistance to chlorsulfuron; and hygro, that confers resistance to hygromycin. Additional selectable genes that may be useful include trpB, which allows cells to utilize indole in place of tryptophan, or hisD, which allows cells to utilize histinol in place of histidine. Markers that give a visual indication for identification of transformants include anthocyanins, β-glucuronidase and its substrate, GUS, and luciferase and its substrate, luciferin.

Many of the hybrid polypeptides of the present invention may be produced using a combination of both automated peptide synthesis and recombinant techniques. For example, a hybrid polypeptide of the present invention may contain a combination of modifications including deletion, substitution, and insertion by PEGylation. Such a hybrid polypeptide may be produced in stages. In the first stage, an intermediate polypeptide containing the modifications of deletion, substitution, insertion, and any combination thereof, may be produced by recombinant techniques as described. Then after an optional purification step as described herein, the intermediate polypeptide is PEGylated through chemical modification with an appropriate PEGylating reagent (*e.g*., from Nektar Therapeutics, San Carlos, California) to yield the desired hybrid polypeptide. One skilled in the art will appreciate that the above-described procedure may be generalized to apply to a hybrid polypeptide containing a combination of modifications selected from deletion, substitution, insertion, derivation, and other means of modification well known in the art and contemplated by the present invention.

It may be desirable to purify the hybrid polypeptides generated by the present invention. Peptide purification techniques are well known to those of skill in the art. These techniques involve, at one level, the crude fractionation of the cellular milieu to polypeptide and non-polypeptide fractions. Having separated the polypeptide from other proteins, the polypeptide of interest may be further purified using chromatographic and electrophoretic techniques to achieve partial or complete purification (or purification to homogeneity). Analytical methods particularly suited to the preparation of a pure peptide are ion-exchange chromatography, exclusion chromatography, polyacrylamide gel electrophoresis, and isoelectric focusing. A particularly efficient method of purifying peptides is reverse phase HPLC, followed by characterization of purified product by liquid chromatography/mass spectrometry (LC/MS) and Matrix-Assisted Laser Desorption Ionization (MALDI) mass spectrometry. Additional confirmation of purity is obtained by determining amino acid analysis.

Certain aspects of the present invention concern the purification, and in particular embodiments, the substantial purification, of an encoded protein or peptide. The term "purified peptide" as used herein, is intended to refer to a composition, isolatable from other components, wherein the peptide is purified to any degree relative to its naturally obtainable state. A purified peptide therefore also refers to a peptide, free from the environment in which it may naturally occur.

Generally, "purified" will refer to a peptide composition that has been subjected to fractionation to remove various other components, and which composition substantially retains its expressed biological activity. Where the term "substantially purified" is used, this designation will refer to a composition in which the peptide forms the major component of the composition, such as constituting about 50%, about 60%, about 70%, about 80%, about 90%, about 95% or more of the peptides in the composition.

Various techniques suitable for use in peptide purification will be well known to those of skill in the art. These include, for example, precipitation with ammonium sulphate, PEG, antibodies, and the like; heat denaturation, followed by centrifugation; chromatography steps such as ion exchange, gel filtration, reverse phase, hydroxylapatite and affinity chromatography; isoelectric focusing; gel electrophoresis; and combinations of such and other techniques. As is generally known in the art, it is believed that the order of conducting the various purification steps may be changed, or that certain steps may be omitted, and still result in a suitable method for the preparation of a substantially purified protein or peptide.

There is no general requirement that the peptides always be provided in their most purified state. Indeed, it is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed, utilizing an HPLC apparatus, will generally result in a greater "-fold" purification than the same technique utilizing a low pressure chromatography system. Methods exhibiting a lower degree of relative purification may have advantages in total recovery of protein product, or in maintaining the activity of an expressed protein.

One may optionally purify and isolate such hybrid polypeptides from other components obtained in the process. Methods for purifying a polypeptide can be found in U.S. Patent No. 5,849,883. These documents describe specific exemplary methods for the isolation and purification of G-CSF compositions that may be useful in isolating and purifying the hybrid polypeptides of the present invention. Given the disclosure of these patents, it is evident that one of skill in the art would be well aware of numerous purification techniques that may be used to purify hybrid polypeptides from a given source.

Also it is contemplated that a combination of anion exchange and immunoaffinity chromatography may be employed to produce purified hybrid polypeptide compositions of the present invention.

### Pharmaceutical Compositions

The present invention also relates to pharmaceutical compositions comprising a therapeutically or prophylactically effective amount of at least one hybrid polypeptide of the invention, or a pharmaceutically acceptable salt thereof, together with pharmaceutically acceptable diluents, preservatives, solubilizers, emulsifiers, adjuvants and/or carriers useful in the delivery of the hybrid polypeptides. Such compositions may include diluents of various buffer content (*e.g.,* Tris-HCl, acetate, phosphate), pH and ionic strength; additives such as detergents and solubilizing agents (*e.g.,* Tween 80, Polysorbate 80), anti-oxidants (*e.g.,* ascorbic acid, sodium metabisulfite), preservatives (*e.g.,* thimersol, benzyl alcohol), and bulking substances (*e.g.,* lactose, mannitol); incorporation of the material into particulate preparations of polymeric compounds, such as polylactic acid, polyglycolic acid, *etc.,* or in association with liposomes. Such compositions will influence the physical state, stability, rate of *in vivo* release, and rate of in vivo clearance of the present hybrid polypeptides. *See, e.g.,* Remington's Pharmaceutical Sciences 1435-712, 18th ed., Mack Publishing Co., Easton, Pennsylvania (1990).

In general, the present hybrid polypeptides will be useful in the same way that the individual component polypeptides are useful in view of their pharmacological properties. One preferred use is to peripherally administer such hybrid polypeptides for the treatment or prevention of metabolic conditions and disorders. In particular, the compounds of the invention possess activity as agents to reduce nutrient availability, reduce food intake, suppress appetite, and effect weight loss. In another embodiment, a preferred use is to administer such hybrid polypeptides for the treatment of diabetes or diabetes related conditions and disorders.

The present hybrid polypeptides may be formulated for peripheral administration, including formulation for injection, oral administration, nasal administration, pulmonary administration, topical administration, or other types of administration as one skilled in the art will recognize. More particularly, administration of the pharmaceutical compositions according to the present invention may be *via* any common route so long as the target tissue is available via that route. In a preferred embodiment, the pharmaceutical compositions may be introduced into the subject by any conventional peripheral method, *e.g*., by intravenous, intradermal, intramusclar, intramammary, intraperitoneal, intrathecal, retrobulbar, intrapulmonary (*e.g*., term release); by oral, sublingual, nasal, anal, vaginal, or transdermal delivery, or by surgical implantation at a particular site. The treatment may consist of a single dose or a plurality of doses over a period of time. Controlled continual release of the compositions of the present invention is also contemplated.

The formulation may be liquid or may be solid, such as lyophilized, for reconstitution. Aqueous compositions of the present invention comprise an effective amount of the hybrid polypeptide, dissolved or dispersed in a pharmaceutically acceptable carrier or aqueous medium. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in therapeutic compositions is contemplated. Supplementary active ingredients also can be incorporated into the compositions. In some cases, it will be convenient to provide a hybrid polypeptide of the invention and another food-intake-reducing, diabetes treating, plasma glucose-lowering, or plasma lipid-altering agent, such as an amylin, an amylin agonist analog, a CCK or CCK agonist, or a leptin or leptin agonist, or an exendin or exendin agonist analog, in a single composition or solution for administration together. In other cases, it may be more advantageous to administer the additional agent separately from said hybrid polypeptide.

The hybrid polypeptide of the invention may be prepared for administration as solutions of free base, or pharmacologically acceptable salts in water suitably mixed with a surfactant, such as hydroxypropylcellulose. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, histidine, procaine and the like. Such products are readily prepared by procedures well known to those skilled in the art. Dispersions also can be prepared in glycerol, liquid polyethylene glycols, and mixtures thereof and in oils. Under ordinary conditions of storage and use, these preparations contain a preservative to prevent the growth of microorganisms.

In one embodiment, the pharmaceutical compositions of the present invention are formulated so as to be suitable for parenteral administration, *e.g., via* injection or infusion. Preferably, the hybrid polypeptide is suspended in an aqueous carrier, for example, in an isotonic buffer solution at a pH of about 3.0 to about 8.0, preferably at a pH of about 3.5 to about 7.4, 3.5 to 6.0, or 3.5 to about 5.0. Useful buffers include sodium citrate-citric acid and sodium phosphate-phosphoric acid, and sodium acetate/acetic acid buffers. A form of repository or "depot" slow release preparation may be used so that therapeutically effective amounts of the preparation are delivered into the bloodstream over many hours or days following transdermal injection or delivery.

The pharmaceutical compositions suitable for injectable use include sterile aqueous solutions or dispersions and sterile powders for the extemporaneous preparation of sterile injectable solutions or dispersions. In all cases, the form should be sterile and should be fluid to the extent that easy syringability exists. It is also desirable for the hybrid polypeptide of the invention to be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents (for example, sugars or sodium chloride). Prolonged absorption of the injectable compositions can be brought about by the use in the compositions of agents delaying absorption (for example, aluminum monostearate and gelatin).

Sterile injectable solutions may be prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle that contains the basic dispersion medium and the required other ingredients from those enumerated above. In the case of sterile powders for the preparation of sterile injectable solutions, the preferred methods of preparation are vacuum-drying and freeze-drying techniques that yield a powder of the active ingredient plus any additional desired ingredient from a previously sterile-filtered solution thereof.

Generally, a therapeutically or prophylactically effective amount of the present hybrid polypeptides will be determined by the age, weight, and condition or severity of the diseases, conditions or disorders of the recipient. *See, e.g.,* Remington's Pharmaceutical Sciences 697-773. *See also* Wang and Hanson, Parenteral Formulations of Proteins and Peptides: Stability and Stabilizers, Journal of Parenteral Science and Technology, Technical Report No. 10, Supp. 42:2S (1988). Typically, a dosage of between about 0.001 µg/kg body weight/day to about 1000 µg/kg body weight/day, may be used, but more or less, as a skilled practitioner will recognize, may be used. Dosing may be one or more times daily, or less frequently, and may be in conjunction with other compositions as described herein. It should be noted that the present invention is not limited to the dosages recited herein.

Appropriate dosages may be ascertained through the use of established assays for determining level of metabolic conditions or disorders in conjunction with relevant dose-response data. The final dosage regimen will be determined by the attending physician, considering factors that modify the action of drugs, *e.g.,* the drug's specific activity, severity of the damage and the responsiveness of the patient, the age, condition, body weight, sex and diet of the patient, the severity of any infection, time of administration and other clinical factors. As studies are conducted, further information will emerge regarding appropriate dosage levels and duration of treatment for specific diseases and conditions.

An effective dose will typically be in the range of about 1 to 30 µg to about 5 mglday, preferably about 10 to 30 µg to about 2 mg/day and more preferably about 5 to 100 µg to about 1 mg/day, most preferably about 5 µg to about 500 µg/day, for a 50 kg patient, administered in a single or divided doses. Preferably, dosages are between about 0.01 to about 100 µg/kg/dose. The exact dose to be administered may be determined by one of skill in the art and is dependent upon the potency of the particular compound, as well as upon the age, weight and condition of the individual. Administration should begin whenever, *e.g.,* suppression of nutrient availability, food intake, weight, blood glucose or plasma lipid modulation is desired, for example, at the first sign of symptoms or shortly after diagnosis of obesity, diabetes mellitus, or insulin-resistance syndrome. Administration may be by any route, *e.g.,* injection, preferably subcutaneous or intramuscular, oral, nasal, transdermal, *etc*. Dosages for certain routes, for example oral administration, may be increased to account for decreased bioavailablity, for example, by about 5-100 fold.

Parenteral administration may be carried out with an initial bolus followed by continuous infusion to maintain therapeutic circulating levels of drug product. Those of ordinary skill in the art will readily optimize effective dosages and administration regimens as determined by good medical practice and the clinical condition of the individual patient.

The frequency of dosing will depend on the pharmacokinetic parameters of the agents and the routes of administration. The optimal pharmaceutical formulation will be determined by one of skill in the art depending on the route of administration and the desired dosage. *See, e.g.,* Remington's Pharmaceutical Sciences, supra, pages 1435-1712. Such formulations may influence the physical state, stability, rate of in vivo release and rate of in vivo clearance of the administered agents. Depending on the route of administration, a suitable dose may be calculated according to body weight, body surface areas or organ size. Further refinement of the calculations necessary to determine the appropriate treatment dose is routinely made by those of ordinary skill in the art without undue experimentation, especially in light of the dosage information and assays disclosed herein, as well as the pharmacokinetic data observed in animals or human clinical trials.

It will be appreciated that the pharmaceutical compositions and treatment methods of the invention may be useful in fields of human medicine and veterinary medicine. Thus the subject to be treated may be a mammal, preferably human or other animal. For veterinary purposes, subjects include for example, farm animals including cows, sheep, pigs, horses and goats, companion animals such as dogs and cats, exotic and/or zoo animals, laboratory animals including mice, rats, rabbits, guinea pigs and hamsters; and poultry such as chickens, turkeys, ducks and geese.

In addition, the present invention contemplates a kit comprising a hybrid polypeptide of the invention, components suitable for preparing said hybrid polypeptide of the invention for pharmaceutical application, and instructions for using said hybrid polypeptide and components for pharmaceutical application.

To assist in understanding the present invention, the following examples are included. The experiments relating to this invention should not, of course, be construed as specifically limiting the invention and such variations of the invention, now known or later developed, which would be within the purview of one skilled in the art are considered to fall within the scope of the invention as described herein and hereinafter claimed.

### EXAMPLES

The present invention is described in more detail with reference to the following non-limiting examples, which are offered to more fully illustrate the invention, but are not to be construed as limiting the scope thereof. The examples illustrate the preparation of the present hybrid polypeptides, and the testing of these hybrid polypeptides of the invention *in vitro* and/or *in vivo.* Those of skill in the art will understand that the techniques described in these examples represent techniques described by the inventors to function well in the practice of the invention, and as such constitute preferred modes for the practice thereof. However, it should be appreciated that those of skill in the art should in light of the present disclosure, appreciate that many changes can be made in the specific methods that are disclosed and still obtain a like or similar result without departing from the scope of the invention.

### Example 1. Preparation of Hybrid Polypeptides

Peptides of the invention may be assembled on a Symphony peptide synthesizer (Protein Technologies, Inc.) using Rink amide resin (Novabiochem) with a loading of 0.43-0.49 mmol/g at 0.050-0.100 mmol or a pre-loaded Wang Resin (Fmoc-Tyr(tBu)-Wang resin) 0.63 mmol/g (Novabiochem). Fmoc amino acid (5.0 eq, 0.250-.500 mmol) residues are dissolved at a concentration of 0.10 M in 1-methyl-2-pyrrolidinone. All other reagents (HBTU, 1-Hydroxybenzotriazole hydrate and N,N-Diisopropylethylamine ) are prepared as 0.55 M Dimethylformamide solutions. The Fmoc protected amino acids are then coupled to the resin-bound amino acid using, HBTU (2.0 eq, 0.100-0.200 mmol), 1-Hydroxybenzotriazole hydrate (1.8 eq, 0.090-0.18 mmol), N,N-Diisopropylethylamine (2.4 eq, 0.120-0.240 mmol) for 2 hours. Following the last amino acid coupling, the peptide is deprotected using 20% (v/v) piperidine in dimethylformamide for 1 hour. Once peptide sequence is complete, the Symphony peptide synthesizer is programmed to cleave the resin. Trifluoroacetic acid (TFA) cleavage of the peptide from resin is carried out using 93% TFA, 3% phenol, 3% water and 1% triisopropylsilane for 1 hour. The cleaved peptide is precipitated using tert-butyl methyl ether, pelleted by centrifugation and lyophilized. The pellet is re-dissolved in water (10-15 mL), filtered and purified via reverse phase HPLC using a C18 column and an acetonitrile/water gradient containing 0.1% TFA.

A general procedure for N-capping the peptides of the invention with fatty acids (*e.g*., octanoic and stearic acids) is as follows: Peptide on rink amide resin (0.1 mmol) is suspended in NMP (5 mL). In a separate vial, HBTU (0.3 mmol), HOBt (0.3 mmol) is dissolved in DMF (5 mL) followed by the addition of DIEA (0.6 mmol). This solution is added to the resin and this suspension is shaken for 2 hrs. The solvent is filtered and washed thoroughly with NMP (5 mLx4) and CH₂Cl₂ (20 mL), dried and is subjected to the TFA cleavage for 1 hr. The yield of the desired peptide is ca. 40 mg after cleavage and purification.

PEG modification may be carried out in solution on a free epsilon-amino group of lysine or a terminal amino group of a purified peptide using commercially available activated PEG esters. The resulting PEGylated derivatives are purified to homogeneity by reverse phase HPLC and the purity is confirmed by LC/MS and MALDI-MS.

Certain exemplary hybrid polypeptides of the disclosure are shown in Table 1-1. Various modifications to the embodied compounds are envisioned, such as chemical modifications such as glycosylation, PEG modifications, etc.; amino acid modifications such as substitutions, insertions and deletions, etc. Further, even though represented as C-terminally amidated, it is understood that the hybrid polypeptides of the invention may alternatively be in the free acid form.

**Table 1-1: Certain Exemplary Hybrid Compounds of the Disclosure**

| SEQ ID: | |
|---|---|
| 1 | HGEGTFTSDLSKQMEEEAVRLFIEWLKNGGPSSGAPPPS-ASLRHYLNLVTRQRY-NH₂ |
| 2 | HGEGTFTSDLSKQMEEAVRLFMWLKNGGPSSGAPPPS-RHYLNLVTRQRY-NH₂ |
| 3 | HGEGTFTSDLSKQMEEEAVRLFIEWLKNGGPSSGAPPPS-NRYYASLRHYLNLVTRQRY-NH₂ |
| 4 | HGEGTFTSDLSKQMEEEAVRLFIEWLKNGGPSSGAPPPS-βAla-βAla-ASLRHYLNLVTRQRY-NH₂ |
| 5 | HGEGTFTSDLSKQMEEEAVRLFIEWLKNGGPSSGAPPPS-βAla-βAla-RHYLNLVTRQRY-NH₂ |
| 6 | HGEGTFTSDLKQMEEEAVRLFIEWLKNGGPSSGAPPPS-βAla-βAla-VTRQRY-NH₂ |
| 7 | HGEGTFTSDLSKQMEEEAVRLFIEWLKN-ASLRHYLNLVTRQRY-NH₂ |
| 8 | HGEGTFTSDLSKQMEEEAVRLFIEWLKN-RHYLNLVTRQRY-NH₂ |
| 9 | HGEGTFRSDLSKQMEEEAVRLFIEWLKN-NRYYASLRHYLNLVTRQRY-NH₂ |
| 10 | HGEGTFTSDLSKQMEEEAVRLFIEWLKN-βAla-βAla-ASLRHYLNLVTRQRY-NH₂ |
| 11 | HGEGTFTSDLSKQMEEEAVRLFIEWLKN-βAla-βAla-RHYLNLVTRQRY-NH₂ |
| 12 | HGEGTFTSDLSKQMEEEAVRLFIEWLKN-βAla-βAla-VTRQRY-NH₂ |
| 13 | HGEGAFTSDLSKQLEEEAVRLFIEWFLKNNRYYASLRHYLNLVTRQRY-NH₂ |
| 14 | HGEGAFTSTDLQLEEEAVRLFIEFLKNASLRHYLNLVTRQRY-NH₂ |
| 15 | HGEGAFTSDLSKQLEEEAVRLFIEFLKNRHYLNLVTRQRY-NH₂ |
| 16 | HGEGAFTSDLSKQLEEENRYYASLRHYLNLVTRQRY-NH₂ |
| 17 | HGEGAFTSDLSKQLEEEAVRLFIEFLKN-βAla-βAla-ASLRHYLNLVTRQRY-NH₂ |
| 18 | HGEGAFTSDLSKQLEEEAVRLFIEFLKN-βAla-βAla-RHYLNLVTRQRY-NH₂ |
| 19 | HGEGAFTSDLSKQLEEEAVRLFIEFLKN-βAla-βAla-VTRQRY-NNH₂ |
| 20 | HGEGTFTSDLSKQMEEEAVRLFIEWLKNGGPSSGAPPPS-DY(SO₃)MGWMDF-NH₂ |
| 21 | HGEGTFTSDLSKQMEEEAVRLFIEWLKN-DY(SO₃)MGWMDF-NH₂ |
| 22 | HGEGTFTSDLSKQMEEEAVRLFIEWLKN-DF(CH₂SO₃)MGWMDF-NH₂ |
| 23 | HGEGTFTSDLSKQMEEEAVRLFIEWLKN-[8-amino-3,6-dioxaoctanoyl]-DY(SO₃)MGWMDF-NH2 |
| 24 | HGEGTFTSDLSKQMEEEAVRLFIEWLKN-[8-amino-3,6-dioxactoanoyl]-DF(CH₂SO₃)MGWMDF-NH₂ |
| 25 | HGEGTFTSDLSKQMEEEAVRLFIEWLKKCNTATCVLGRLSQELHRLQTYPRTNTGSNTY-NH₂ |
| 26 | HGEGTFTSDLSKQMEEEAVRLFIEWLKKANTATAVLG-NH₂ |
| 27 | |
| 28 | HGEGTFTSDLSKQMEEEAVRLFIEWLKN-12-Ado-CNTATCVLGRLSQELHRLQTYPRTNTGSNTY-NH₂ |
| 29 | |
| 30 | |
| 31 | HGEGTFTSDLSKQMEEEAVRLFIEWLKN-5-Apa-KCNTATCVLGRLSQELHRLQTYPRTNTGSNTY-NH₂ |
| 32 | HGEGTFTSDLSKQMEEEAVRLFIEWLKN-5-Apa-CNTATCVLGRLSQELHRLQTYPRTNTGSNTY-NH2 |
| 33 | |
| 34 | |
| 35 | |
| 36 | |
| 203 | DF(CH2SO3)MGWMDF-*GKR*-KCNTATCATQRLANELVRLQTYPRTNVGSNTY-NH₂ |
| 38 | KCNTATCATQRLANFLVR-RYYASLRHYLNLVTRQRY-NH₂ |
| 39 | isocaproyl-STAVL-(Aib)-K(formyl)-LSQEL-(Aib)-K(formyl)-LQT-NRYYASLRHYLNLVTRQRY-NH₂ |
| 40 | isocaproyl-STAVL-(Aib)-K(formyl)-LSQEL-(Aib)-K(formyl)-L-ELNRYYASLRHYILNLVTRQRY-NH₂ |
| 41 | 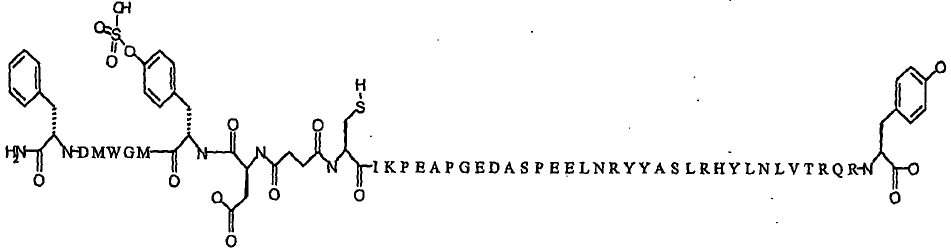 |
| 42 | 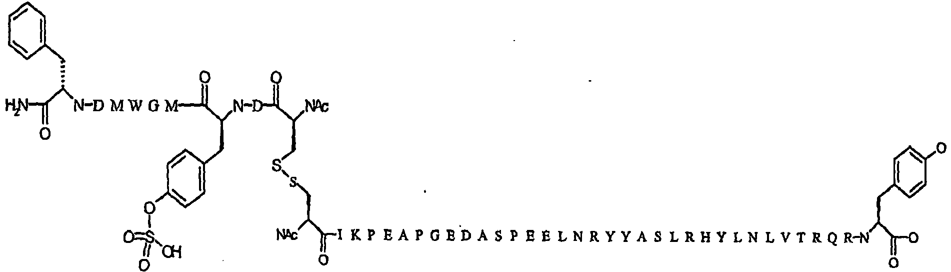 |
| 43 | 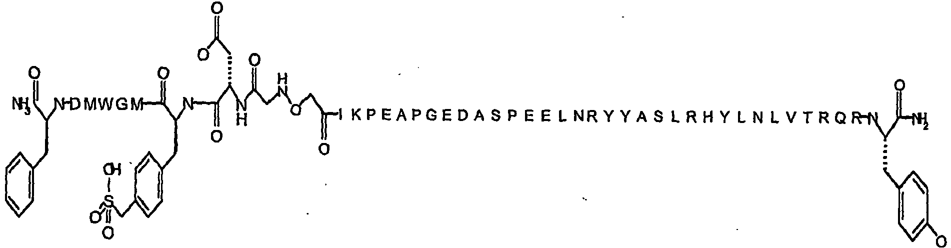 |
| 309 | 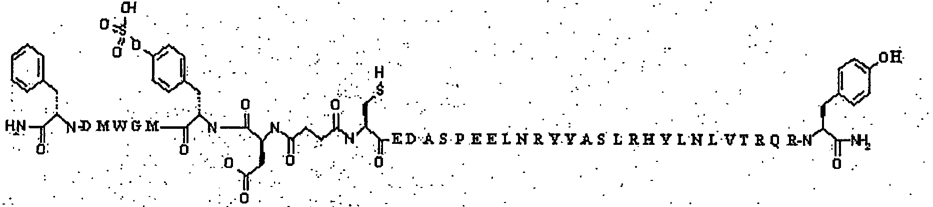 |
| 310 | 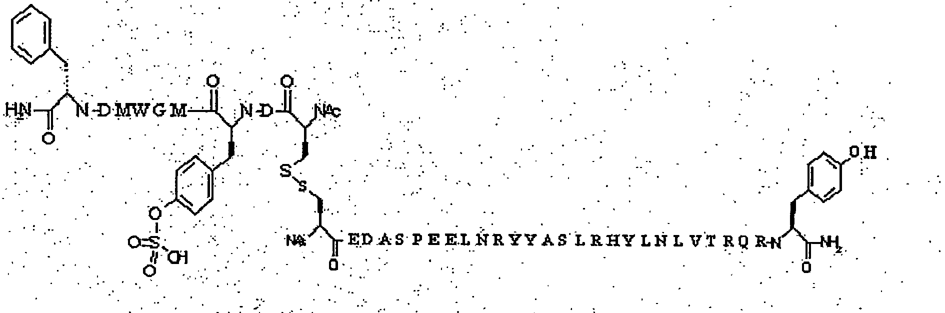 |
| 311 | 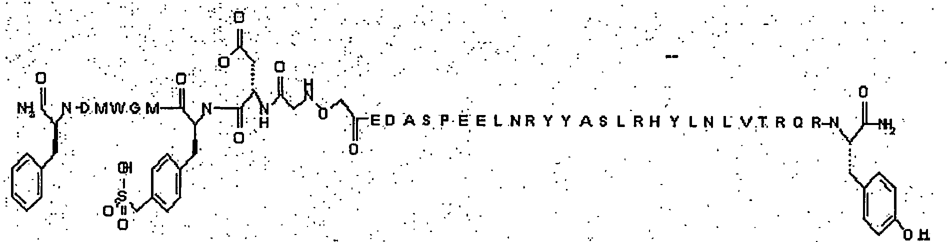 |
| 27 | ²⁹12 Ado-Exendin(1-28)-hAmylin(1-7)-^{11,18}Arg-sCt(8-27)-hAmylin(33-37) |
| 28 | ²⁹12 Ado-Exendin(1-28)-¹des-Lys-hAmylin(1-7)-^{11,18}Arg-sCt(8-27)-hAmylin(33-37) |
| 29 | ²⁹3,6-dioxaoctanoyl-Exendin(1-28)-hAmylin(1-7)-^{11,18}Arg-sCt(8-27)-hAmylin(33-37) |
| 30 | ²⁹3,6-dioxaoctanoyl-Exendin(1-28)-¹des-Lys-hAmylm(1-7),^{11,18}Arg-sCt(8-27)-hAmylin(33-37) |
| 31 | ²⁹5 Apa-Exendin(1-28)-hAmylin(1-7)-^{11,18}Arg-sCt(8-27)-hAmylin(33-37) |
| 32 | ²⁹5 Apa-Exendin(1-28)-¹des-Lys-hAmylin(1-7)^{11,18}Arg-sCt(8-27)-hAmylin(33-37) |
| 33 | ²⁹betaAla-betaAla-Exendin(1-28),hAmylin(1-7)-^{11,18}Arg-sCt(8-27)-hAmylin(33-37) |
| 34 | ²⁹betaAla-betaAla-Exendin(1-28)-¹des-Lys-hAmylin(1-7)-^{11,18}Arg-sCt(27)-hAmylin(33-37) |
| 35 | ²⁹4,7,10-trioxa-13-tridecanamine succinimidyl-Exendin(1-28)-hAmylin(1-7)^{11,18}Arg-sCt(8-27)-hAmylin(33-37) |
| 36 | ²⁹4,7,10-trioxa-13-tridecanamine succinimidyl-Exendin(1-28)-¹des-Lys-hAmylin(1-7)-^{11,18}Arg-sCt(8-27)-hAmylin(33-37) |
| 312 | ²⁹(Gly-Gly-Gly)-Exendin(1-28),hAmylin(1-7)-^{11,18}Arg-sCt(8-27)-hAmylin(33-37) |
| 313 | ²⁹(Gly-Gly-Gly)-Exendin(1-28)-¹des-Lys-hAmylin(1-7)-^{11,18}Arg-sCt(27)-hAmylin(33-37) |

Further exemplary hybrids comprising various combination of component peptide families are found in the following table. Their analog and derivative hybrids as discussed herein are also specifically contemplated. The "#" character indicates the linkage location for each component. Linkers indicated are as described herein. CCK-8 is DY(SO3H)MGWMDF-NH2 whereas "CCK-8*" is the sulfated phenylalanine form DF(CH2SO3H)MGWMDF-NH2.

| **Compound No.** | **Family** | **Name/Sequence** |
|---|---|---|
| 4240 | Exendin | Exendin4(1-27) |
| | Amylin | hAmy(1-7),11Arg)sCT(8-27),hAmy(33-77) |
| | | |
| 4487 | Exendin | EX(1-28) |
| | CCK8 | |
| 4489 | Exendin | EX(1-28) |
| | CCK8 | |
| 5133 | Exendin | Exendin-4-(1-28) |
| | Amylin | Gly-Gly-Gly-hAmy-(1-7)-[Arg11,Arg18]sCT-(8-27)-hAmy-(33-37), amide |
| | | |
| 5453 | Exendin4 Amylin | Exendin-4-(1-28)-bAla-bAla-Rat Amylin, amide |
| | | |
| 5138 | Exendin4 Amylin-sCT-amylin | Exendin-4-(1-28)-betaAla-betaAla-Compound 10- amide |
| | | |
| 5401 | PYY (3-36 Amylin-sCT-amylin | [#Suc-(bAla)-PYY-(3-36), amide] |
| | | [#NH-des-Lys1-Compound 10-amide] |
| | | |
| 5403 | PYY (3-36) | [#Mal-Bu-(bAla)-PYY-(3-36), amide] |
| | Amylin-sCT-amylin | [#S-Prp-(bAla)-Compound 10, amide] |
| | | |
| 5426 | PYY (3-36) | [#Suc-(bAla)-PYY-(3-36), amide] |
| | Amylin | |
| 5457 | Amylin-sCT-amylin | [#S-Prp-(bAla)-Compound 10, amide] |
| | CCK8 | [#Mal-Bu-(bAla)-CCK-8, amide] |
| | | |
| 4277 | CCK8 | 27(p-CH2SO3)-PheCCK8(26-33),G,K,R,15Glu,hAmy(1-17), 18Arg,Sct(18-26),hAmy(32-37 |
| | Amylin | ,hAmy(1-17), 18Arg,Sct(18-26),hAmy(32-37 |
| | | |
| 5498 | Amylin-sCT-amylin | [#NH-des-Lys1-Compound 10-amide] |
| | CCK8 | [#Suc-(bAla)-CCK8, amide] |
| | | |
| 5666 | Amylin-sCT-amylin | [#NH-des-Lys1-Compound 10, amide] |
| | CCK8 | [#Suc-(bAla)-Phe(CH2S03H)2-des-MGWMDF-CCK-8, amide] |
| | | |
| 5493 | aMSH | [Ac-alpha-MSH-(bAla)-(bAla)-K=Lys#] |
| | Exendin4 | [Exendin-4-(1-28)-(bAla)-(bAla)-Lys(#C-ys), amide |
| | | |
| 5499 | aMSH Amylin-sCT-amylin | Ac-alpha-MSH-bAla-bAla-Compound 10-amide |
| | | |
| 5527 | aMSH PYY(3-36) | [Ac-alpha-MSH-bAla-bAla-PYY-(3-36), amide] |
| | | |
| 5522 | FN38 | [#S-Prp-(bAla)-FN38, amide] |
| | CCK8 | [#Mal-Bu-(bAla)-CCK8, amide] |
| | | |
| 5528 | FN38 | [#Suc-bAla-FN38, amide] |
| | Amylin-sCT-amylin | [#NH-des-Lys1-Compound 10- amide] |
| | | |
| 5497 | FN38 | [#S-Prp-(bAla)-FN38, amide] |
| | PYY(3-36) | [#Mal-Bu-(bAla)-PYY-(3-36), amide] |
| | | |
| 5561 | PYY-NPY | [#Suc-Compound 4883, amide] |
| | Amylin-sCT-amylin | [#NH-des-Lys1-Compound 10, amide] |
| | | |
| 5562 | PYY-NPY | [#Suc-bAla-Compound 4883, amide] |
| | Amylin-sCT-amylin | [#NH-des-Lys1-Compound 10, amide] |
| | | |
| 5582 | Amylin-sCT-amylin | [#Des-Lys1 Compound 10, amide] |
| | PYY (3-36) | [Asp(#)-Pro-Pro-Pro-Pro-Pro-Pro-PYY (3-36), amide] |
| | | |
| 5598 | Amylin-sCT-amylin | [#Des-Lys1-Compound 10, amide] |
| | PYY(3-36) | [Asp(#)-miniPEG3-Gly-PYY(3-36), amide] |
| | | |
| 5454 | PYY(3-36) | [#Mal-Bu-(bAla)-PYY-(3-36), amide] |
| | CCK | [#S-Prp-(bAla)-CCK-8, amide] |
| | | |
| 5603 | CCK8* | [#S-PrD-(bAla)-Phe(CH2SO3H)2-CCK8-(1-2), amide] |
| | PYY(3-36) | [#Mal-Bu-(bAla)-PYY(3-36), amide] |
| | | |
| 5604 | CCK8* | [#S-Prp-(bAla)-Tyr2-CCK-8, amide] |
| | PYY(3-36) | [#Mal-Bu-(bAla)-PYY(3-36), amide] |
| | | |
| 5550 | CCK-8 | [#S-Prp-(bAla)-CCK-8, amide] |
| | PYY-NPY | [#Mal-Bu-(bAla)-Compound 4883, amide] |
| | | |
| 5626 | FN38 | [#Suc-(bAla)- FN38, amide] |
| | PYY-NPY | [#NH-Gly-(bAla)-Compound 4883, amide] |
| | | |
| 5567 | PYY (3-36) | [#Cys-Gly-Gly-PYY (3-36), amide] |
| | PYY (3-36) | |
| 5568 | PYY (3-36) | [Asp(#)-miniPEG-Gly-PYY (3-36), amide] |
| | PYY (3-36) | |
| 5581 | PYY (3-36) | [#Cys-Gly-Gly-PYY (3-36), amide] |
| | PYY (3-36) | |
| 5425 | PYY (3-36) | [#NH-Cys-(bAla)-PYY-(3-36), amide] |
| | PYY (3-36) | [#Suc-(bAla)-PYY-(3-36), amide] |
| | | |

### Example 2. Binding Assays

The hybrid polypeptides of the invention may be tested in a variety of receptor binding assays using binding assay methodologies generally known to those skilled in the art. Such assays include those described herein.

Amylin binding assay: Evaluation of the binding of some exemplary compounds of the invention to amylin receptors may be carried out as follows in nuclueus accumbens membranes prepared from rat brain. Male Sprague-Dawley® rats (200-250) grams are sacrificed by decapitation. Brains are removed and place in cold phosphate-buffered saline (PBS). From the ventral surface, cuts are made rostral to the hypothalamus, bounded laterally by the olfactory tracts and extending at a 45° angle medially from these tracts. This basal forebrain tissue, containing the nucleus accumbens and surrounding regions, is weighed and homogenized in ice-cold 20 mM HEPES buffer (20 mM HEPES acid, pH adjusted to 7.4 with NaOH at 23°C). Membranes are washed three times in fresh buffer by centrifugation for 15 minutes at 48,000 x g. The final membrane pellet is resuspended in 20 mM HEPES buffer containing 0.2 mM phenylmethylsulfonyl fluoride (PMSF).

To measure ¹²⁵I-amylin binding (see, Beaumont K et al. Can J Physiol Pharmacol. 1995 Jul; 73(7):1025-9), membranes from 4 mg original wet weight of tissue are incubated with ¹²⁵I -amylin at 12-16 pM in 20 mM HEPES buffer containing 0.5 mg/ml bacitracin, 0.5 mg/ml bovine serum albumin, and 0.2 mM PMSF. Solutions are incubated for 60 minutes at 2°C. Incubations are terminated by filtration through GF/B glass fiber filters (Whatman Inc., Clifton, N.J.) that are presoaked for 4 hours in 0.3% poylethyleneimine in order to reduce nonspecific binding of radiolabeled peptides. Filters are washed immediately before filtration with 5 ml cold PBS, and immediately after filtration with 15 ml cold PBS. Filters are removed and radioactivity assessed in a gamma-counter at a counting efficiency of 77%. Competition curves are generated by measuring binding in the presence of 10⁻¹² to 10⁻⁶ M unlabeled test compound and are analyzed by nonlinear regression using a 4-parameter logistic equation (Inplot program; GraphPAD Software, San Diego).

CGRP receptor binding assay: Evaluation of the binding of compounds of the invention to CGRP receptors are essentially as described for amylin except using membranes prepared from SK-N-MC cells, known to express CGRP receptors (Muff, R. et.al., Ann NY Acad. Sci. 1992: 657, 106-16). Binding assays are performed as described for amylin except using 13,500 cpm 125I-hCGRP /well or 21.7 pM/well (Amersham).

Adrenomedullin binding assay: Binding to the adrenomedullin receptor may be investigated using HUVECs that contain the adrenomedullin receptor (Kato J et. al., Eur J Pharmacol. 1995, 289:383-5) using the Perkin Elmer AlphaScreen™ assay for cyclic AMP using an optimum of 25-30,000 cells per well. Elevation of cAMP levels is not large for HUVEC compared to CHO cells. As such, CHO cells may be chosen as a negative control since they do not express the adrenomedullin receptor if desired.

Calcitonin receptor binding assay: Binding to the calcitonin receptor may be investigated using CHO cells or T47D cells, which also express the calcitonin receptor (Muff R. et.al, Ann N Y Acad Sci. 1992, 657:106-16 and Kuestner R.E. et. al. Mol Pharmacol. 1994, 46:246-55), as known in the art.

Leptin binding assay: Two *in vitro* bioassays are routinely used to assess leptin binding and receptor activation (see *e.g*., White, et al., 1997. Proc.Natl. Acad. Sci. U. S. A. 94: 10657-10662). An alkaline phosphatase("AP")-leptin ("OB") fusion protein ("AP-OB") may be used to measure inhibition of leptin binding in the absence or presence of recombinant mouse leptin (positive control) or peptide, by COS-7 cells transfected with the long (signaling) form of the mouse OB receptor ("OB-RL"). Signal transduction assays may be done in GT1-7 cells cotransfected with AP reporter and OB-RL constructs. Secreted alkaline phosphatase("SEAP") activity in response to stimulation with mouse leptin or peptide may be measured by chemiluminescence.

Y1 receptor binding assay: Membranes are prepared from confluent cultures of SK-N-MC cells that endogenously expresses the neuropeptide Y1 receptors. Membranes are incubated with 60 pM [¹²⁵I]- human Peptide YY (2200 Ci/mmol, PerkinElmer Life Sciences), and with unlabeled active compound for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

Y2 receptor binding assay: Membranes are prepared from confluent cultures of SK-N-BE cells that endogenously expresses the neuropeptide Y2 receptors. Membranes are incubated with 30 pM [¹²⁵I]- human Peptide YY (2200 Ci/mmol, PerkinElmer Life Sciences), and with unlabeled active compound for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

Y4 receptor binding assay: CHO-K1 cells are transiently transfected with cDNA encoding neuropeptide Y4 gene, and then forty-eight hours later membranes are prepared from confluent cell cultures. Membranes are incubated with 18 pM [¹²⁵I]- human Pancreatic Polypeptide (2200 Ci/mmol, PerkinElmer Life Sciences), and with unlabeled active compound for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

Y5 receptor binding assay: CHO-K1 cells are transiently transfected with cDNA encoding neuropeptide Y5 gene, and then forty-eight hours later membranes are prepared from confluent cell cultures. Membranes are incubated with 44 pM [125I]- human Peptide YY (2200 Ci/mmol, PerkinElmer Life Sciences), and with active compound for 60 minutes at ambient temperature in a 96 well polystyrene plate. Then well contents are harvested onto a 96 well glass fiber plate using a Perkin Elmer plate harvestor. Dried glass fiber plates are combined with scintillant and counted on a Perkin Elmer scintillation counter.

GLP-1 receptor binding assay: GLP-1 receptor binding activity and affinity may be measured using a binding displacement assay in which the receptor source is RINm5F cell membranes, and the ligand is [¹²⁵I]GLP-1. Homogenized RINm5F cell membranes are incubated in 20 mM HEPES buffer with 40,000 cpm [¹²⁵I]GLP-1 tracer, and varying concentrations of test compound for 2 hours at 23° C with constant mixing. Reaction mixtures are filtered through glass filter pads presoaked with 0.3% PEI solution and rinsed with ice-cold phosphate buffered saline. Bound counts are determined using a, scintillation counter. Binding affinities are calculated using GraphPad Prism software (GraphPad Software, Inc., San Diego, CA).

In vitro receptor binding and receptor activation, including specific receptor activation of the component modules, for exemplary hybrids are shown in the following table.

| **Cmpd No./Name** | **Receptor Binding** | | | | | **Receptor Activation** | | | | |
|---|---|---|---|---|---|---|---|---|---|---|
| | GLP-1 | Amylin | CGRP | CT | Y2 | GLP1 cyclase | GLP-1/GIP/ CT cyclase | CT cyclase | Y1 | CCK |
| **---Parent---** | | | | | | | | | | |
| Rat amylin | >1000 | 0.05 | 21 | 3 | >1000 | | 5 | 326 | | |
| Human NPY | | | | | 0.2 | | | | | |
| Cmpd 10 | >1000 | 0.03 | 2.3 | 0.03 | >1000 | | 1.4 | 0.7 | | 1000 |
| Exendin-4 amide | 0.5 | | >1000 | >1000 | | | 0.3 | 10000 | | 1000 |
| Exendin-(1-28) amide | 0.6 | >1000 | >1000 | >1000 | | | 0.8 | .tbd | | |
| PYY(3-36) | | | | | 0.08 | | | | 874 | 1000 |
| rat NMU 24 | | | | | | | | | | |
| CCK8 (Sulfated tyrosine) | | | | | | | | | | 7.8 |
| FN-38 | | | | | | | | | | |
| CCK8 | | | | | | | | | | 1000 |
| **---Hybrids---** | | | | | | | | | | |
| 4240 | 1.7 | 0.6 | 178 | 0.5 | >1000 | | 8 | 28 | | |
| 4277 | | | 4.4 | 0.04 | | | 1000 | | | |
| NH2-HGEGTFRSDL SKQMEEEAV RLFIEWLKK ANTATAVLG -NH2 | tbd | >1000 | >1000 | >1000 | | | 2.9 | - | | |
| 4487 | 49 | | 1000 | | 1000 | | | 1000 | | 42.1 |
| 4489 | 31 | | | | 1000 | | | | | 229.5 |
| 5133 | 0.4 | 0.2 | 63 | 0.03 | | | 6.4 | 8 | | |
| 5138 | 0.3 | 0.2 | 113 | 0.1 | | | 7.4 | 7.8 | | |
| 5401 | | | | 0.09 | 0.48 | | | 8.3 | | |
| 5403 | | | | 0.052 | 0.2 | | | 7.5 | | |
| 5425 | | | | 643 | 1.2 | | | 2271 | | |
| 5426 | | | | 46 | 0.39 | | | 645 | | |
| 5453 | | | | 6.6 | | 5.8 | | 108.3 | | |
| 5454 | | | | | 98.9 | | | | | 83.3 |
| 5455 | | | | | | | | | | |
| 5457 | | | | 0.21 | | | | 1.6 | | 58 |
| 5493 | | | | | | | 13 | | | |
| 5497 | | | | | 0.163 | | | | | |
| 5498 | | | | 0.5 | | | | 0.8 | | 57 |
| 5499 | | | | 0.02 | | | | 3 | | |
| 5522 | | | | | | | | | | 61.7 |
| 5527 | | | | | 0.2 | | | | | |
| 5528 | | | | | | | | 2.9 | | |
| 5550 | | | | | 151.6 | | | | | 175 |
| 5561 | | | | 0.11 | 2.3 | | | 27 | | |
| 5562 | | | | 0.084 | 1.3 | | | 3.7 | | |
| 5567 | | | | | 0.8 | | | | | |
| 5568 | | | | | 0.8 | | | | | |
| 5581 | | | | | 0.8 | | | | | |
| 5582 | | | | 0.064 | 0.5 | | | 10 | | |
| 5598 | | | | | 0.3 | | | 7.6 | | |
| 5603 | | | | | 12.2 | | | | | 1000 |
| 5604 | | | | | 44.8 | | | | | 1000 |
| 5626 | | | | | 0.2 | | | | | |
| 5666 | | | | 0.16 | | | | 0.43 | | 1000 |

### Example 3: Mouse Food Intake Assay

The hybrid polypeptides of the invention may be tested for appetite suppression in the mouse food intake assay and for their effect on body weight gain in diet-induced obesity (DIO) mice. The experimental protocols for the screens are described below.

Female NIH/Swiss mice (8-24 weeks old) are group housed with a 12:12 hour light:dark cycle with lights on at 0600. Water and a standard pelleted mouse chow diet are available ad libitum, except as noted. Animals are fasted starting at approximately 1500 hrs, 1 day prior to experiment. The morning of the experiment, animals are divided into experimental groups. In a typical study, n=4 cages with 3 mice/cage.

At time=0 min, all animals are given an intraperitoneal injection of vehicle or compound, typically in an amount ranging from about 10 nmol/kg to 75 nmol/kg, and immediately given a pre-weighed amount (10-15g) of the standard chow. Food is removed and weighed at various times, typically 30, 60, and 120 minutes, to determine the amount of food consumed (Morley, Flood et al., Am. J. Physiol. 267: R178-R184, 1994). Food intake is calculated by subtracting the weight of the food remaining at the *e.g*., 30, 60, 120, 180 and/or 240 minute time point, from the weight of the food provided initially at time=0. Significant treatment effects are identified by ANOVA (p<0.05). Where a significant difference exists, test means are compared to the control mean using Dunnett's test (Prism v. 2.01, GraphPad Software Inc., San Diego, California).
Activity in the food intake assay and sequence of parent molecules used for the synthesis of hybrids herein are:

| | | | | *Mouse Food Intake, % basal* | | | | |
|---|---|---|---|---|---|---|---|---|
| *Description* | *mpd* # | *60 min ED50 (nmol*/*kg)* | *Sequence* | *30min* | *60min* | *120 min* | *180 min* | *Dose* |
| PYY(3-36) | 1 | 3 | IKPEAPGEDASPEELN RYYASLRHYLNLVTR QRY-NH2 (SEQ ID NO: 58) | -31 | -38 | -40 | -26 | 10 nmol/ Kg |
| Exendin-4 | | 5 | HGEGTFTSDLSKQME EEAVRLFIEWLKNGG PSSGAPPPS-NH2 (SEQ ID NO: 66) | -41 | -60 | -61 | -60 | 4.8 nmol/ Kg |
| Exendin-4 (1-28) | 11 | 0.3 | HGEGTFTSDLSKQME EEAVRLFIEWLKN-NH2 (SEQ ID NO: 237) | -50 | -62 | -49 | -49 | 16.3 nmol/ Kg |
| Exendin-4 (1-28) [Ala5, Leul4, Phe25] | 12 | 13 | HGEGAFTSDLSKQLE EEAVRLFIEFLKN-NH2 (SEQ ID NO: 240) | -53 | -61 | -50 | -53 | 16.7 nmol/ Kg |
| Rat Amylin | | 9 | KCNTATCATQRLANF LVRSSNNLGPVLPPTN VGSNTY-NH2 (SEQ ID NO: 44) | -58 | -40 | -36.5 | -35.5 | 25 nmol/ Kg |
| hAmylin(1-7)-^{11,18}Arg-sCT(8-27)-Amylin(33-37) | 10 | 26 | KCNTATCVLGRLSQE LHRLQTYPRTNTGSN TY-NH2 (SEQ ID NO: 396) | -60 | -47 | -42.5 | -32 | 25 nmol/ Kg |
| CCK-8 | | 26 | DY(S03) MGWMDF-NH2 (SEQ ID NO: 55) | -92 | -56 | -27 | | 10 nmol/ Kg |

### Example 4: Body Weight. Fat Redistribution, and Lean Body Mass Assays

Assays for body weight and related effects can be performed as follows.

Diet-induced obesity (DIO) in the in the Sprague-Dawley rat is a valuable model for the study of obesity and regulation of energy homeostasis. These rats were developed from a line of (Crl:CD®(SD)BR) rats that are prone to become obese on a diet relatively high in fat and energy. See, for example, Levin (1994) Am. J. Physiol. 267:R527-R535, Levin et al. (1997) Am. J. Physiol. 273:R725-R730. DIO male rats are obtained from Charles River Laboratories, Inc. (Wilmington, MA). The rats are housed individually in shoebox cages at 22 °C in a 12/12-hour light dark cycle. Rats are maintained ad-libitum on a moderately high fat diet (32% kcal from fat; Research Diets D1226B). The animals typically achieve a mean body weight of about 500 g. Levin DIO rats are habituated to caging environment for 7 days. During the 3 nights of habituation, animals receive a single intraperitoneal (IP) injection of vehicle. On test day, rats are administered a single IP injection of compound or vehicle (10% DMSO), at the onset of the dark cycle for 6-14 consecutive nights. Food intake is measured by an automated food intake measuring system (BioDAQ, Research Diets) at 5 sec intervals throughout the course of the study. Body weight is recorded nightly.

Body composition was measured prior to and after drug treatment using NMR (Echo Medical Systems, Houston, TX). For body composition measurements, rats are briefly placed (∼1 min) in a well-ventilated plexiglass tube that was then inserted into a specialized rodent NMR machine. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment % body fat at baseline = change in % body fat). All data are represented as mean ± SEM. Analysis of variance (ANOVA) and post-hoc tests were used to test for group difference. A P-value <0.05 was considered significant. Statistical analysis and graphing were performed using PRISM® 4 for Windows (GraphPad Software, Inc., San Diego, CA). In some early studies, SYSTAT® for Windows (Systat Software, Inc., Point Richmond CA) was used for analysis. For hybrids containing an amylinomimetic, an exendin, a PYY analog and/or a leptin, changes in body fat are not accompanied by significant decreases in lean tissue. Graphs and results are typically presented as vehicle-corrected changes in percent body weight, body fat and changes in body protein

In other experiments, male C57BL/6 mice (4 weeks old at start of study) are fed high fat (HF, 58% of dietary kcal as fat) or low fat (LF, 11% of dietary kcal as fat) chow. After 4 weeks on chow, each mouse is implanted with an osmotic pump (Alzet # 2002) that subcutaneously delivers a predetermined dose of hybrid polypeptide continuously for two weeks. Body weight and food intake are measured weekly (Surwit et al., Metabolism-Clinical and Experimental, 44: 645-51, 1995). Effects of the test compound are expressed as the mean +/- sd of % body weight change (*i.e*., % change from starting weight) of at least 14 mice per treatment group (p<0.05 ANOVA, Dunnett's test, Prism v. 2.01, GraphPad Software Inc., San Diego, California).

ExendinlPYY Hybrids. Exemplary hybrid polypeptides of the disclosure were synthesized using a C-terminally truncated exendins (*e.g*., exendin-4(1-28) or ⁵Ala,¹⁴Leu,²⁵Phe-exendin-4(1-28)) and an N-terminally truncated PYY spanning the 18-36 to 31-36 regions. As such, the exemplary hybrid polypeptides generally comprise two modules, wherein the first module is a fragment of an exendin-4 analog and the second module is a peptidic enhancer selected from PYY truncations. For comparison, a β-alanine dipeptide spacers were also incorporated between the peptide building blocks in several variants (see Table 4-1).

**Table 4-1: Exendin/PYY Hybrids, Receptor Binding Data, and Their Effects in the Food Intake Assay**

| | | *Receptor Binding (IC50) nM* | | *Mouse Food Intake % basal* | | | |
|---|---|---|---|---|---|---|---|
| *Description* | *Cmpd #* | *Y2* | *GLP1R* | *30min* | *60min* | *120min* | *Dose* |
| PYY(3-36) (SEQ ID NO: 58) | | 0.04 | | -31 | -38 | -40 | -26 |
| ⁵Ala,¹⁴Leu,²⁵Phe-exendin-4(1-28) (SEQ ID NO: 240) | | - | 1.9 | -50 | -62 | 49 | -49 |
| ⁵Ala,¹⁴Leu,²⁵Phe-exendin-4(1-17)-PYY(18-36) (SEQ ID NO: 16) | - | 1.7 | 1000 | -4 | -11 | -10 | 10 nmol/Kg |
| ⁵Ala,¹⁴Leu,²⁵Phe-exendin-4(1-28)-PYY(22-36) (SEQ ID NO: 14) | - | 17 | 2.1 | 21 | 9 | -4 | 10 nmol/Kg |
| ⁵Ala,¹⁴Leu,²⁵Phe-exendin-4(1-28)-βAla-βAla-PYY(22-36) (SEQ ID NO: 17) | - | 9 | 0.81 | -3 | -5 | -22 | 10 nmol/Kg |
| ⁵Ala,¹⁴Leu,²⁵Phe-exendin-4(1-28)-PYY(25-36) (SEQ ID NO: 15) | 2 | nd | nd | 8 | -13 | -30 | 10 nmol/Kg |
| ⁵Ala,¹⁴Leu,²⁵Phe-exendin-4(1-28)- βAla-βAla-PYY(25-36) (SEQ ID NO: 18) | 3 | 13 | 0.22 | -9 | -25 | -42 | 10 nmol/Kg |
| ⁵Ala,¹⁴Leu,²⁵Phe-exendin-4(1-28)- βAla-βAla-PYY(31-36) (SEQ ID NO: 19) | 4 | 1000 | 0.25 | -14 | -36 | -52 | 10 nmol/Kg |
| exendin-4(1-28)-PYY(25-36) (SEQ ID NO: 8) | - | 16 | 0.29 | -30 | -37 | -45 | 10 nmol/Kg |
| exendin-4(1-28)- βAla-βAla-PYY(25-36) (SEQ ID NO: 5) | - | 7.8 | 0,16 | -24 | -40 | -52 | 10 mnol/Kg |
| exendin-4(1-28)-βAla-βAla-PYY(31-36) (SEQ ID NO: 6) | - | 1000 | 0.19 | -49 | -56 | -61 | 10 nmol/Kg |

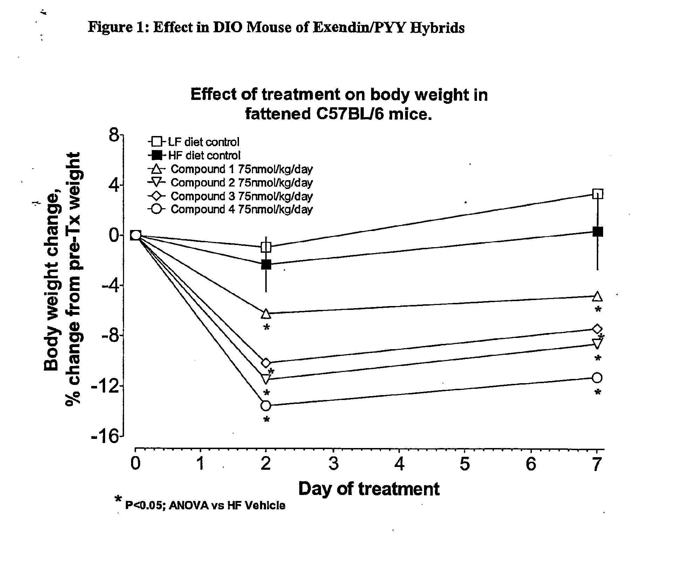

As shown in Table 4-1, certain exemplary compounds of the disclosure showed efficacy in the food intake assay. Certain peptides were also tested at 75 nmol/kg in the DIO assay and proved to be more efficacious than PYY (Figure 1). As observed for other hybrids herein, hybrids can retain binding to one, two or more receptors that recognize the parent molecules. Hybrids were designed that recognize at least one receptor from each parent or from only one parent, as desired. As observed for other hybrids herein, use of a linker (which can act as a spacer between each adjacent hormone portion) can provide increased activity, including receptor(s) binding and in vitro and in vivo activity, such as weight loss. The results herein indicate that a C-terminal portion of PYY can modulate activity.

Exendin/Amylin Hybrids. Exendin/Amylin Hybrids. Exemplary hybrid polypeptides of the invention were prepared from C-terminally truncated exendin (1-27) (SEQ ID NO: 236), C-terminally truncated amylin peptides (*e.g*., amylin(1-7) (SEQ ID NO: 217), ^{2,7}Ala-Amylin(1-7) (SEQ ID NO: 285), and Amylin(33-27) (SEQ ID NO: 243), and optional sCT fragments (e.g., sCT(8.10), ^{11,18}Arg-sCT(8-27) (SEQ ID NO: 289) and ¹⁴Glu,^{11,18}Arg-sCT(8-27) (SEQ ID NO: 286). Whereas both hybrid polypeptides were very active in appetite suppression (see Table 4-2), superior to the same dose of rat amylin, the onset of action differed from the activity profiles of the parent molecules (data not shown). At a dose of 1 nmol/kg, Compound 5 was as effective as rat amylin.

**Table 4-2: Exendin/Amylin Hybrids and Their Effect in the FI Assay**

| | | *Receptor Binding Assay* | | | | *Mouse Food Intake % basal* | | | |
|---|---|---|---|---|---|---|---|---|---|
| *Description* | *Cmpd#* | *GLP-1* | *Amylin* | *CGRP* | *CT* | *30min* | *60min* | *120min* | *Dose* |
| Extendin-4(1-27)-Amylin(1-7)-^{11,18}Arg-sCT(8-27)-Amylin(33-37) | 5 (SEQ ID No. 25) | | | | | -24 | -40 | -48 | 25 nmol/Kg |
| Exendin-4(1-27)-^{2,7}Ala-Amylin(1-7)-sCT(8-10) | 6 (SEQ ID NO. 26) | | | | | -40 | -59 | -66 | 25 nmol/Kg |
| Exendin-4(1-28)-betaAla-betaAla-Amylin(1-7)-11,18Arg-sCT(8-27)-Amylin(33-37) | 14 (SEQ ID NO: 33) | 0.3 | 0.2 | 113 | 0.1 | -5 | -30 | -51 | 3 nmol/Kg |
| Exendin-4(1-28)-Gly-Gly-Gly-Amylin(1-7)-11,18Arg-sCT(8-27)-Amylin(33-37) | 15 (SEQ ID NO: 312) | 0.4 | 0.2 | 63 | 0.03 | -8 | -36 | -51 | 3 nmol/Kg |
| Exendin-4(1-27)-Amylin(1-7)-11,18Arg-sCT(8-27)-Amylin(33-37) | 5 (SEQ ID NO: 25) | 1.7 | 0.6 | 178 | 0.5 | -20 | -10 | -26 | 3 nmol/Kg |

Both compounds also showed excellent efficacy when screened in the DIO assay (Figure 2).

Further exemplary compounds were assayed for effect on blood glucose levels and in a food intake assay. These tests included compounds 14 and 15. Compound 14, which includes a betaAla linker, is ²⁹βAla-βAla-Exendin(1-28),hAmylin(1-7)-^{11,18}Arg-sCt(8-27)-hAmylin(33-37) (SEQ ID NO: 33), (alternatively written as Exendin(1-28)-βAla-βAla-hAmylin(1-7)-^{11,18}Arg-sCt(8-27)-hAmylin(33-37), while Compound 15 contains a Gly linker: ²⁹GlyGlyGly-Exendin(1-28),hAmylin(1-7)^{11,18}Arg-sCt(8-27)-hAmylin(33-37) (SEQ ID NO: 312). The longer exendin(1-28) provided increased activity compared to exendin(1-27).

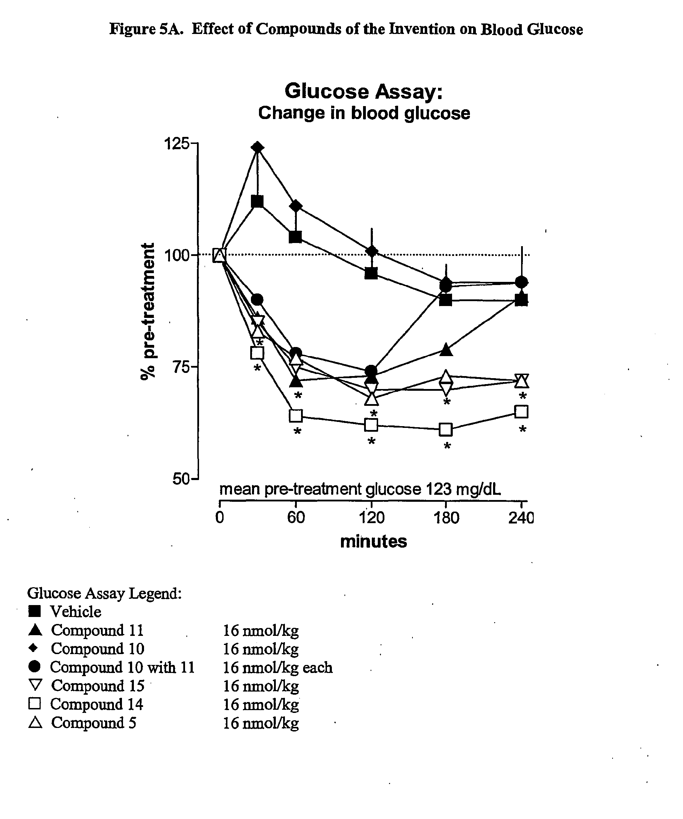

A Blood Glucose Assay was performed to test effect on lowering blood glucose levels. Female NIH/Swiss mice (8-20 weeks old) were group housed with a 12:12 hour light:dark cycle with lights on at 0600. Water and a standard pelleted mouse chow diet were available ad libitum, except as noted. The morning of the experiment, animals were divided into experimental groups and fasted starting at approximately 0630 hrs. In a typical study, n=2 cages with 3 mice/cage. At time=0 min, a blood glucose sample was taken and immediately followed by an intraperitoneal injection of vehicle or compound in an amount ranging from about 1 nmol/kg to 25 nmol/kg. Blood glucose was measured at 30, 60, 120, 180, and 240 min. Percent pre-treatment was calculated by dividing the blood glucose at the e.g., 30, 60, 120, 180 and/or 240 minute time point by the blood glucose at time=0 min. Significant treatment effects were identified by ANOVA (p<0.05). Where a significant difference exists, test means were compared to the control mean using Dunnett's test (Prism v. 4.01, GraphPad Software Inc., San Diego, California). The results on exemplary compounds are presented in Figure 5A. Points represent mean ± sd. Peptide was injected intraperitoneally (IP) at t=0 immediately following baseline sample into 2-hour fasted NIH/Swiss mice. Samples were taken at t= 30, 60, 120, 180 and 240 min. Blood glucose was measured with a OneTouch® Ultra® (LifeScan, Inc., a Johnson & Johnson Company, Milpitas, CA). * p<0.05 vs. vehicle control; ANOVA, Dunnett's test.

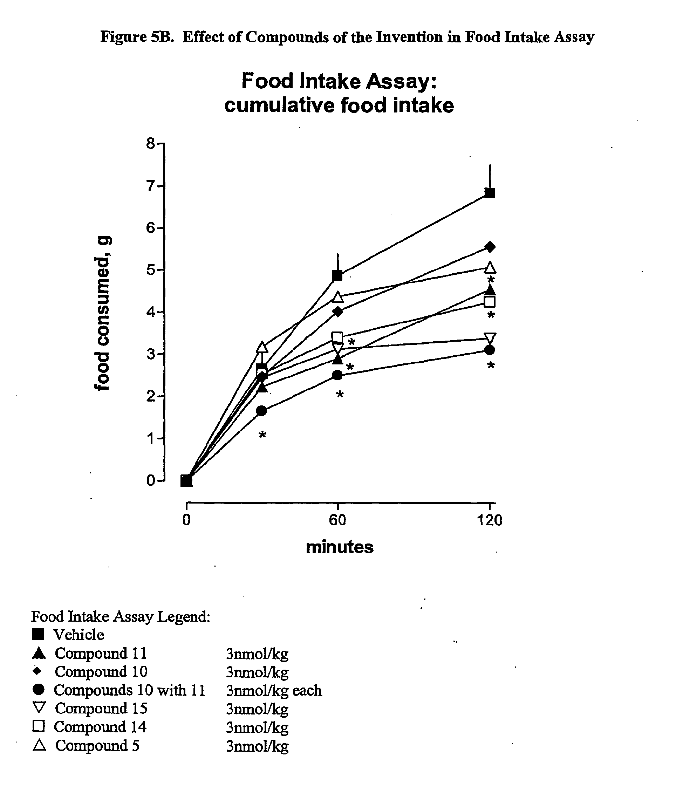

A food intake assay was performed as previously described herein. The results are presented in Figure 5B. Points represent mean ± sd of n=4 cages (3 mice/cage). Peptide was injected intraperitoneally (IP) at t=0 into overnight-fasted NIH/Swiss mice. Food was introduced immediately after injection and amount consumed measured at t=30, 60, and 120 min. * p<0.05 vs. vehicle control; ANOVA, Dunnett's test.

Parental Compound 10 and exendin compounds have opposing effects in the Glucose Assay. One would expect that joining them would result in counteracting effects or, at best, a dilution of the effect seen with the more potent parent compound. However, in the Glucose Assay, the exemplary hybrid compounds were just as efficacious as exendin(1-28) and had a longer duration of action.

From the food intake data, the exemplary compounds were anorexigenic. Activity was generally better than the parent compounds dosed individually. Activity was comparable to the parent compounds dosed together but at half the concentration of drug (3 nmol/kg hybrid versus 6 nmol/kg total for co-dosed parent compounds); thus further demonstrating hybrid superiority. The addition of a linker, increased activity of the hybrids. The Gly-Gly-Gly linker was more effective than the betaAla-betaAla linker in this case.

Exendin/CCK-8 Hybrids. Exemplary hybrid polypeptides of the disclosure were prepared from full length or C-terminally truncated exendin-4 attached to the N-terminus of CCK-8 either directly or *via* a linker, preserving the N-terminal amide of the CCK-8. (Table 4-3). Further, certain hybrids were prepared incorporating the naturally occurring Tyr(SO₃), while another hybrid incorporating the more stable Phe(CH₂SO₃) group was prepared. All the prepared hybrid polypeptides were active in inhibiting food intake (Table 4-3).

**Table 4-3: Exendin/CCK-8 Hybrids and Their Effect in the Food Intake Assay**

| | | | *Receptor Binding (IC50) nM* | *Mouse Food Intake* % *basal* | | | |
|---|---|---|---|---|---|---|---|
| *Description* | *Cmpd#* | | *GLP1-R* | *30min* | *60min* | *120min* | *Dose* |
| Exendin-4(1-28) amide | 11 (SEQ ID NO: 237) | | 0.63 | | | | |
| Exendin-4-CCK-8 | - (SEQ ID NO. 20) | | | -12 | -28 | -28 | 10 nmol/ Kg |
| Exendin-4(1-28)-CCK-8 | 7 (SEQ ID NO. 21) | | 75 | -20 | -36 | -45 | 10 nmol/ Kg |
| Exendin-4(1-28)-CCK-8 [Phe(CH₂SO₃)] | 8 (SEQ ID NO. 22) | | 31 | -24 | -47 | -66 | 10 nmol/ Kg |
| Exendin-4(1-28)- [8-amino- 3,6-dioxaoctanoyl]-CCK-8 | 9 (SEQ ID NO. 23) | | | -12 | -28 | -40 | 10 nmol/ Kg |

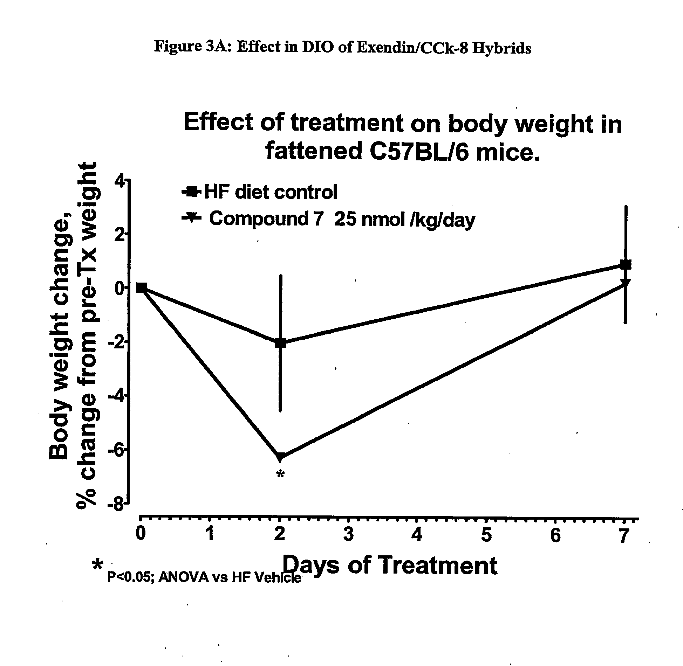

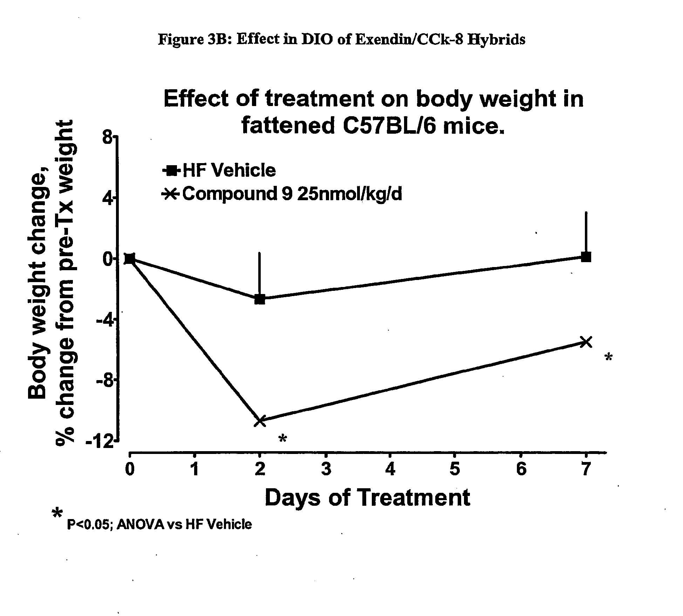

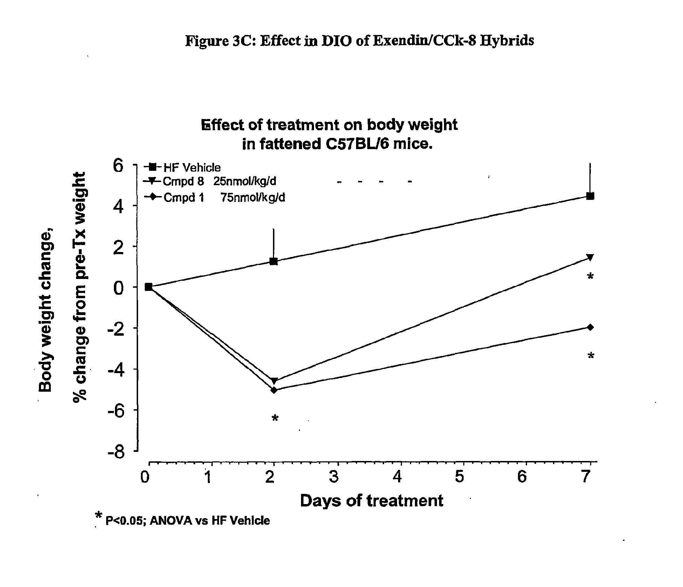

Exemplary exendin/CCK-8 hybrid polypeptides were tested in the DIO assay at 25 nmol/kg (Figures 3A and 3B). The data shows an initial weight loss, followed by a rebound effect in all compounds. Interestingly, the rebound effect appears to be diminished in hybrids incorporating the more hydrolytically stable Phe(CH₂SO₃) residue (compare Figures 3A and 3C), as well as hybrids incorporating a linker, for example the linker 8-amino-3,6-dioxaoctanoyl, between the exendin and the CCK residues (compare Figures 3A and 3B). A ten-fold greater amount of CCK-8 (250 nmol/kg/day) was needed to produce about a -2.8% change at day 2, which rebounded to the HF diet control level at day 7.

Amylin/PYY Hybrids. An Amylin/PYY hybrid polypeptide was synthesized that contained truncated segments of each peptide. *In-vivo* activity in the food intake assay is shown in Table 4-4.

**Table 4-4: Anaylin/PYY Phybrid**

| | Mouse Food Intake % Basal | | | |
|---|---|---|---|---|
| Description | 30min | 60min | 120min | Dose |
| Amylin(1-18)-PYY(19-36) (SEQ ID NO. 38) | -13 | -14 | -13 | 25 nmol/Kg |

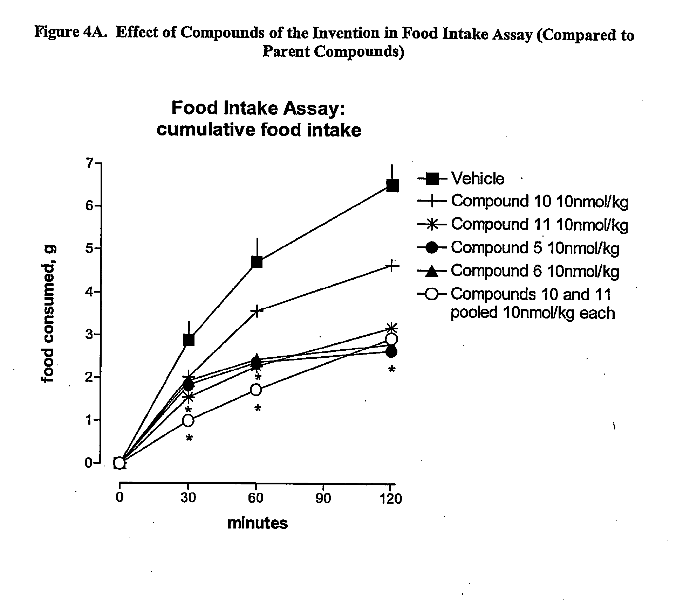

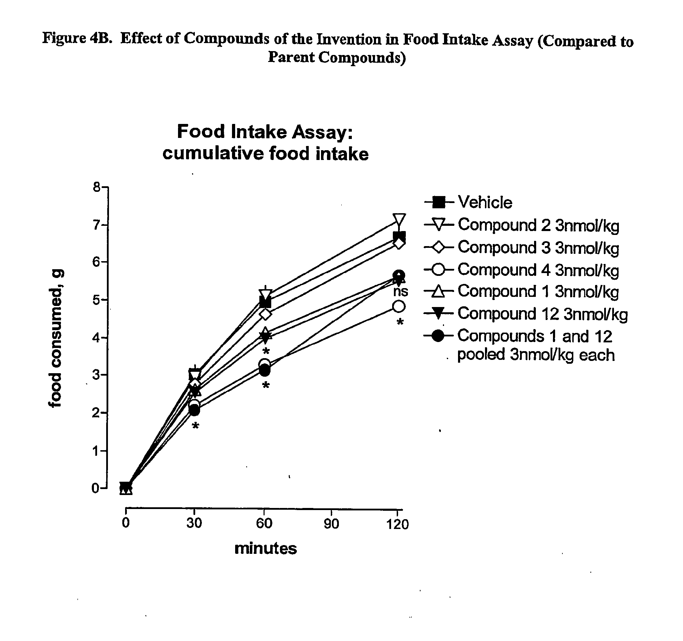

To ascertain if exemplary hybrid polypeptides of the invention are more potent than their parent component peptide hormones, exemplary compounds were tested in the food intake assay at the minimum efficacious dose of the more active parent molecule. The results are shown in Figures 4A and 4B, which also compares the effects of pooled parent peptides (Compounds 1, 11, and 12 are component peptide hormones, analogs or fragments thereof). The data indicate that several peptides are at least as equipotent as the pooled parent peptides. In parallel with the *in vivo* studies, *in vitro* receptor binding and functional assays (cyclase activity) have been performed for all the compounds (data not shown).

Amylin-sCT/leptin hybrids. Further exemplary hybrids were made which contained a leptin peptide fragment joined to Compound 10, an amylin-sCT-amylin chimera described herein. Compound 16 is [Ser117, dLeu119]leptin(116-122)-Amylin(1-7)-[^{11,18}Arg]sCT(8-27)-Amylin(33-37) (SEQ ID NO: 397). The compound bound (RBA = receptor binding assay) the amylin and CT receptors, with some binding to the CGRP receptor. The compound was also able to activate the CT receptor (C1A assay).

| Compound | Cmpd# | Assay | IC50 |
|---|---|---|---|
| [Ser117,dLeu119]leptin(116-122)-Amylin(1-7)-[^{11,18}Arg]sCT(8-27)-amylin(33-37) (SEQ ID NO: 397) | 16 | amylin RBA | 0.04 nM |
| | 16 | CGRP RBA | 81 nM |
| | 16 | CT CYCLASE(C1A) | 2.2 nM |
| | 16 | CT RBA (C1A) | 0.063 nM |
| | 16 | GLP RBA (RIN) | 1000 nM |

This representative molecule was tested for activity in a food intake assay as described herein. Although leptin was not active in this assay, Compound 16 was anorexigenic at 1 mg/kg. Compound 16 was also superior to rat amylin (at 25 nmollkg) in its anorexigenic effect. While Compound 10 reduced food intake 91-95% compared to controls, much more effectively; Compound 16 reduced the cumulative intake to 34-38% that of controls. Further exemplary embosiments include a head-to-head joining of the N-terminus of the leptin peptide with that of the Amylin(1-7)-[^{11,18}Arg]sCT(8-27)-amylin(33-37) compound.

CCK/Amylin-sCT Hybrids. An exemplary hybrid of CCK with an amylin-sCT chimera demonstrated relevant receptor specificity and activation. The exemplary compound having sequence DF(P-CH₂SO₃)MGWMDFGKR KCNTATCATQRLANELVRLQTYPRTNVGSNTY (SEQ ID NO: 398) demonstrated an IC50 of 0.044 nM in a CT receptor binding assay, 4.4nM in a CGRP receptor binding assay, 0.083 nM in an amylin receptor binding assay, and 1000 nM in a GLP Receptor cyclase (RIN) assay.

### Example 5. Reduction of Food Intake, Body Weight and Weight Gain

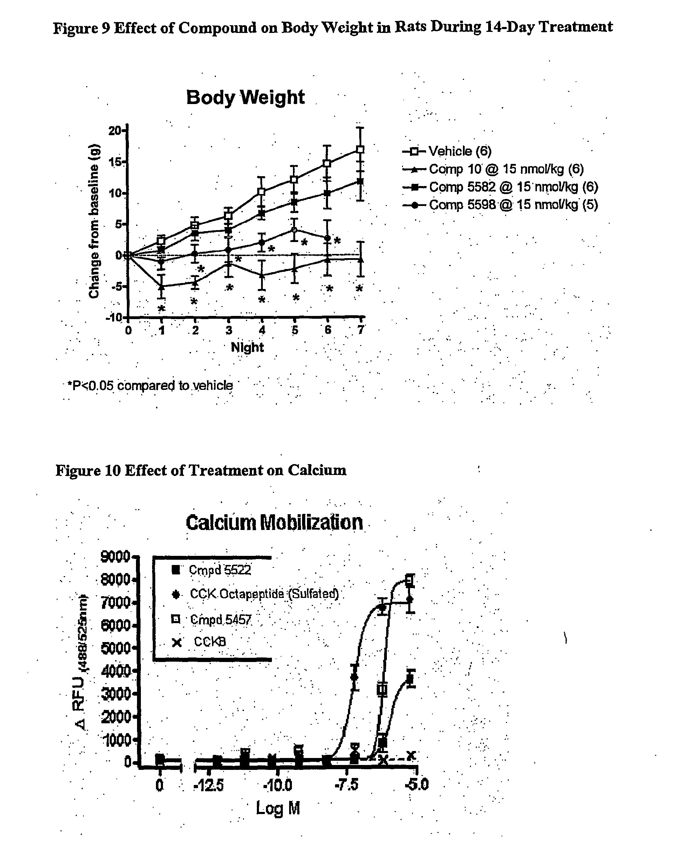

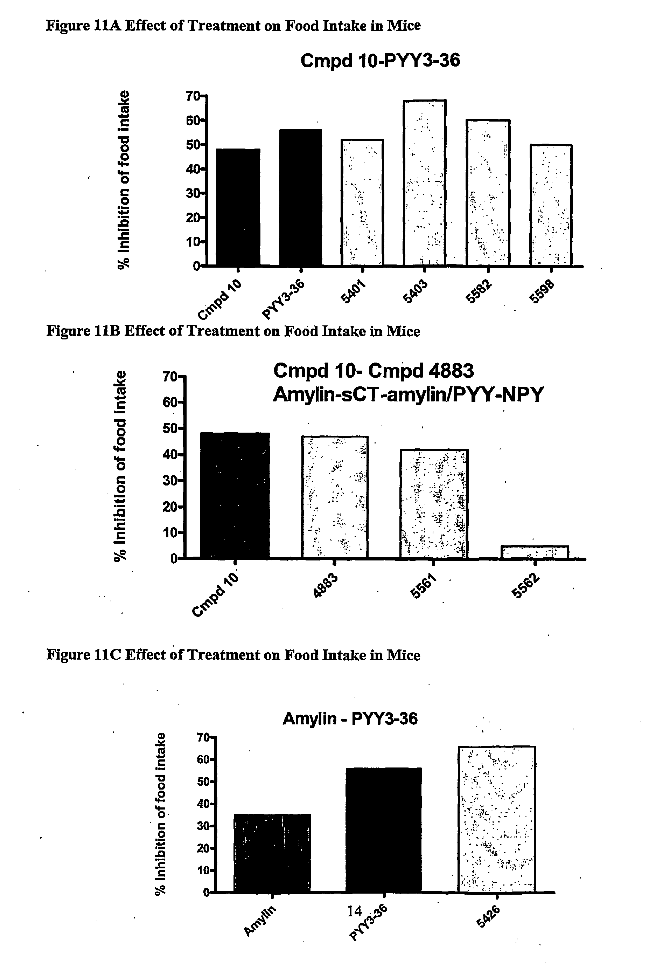

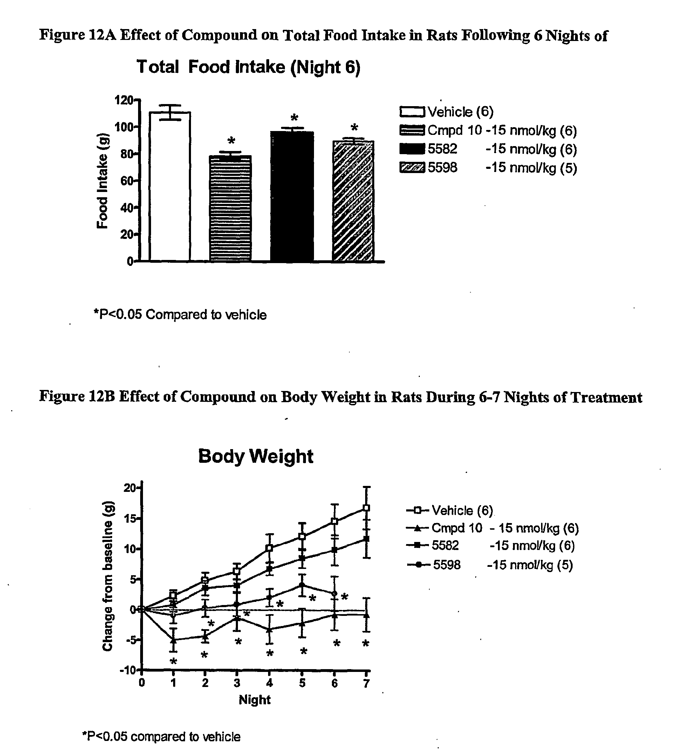

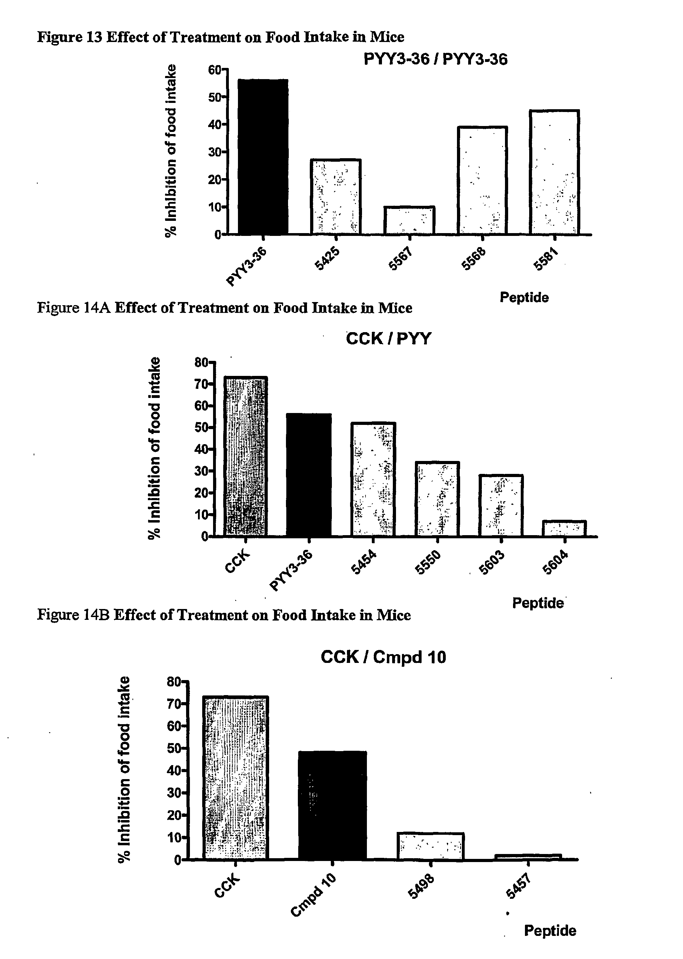

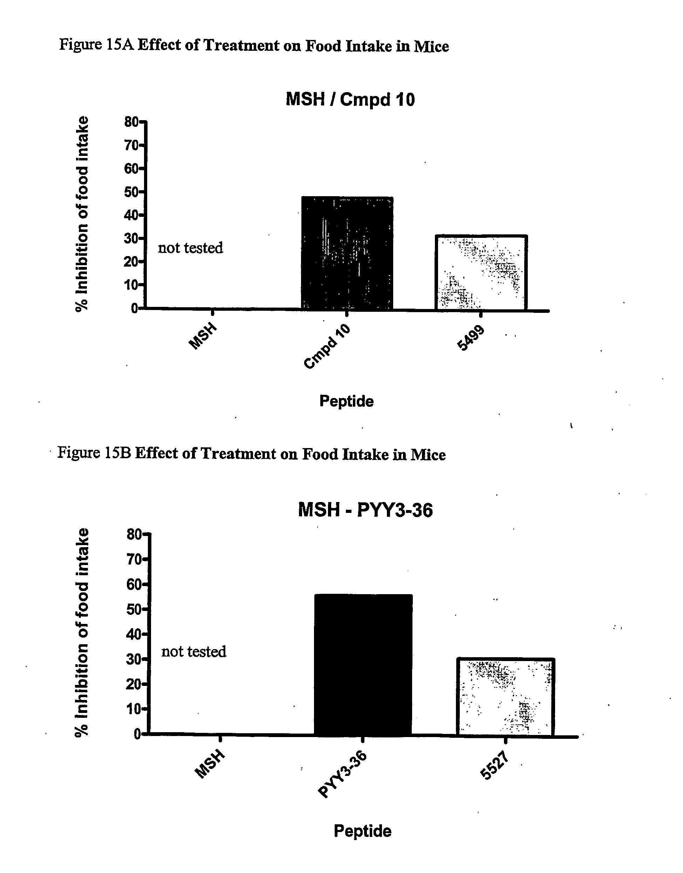

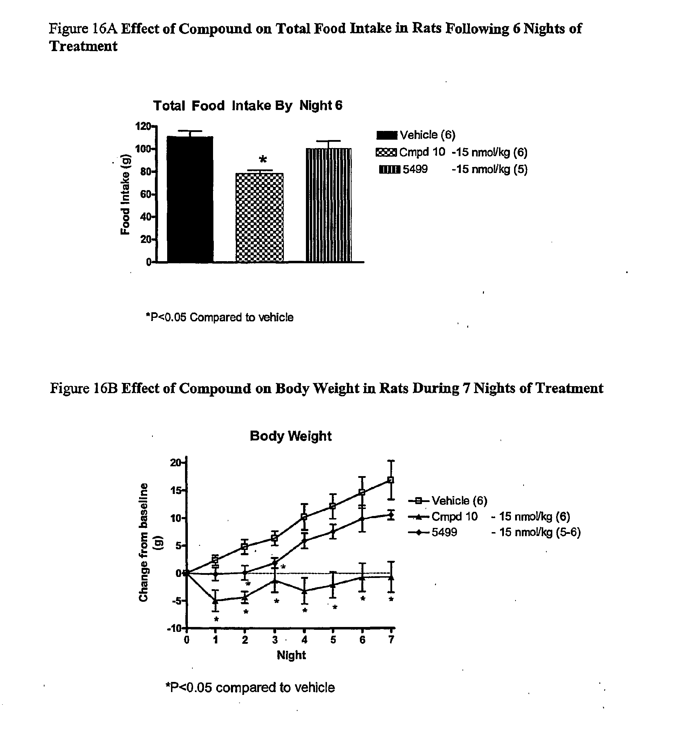

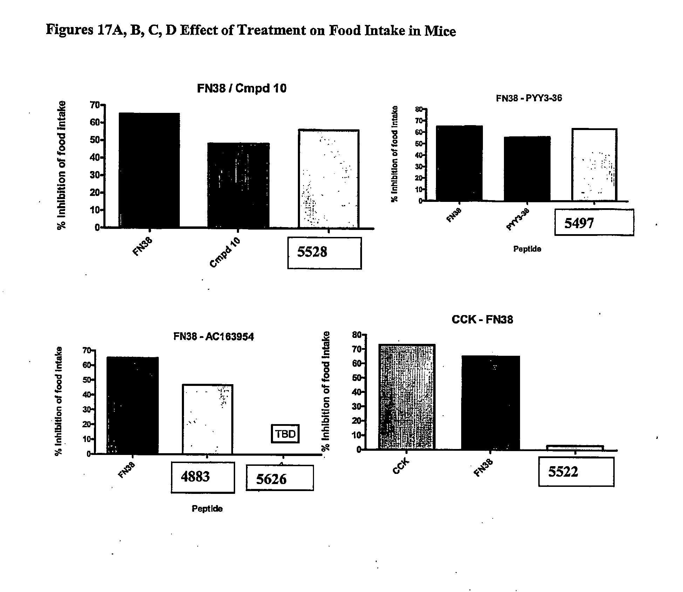

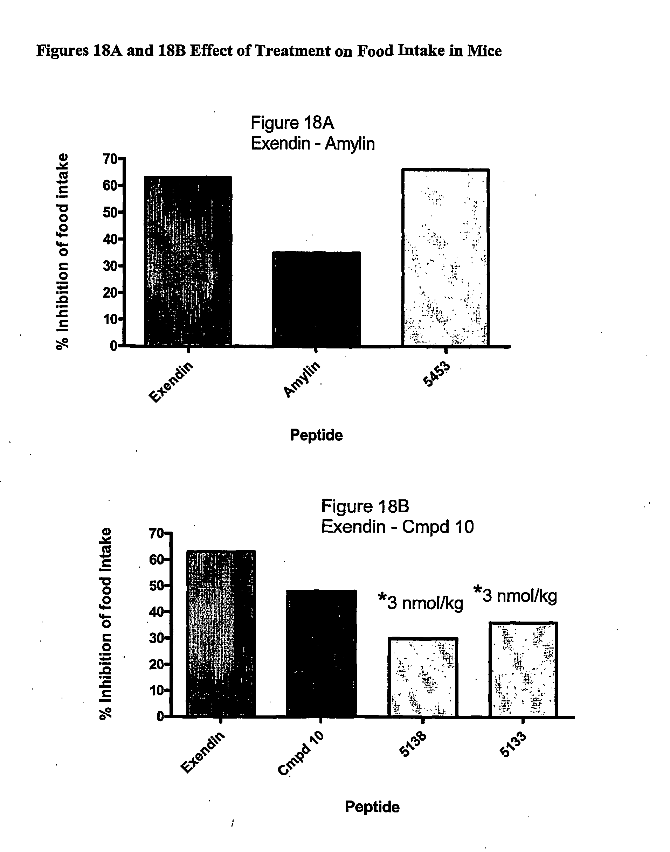

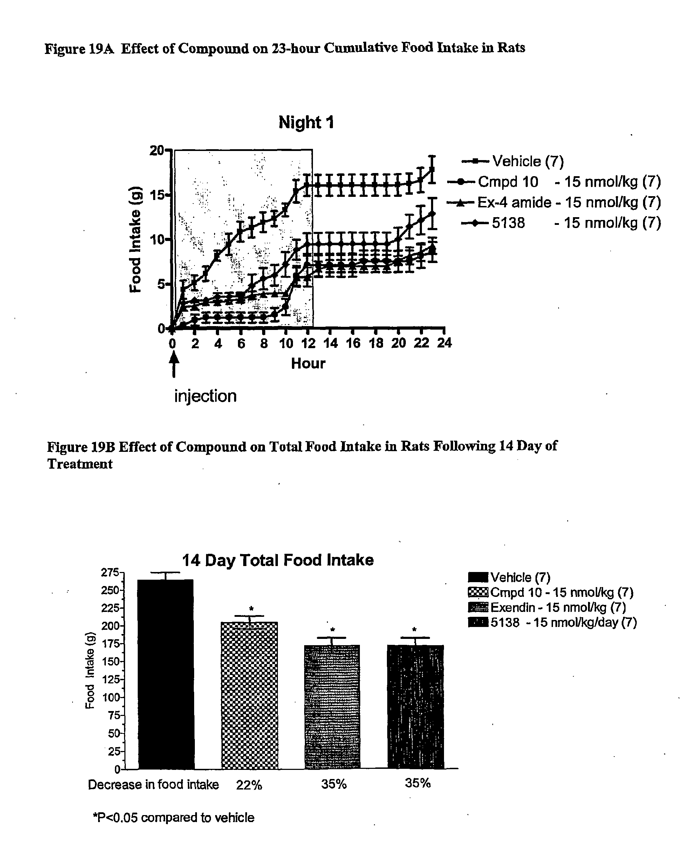

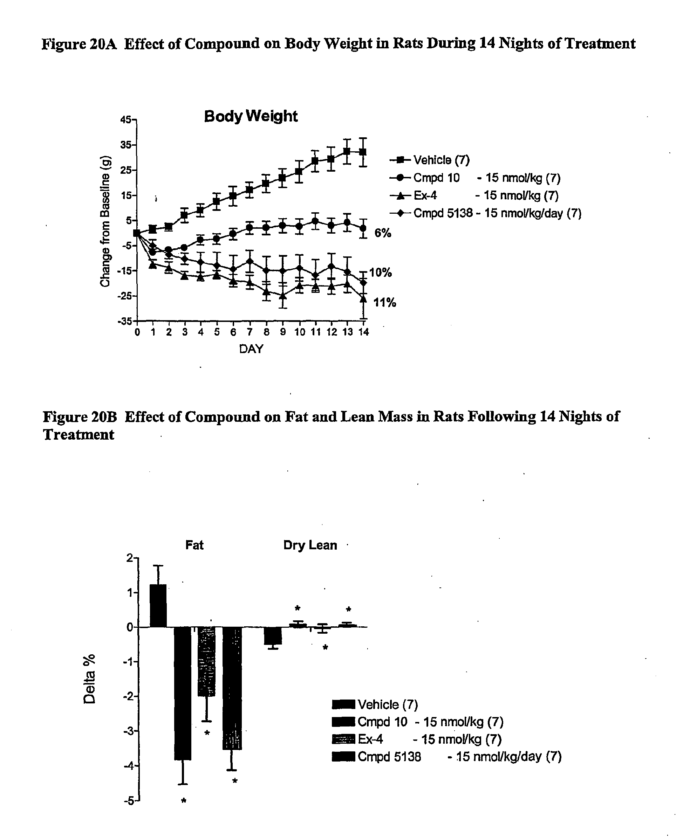

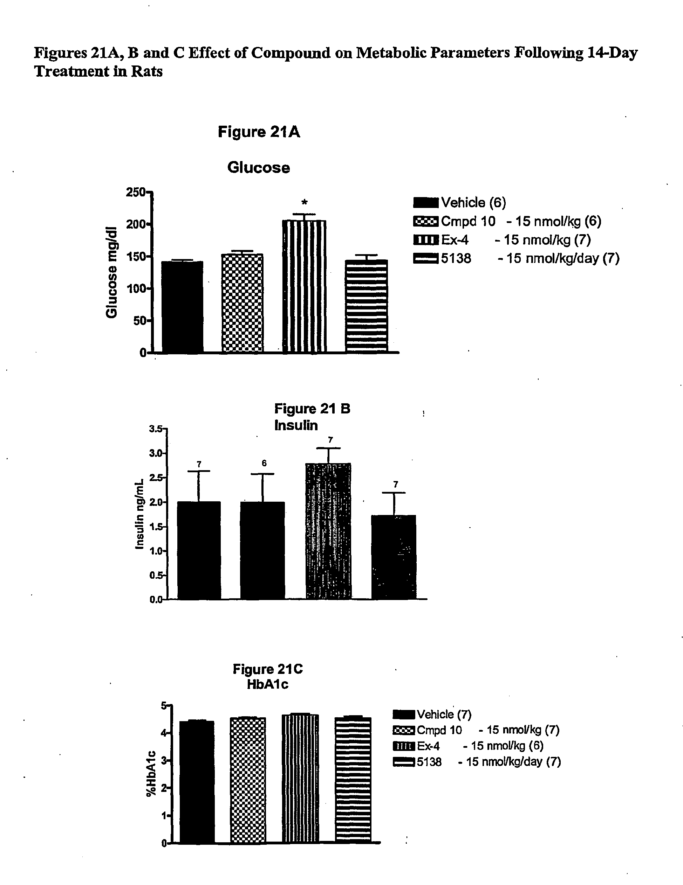

Exemplary hybrid polypeptides of the invention were tested further for appetite suppression, recution of body weight and reduction of body weight gain in mice and rats as decribed herein. Parent compounds were also assayed either alone or in combination. Figures 9-21 demonstrate in vivo actions of exemplary hybrids in the assays as described herein.

In vivo activity of inhibition of food intake in a mouse for exemplary hybrids and their parent compounds is shown in the following table. Values are as provided as percent inhibition of food intake after infusion for the time periods indicated. "ns" means "not significant" compared to vehicle.

| **Compound/Dose** | **Time point (min)** | | | | |
|---|---|---|---|---|---|
| **Rat Amylin** | 30 | 60 | 120 | 180 | 240 |
| 25 nmol/kg | 46 | 35 | 21 | 17 | 7 |
| | | | | | |
| 250 nmol/kg | 70 | 53 | 38 | 30 | |
| | | | | | |
| 300 mnol/kg | 88 | 90 | 51 | 21 | |
| | | | | | |
| **Human NPY** | | | | | |
| 37.5 nmol/kg | 21 | 29 | 0 | 0 | |
| | | | | | |
| **Cmpd 10** | 30 | 60 | 120 | 180 | 240 |
| 10 nmol/kg | 24 | 26 | 27 | 21 | |
| | | | | | |
| 25 nmol/kg | 57 | 46 | 31 | 19 | 9 |
| | | | | | |
| 30 mnol/kg | 64 | 55 | 33 | | |
| 100 nmol/kg | 81 | 89 | 55 | | |
| | | | | | |
| 300 nmol/kg | 93 | 96 | 88 | | |
| | | | | | |
| **Exendin-4 amide** | 30 | 60 | 120 | 180 | 240 |
| 3 nmol/kg | 40 | 48 | 61 | | |
| | | | | | |
| 10 nmol/kg | 46 | 61 | 67 | 67 | |
| | | | | | |
| 30 nmol/kg | 48 | 61 | 68 | | |
| | | | | | |
| **Exendin-4 (1-28) amide** | 30 | 60 | 120 | 180 | 240 |
| 3nmol/kg | 7 | 24 | 16.3 | | |
| | | | | | |
| 10 mnol/kg | 23 | 41 | 41 | | |
| | | | | | |
| 30 nmollkg | 34 | 51 | 60 | | |
| | | | | | |
| 100 nmol/kg | 45 | 60 | 72 | | |
| | | | | | |
| 300 nmol/kg | 41 | 57 | 65 | | |
| | | | | | |
| **PYY(3-36)** | 30 | 60 | 120 | 180 | 240 |
| 3 nmol/kg | n.s. | 7.3 | 10 | 6 | 0 |
| | | | | | |
| 7.5 nmol/kg | 32 | 39 | 34 | 31 | |
| | | | | | |
| 8 nmol/kg | 48 | 40 | 20 | 13 | |
| | | | | | |
| 10 nmol/kg | 28 | 36 | 38 | 30 | 30 |
| | | | | | |
| 25 nmo/kg | 40 | 51 | 54 | 43 | |
| | | | | | |
| 30 nmol/kg | 45 | 33 | 58 | 54 | 46 |
| | | | | | |
| 100 nmol/kg | 41 | 35 | 56 | 56 | 52 |
| | | | | | |
| 300 nmol/kg | 37 | 34 | 52 | 51 | 51 |
| | | | | | |
| **CCK8 sulfated Tyr** | 30 | 60 | 120 | | |
| 3 nmol/kg | 49 | 26 | 0 | | |
| | | | | | |
| 10 nmol/kg | 92 | 56 | 27 | | |
| | | | | | |
| 30 nmol/kg | 92 | 73 | 43 | | |
| | | | | | |
| 100 nmol/kg | 100 | 78 | 59 | | |
| | | | | | |
| **CCK8** | 30 | 60 | 120 | | |
| 1000 µg/kg | ns | ns | ns | | I |
| | | | | | |
| **--HYBRIDS--** | | | | | |
| **4240** | 30 | 60 | 120 | 180 | 240 |
| 10 nmol/kg | 37 | 50 | 60 | | |
| | | | | | |
| 25 nmol/kg | 24 | 40 | 48 | | |
| | | | | | |
| **4277** | 30 | 60 | 120 | 180 | 240 |
| 172 nmol/kg (1000 µg/kg) | 76 | 70 | 50 | | |
| | | | | | |
| NH2-HGEGTFTSDLSKQ MEEEAVRLFIEW LKKANTATAVLG -NH2 | 30 | 60 | 120 | 180 | 240 |
| 10 nmol/kg | 33 | 49 | 57 | | |
| | | | | | |
| 25 mnol/kg | 40 | 59 | 66 | | |
| | | | | | |
| **4487** | 30 | 60 | 120 | | |
| 10 nmol/kg | 0 | 36 | 45 | | |
| | | | | | |
| **4489** | 30 | 60 | 120 | | |
| 3 nmol/kg | ns | 25 | ns | | |
| | | | | | |
| 10 nmol/kg | ns | 47 | 66 | | |
| | | | | | |
| **5133** | 30 | 60 | 120 | 180 | 240 |
| 3 nmol/kg | 7.5 | 36 | 51 | | |
| | | | | | |
| **5138** | 30 | 60 | 120 | 180 | 240 |
| 3 nmol/kg | 4.7 | 30 | 38 | | |
| | | | | | |
| **5401** | 30 | 60 | 120 | 180 | 240 |
| 25 nmol/kg | 32 | 52 | 59 | 57 | |
| | | | | | |
| **5403** | 30 | 60 | 120 | 180 | 240 |
| 25 nmol/kg | 52 | 68 | 65 | 55 | |
| | | | | | |
| **5425** | 30 | 60 | 120 | 180 | 240 |
| 25 nmol/kg | 8 | 27 | 31 | 18 | |
| | | | | | |
| **5426** | 30 | 60 | 120 | 180 | 240 |
| 25 nmol/kg | 49 | 66 | 55 | 45 | |
| | | | | | |
| **5453** | 30 | 60 | 120 | 180 | 240 |
| 25 nmol/kg | 55 | 66 | 71 | 61 | |
| | | | | | |
| **5454** | 30 | 60 | 120 | 180 | 240 |
| 25nmol/kg | 39 | 33 | 28 | 20 | |
| **5455** | 30 | 60 | 120 | 180 | 240 |
| 25 nmol/kg | 28 | 33 | 39 | | |
| | | | | | |
| **5457** | 30 | 60 | 120 | 180 | 240 |
| 25 mnol/kg | ns | ns | ns | | |
| | | | | | |
| **5493** | 30 | 60 | 120 | 180 | 240 |
| 25 mnol/kg | 54 | 59 | 68 | 64 | |
| | | | | | |
| **5497** | 30 | 60 | 120 | 180 | 240 |
| 25 nmol/kg | 54 | 63 | 41 | ns | |
| | | | | | |
| **5498** | 30 | 60 | 120 | 180 | 240 |
| 25 nmol/kg | ns | ns | ns | ns | |
| | | | | | |
| **5499** | 30 | 60 | 120 | 180 | 240 |
| 25 nmol/kg | 40 | 32 | 25 | | |
| | | | | | |
| **5522** | 30 | 60 | 120 | 180 | 240 |
| 25 nmol/kg | | | | | |
| | | | | | |
| **5527** | 30 | 60 | 120 | 180 | 240 |
| 25 nmol/kg | 40 | 32 | 25 | | |
| | | | | | |
| **5528** | 30 | 60 | 120 | 180 | 240 |
| 25 nmol/kg | 56 | 55 | 50 | | |
| | | | | | |
| **5550** | 30 | 60 | 120 | 180 | 240 |
| 25 nmol/kg | 0 | 34 | 0 | | |
| | | | | | |
| **5561** | 30 | 60 | 120 | 180 | 240 |
| 25 nmol/kg | 0 | 42 | 47 | | |
| | | | | | |
| **5562** | 30 | 60 | 120 | 180 | 240 |
| 25 mmol/kg | | | | | |
| | | | | | |
| **5567** | 30 | 60 | 120 | 180 | 240 |
| 25 nmol/kg | 0 | 0 | 0 | | |
| | | | | | |
| **5568** | 30 | 60 | 120 | 180 | 240 |
| 25 nmol/kg | 0 | 39 | 48 | | |
| | | | | | |
| **5581** | 30 | 60 | 120 | 180 | 240 |
| 25 nmol/kg | 0 | 45 | 32 | | |
| | | | | | |
| **5582** | 30 | 60 | 120 | 180 | 240 |
| 25 nmol/kg | 0 | 58 | 57 | | |
| | | | | | |
| **5598** | 30 | 60 | 120 | 180 | 240 |
| 25 nmol/kg | 0 | 50 | 58 | | |
| **5603** | 30 | 60 | 120 | 180 | 240 |
| 25 nmol/kg | 0 | 0 | 0 | | |
| | | | | | |
| **5604** | 30 | 60 | 120 | 180 | 240 |
| 25 nmol/kg | 0 | 0 | 0 | | |

In vivo activity of inhibition of body weight in a mouse DIO model for exemplary hybrids and their parent compounds is shown in the following table. Values are as provided as percent inhibition of body weight after infusion for the time periods indicated.

| | | | Day | | | | | | | | | | | |
|---|---|---|---|---|---|---|---|---|---|---|---|---|---|---|
| Compo und | units | 2 | 7 | 14 | 21 | 28 | 35 | 42 | 49 | 56 | 63 | 70 | 77 | 84 |
| RAT AMYLIN | Nmo 1/kg/d | | | | | | | | | | | | | |
| 30 | | 1.9 | 1.5 | 2.1 | 2.5 | 2.2 | | | | | | | | |
| 75 | | 1.5 | 1.4 | 2.1 | | | | | | | | | | |
| 100 | | 1.6 | 1.8 | 3.1 | 3.8 | 4.4 | 6.9 | 8.6 | 9.6 | 10.4 | 10.3 | 11.4 | 12 | 13.4 |
| 300 | | 1.1 | -0.7 | -0.9 | -1.5 | -1.5 | | | | | | | | |
| | | | | | | | | | | | | | | |
| | | 2 | 7 | | | | | | | | | | | |
| CMPD 10 | nmol/kg/d | | | | | | | | | | | | | |
| 150 | | 2.2 | -2.4 | | | | | | | | | | | |
| | | | | | | | | | | | | | | |
| | | 2 | 7 | 14 | | | | | | | | | | |
| EXEN DIN-4 | nmol/kg/d | | | | | | | | | | | | | |
| 2.5 | | 7 | 6.3 | 7.6 | | | | | | | | | | |
| | | | | | | | | | | | | | | |
| | | | | | | | | | | | | | | |
| PYY(3-36) | nmol/kg/d | 2 | 7 | 14 | | | | | | | | | | |
| 25 | | | 2.6 | 3.1 | | | | | | | | | | |
| 75 | | 4.6 | 4.7 | 5.9 | | | | | | | | | | |
| 100 | | 4.4 | 3.4 | 3.9 | | | | | | | | | | |
| 250 | | | 9 | 10.1 | | | | | | | | | | |
| | | | | | | | | | | | | | | |
| 4240 | nmol/kg/d | 2 | 7 | | | | | | | | | | | |
| 75 | | 9.6 | 10.1 | | | | | | | | | | | |
| | | | | | | | | | | | | | | |
| | | | | | | | | | | | | | | |
| NH2-HGEG TFTSD LSKQ MEEE AVRL FIEWL KKAN TATA VLG-NH2 | nmol/kg/d | 2 | 7 | | | | | | | | | | | |
| 75 | | 11.6 | 12.1 | | | | | | | | | | | |

In vivo activity is demonstrated for inhibition of body weight in a rat DIO model for exemplary hybrids and their parent compounds in the following table. Values are as provided as percent inhibition of body weight after infusion for the time periods indicated.

| | Day | | | | | | | | | | | | |
|---|---|---|---|---|---|---|---|---|---|---|---|---|---|
| Compound/Dose (nmol/kg/d) | 3 | 7 | 14 | 21 | 28 | 35 | 42 | 49 | 56 | 63 | 70 | 77 | 8 4 |
| RAT AMYLIN | | | | | | | | | | | | | |
| 0.7 | | 1.6 | 1.3 | 1.6 | 2.1 | | | | | | | | |
| 1.8 | | 2.2 | 2.2 | 2.2 | 2.2 | | | | | | | | |
| 2.3 | | 4 | 5.4 | 5.4 | 6.2 | | | | | | | | |
| 5.6 | | 2.9 | 3.9 | 4.2 | 4.8 | | | | | | | | |
| 7 | | 5.7 | 7.3 | 7.9 | 8.3 | | | | | | | | |
| 18.4 | | 5.9 | 7.5 | 6.7 | 6.5 | | | | | | | | |
| 23 | | 7.8 | 9.4 | 8.8 | 8.8 | 9.5 | 10 | 9.8 | 9.1 | | | | |
| 69 | | 7.6 | 9.3 | 11.1 | 12.2 | 14.4 | 14.9 | 15.4 | 15.7 | 16.2 | 15.8 | | |
| 70 | | 7.6 | 8.9 | 10.2 | 11.8 | | | | | | | | |
| | | | | | | | | | | | | | |
| | 3 | 7 | 14 | 21 | 28 | 35 | 42 | 49 | 56 | 63 | 70 | 77 | 84 |
| CMPD 10 | | | | | | | | | | | | | |
| 0.29 | | 2.3 | 3.8 | | | | | | | | | | |
| 0.9 | | 5.1 | 7.1 | | | | | | | | | | |
| 2.9 | | 7.7 | 10.0 | 12.0 | 11.5 | 11.6 | 12.1 | 11.7 | 12.3 | | | | |
| 7.5 | | 11.6 | 14.9 | 17.9 | 19.5 | 20.8 | 22.1 | 23.3 | 24.2 | 26.2 | 26 | | |
| 11 | 8.5 | 9.9 | | | | | | | | | | | |
| 69 | | 11.5 | 13.7 | 15.6 | 17 | | | | | | | | |
| 69 | | 11.5 | 13.7 | 15.6 | 17 | | | | | | | | |
| | | | | | | | | | | | | | |
| | 3 | 7 | 14 | 21 | 28 | | | | | | | | |
| EXENDIN-4(1-28) | | | | | | | | | | | | | |
| 0.8 | | h.s. | | | | | | | | | | | |
| 2.6 | | 1.4 | n.s. | n.s. | n.s. | | | | | | | | |
| 8 | | 7.5 | 8.3 | 8.5 | 8.2 | | | | | | | | |
| 11 | 6.5 | 6.8 | | | | | | | | | | | |
| 26 | | 10.4 | 13 | 14.6 | 15.7 | | | | | | | | |
| | | | | | | | | | | | | | |
| | 3 | 7 | 14 | 21 | 28 | 35 | 42 | 49 | 56 | 63 | 70 | 77 | 84 |
| EXENDIN-4 | | | | | | | | | | | | | |
| 0.7 | | 9.6 | 8.4 | 6.3 | 4.3 | | | | | | | | |
| 2.4 | | 12.1 | 10.8 | 10 | 8.4 | | | | | | | | |
| 7 | | 13.9 | 12.8 | 11.4 | 10.6 | | | | | | | | |
| 6.3 | | 12.2 | 13.7 | 14.4 | 14.4 | 19.1 | 18.6 | 18.9 | 18.5 | 19 | 18.1 | | |
| 21 | | 11.9 | 13.2 | 13.3 | 12.6 | | | | | | | | |
| | | | | | | | | | | | | | |
| RAT NMU 24 | 2 | 7 | | | | | | | | | | | |
| 300 | 2.3 | 1.6 | | | | | | | | | | | |
| | | | | | | | | | | | | | |
| | 3 | 7 | 14 | 21 | 28 | | | | | | | | |
| PYY(3-36) | | | | | | | | | | | | | |
| 67 | | 6.7 | 7.3 | 6.2 | 5.8 | | | | | | | | |
| 67 | | 5.5 | 7.3 | 7.8 | 9 | | | | | | | | |
| 67 | | 4.9 | 5.2 | 5 | 4.6 | | | | | | | | |
| | | | | | | | | | | | | | |
| FN-38 | 2 | 7 | | | | | | | | | | | |
| 300 | 3.7 | 2.7 | | | | | | | | | | | |
| | | | | | | | | | | | | | |
| | | | | | | | | | | | | | |
| | 3 | 7 | | | | | | | | | | | |
| 5138 | | | | | | | | | | | | | |
| 11 | 7.5 | 9.3 | | | | | | | | | | | |

In vivo activity is demonstrated for exemplary hybrids and their parent compounds for inhibition of body weight in a rat model (daily injection) in the following table. Values are given as percent inhibition of body weight. Bold value indicates significant from vehicle.

| | Dose | Day 1 | Day 5 | Day 6 | Day 7 | Day 10 | Day 14 |
|---|---|---|---|---|---|---|---|
| RAT AMYLIN | 25 nmol/kg | NS | NS | NS | NS | NS | NS |
| CMPD 10 | 15 nmol/kg | **1.7** | **3.0** | | **3.2** | **4.5** | **6.2** |
| EXENDIN-4 | 15 nmol/kg | **2.8** | **6.1** | | **7.8** | **9.3** | **12.0** |
| 5138 | 15 nmol/kg | **1.3** | **5.2** | | **5.8** | **7.9** | **10.7** |
| 5401 | nmol/kg | | | | | | |
| 5403 | 15 nmol/kg | | | | | | |
| 5426 | 15 nmol/kg | | | | | | |
| 5454 | 15 nmol/kg | | | | | | |
| 5455 | 15 nmol/kg | 0.7 | **2.6** | | **3.2** | | |
| 5493 | 15 nmol/kg | | **3.3** | | **5.1** | | |
| 5499 | 15 mnol/kg | 0.5 | **0.9** | 1 | 1.3 | | |
| 5568 | 15 nmol/kg | 0.5 | 0.5 | | 0.5 | | |
| 5581 | 15 nmol/kg | 0.6 | 0.8 | | 1.2 | | |
| 5582 | 15 nmol/kg | 0.3 | 0.7 | 1 | 1 | | |
| 5598 | 15 nmol/kg | 0.7 | **1.7** | **2.5** | | | |

In vivo activity of reduction of body weight and body weight gain is demonstrated for exemplary hybrids and their parent compounds for inhibition of body weight in a rat model for both infusion over the indicated period and once daily injection over the indicated period, in the following table. Values are given as percent inhibition of body weight. Percent inhibition of food intake over an 8-hour period of night-time feeding on Day 6 was also measured. Hybrids were provided at 15 nmol/kg/day unless otherwise noted. Body Weight: ∼2% difference is statistically significant. Food Intake: ∼20% difference is statistically significant.

| | | **Sustained Infusion** | | **Once Daily Injection** | |
|---|---|---|---|---|---|
| | | ***1 Wk BW Loss*** | | ***1 Wk BW Loss*** | ***FI Inhib* @ *8 hr*** |
| **Parents** | | | | | |
| Exenatide | | 11% | | 8% | 42%-52% |
| Amylin | | 6% | | 0% | 0% |
| Compound 10 | | 11% | | 3% | 32%-57% |
| PYY3-36 | | 0-1% | | 0% | 0% |
| Compound 4883 | | | | nd | nd |
| **PYY-** | | | | | |
| **Amylin** | | | | | |
| 5401 | *PYY-cmpd 10* | 8% | | | |
| 5403 | *PYY-cmpd 10* | 7% | | | |
| 5582 | *PYY-cmpd 10* | | | 1% | 15% |
| 5598 | *PYY-cmpd 10* | | 3% | 3% | 54% |
| 5561 | *PYY*/*NPY-cmpd 10* | 7% | | | |
| 5562 | *PYY-NPY*/*cp10 PPY*/*NPY-amylin* | 9% | | | |
| 5426 | *PYY-amylin* | 1% | | | |
| Exendin-amylin | | | | | |
| 5138 | *EX-cmpd 10* | 10% (11 nmol) | | 6% | 54% |
| 5133 | *EX-cmpd 10* | | | | |
| 5454 | *EX-amylin* | 13% | 12% | | |

### Example 7. Blood Glucose Lowering

In vivo activity is demonstrated for exemplary hybrids and their parent compounds for an blood glucose lowering in an OGTT assay over the periods indicated in Levin rats, in the following table. Values are given as percent reduction of blood glucose level. Bold value indicates significant from vehicle.

| | | | | | %Reduction | | |
|---|---|---|---|---|---|---|---|
| Compound | Dose | Time point (day) | Basal | 15 | 30 | 60 | 12 0 |
| | | | | | | | |
| RAT AMYLIN | 23 nmol/kg/d | day 26 | 4 | na | 2.3 | 19.2 | 2.1 |
| | 69 nmol/kg/d | day 14 | **6** | -0.2 | 11 | 5.9 | 1.2 |
| | | | | | | | |
| CMPD 10 | 11 nmol/kg/d | day 7 | 5.1 | **13** | **14** | **30** | **26** |
| | 69 nmol/kg/d | day 14 | 5 | 7 | 5.5 | 9.8 | 5.9 |
| | | | | | | | |
| EXENDIN-4(1-28) | 7.8 nmol/kg | day 26 | 11.6 | na | -21.4 | 2 | -1.5 |
| | 11nmol/kg/d | day 7 | **15** | -1 | -8 | 5 | -1 |
| | | | | | | | |
| | | | | | | | |
| 5138 | 11nmol/kg/d | day 7 | **13** | -1 | 2.6 | **14** | **16** |

### Example 8. Gastric Emptying Activity

In vivo activity is demonstrated for exemplary hybrids and their parent compounds for gastric emptying in Levin rats over the periods indicated in the following table. Values are given as percent inhibition of gastric emptying and percent acetaminophen passed.

| Compound | Dose | time on study | %passed | %inhibition |
|---|---|---|---|---|
| | | | | |
| RAT AMYLIN | 69 nmol/kg/d | 21 days | 55 | 45 |
| | 100 µg/kg/d | 26 days | 76 | 24 |
| | | | | |
| CMPD 10 | 69 nmol/kg/d | 21 days | 87 | 13 |
| | | | | |
| EXENDIN-4(1-28) | 30 µg/kg/d | 26 days | 94 | 6 |
| | | | | |

### Example 9. PPF Polypeptides Suppress Acute and Chronic Food Intake

Female NIH/Swiss mice (8-24 weeks old) were group housed with a 12:12 hour light:dark cycle with lights on at 0600. Water and a standard pelleted mouse chow diet were available ad libitum, except as noted. Animals were fasted starting at approximately 1500 hrs, one day prior to experiment. The morning of the experiment, animals were divided into experimental groups. In a typical study, n=4 cages with 3 mice/cage.

At time=0 min, all animals were given an intraperitoneal injection of vehicle or compound in an amount ranging from about 10 nmol/kg to 100 nmol/kg, and immediately given a pre-weighed amount (10-15g) of the standard chow. Food was removed and weighed at 30, 60, and 120 min to determine the amount of food consumed (Morley, Flood et al., Am. J. Physiol. 267: R178-R184, 1994). For studies of the acute effects of PPF polypeptides on food intake, the amount of food ingested was calculated by subtracting the weight of the food remaining at the 30, 60, 120 and 180 minute time points, from the weight of the food provided initially at time=0. Similarly, water intake was calculated by subtracting the weight in grams of water remaining at these time points from the weight of the water in grams provided initially. For studies of the effects of PPF polypeptides on longer-term (chronic) food intake and/or body composition, the amount of food consumed over a period of two weeks was measured. For these studies of the effects of PPF polypeptides on longer-term food intake and/or body composition, mice were housed singly for 1 week prior to the beginning of treatment. Throughout the experiments, food intake and body weight were monitored daily. During the treatment period, vehicle (50% dimethylsulfoxide in water) and PYY(3-36) (1 mg/kg/day) were administered by continuous subcutaneous (s.c.) infusion using Alzet® osmotic pumps (Durect Corp., Cupertino, CA; Models 1003D, 2001, & 2004 for 3, 7, & 28 day studies, respectively) placed in the intrascapular region under isoflurane anesthesia. At the end of each study, animals were sacrificed after a 2-4 hr fast by isoflurane overdose. Blood was collected into Na-heparin-flushed syringes by cardiac puncture, and plasma was immediately frozen. In some studies, body composition was determined using dual-energy X-ray absorptiometry (DEXA; PixiMus, GE Lunar). In some studies, body composition (such as the amounts of fat and protein) were measured pre- and post treatment using a rodent NMR machine (EchoMRI-700™), in which animals are placed in a restraining tube and placed in the NMR for 2 minutes, and the amount of fat and lean mass, in grams, is quantified. Bilateral epididymal fat pads and intrascapular brown adipose tissue (BAT) can be dissected out of animals and the weights of these organs determined. Excised liver samples can be placed in RNALater (Ambion, Austin, TX), and stored at -20°C.

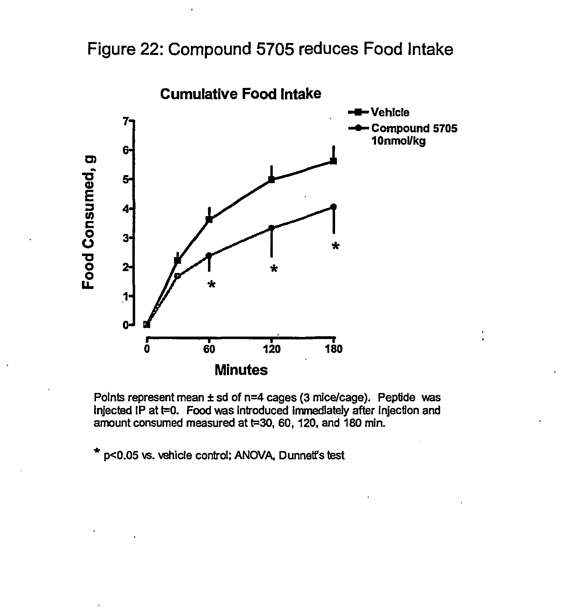

Figures 22-29 show the ability of PPF polypeptide chimeras, particulary PPY-NPY chimeras, for example, Compounds 4883 and 5705, to reduce cumulative food intake in the food intake assay described above.

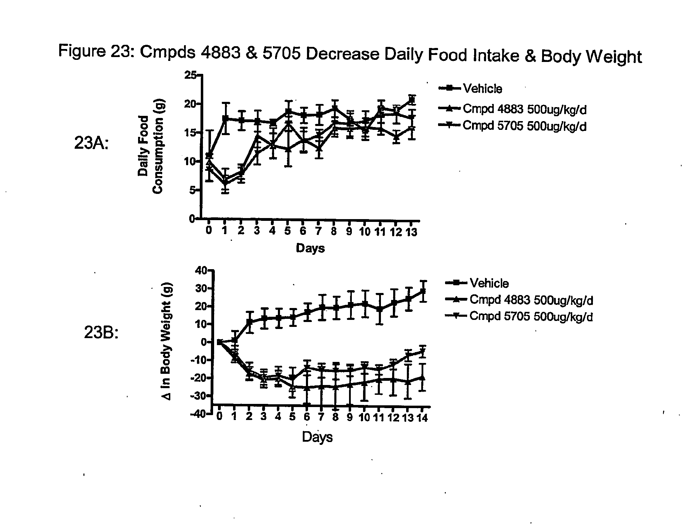

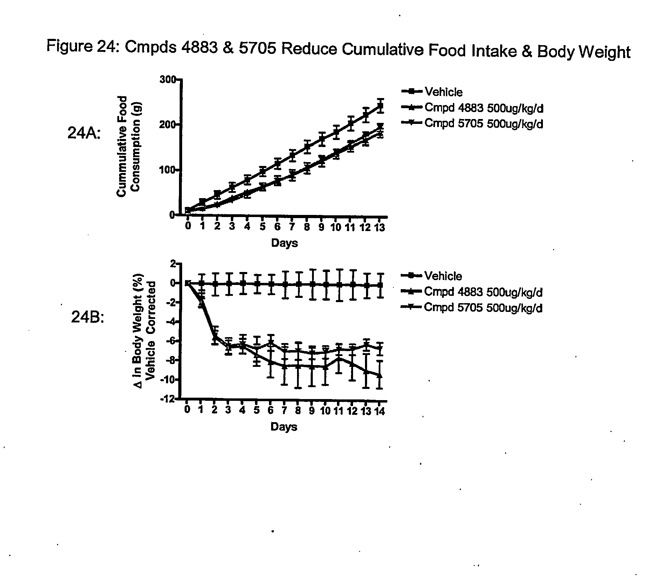

Figure 22 shows the reduction in cumulative food intake over three hours upon administration of Compound 5705 as compared to vehicle alone. Figures 23A and 24A show the change in food intake (grams), and Figures 23B and 24B show the change in body weight (change in percentage of vehicle-corrected body weight), in mice continuously administered vehicle or Compounds 4883 or 5705 (at dosages of 500 µg/kg/d) for 13 days. As early as day one after beginning administration of the PPF polypeptide, there appears to be a trend for reduced food intake and body weight in animals treated with Compound 4883 or Compound 5705.

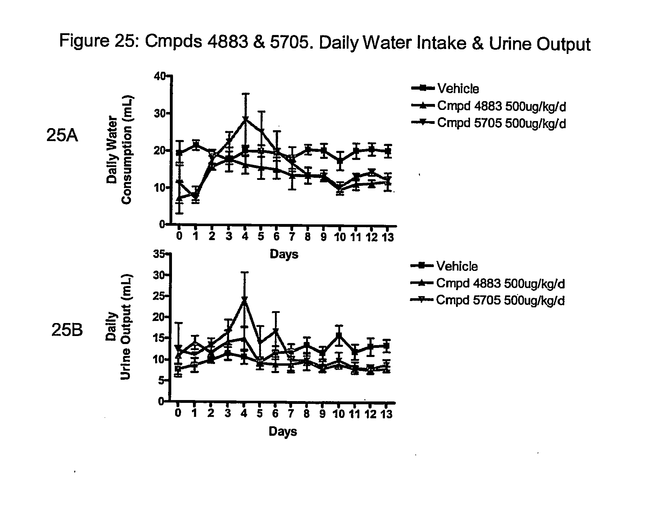

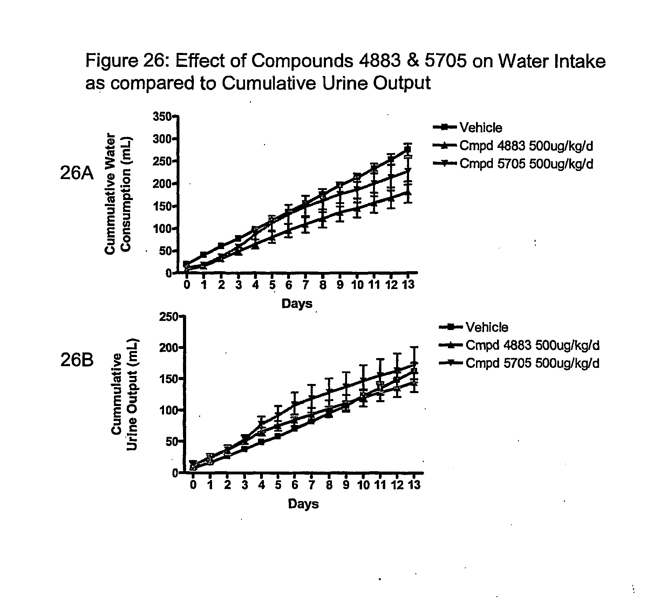

Figures 25 and 26 show the changes in daily water intake and urine output upon administration of Compound 4883 or Compound 5705 in mice continuously administered vehicle or Compounds 4883 or 5705 (at dosages of 500 µg/kg/d) for 13 days.

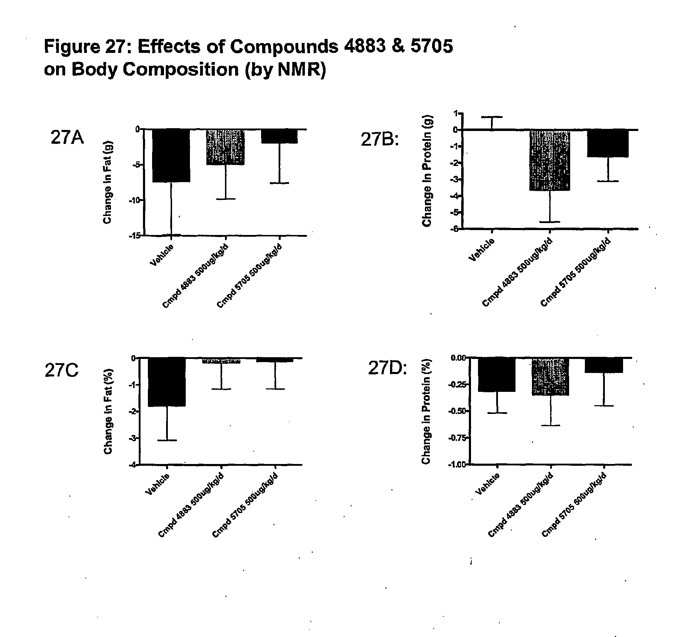

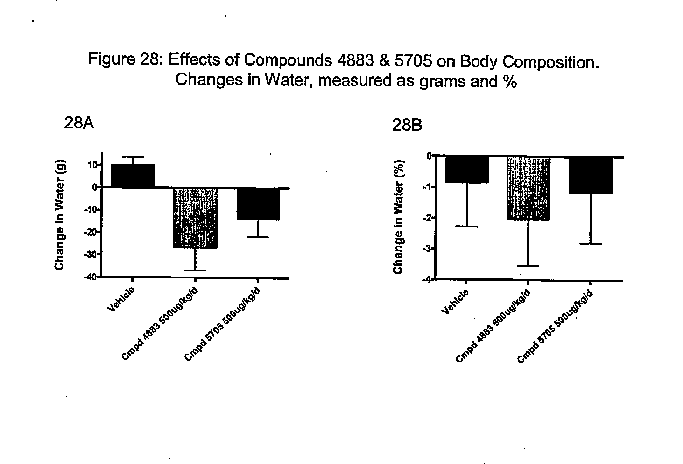

Figure 27 shows the effects of continuous administration vehicle or Compounds 4883 or 5705 (at dosages of 500 µg/kg/d) for 13 days upon body composition as assessed by NMR. Figures 28A and 28B show changes in water as measured in grams as well as percent in mice continuously administered vehicle or Compounds 4883 or 5705 (at dosages of 500 µg/kg/d) for 13 days.

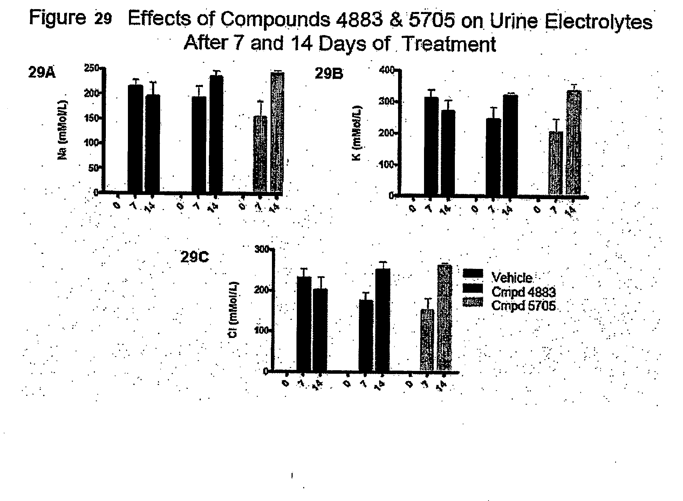

Figure 29 shows the effects of 14 days of continuous administration of Compounds 4883 or 5705 (at dosages of 500 µg/kg/d) upon urine electrolytes.

While the present invention has been described in terms of preferred examples and embodiments, it is understood that variations and modifications will occur to those skilled in the art. Therefore, it is intended that the appended claims cover all such equivalent variations which come within the scope of the invention as claimed.

## Claims

1. A hybrid polypeptide exhibiting at least two hormonal activities, said polypeptide comprising a first bio-active peptide hormone module covalently linked to at least one additional bio-active peptide hormone module, wherein:
the first bio-active peptide hormone is exendin-4 or an analog thereof having at least 50% sequence identity to exendin-4 (SEQ ID NO:66);
the at least one additional bio-active peptide hormone module is amylin, an analog thereof having at least 50% sequence identity to amylin (SEQ ID NO:45), or hAmylin(1-7)-^{11,18}Arg-sCT(8- 27)-Amylin(33-37) (SEQ ID NO:396);
the bio-active peptide hormone modules are covalently attached using a glycine linker;
the first bio-active peptide hormone is an agonist of the GLP-1 receptor and exhibits the insulin secretion activity of exendin-4; and
the at least one additional bio-active peptide hormone exhibits the food intake reduction activity of amylin.

2. The hybrid polypeptide according to claim 1, wherein the at least one additional bio-active peptide hormone binds the amylin receptor.

3. The hybrid polypeptide according to claim 1, wherein the at least one additional bio-active peptide hormone has at least 50% sequence identity to hAmylin(1-7)-^{11,18}Arg-sCT(8- 27)-Amylin(33-37) and is an agonist of the calcitonin receptor (CTR).

4. The hybrid polypeptide of claim 1 wherein the at least one additional bio-active peptide hormone module is selected from the group consisting of: hAmylm(1-7)-^{11,18}Arg-sCT(8- 27)-Amylin(33-37) and ^{25,28,29}Pro-h-amylin.

5. The hybrid polypeptide of any one of claims 1 to 4 wherein the first bioactive peptide is linked by its C-terminus to the N-terminus of the at least one additional bioactive peptide.

6. The hybrid polypeptide of claim 5, wherein the glycine linker is 1 to 30 residues long.

7. The hybrid polypeptide of claim 6, wherein the linker is 3 to 30 residues long.

8. The hybrid polypeptide of claim 7, wherein the linker comprises Gly-Gly-Gly.

9. The hybrid polypeptide of any one of claims 1 to 8, wherein the C-terminal end of the hybrid polypeptide is amidated.

10. The hybrid polypeptide of any one of claims 1 to 9 wherein the exendin analog has at least 80% amino acid sequence identity to exendin-4.

11. The hybrid polypeptide of any one of claims 1 to 10 wherein the exendin analog has at least 90% amino acid sequence identity to exendin-4.

12. The hybrid polypeptide of any one of claims 1 to 11 wherein the amylin analog has at least 80% amino acid sequence identity to amylin.

13. A pharmaceutical composition comprising the hybrid polypeptide of any one of claims 1 to 10.

14. The pharmaceutical composition of claim 13 for use in the treatment of diabetes.

15. The pharmaceutical composition for the use of claim 14 wherein the diabetes is type II diabetes.

## Patentansprüche

1. Hybridpolypeptid, das zumindest zwei Hormonaktivitäten zeigt,
wobei das Polypeptid ein erstes bioaktives Peptidhormonmodul umfasst, das kovalent mit zumindest einem zusätzlichen bioaktiven Peptidhormonmodul verbunden ist, wobei:
das erste bioaktive Peptidhormon Exendin-4 oder ein Analog davon mit einer zumindest 50%igen Sequenzidentität zu Exendin-4 (SEQ ID Nr. 66) ist;
das zumindest eine zusätzliche bioaktive Peptidhormonmodul Amylin, ein Analog davon mit einer zumindest 50%igen Sequenzidentität zu Amylin (SEQ ID Nr. 45) oder hAmylin(1-7)-^{11,18}Arg-sCT(8-27)-Amylin(33-37) (SEQ ID Nr. 396) ist;
die bioaktiven Peptidhormonmodule kovalent mit Hilfe eines Glycin-Linkers angelagert sind;
das erste bioaktive Peptidhormon ein Agonist des GLP-1-Rezeptors ist und die Insulinsekretionsaktivität von Exendin-4 zeigt; und
das zumindest eine zusätzliche bioaktive Peptidhormon die Nahrungsaufnahmereduktionsaktivität von Amylin zeigt.

2. Hybridpolypeptid nach Anspruch 1, wobei das zumindest eine zusätzliche bioaktive Peptidhormon den Amylinrezeptor bindet.

3. Hybridpolypeptid nach Anspruch 1, wobei das zumindest eine zusätzliche bioaktive Peptidhormon eine zumindest 50%ige Sequenzidentität zu hAmylin(1-7)-^{11,18}Arg-sCT(8-27)-Amylin(33-37) aufweist und ein Agonist des Calcitoninrezeptors (CTR) ist.

4. Hybridpolypeptid nach Anspruch 1, wobei das zumindest eine zusätzliche bioaktive Peptidhormonmodul aus der Gruppe ausgewählt ist, die aus hAmylin(1-7)-^{11,18}Arg-sCT(8-27)-Amylin(33-37) und ^{25,28,29}Pro-h-Amylin besteht.

5. Hybridpolypeptid nach irgendeinem der Ansprüche 1 bis 4, wobei das erste bioaktive Peptid durch seinen C-Terminus mit dem N-Terminus des zumindest einen zusätzlichen bioaktiven Peptids verbunden ist.

6. Hybridpolypeptid nach Anspruch 5, wobei der Glycin-Linker 1 bis 30 Rückstände lang ist.

7. Hybridpolypeptid nach Anspruch 6, wobei der Linker 3 bis 30 Rückstände lang ist.

8. Hybridpolypeptid nach Anspruch 7, wobei der Linker Gly-Gly-Gly umfasst.

9. Hybridpolypeptid nach irgendeinem der Ansprüche 1 bis 8, wobei das C-terminale Ende des Hybridpolypeptids amidiert ist.

10. Hybridpolypeptid nach irgendeinem der Ansprüche 1 bis 9, wobei das Exendin-Analog eine zumindest 80%ige Aminosäure-Sequenzidentität zu Exendin-4 aufweist.

11. Hybridpolypeptid nach irgendeinem der Ansprüche 1 bis 10, wobei das Exendin-Analog eine zumindest 90%ige Aminosäure-Sequenzidentität zu Exendin-4 aufweist.

12. Hybridpolypeptid nach irgendeinem der Ansprüche 1 bis 11, wobei das Amylin-Analog eine zumindest 80%ige Aminosäure-Sequenzidentität zu Amylin aufweist.

13. Pharmazeutische Zusammenfassung umfassend das Hybridpolypeptid nach irgendeinem der Ansprüche 1 bis 10.

14. Pharmazeutische Zusammenfassung nach Anspruch 13 zur Verwendung bei der Behandlung von Diabetes.

15. Pharmazeutische Zusammenfassung zur Verwendung nach Anspruch 14, wobei der Diabetes Typ-II-Diabetes ist.

## Revendications

1. Polypeptide hybride présentant au moins deux activités hormonales, ledit polypeptide comprenant un premier module d'hormone peptidique bioactive lié par covalence à au moins un module d'hormone peptidique bioactive supplémentaire, dans lequel :
la première hormone peptidique bioactive est l'exendine 4 ou un analogue correspondant ayant une identité de séquence d'au moins 50 % avec l'exendine 4 (SEQ ID NO 66) ;
l'au moins un module d'hormone peptidique bioactive supplémentaire est l'amyline, un analogue correspondant ayant une identité de séquence d'au moins 50 % avec l'amyline (SEQ ID NO 45), ou hAmyline(1-7)-^{11,18}Arg-sCT(8-27)-Amyline(33-37) (SEQ ID NO 396) ;
les modules d'hormone peptidique bioactive sont liés par covalence au moyen d'un lieur de glycine ;
la première hormone peptidique bioactive est un agoniste du récepteur GLP-1 et présente l'activité de sécrétion d'insuline de l'exendine 4 ; et
l'au moins une hormone peptidique bioactive supplémentaire présente l'activité de réduction de prise de nourriture de l'amyline.

2. Polypeptide hybride selon la revendication 1, dans lequel l'au moins une hormone peptidique bioactive supplémentaire se lie au récepteur à l'amyline.

3. Polypeptide hybride selon la revendication 1, dans lequel l'au moins une hormone peptidique bioactive supplémentaire a une séquence d'identité d'au moins 50 % avec hAmylin(1-7)-^{11,18}Arg-sCT(8-27)-Amylin(33-37) et est un agoniste du récepteur à la calcitonine (CTR).

4. Polypeptide hybride selon la revendication 1, dans lequel l'au moins un module d'hormone peptidique bioactive supplémentaire est choisi dans le groupe constitué de : hAmylin(1-7)-^{11,18}Arg-sCT(8-27)-Amylin(33-37) et ^{25,28,29}Pro-h-amyline.

5. Polypeptide hybride selon l'une quelconque des revendications 1 à 4 dans lequel le premier peptide bioactif est lié par son extrémité C à l'extrémité N de l'au moins un peptide bioactif supplémentaire.

6. Polypeptide hybride selon la revendication 5, dans lequel le lieur de glycine a une longueur de 1 à 30 résidus.

7. Polypeptide hybride selon la revendication 6, dans lequel le lieur a une longueur de 3 à 30 résidus.

8. Polypeptide hybride selon la revendication 7, dans lequel le lieur consiste en Gly-Gly-Gly.

9. Polypeptide hybride selon l'une quelconque des revendications 1 à 8, l'extrémité C-terminale du polypeptide hybride étant amidée.

10. Polypeptide hybride selon l'une quelconque des revendications 1 à 9, dans lequel l'analogue de l'exendine a une identité de séquence d'acide aminé d'au moins 80 % avec l'exendine 4.

11. Polypeptide hybride selon l'une quelconque des revendications 1 à 10, dans lequel l'analogue de l'exendine a une identité de séquence d'acide aminé d'au moins 90 % avec l'exendine 4.

12. Polypeptide hybride selon l'une quelconque des revendications 1 à 11, dans lequel l'analogue de l'amyline a une identité de séquence d'acide aminé d'au moins 80 % avec l'amyline.

13. Composition pharmaceutique comprenant le polypeptide hybride selon l'une quelconque des revendications 1 à 10.

14. Composition pharmaceutique selon la revendication 13, destinée à être utilisé dans le traitement de diabètes.

15. Composition pharmaceutique destinée à être utilisée selon la revendication 14, dans les diabètes sont des diabètes de type II.


### SEQUENCE LISTING

<110> LEVY, ODILE ESTHER HANLEY, MICHAEL R. JODKA, CAROLYN M. LEWIS, DIANA Y. SOARES, CHRISTOPHER J. GHOSH, SOUMITRA S. D'SOUZA, LAWRENCE J. PARKES, DAVID MACK, CHRISTINE M. FOROOD, BRUCE B.
<120> HYBRID POLYPEPTIDES WITH SELECTABLE PROPERTIES
<130> 0701WO2
<140> 11/201,664 <141> 2005-08-11
<140> 11/206,903 <141> 2005-08-17
<140> 11/301,744 <141> 2005-12-12
<160> 457
<170> PatentIn Ver. 3.3
<210> 1
   <211> 54
   <212> PRT
   <213> Artificial Sequence
<220>
   <223> Description of Artificial Sequence: Chimeric lizard and human construct
<220>
   <223> C-term amidated
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Ala Ser Leu Arg His Tyr Leu Asn Leu
           35                  40                  45
   Val Thr Arg Gln Arg Tyr
       50
<210> 2
   <211> 51
   <212> PRT
   <213> Artificial Sequence
<220>
   <223> Description of Artificial Sequence: Chimeric lizard and human construct
<220>
   <223> C-term amidated
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Arg His Tyr Leu Asn Leu Val Thr Arg
           35                  40                  45
   Gln Arg Tyr
       50
<210> 3
   <211> 58
   <212> PRT
   <213> Artificial Sequence
<220>
   <223> Description of Artificial sequence: Chimeric lizard and human construct
<220>
   <223> C-term amidated
<400> 3
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Asn Arg Tyr Tyr Ala Ser Leu Arg His
           35                  40                  45
   Tyr Leu Asn Leu Val Thr Arg Gln Arg Tyr
       50                  55
<210> 4
   <211> 56
   <212> PRT
   <213> Artificial Sequence
<220>
   <223> Description of Artificial Sequence: Chimeric lizard and human construct
<220>
   <221> MOD_RES
   <222> (40)..(41)
   <223> Beta-alanine
<220>
   <223> C-term amidated
<400> 4
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Xaa Xaa Ala Ser Leu Arg His Tyr Leu
           35                  40                  45
   Asn Leu Val Thr Arg Gln Arg Tyr
       50                  55
<210> 5
   <211> 53
   <212> PRT
   <213> Artificial Sequence
<220>
   <223> Description of Artificial Sequence: Chimeric lizard and human construct
<220>
   <221> MOD_RES
   <222> (40)..(41)
   <223> Beta-alanine
<220>
   <223> C-term amidated
<400> 5
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Xaa Xaa Arg His Tyr Leu Asn Leu Val
           35                  40                  45
   Thr Arg Gln Arg Tyr
       50
<210> 6
   <211> 47
   <212> PRT
   <213> Artificial Sequence
<220>
   <223> Description of Artificial Sequence: Chimeric lizard and human construct
<220>
   <221> MOD_RES
   <222> (40)..(41)
   <223> Beta-alanine
<220>
   <223> C-term amidated
<400> 6
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Xaa Xaa Val Thr Arg Gln Arg Tyr
           35                  40                  45
<210> 7
   <211> 43
   <212> PRT
   <213> Artificial Sequence
<220>
   <223> Description of Artificial Sequence: Chimeric lizard and human construct
<220>
   <223> C-term amidated
<400> 7
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Ala Ser Leu Arg
   20 25 30
   His Tyr Leu Asn Leu Val Thr Arg Gln Arg Tyr
   35 40
<210> 8
   <211> 40
   <212> PRT
   <213> Artificial Sequence
<220>
   <223> Description of Artificial Sequence: Chimeric lizard and human construct
<220>
   <223> C-term amidated
<400> 8
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Arg His Tyr Leu
   20 25 30
   Asn Leu Val Thr Arg Gln Arg Tyr
   35 40
<210> 9
   <211> 47
   <212> PRT
   <213> Artificial sequence
<220>
   <223> Description of Artificial Sequence: Chimeric lizard and human construct
<220>
   <223> C-term amidated
<400> 9
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Asn Arg Tyr Tyr
   20 25 30
   Ala Ser Leu Arg His Tyr Leu Asn Leu Val Thr Arg Gln Arg Tyr
   35 40 45
<210> 10
   <211> 45
   <212> PRT
   <213> Artificial Sequence
<220>
   <223> Description of Artificial sequence: Chimeric lizard and human construct
<220>
   <221> MOD_RES
   <222> (29)..(30)
   <223> Beta-alanine
<220>
   <223> C-term amidated
<400> 10
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Ala Ser
               20                  25                  30
   Leu Arg His Tyr Leu Asn Leu Val Thr Arg Gln Arg Tyr
           35                  40                  45
<210> 11
   <211> 42
   <212> PRT
   <213> Artificial Sequence
<220>
   <223> Description of Artificial Sequence: Chimeric lizard and human construct
<220>
   <221> MOD_RES
   <222> (29)..(30)
   <223> Beta-alanine
<220>
   <223> C-term amidated
<400> 11
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Arg His
               20                  25                  30
   Tyr Leu Asn Leu Val Thr Arg Gln Arg Tyr
           35                  40
<210> 12
   <211> 36
   <212> PRT
   <213> Artificial Sequence
<220>
   <223> Description of Artificial Sequence: Chimeric lizard and human construct
<220>
   <221> MOD_RES
   <222> (29)..(30)
   <223> Beta-alanine
<220>
   <223> C-term amidated
<400> 12
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Val Thr
               20                  25                  30
   Arg Gln Arg Tyr
           35
<210> 13
   <211> 47
   <212> PRT
   <213> Artificial Sequence
<220>
   <223> Description of Artificial Sequence: Chimeric lizard and human construct
<220>
   <223> C-term amidated
<400> 13
   His Gly Glu Gly Ala Phe Thr Ser Asp Leu Ser Lys Gln Leu Glu Glu
   1               5                   10                  15
   Glu Ala Val Arg Leu Phe Ile Glu Phe Leu Lys Asn Asn Arg Tyr Tyr
               20                  25                  30
   Ala Ser Leu Arg His Tyr Leu Asn Leu Val Thr Arg Gln Arg Tyr
           35                  40                  45
<210> 14
   <211> 43
   <212> PRT
   <213> Artificial Sequence
<220>
   <223> Description of Artificial sequence: Chimeric lizard and human construct
<220>
   <223> C-term amidated
<400> 14
   His Gly Glu Gly Ala Phe Thr Ser Asp Leu Ser Lys Gln Leu Glu Glu
   1 5 10 15
   Glu Ala Val Arg Leu Phe Ile Glu Phe Leu Lys Asn Ala Ser Leu Arg
   20 25 30
   His Tyr Leu Asn Leu Val Thr Arg Gln Arg Tyr
   35 40
<210> 15
   <211> 40
   <212> PRT
   <213> Artificial sequence
<220>
   <223> Description of Artificial Sequence: Chimeric lizard and human construct
<220>
   <223> C-term amidated
<400> 15
   His Gly Glu Gly Ala Phe Thr Ser Asp Leu Ser Lys Gln Leu Glu Glu
   1 5 10 15
   Glu Ala Val Arg Leu Phe Ile Glu Phe Leu Lys Asn Arg His Tyr Leu
   20 25 30
   Asn Leu Val Thr Arg Gln Arg Tyr
   35 40
<210> 16
   <211> 36
   <212> PRT
   <213> Artificial Sequence
<220>
   <223> Description of Artificial Sequence: Chimeric lizard and human construct
<220>
   <223> C-term amidated
<400> 16
   His Gly Glu Gly Ala Phe Thr Ser Asp Leu Ser Lys Gln Leu Glu Glu
   1 5 10 15
   Glu Asn Arg Tyr Tyr Ala Ser Leu Arg His Tyr Leu Asn Leu Val Thr
   20 25 30
   Arg Gln Arg Tyr
   35
<210> 17
   <211> 45
   <212> PRT
   <213> Artificial Sequence
<220>
   <223> Description of Artificial sequence: Chimeric lizard and human construct
<220>
   <221> MOD_RES
   <222> (29)..(30)
   <223> Beta-alanine
<220>
   <223> C-term amidated
<400> 17
   His Gly Glu Gly Ala Phe Thr Ser Asp Leu Ser Lys Gln Leu Glu Glu
   1               5                   10                  15
   Glu Ala Val Arg Leu Phe Ile Glu Phe Leu Lys Asn Xaa Xaa Ala Ser
               20                  25                  30
   Leu Arg His Tyr Leu Asn Leu Val Thr Arg Gln Arg Tyr
           35                  40                  45
<210> 18
   <211> 42
   <212> PRT
   <213> Artificial Sequence
<220>
   <223> Description of Artificial sequence: Chimeric lizard and human construct
<220>
   <221> MOD_RES
   <222> (29)..(30)
   <223> Beta-alanine
<220>
   <223> C-term amidated
<400> 18
   His Gly Glu Gly Ala Phe Thr Ser Asp Leu Ser Lys Gln Leu Glu Glu
   1               5                   10                  15
   Glu Ala Val Arg Leu Phe Ile Glu Phe Leu Lys Asn Xaa Xaa Arg His
               20                  25                  30
   Tyr Leu Asn Leu Val Thr Arg Gln Arg Tyr
           35                  40
<210> 19
   <211> 36
   <212> PRT
   <213> Artificial Sequence
<220>
   <223> Description of Artificial Sequence: Chimeric lizard and human construct
<220>
   <221> MOD_RES
   <222> (29)..(30)
   <223> Beta-alanine
<220>
   <223> C-term amidated
<400> 19
   His Gly Glu Gly Ala Phe Thr Ser Asp Leu Ser Lys Gln Leu Glu Glu
   1               5                   10                  15
   Glu Ala Val Arg Leu Phe Ile Glu Phe Leu Lys Asn Xaa Xaa Val Thr
               20                  25                  30
   Arg Gln Arg Tyr
           35
<210> 20
   <211> 47
   <212> PRT
   <213> Artificial Sequence
<220>
   <223> Description of Artificial sequence: Chimeric lizard and human construct
<220>
   <221> MOD_RES
   <222> (41)
   <223> Tyr (S03)
<220>
   <223> C-term amidated
<400> 20
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser Asp Tyr Met Gly Trp Met Asp Phe
           35                  40                  45
<210> 21
   <211> 36
   <212> PRT
   <213> Artificial Sequence
<220>
   <223> Description of Artificial Sequence: chimeric lizard and human construct
<220>
   <221> MOD_RES
   <222> (30)
   <223> Tyr(S03)
<220>
   <223> C-term amidated
<400> 21
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Asp Tyr Met Gly
               20                  25                  30
   Trp Met Asp Phe
           35
<210> 22
   <211> 36
   <212> PRT
   <213> Artificial Sequence
<220>
   <223> Description of Artificial Sequence: Chimeric lizard and human construct
<220>
   <221> MOD_RES
   <222> (30)
   <223> Phe(CH2SO3)
<220>
   <223> C-term amidated
<400> 22
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Asp Phe Met Gly
               20                  25                  30
   Trp Met Asp Phe
           35
<210> 23
   <211> 37
   <212> PRT
   <213> Artificial Sequence
<220>
   <223> Description of Artificial sequence: Chimeric lizard and human construct
<220>
   <221> MOD_RES
   <222> (29)
   <223> 8-amino-3,6-dioxactoanoyl
<220>
   <221> MOD_RES
   <222> (31)
   <223> Tyr(S03)
<220>
   <223> C-term amidated
<400> 23
   His Gly Glu Gly Thr Phe Thr Ser Asp Leu Ser Lys Gln Met Glu Glu
     1               5                  10                  15
   Glu Ala Val Arg Leu Phe Ile Glu Trp Leu Lys Asn Xaa Asp Tyr Met
                20                  25                  30
   Gly Trp Met Asp Phe
            35
<210> 24
   <211> 37
   <212> PRT
   <213> Artificial Sequence
<220>
   <223> Description of Artificial Sequence: Chimeric lizard and human construct
<220>
   <221> MOD_RES
   <222> (29)
   <223> 8-amino-3,6-dioxactoanoyl
<220>
   <221> MOD_RES
   <222> (31)
   <223> Phe(CH2SO3)
<220>
   <223> C-term amidated
<400> 24
   His Gly Glu Gly Thr Phe Thr Ser Asp Leu Ser Lys Gln Met Glu Glu
     1               5                  10                  15
   Glu Ala Val Arg Leu Phe Ile Glu Trp Leu Lys Asn Xaa Asp Phe Met
                20                  25                  30
   Gly Trp Met Asp Phe
            35
<210> 25
   <211> 59
   <212> PRT
   <213> Artificial Sequence
<220>
   <223> Description of Artificial Sequence: Chimeric lizard, human and salmon construct
<220>
   <223> C-term amidated
<400> 25
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Lys Cys Asn Thr Ala
   20 25 30
   Thr Cys Val Leu Gly Arg Leu Ser Gln Glu Leu His Arg Leu Gln Thr
   35 40 45
   Tyr Pro Arg Thr Asn Thr Gly Ser Asn Thr Tyr
   50 55
<210> 26
   <211> 37
   <212> PRT
   <213> Artificial Sequence
<220>
   <223> Description of Artificial sequence: Chimeric lizard, human and salmon construct
<220>
   <223> C-term amidated
<400> 26
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Lys Ala Asn Thr Ala
   20 25 30
   Thr Ala Val Leu Gly
   35
<210> 27
   <211> 61
   <212> PRT
   <213> Artificial Sequence
<220>
   <223> Description of Artificial Sequence: Chimeric lizard, human and salmon construct
<220>
   <221> MOD_RES
   <222> (29)
   <223> 12-Ado
<220>
   <223> C-term amidated
<400> 27
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Xaa Lys Cys Asn
   20 25 30
   Thr Ala Thr Cys Val Leu Gly Arg Leu Ser Gln Glu Leu His Arg Leu
   35 40 45
   Gln Thr Tyr Pro Arg Thr Asn Thr Gly Ser Asn Thr Tyr
   50 55 60
<210> 28
   <211> 60
   <212> PRT
   <213> Artificial Sequence
<220>
   <223> Description of Artificial sequence: Chimeric lizard, human and salmon construct
<220>
   <221> MOD_RES
   <222> (29)
   <223> 12-Ado
<220>
   <223> C-term amidated
<400> 28
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Xaa Cys Asn Thr
   20 25 30
   Ala Thr Cys Val Leu Gly Arg Leu Ser Gln Glu Leu His Arg Leu Gln
   35 40 45
   Thr Tyr Pro Arg Thr Asn Thr Gly Ser Asn Thr Tyr
   50 55 60
<210> 29
   <211> 61
   <212> PRT
   <213> Artificial Sequence
<220>
   <223> Description of Artificial Sequence: Chimeric lizard, human and salmon construct
<220>
   <221> MOD_RES
   <222> (29)
   <223> 3,6-dioxactoanoyl
<220>
   <223> C-term amidated
<400> 29
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Lys Cys Asn
               20                  25                  30
   Thr Ala Thr Cys Val Leu Gly Arg Leu Ser Gln Glu Leu His Arg Leu
                   35                  40                  45
   Gln Thr Tyr Pro Arg Thr Asn Thr Gly Ser Asn Thr Tyr
       50                  55                  60
<210> 30
   <211> 60
   <212> PRT
   <213> Artificial Sequence
<220>
   <223> Description of Artificial sequence: Chimeric lizard, human and salmon construct
<220>
   <221> MOD_RES
   <222> (29)
   <223> 3,6-dioxactoanoyl
<220>
   <223> C-term amidated
<400> 30
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Cys Asn Thr
               20                  25                  30
   Ala Thr Cys Val Leu Gly Arg Leu Ser Gln Glu Leu His Arg Leu Gln
           35                  40                  45
   Thr Tyr Pro Arg Thr Asn Thr Gly Ser Asn Thr Tyr
         50                  55                  60
<210> 31
   <211> 61
   <212> PRT
   <213> Artificial Sequence
<220>
   <223> Description of Artificial sequence: Chimeric lizard, human and salmon construct
<220>
   <221> MOD_RES
   <222> (29)
   <223> 5-Apa
<220>
   <223> C-term amidated
<400> 31
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Xaa Lys Cys Asn
   20 25 30
   Thr Ala Thr Cys Val Leu Gly Arg Leu Ser Gln Glu Leu His Arg Leu
   35 40 45
   Gln Thr Tyr Pro Arg Thr Asn Thr Gly Ser Asn Thr Tyr
   50 55 60
<210> 32
   <211> 60
   <212> PRT
   <213> Artificial Sequence
<220>
   <223> Description of Artificial Sequence: chimeric lizard, human and salmon construct
<220>
   <221> MOD_RES
   <222> (29)
   <223> 5-Apa
<220>
   <223> C-term amidated
<400> 32
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Xaa Cys Asn Thr
   20 25 30
   Ala Thr Cys Val Leu Gly Arg Leu Ser Gln Glu Leu His Arg Leu Gln
   35 40 45
   Thr Tyr Pro Arg Thr Asn Thr Gly Ser Asn Thr Tyr
   50 55 60
<210> 33
   <211> 62
   <212> PRT
   <213> Artificial sequence
<220>
   <223> Description of Artificial Sequence: Chimeric lizard, human and salmon construct
<220>
   <221> MOD_RES
   <222> (29)..(30)
   <223> Beta-alanine
<220>
   <223> C-term amidated
<400> 33
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Lys Cys
               20                  25                  30
   Asn Thr Ala Thr Cys Val Leu Gly Arg Leu Ser Gln Glu Leu His Arg
           35                  40                  45
   Leu Gln Thr Tyr Pro Arg Thr Asn Thr Gly Ser Asn Thr Tyr
       50                  55                  60
<210> 34
   <211> 61
   <212> PRT
   <213> Artificial Sequence
<220>
   <223> Description of Artificial Sequence: Chimeric lizard, human and salmon construct
<220>
   <221> MOD_RES
   <222> (29)..(30)
   <223> Beta-alanine
<220>
   <223> C-term amidated
<400> 34
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Cys Asn
               20                  25                  30
   Thr Ala Thr Cys Val Leu Gly Arg Leu Ser Gln Glu Leu His Arg Leu
           35                  40                  45
   Gln Thr Tyr Pro Arg Thr Asn Thr Gly Ser Asn Thr Tyr
       50                  55                  60
<210> 35
   <211> 61
   <212> PRT
   <213> Artificial sequence
<220>
   <223> Description of Artificial Sequence: Chimeric lizard, human and salmon construct
<220>
   <221> MOD_RES
   <222> (29)
   <223> 4,7,10-trioxa-13-tridecanamine succinimidyl
<220>
   <223> C-term amidated
<400> 35
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Lys Cys Asn
               20                  25                  30
   Thr Ala Thr Cys Val Leu Gly Arg Leu Ser Gln Glu Leu His Arg Leu
           35                  40                  45
   Gln Thr Tyr Pro Arg Thr Asn Thr Gly Ser Asn Thr Tyr
       50                  55                  60
<210> 36
   <211> 60
   <212> PRT
   <213> Artificial sequence
<220>
   <223> Description of Artificial Sequence: Chimeric lizard, human and salmon construct
<220>
   <221> MOD_RES
   <222> (29)
   <223> 4,7,10-trioxa-13-tridecanamine succinimidyl
<220>
   <223> C-term amidated
<400> 36
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Cys Asn Thr
               20                  25                  30
   Ala Thr Cys Val Leu Gly Arg Leu Ser Gln Glu Leu His Arg Leu Gln
           35                  40                  45
   Thr Tyr Pro Arg Thr Asn Thr Gly Ser Asn Thr Tyr
       50                  55                  60
<210> 37
   <211> 43
   <212> PRT
   <213> Artificial Sequence
<220>
   <223> Description of Artificial sequence: Chimeric human and salmon construct
<220>
   <221> MOD_RES
   <222> (2)
   <223> Tyr(SO3)
<220>
   <223> C-term amidated
<400> 37
   Asp Tyr Met Gly Trp Met Asp Phe Gly Lys Arg Lys Cys Asn Thr Ala
   1               5                   10                  15
   Thr Cys Ala Thr Gln Arg Leu Ala Asn Glu Leu Val Arg Leu Gln Thr
               20                  25                  30
   Tyr Pro Arg Thr Asn Val Gly Ser Asn Thr Tyr
           35                  40
<210> 38
   <211> 36
   <212> PRT
   <213> Homo sapiens
<220>
   <223> C-term amidated
<400> 38
   Lys Cys Asn Thr Ala Thr Cys Ala Thr Gln Arg Leu Ala Asn Phe Leu
   1               5                   10                  15
   Val Arg Arg Tyr Tyr Ala Ser Leu Arg His Tyr Leu Asn Leu Val Thr
               20                  25                  30
   Arg Gln Arg Tyr
           35
<210> 39
   <211> 36
   <212> PRT
   <213> Artificial Sequence
<220>
   <223> Description of Artificial Sequence: Synthetic polypeptide
<220>
   <221> MOD_RES
   <222> (1)
   <223> Isocaproyl-Ser
<220>
   <221> MOD_RES
   <222> (6)
   <223> Aib
<220>
   <221> MOD_RES
   <222> (7)
   <223> Lys(formyl)
<220>
   <221> MOD_RES
   <222> (13)
   <223> Aib
<220>
   <221> MOD_RES
   <222> (14)
   <223> Lys(formyl)
<220>
   <223> C-term amidated
<400> 39
   Ser Thr Ala Val Leu Xaa Lys Leu Ser Gln Glu Leu Xaa Lys Leu Gln
   1               5                   10                  15
   Thr Asn Arg Tyr Tyr Ala Ser Leu Arg His Tyr Leu Asn Leu Val Thr
               20                  25                  30
   Arg Gln Arg Tyr
           35
<210> 40
   <211> 36
   <212> PRT
   <213> Artificial Sequence
<220>
   <223> Description of Artificial Sequence: Synthetic polypeptide
<220>
   <221> MOD_RES
   <222> (1)
   <223> Isocaproyl-Ser
<220>
   <221> MOD_RES
   <222> (6)
   <223> Aib
<220>
   <221> MOD_RES
   <222> (7)
   <223> Lys(formyl)
<220>
   <221> MOD_RES
   <222> (13)
   <223> Aib
<220>
   <221> MOD_RES
   <222> (14)
   <223> Lys(formyl)
<220>
   <223> C-term amidated
<400> 40
   Ser Thr Ala Val Leu Xaa Lys Leu Ser Gln Glu Leu Xaa Lys Leu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 41
   <211> 43
   <212> PRT
   <213> Homo sapiens
<220>
   <221> MOD_RES
   <222> (1)
   <223> Amidated terminus
<220>
   <221> MOD_RES
   <222> (7)
   <223> Sulfonated Tyr
<220>
   <221> MOD_RES
   <222> (9)
   <223> Succinoyl-Cys
<400> 41
   Phe Asp Met Trp Gly Met Tyr Asp Cys Ile Lys Pro Glu Ala Pro Gly
   1               5                   10                  15
   Glu Asp Ala Ser Pro Glu Glu Leu Asn Arg Tyr Tyr Ala Ser Leu Arg
               20                  25                  30
   His Tyr Leu Asn Leu Val Thr Arg Gln Arg Tyr
           35                  40
<210> 42
   <211> 43
   <212> PRT
   <213> Homo sapiens
<220>
   <221> MOD_RES
   <222> (1)
   <223> Amidated terminus
<220>
   <221> MOD_RES
   <222> (7)
   <223> Sulfonated Tyr
<220>
   <221> MOD_RES
   <222> (9)
   <223> Bis-Cys(N-Acetyl)
<400> 42
   Phe Asp Met Trp Gly Met Tyr Asp Cys Ile Lys Pro Glu Ala Pro Gly
   1 5 10 15
   Glu Asp Ala Ser Pro Glu Glu Leu Asn Arg Tyr Tyr Ala Ser Leu Arg
   20 25 30
   His Tyr Leu Asn Leu Val Thr Arg Gln Arg Tyr
   35 40
<210> 43
   <211> 43
   <212> PRT
   <213> Homo sapiens
<220>
   <221> MOD_RES
   <222> (1)
   <223> Amidated terminus
<220>
   <221> MOD_RES
   <222> (7)
   <223> Sulfonated Tyr
<220>
   <221> MOD_RES
   <222> (9)
   <223> Gly-Aminoxymethylcarbonyl
<220>
   <223> C-term amidated
<400> 43
   Phe Asp Met Trp Gly Met Tyr Asp Gly Ile Lys Pro Glu Ala Pro Gly
   1 5 10 15
   Glu Asp Ala Ser Pro Glu Glu Leu Asn Arg Tyr Tyr Ala Ser Leu Arg
   20 25 30
   His Tyr Leu Asn Leu Val Thr Arg Gln Arg Tyr
   35 40
<210> 44
   <211> 37
   <212> PRT
   <213> Rattus sp.
<400> 44
   Lys Cys Asn Thr Ala Thr Cys Ala Thr Gln Arg Leu Ala Asn Phe Leu
   1 5 10 15
   Val Arg Ser Ser Asn Asn Leu Gly Pro Val Leu Pro Pro Thr Asn Val
   20 25 30
   Gly Ser Asn Thr Tyr
   35
<210> 45
   <211> 37
   <212> PRT
   <213> Homo sapiens
<400> 45
<210> 46
   <211> 52
   <212> PRT
   <213> Homo sapiens
<400> 46
   Tyr Arg Gln Ser Met Asn Asn Phe Gln Gly Leu Arg Ser Phe Gly Cys
   1 5 10 15
   Arg Phe Gly Thr Cys Thr Val Gln Lys Leu Ala His Gln Ile Tyr Gln
   20 25 30
   Phe Thr Asp Lys Asp Lys Asp Asn Val Ala Pro Arg Ser Lys Ile Ser
   35 40 45
   Pro Gln Gly Tyr
   50
<210> 47
   <211> 32
   <212> PRT
   <213> Oncorhynchus sp.
<400> 47
   Cys Ser Asn Leu Ser Thr Cys Val Leu Gly Lys Leu Ser Gln Glu Leu
   1 5 10 15
   His Lys Leu Gln Thr Tyr Pro Arg Thr Asn Thr Gly Ser Gly Thr Pro
   20 25 30
<210> 48
   <211> 32
   <212> PRT
   <213> Homo sapiens
<400> 48
   Cys Gly Asn Leu Ser Thr Cys Met Leu Gly Thr Tyr Thr Gln Asp Phe
   1               5                   10                  15
   Asn Lys Phe His Thr Phe Pro Gln Thr Ala Ile Gly Val Gly Ala Pro
               20                  25                  30
<210> 49
   <211> 37
   <212> PRT
   <213> Homo sapiens
<400> 49
   Ala Cys Asp Thr Ala Thr Cys Val Thr His Arg Leu Ala Gly Leu Leu
   1               5                   10                  15
   Ser Arg Ser Gly Gly Val Val Lys Asn Asn Phe Val Pro Thr Asn Val
               20                  25                  30
   Gly Ser Lys Ala Phe
           35
<210> 50
   <211> 37
   <212> PRT
   <213> Homo sapiens
<400> 50
   Ala Cys Asn Thr Ala Thr Cys Val Thr His Arg Leu Ala Gly Leu Leu
   1               5                   10                  15
   Ser Arg Ser Gly Gly Met Val Lys Ser Asn Phe Val Pro Thr Asn Val
               20                  25                  30
   Gly Ser Lys Ala Phe
           35
<210> 51
   <211> 47
   <212> PRT
   <213> Homo sapiens
<400> 51
   Thr Gln Ala Gln Leu Leu Arg Val Gly Cys Val Leu Gly Thr Cys Gln
   1               5                   10                  15
   Val Gln Asn Leu Ser His Arg Leu Trp Gln Leu Met Gly Pro Ala Gly
               20                  25                  30
   Arg Gln Asp Ser Ala Pro Val Asp Pro Ser Ser Pro His Ser Tyr
           35                  40                  45
<210> 52
   <211> 40
   <212> PRT
   <213> Homo sapiens
<400> 52
   Val Gly Cys Val Leu Gly Thr Cys Gln Val Gln Asn Leu Ser His Arg
   1               5                   10                  15
   Leu Trp Gln Leu Met Gly Pro Ala Gly Arg Gln Asp Ser Ala Pro Val
               20                  25                  30
   Asp Pro Ser Ser Pro His Ser Tyr
           35                  40
<210> 53
   <211> 47
   <212> PRT
   <213> Mus musculus
<400> 53
   Pro His Ala Gln Leu Leu Arg Val Gly Cys Val Leu Gly Thr Cys Gln
   1               5                   10                  15
   Val Gln Asn Leu Ser His Arg Leu Trp Gln Leu Val Arg Pro Ala Gly
               20                  25                  30
   Arg Arg Asp Ser Ala Pro Val Asp Pro Ser Ser Pro His Ser Tyr
           35                  40                  45
<210> 54
   <211> 40
   <212> PRT
   <213> Mus musculus
<400> 54
   Val Gly Cys Val Leu Gly Thr Cys Gln Val Gln Asn Leu Ser His Arg
   1               5                   10                  15
   Leu Trp Gln Leu Val Arg Pro Ala Gly Arg Arg Asp Ser Ala Pro Val
               20                  25                  30
   Asp Pro Ser Ser Pro His Ser Tyr
           35                  40
<210> 55
   <211> 8
   <212> PRT
   <213> Homo sapiens
<220>
   <221> MOD_RES
   <222> (2)
   <223> Tyr(SO3)
<400> 55
<210> 56
   <211> 167
   <212> PRT
   <213> Homo sapiens
<400> 56
<210> 57
   <211> 36
   <212> PRT
   <213> Homo sapiens
<400> 57
   Tyr Pro Ile Lys Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Val Thr
   20 25 30
   Arg Gln Arg Tyr
   35
<210> 58
   <211> 34
   <212> PRT
   <213> Homo sapiens
<400> 58
   Ile Lys Pro Glu Ala Pro Gly Glu Asp Ala Ser Pro Glu Glu Leu Asn
   1               5                   10                  15
   Arg Tyr Tyr Ala Ser Leu Arg His Tyr Leu Asn Leu Val Thr Arg Gln
               20                  25                  30
   Arg Tyr
<210> 59
   <211> 37
   <212> PRT
   <213> Homo sapiens
<400> 59
   His Asp Glu Phe Glu Arg His Ala Glu Gly Thr Phe Thr Ser Asp Val
   1               5                   10                  15
   Ser Ser Thr Leu Glu Gly Gln Ala Ala Leu Glu Phe Ile Ala Trp Leu
               20                  25                  30
   Val Lys Gly Arg Gly
           35
<210> 60
   <211> 37
   <212> PRT
   <213> Xenopus laevis
<220>
   <221> MOD_RES
   <222> (37)
   <223> Ser-OH
<400> 60
   His Ala Glu Gly Thr Tyr Thr Asn Asp Val Thr Glu Tyr Leu Glu Glu
   1               5                   10                  15
   Lys Ala Ala Lys Glu Phe Ile Glu Trp Leu Ile Lys Gly Lys Pro Lys
               20                  25                  30
   Lys Ile Arg Tyr Ser
           35
<210> 61
   <211> 30
   <212> PRT
   <213> Homo sapiens
<400> 61
   His Ala Glu Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg
   20 25 30
<210> 62
   <211> 33
   <212> PRT
   <213> Homo sapiens
<400> 62
   His Ala Asp Gly Ser Phe Ser Asp Glu Met Asn Thr Ile Leu Asp Asn
   1 5 10 15
   Leu Ala Ala Arg Asp Phe Ile Asn Trp Leu Ile Glu Thr Lys Ile Thr
   20 25 30
   Asp
<210> 63
   <211> 31
   <212> PRT
   <213> Xenopus sp.
<220>
   <221> MOD_RES
   <222> (31)
   <223> Pro-OH
<400> 63
   His Ala Glu Gly Thr Phe Thr Asn Asp Met Thr Asn Tyr Leu Glu Glu
   1 5 10 15
   Lys Ala Ala Lys Glu Phe Val Gly Trp Leu Ile Lys Gly Arg Pro
   20 25 30
<210> 64
   <211> 37
   <212> PRT
   <213> Homo sapiens
<400> 64
   His Ser Gln Gly Thr Phe Thr Ser Asp Tyr Ser Lys Tyr Leu Asp Ser
   1 5 10 15
   Arg Arg Ala Gln Asp Phe Val Gln Trp Leu Met Asn Thr Lys Arg Asn
   20 25 30
   Arg Asn Asn Ile Ala
   35
<210> 65
   <211> 39
   <212> PRT
   <213> Heloderma suspectum
<400> 65
   His Ser Asp Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 66
   <211> 39
   <212> PRT
   <213> Heloderma suspectum
<400> 66
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 67
   <211> 37
   <212> PRT
   <213> Homo sapiens
<400> 67
   Lys Cys Asn Thr Ala Thr Cys Ala Thr Gln Arg Leu Ala Asn Phe Leu
   1 5 10 15
   Val His Ser Ser Asn Asn Phe Gly Pro Ile Leu Pro Pro Thr Asn Val
   20 25 30
   Gly Ser Asn Thr Tyr
   35
<210> 68
   <211> 36
   <212> PRT
   <213> Homo sapiens
<400> 68
   Cys Asn Thr Ala Thr Cys Ala Thr Gln Arg Leu Ala Asn Phe Leu Val
   1 5 10 15
   His Ser Ser Asn Asn Phe Gly Ala Ile Leu Ser Ser Thr Asn Val Gly
   20 25 30
   Ser Asn Thr Tyr
   35
<210> 69
   <211> 37
   <212> PRT
   <213> Homo sapiens
<400> 69
   Lys Cys Asn Thr Ala Thr Cys Ala Thr Gln Arg Leu Ala Asn Phe Leu
   1 5 10 15
   Val His Ser Ser Asn Asn Phe Gly Pro Val Leu Pro Pro Thr Asn Val
   20 25 30
   Gly Ser Asn Thr Tyr
   35
<210> 70
   <211> 37
   <212> PRT
   <213> Homo sapiens
<400> 70
   Lys Cys Asn Thr Ala Thr Cys Ala Thr Gln Arg Leu Ala Asn Phe Leu
   1 5 10 15
   Val Arg Ser Ser Asn Asn Phe Gly Pro Ile Leu Pro Ser Thr Asn Val
   20 25 30
   Gly Ser Asn Thr Tyr
   35
<210> 71
   <211> 36
   <212> PRT
   <213> Homo sapiens
<400> 71
   Cys Asn Thr Ala Thr Cys Ala Thr Gln Arg Leu Ala Asn Phe Leu Val
   1 5 10 15
   Arg Ser Ser Asn Asn Phe Gly Pro Ile Leu Pro Ser Thr Asn Val Gly
   20 25 30
   Ser Asn Thr Tyr
   35
<210> 72
   <211> 37
   <212> PRT
   <213> Homo sapiens
<400> 72
   Lys Cys Asn Thr Ala Thr Cys Ala Thr Gln Arg Leu Ala Asn Phe Leu
    1               5                  10                  15
   Val Arg Ser Ser Asn Asn Phe Gly Pro Ile Leu Pro Pro Thr Asn Val
               20                  25                  30
   Gly Ser Asn Thr Tyr
                   35
<210> 73
   <211> 36
   <212> PRT
   <213> Homo sapiens
<400> 73
   Cys Asn Thr Ala Thr Cys Ala Thr Gln Arg Leu Ala Asn Phe Leu Val
    1               5                  10                  15
   Arg Ser Ser Asn Asn Phe Gly Pro Ile Leu Pro Pro Thr Asn Val Gly
               20                  25                  30
   Ser Asn Thr Tyr
           35
<210> 74
   <211> 36
   <212> PRT
   <213> Homo sapiens
<400> 74
   Cys Asn Thr Ala Thr Cys Ala Thr Gln Arg Leu Ala Asn Phe Leu Val
    1               5                  10                  15
   His Ser Ser Asn Asn Phe Gly Pro Ile Leu Pro Pro Thr Asn Val Gly
               20                  25                  30
   Ser Asn Thr Tyr
           35
<210> 75
   <211> 37
   <212> PRT
   <213> Homo sapiens
<400> 75
   Lys Cys Asn Thr Ala Thr Cys Ala Thr Gln Arg Leu Ala Asn Phe Leu
   1 5 10 15
   Val His Ser Ser Asn Asn Phe Gly Pro Val Leu Pro Pro Thr Asn Val
   20 25 30
   Gly Ser Asn Thr Tyr
   35
<210> 76
   <211> 37
   <212> PRT
   <213> Homo sapiens
<220>
   <223> 2,7-Cyclo bridge
<400> 76
   Lys Asp Asn Thr Ala Thr Lys Ala Thr Gln Arg Leu Ala Asn Phe Leu
   1 5 10 15
   Val His Ser Ser Asn Asn Phe Gly Ala Ile Leu Pro Ser Thr Asn Val
   20 25 30
   Gly Ser Asn Thr Tyr
   35
<210> 77
   <211> 36
   <212> PRT
   <213> Homo sapiens
<400> 77
   Cys Asn Thr Ala Thr Cys Ala Thr Gln Arg Leu Ala Asn Phe Leu Val
   1 5 10 15
   His Ser Ser Asn Asn Phe Gly Ala Ile Leu Ser Ser Thr Asn Val Gly
   20 25 30
   Ser Asn Thr Tyr
   35
<210> 78
   <211> 37
   <212> PRT
   <213> Homo sapiens
<400> 78
   Ala Cys Asn Thr Ala Thr Cys Ala Thr Gln Arg Leu Ala Asn Phe Leu
   1 5 10 15
   Val His Ser Ser Asn Asn Phe Gly Ala Ile Leu Ser Ser Thr Asn Val
   20 25 30
   Gly Ser Asn Thr Tyr
   35
<210> 79
   <211> 37
   <212> PRT
   <213> Homo sapiens
<400> 79
   Lys Ala Asn Thr Ala Thr Cys Ala Thr Gln Arg Leu Ala Asn Phe Leu
   1 5 10 15
   Val His Ser Ser Asn Asn Phe Gly Ala Ile Leu Ser Ser Thr Asn Val
   20 25 30
   Gly Ser Asn Thr Tyr
   35
<210> 80
   <211> 37
   <212> PRT
   <213> Homo sapiens
<400> 80
   Lys Ala Asn Thr Ala Thr Ala Ala Thr Gln Arg Leu Ala Asn Phe Leu
   1 5 10 15
   Val His Ser Ser Asn Asn Phe Gly Ala Ile Leu Ser Ser Thr Asn Val
   20 25 30
   Gly Ser Asn Thr Tyr
   35
<210> 81
   <211> 37
   <212> PRT
   <213> Homo sapiens
<400> 81
   Ser Cys Asn Thr Ala Thr Cys Ala Thr Gln Arg Leu Ala Asn Phe Leu
   1 5 10 15
   Val His Ser Ser Asn Asn Phe Gly Ala Ile Leu Ser Ser Thr Asn Val
   20 25 30
   Gly Ser Asn Thr Tyr
   35
<210> 82
   <211> 37
   <212> PRT
   <213> Homo sapiens
<400> 82
   Lys Cys Asn Thr Ala Thr Cys Ala Thr Gln Arg Leu Ala Asn Phe Leu
   1               5                   10                  15
   Val His Ser Ser Asn Asn Phe Gly Ala Ile Leu Ser Pro Thr Asn Val
               20                  25                  30
   Gly Ser Asn Thr Tyr
           35
<210> 83
   <211> 37
   <212> PRT
   <213> Homo sapiens
<400> 83
   Lys Cys Asn Thr Ala Thr Cys Ala Thr Gln Arg Leu Ala Asn Phe Leu
   1               5                   10                  15
   Val His Ser Ser Asn Asn Phe Gly Pro Ile Leu Pro Ser Thr Asn Val
               20                  25                  30
   Gly Ser Asn Thr Tyr
           35
<210> 84
   <211> 36
   <212> PRT
   <213> Homo sapiens
<400> 84
   Cys Asn Thr Ala Thr Cys Ala Thr Gln Arg Leu Ala Asn Phe Leu Val
   1               5                   10                  15
   His Ser Ser Asn Asn Phe Gly Pro Ile Leu Pro Ser Thr Asn Val Gly
               20                  25                  30
   Ser Asn Thr Tyr
           35
<210> 85
   <211> 37
   <212> PRT
   <213> Homo sapiens
<400> 85
   Lys Cys Asn Thr Ala Thr Cys Ala Thr Gln Arg Leu Ala Asn Phe Leu
   1 5 10 15
   Val His Ser Ser Asn Asn Leu Gly Pro Val Leu Pro Pro Thr Asn Val
   20 25 30
   Gly Ser Asn Thr Tyr
   35
<210> 86
   <211> 37
   <212> PRT
   <213> Homo sapiens
<400> 86
   Lys Cys Asn Thr Ala Thr Cys Ala Thr Gln Arg Leu Ala Asn Phe Leu
   1 5 10 15
   Val His Ser Ser Asn Asn Leu Gly Pro Val Leu Pro Ser Thr Asn Val
   20 25 30
   Gly Ser Asn Thr Tyr
   35
<210> 87
   <211> 36
   <212> PRT
   <213> Homo sapiens
<400> 87
   Cys Asn Thr Ala Thr Cys Ala Thr Gln Arg Leu Ala Asn Phe Leu Val
   1 5 10 15
   His Ser Ser Asn Asn Leu Gly Pro Val Leu Pro Ser Thr Asn Val Gly
   20 25 30
   Ser Asn Thr Tyr
   35
<210> 88
   <211> 37
   <212> PRT
   <213> Homo sapiens
<400> 88
   Lys Cys Asn Thr Ala Thr Cys Ala Thr Gln Arg Leu Ala Asn Phe Leu
   1 5 10 15
   Val Arg Ser Ser Asn Asn Leu Gly Pro Val Leu Pro Ser Thr Asn Val
   20 25 30
   Gly Ser Asn Thr Tyr
   35
<210> 89
   <211> 37
   <212> PRT
   <213> Homo sapiens
<400> 89
   Lys Cys Asn Thr Ala Thr Cys Ala Thr Gln Arg Leu Ala Asn Phe Leu
   1               5                   10                  15
   Val Arg Ser Ser Asn Asn Leu Gly Pro Ile Leu Pro Pro Thr Asn Val
                     20                  25                  30
   Gly Ser Asn Thr Tyr
           35
<210> 90
   <211> 37
   <212> PRT
   <213> Homo sapiens
<400> 90
   Lys Cys Asn Thr Ala Thr Cys Ala Thr Gln Arg Leu Ala Asn Phe Leu
   1               5                   10                  15
   Val Arg Ser Ser Asn Asn Leu Gly Pro Ile Leu Pro Ser Thr Asn Val
               20                  25                  30
   Gly Ser Asn Thr Tyr
           35
<210> 91
   <211> 37
   <212> PRT
   <213> Homo sapiens
<400> 91
   Lys Cys Asn Thr Ala Thr Cys Ala Thr Gln Arg Leu Ala Asn Phe Leu
   1               5                   10                  15
   Ile His Ser Ser Asn Asn Leu Gly Pro Ile Leu Pro Pro Thr Asn Val
               20                  25                  30
   Gly Ser Asn Thr Tyr
           35
<210> 92
   <211> 37
   <212> PRT
   <213> Homo sapiens
<400> 92
   Lys Cys Asn Thr Ala Thr Cys Ala Thr Gln Arg Leu Ala Asn Phe Leu
   1 5 10 15
   Ile His Ser Ser Asn Asn Phe Gly Pro Ile Leu Pro Pro Thr Asn Val
   20 25 30
   Gly Ser Asn Thr Tyr
   35
<210> 93
   <211> 36
   <212> PRT
   <213> Homo sapiens
<400> 93
   Cys Asn Thr Ala Thr Cys Ala Thr Gln Arg Leu Ala Asn Phe Leu Ile
   1 5 10 15
   His Ser Ser Asn Asn Leu Gly Pro Ile Leu Pro Pro Thr Asn Val Gly
   20 25 30
   Ser Asn Thr Tyr
   35
<210> 94
   <211> 37
   <212> PRT
   <213> Homo sapiens
<400> 94
   Lys Cys Asn Thr Ala Thr Cys Ala Thr Gln Arg Leu Ala Asn Phe Leu
   1 5 10 15
   Ile Arg Ser Ser Asn Asn Leu Gly Ala Ile Leu Ser Ser Thr Asn Val
   20 25 30
   Gly Ser Asn Thr Tyr
   35
<210> 95
   <211> 37
   <212> PRT
   <213> Homo sapiens
<400> 95
   Lys Cys Asn Thr Ala Thr Cys Ala Thr Gln Arg Leu Ala Asn Phe Leu
   1 5 10 15
   Ile Arg Ser Ser Asn Asn Leu Gly Ala Val Leu Ser Pro Thr Asn Val
   20 25 30
   Gly Ser Asn Thr Tyr
   35
<210> 96
   <211> 37
   <212> PRT
   <213> Homo sapiens
<400> 96
   Lys Cys Asn Thr Ala Thr Cys Ala Thr Gln Arg Leu Ala Asn Phe Leu
   1               5                   10                  15
   Ile Arg Ser Ser Asn Asn Leu Gly Pro Val Leu Pro Pro Thr Asn Val
               20                  25                  30
   Gly Ser Asn Thr Tyr
           35
<210> 97
   <211> 37
   <212> PRT
   <213> Homo sapiens
<400> 97
   Lys Cys Asn Thr Ala Thr Cys Ala Thr Gln Arg Leu Thr Asn Phe Leu
   1               5                   10                  15
   Val His Ser Ser His Asn Leu Gly Ala Ala Leu Leu Pro Thr Asp Val
               20                  25                  30
   Gly Ser Asn Thr Tyr
           35
<210> 98
   <211> 37
   <212> PRT
   <213> Homo sapiens
<400> 98
   Lys Cys Asn Thr Ala Thr Cys Ala Thr Gln Arg Leu Thr Asn Phe Leu
   1               5                   10                  15
   Val His Ser Ser His Asn Leu Gly Ala Ala Ser Leu Pro Thr Asp Val
               20                  25                  30
   Gly Ser Asn Thr Tyr
           35
<210> 99
   <211> 36
   <212> PRT
   <213> Homo sapiens
<400> 99
   Cys Asn Thr Ala Thr Cys Ala Thr Gln Arg Leu Thr Asn Phe Leu Val
   1               5                   10                  15
   His Ser Ser His Asn Leu Gly Ala Ala Leu Pro Ser Thr Asp Val Gly
               20                  25                  30
   Ser Asn Thr Tyr
           35
<210> 100
   <211> 37
   <212> PRT
   <213> Homo sapiens
<400> 100
   Lys Cys Asn Thr Ala Thr Cys Ala Thr Gln Arg Leu Thr Asn Phe Leu
   1               5                   10                  15
   Val Arg Ser Ser His Asn Leu Gly Ala Ala Leu Ser Pro Thr Asp Val
               20                  25                  30
   Gly Ser Asn Thr Tyr
           35
<210> 101
   <211> 37
   <212> PRT
   <213> Homo sapiens
<400> 101
   Lys Cys Asn Thr Ala Thr Cys Ala Thr Gln Arg Leu Thr Asn Phe Leu
   1               5                   10                  15
   Val Arg Ser Ser His Asn Leu Gly Ala Ile Leu Pro Pro Thr Asp Val
               20                  25                  30
   Gly Ser Asn Thr Tyr
           35
<210> 102
   <211> 37
   <212> PRT
   <213> Homo sapiens
<400> 102
   Lys Cys Asn Thr Ala Thr Cys Ala Thr Gln Arg Leu Thr Asn Phe Leu
   1               5                   10                  15
   Val Arg Ser Ser His Asn Leu Gly Pro Ala Leu Pro Pro Thr Asp Val
               20                  25                  30
   Gly Ser Asn Thr Tyr
           35
<210> 103
   <211> 32
   <212> PRT
   <213> oncorhynchus sp.
<400> 103
   Cys Ser Asn Leu Ser Thr Cys Gly Leu Gly Lys Leu Ser Glu Glu Leu
   1               5                   10                  15
   His Lys Leu Gln Thr Tyr Pro Arg Thr Asn Thr Gly Ser Gly Thr Arg
               20                  25                  30
<210> 104
   <211> 32
   <212> PRT
   <213> Oncorhynchus sp.
<400> 104
   Cys Ser Asn Leu Ser Thr Cys Val Leu Gly Lys Leu Ser Glu Glu Leu
   1               5                   10                  15
   His Lys Leu Gln Thr Leu Pro Arg Thr Asn Thr Gly Ser Gly Thr Arg
               20                  25                  30
<210> 105
   <211> 31
   <212> PRT
   <213> Oncorhynchus sp.
<400> 105
   Cys Gly Ser Leu Ser Thr Cys Gly Leu Gly Lys Leu Ser Glu Glu Leu
   1               5                   10                  15
   His Lys Leu Gln Thr Pro Arg Thr Asn Thr Gly Ser Gly Thr Arg
               20                  25                  30
<210> 106
   <211> 32
   <212> PRT
   <213> Oncorhynchus sp.
<400> 106
   Cys Ser Asn Leu Ser Thr Cys Val Leu Gly Lys Leu Ser Glu Glu Leu
   1               5                   10                  15
   His Lys Leu Gln Thr Tyr Pro Arg Thr Asn Thr Gly Ser Gly Thr Pro
               20                  25                  30
<210> 107
   <211> 32
   <212> PRT
   <213> Oncorhynchus sp.
<400> 107
   Cys Ser Asn Leu Ser Thr Cys Val Leu Gly Lys Leu Ser Gln Glu Leu
   1 5 10 15
   His Arg Leu Gln Thr Tyr Pro Arg Thr Asn Thr Gly Ser Gly Thr Pro
   20 25 30
<210> 108
   <211> 32
   <212> PRT
   <213> Oncorhynchus sp.
<400> 108
   Cys Ser Asn Leu Ser Thr Cys Val Leu Gly Arg Leu Ser Gln Glu Leu
   1 5 10 15
   His Arg Leu Gln Thr Tyr Pro Arg Thr Asn Thr Gly Ser Gly Thr Pro
   20 25 30
<210> 109
   <211> 32
   <212> PRT
   <213> Oncorhynchus sp.
<400> 109
   Cys Ser Asn Leu Ser Thr Cys Val Leu Gly Lys Leu Ser Glu Glu Leu
   1 5 10 15
   His Arg Leu Gln Thr Tyr Pro Arg Thr Asn Thr Gly Ser Gly Thr Pro
   20 25 30
<210> 110
   <211> 32
   <212> PRT
   <213> Oncorhynchus sp.
<400> 110
   Cys Ser Asn Leu Ser Thr Cys Val Leu Gly Arg Leu Ser Glu Glu Leu
   1 5 10 15
   His Arg Leu Gln Thr Tyr Pro Arg Thr Asn Thr Gly Ser Gly Thr Pro
   20 25 30
<210> 111
   <211> 37
   <212> PRT
   <213> Homo sapiens
<220>
   <221> MOD_RES
   <222> (36)
   <223> D-Ser
<400> 111
   Ala Cys Asn Thr Ala Thr Cys Val Thr His Arg Leu Ala Gly Leu Leu
   1 5 10 15
   Ser Arg Ser Gly Gly Met Val Lys Ser Asn Phe Val Pro Thr Asn Val
   20 25 30
   Gly Ser Lys Ser Phe
   35
<210> 112
   <211> 37
   <212> PRT
   <213> Homo sapiens
<220>
   <221> MOD_RES
   <222> (36)
   <223> D-Thr
<400> 112
   Ala Cys Asn Thr Ala Thr Cys Val Thr His Arg Leu Ala Gly Leu Leu
   1 5 10 15
   Ser Arg Ser Gly Gly Met Val Lys Ser Asn Phe Val Pro Thr Asn Val
   20 25 30
   Gly Ser Lys Thr Phe
   35
<210> 113
   <211> 37
   <212> PRT
   <213> Homo sapiens
<220>
   <221> MOD_RES
   <222> (36)
   <223> D-Asp
<400> 113
   Ala Cys Asn Thr Ala Thr Cys Val Thr His Arg Leu Ala Gly Leu Leu
   1 5 10 15
   Ser Arg Ser Gly Gly Met Val Lys Ser Asn Phe Val Pro Thr Asn Val
   20 25 30
   Gly Ser Lys Asp Phe
   35
<210> 114
   <211> 37
   <212> PRT
   <213> Homo sapiens
<220>
   <221> MOD_RES
   <222> (36)
   <223> D-Asn
<400> 114
   Ala Cys Asn Thr Ala Thr Cys Val Thr His Arg Leu Ala Gly Leu Leu
   1               5                   10                  15
   Ser Arg Ser Gly Gly Met Val Lys Ser Asn Phe Val Pro Thr Asn Val
               20                  25                  30
   Gly Ser Lys Asn Phe
           35
<210> 115
   <211> 37
   <212> PRT
   <213> Homo sapiens
<400> 115
   Ala Cys Asn Thr Ala Thr Cys Val Thr His Arg Leu Ala Gly Leu Leu
   1               5                   10                  15
   Ser Arg Ser Gly Gly Met Val Lys Ser Asn Phe Val Pro Thr Asn Val
               20                  25                  30
   Gly Ser Lys Ser Phe
           35
<210> 116
   <211> 37
   <212> PRT
   <213> Homo sapiens
<220>
   <221> MOD_RES
   <222> (36)
   <223> Hse
<400> 116
   Ala Cys Asn Thr Ala Thr Cys Val Thr His Arg Leu Ala Gly Leu Leu
   1               5                   10                  15
   Ser Arg Ser Gly Gly Met Val Lys Ser Asn Phe Val Pro Thr Asn Val
               20                  25                  30
   Gly Ser Lys Xaa Phe
           35
<210> 117
   <211> 37
   <212> PRT
   <213> Homo sapiens
<400> 117
   Ala Cys Asn Thr Ala Thr Cys Val Thr His Arg Leu Ala Gly Leu Leu
   1 5 10 15
   Ser Arg Ser Gly Gly Met Val Lys Ser Asn Phe Val Pro Thr Asn Val
   20 25 30
   Gly Ser Lys Asp Phe
   35
<210> 118
   <211> 37
   <212> PRT
   <213> Homo sapiens
<400> 118
   Ala Cys Asn Thr Ala Thr Cys Val Thr His Arg Leu Ala Gly Leu Leu
   1 5 10 15
   Ser Arg Ser Gly Gly Met Val Lys Ser Asn Phe Val Pro Thr Asn Val
   20 25 30
   Gly Ser Lys Thr Phe
   35
<210> 119
   <211> 37
   <212> PRT
   <213> Homo sapiens
<400> 119
   Ala Cys Asn Thr Ala Thr Cys Val Thr His Arg Leu Ala Gly Leu Leu
   1 5 10 15
   Ser Arg Ser Gly Gly Met Val Lys Ser Asn Phe Val Pro Thr Asn Val
   20 25 30
   Gly Ser Lys Asn Phe
   35
<210> 120
   <211> 48
   <212> PRT
   <213> Mus musculus
<400> 120
   Thr Gln Ala Gln Leu Leu Arg Val Gly Cys Gly Asn Leu Ser Thr Cys
   1 5 10 15
   Gln Val Gln Asn Leu Ser His Arg Leu Trp Gln Leu Met Gly Pro Ala
   20 25 30
   Gly Arg Gln Asp Ser Ala Pro Val Asp Pro Ser Ser Pro His Ser Tyr
   35 40 45
<210> 121
   <211> 47
   <212> PRT
   <213> Mus musculus
<400> 121
   Thr Gln Ala Gln Leu Leu Arg Val Gly Cys Asp Thr Ala Thr Cys Gln
   1               5                   10                  15
   Val Gln Asn Leu Ser His Arg Leu Trp Gln Leu Met Gly Pro Ala Gly
               20                  25                  30
   Arg Gln Asp Ser Ala Pro Val Asp Pro Ser Ser Pro His Ser Tyr
           35                  40                  45
<210> 122
   <211> 47
   <212> PRT
   <213> Mus musculus
<400> 122
   Thr Gln Ala Gln Leu Leu Arg Val Gly Met Val Leu Gly Thr Met Gln
   1               5                   10                  15
   Val Gln Asn Leu Ser His Arg Leu Trp Gln Leu Met Gly Pro Ala Gly
               20                  25                  30
   Arg Gln Asp Ser Ala Pro Val Asp Pro Ser Ser Pro His Ser Tyr
           35                  40                  45
<210> 123
   <211> 47
   <212> PRT
   <213> Mus musculus
<400> 123
   Thr Gln Ala Gln Leu Leu Arg Val Gly Cys Val Leu Gly Thr Cys Gln
   1               5                   10                  15
   Val Gln Asn Leu Ser His Arg Leu Trp Gln Leu Met Gly Pro Ala Gly
               20                  25                  30
   Arg Gln Asp Ser Ala Pro Val Glu Pro Ser Ser Pro His Ser Tyr
           35                  40                  45
<210> 124
   <211> 47
   <212> PRT
   <213> Mus musculus
<400> 124
   Thr Gln Ala Gln Leu Leu Arg Val Gly Cys Val Leu Gly Thr Cys Gln
   1               5                   10                  15
   Val Gln Asn Leu Ser His Arg Leu Trp Gln Leu Met Gly Pro Ala Gly
               20                  25                  30
   Arg Gln Glu Ser Ala Pro Val Glu Pro Ser Ser Pro His Ser Tyr
           35                  40                  45
<210> 125
   <211> 42
   <212> PRT
   <213> Mus musculus
<400> 125
   Thr Gln Ala Gln Leu Leu Arg Val Gly Cys Val Leu Gly Thr Cys Gln
   1               5                   10                  15
   Val Gln Asn Leu Ser His Arg Leu Trp Gln Leu Arg Gln Asp Ser Ala
               20                  25                  30
   Pro Val Asp Pro Ser Ser Pro His Ser Tyr
           35                  40
<210> 126
   <211> 40
   <212> PRT
   <213> Mus musculus
<400> 126
   Thr Gln Ala Gln Leu Leu Arg Val Gly Cys Val Leu Gly Thr Cys Gln
   1               5                   10                  15
   Val Gln Asn Leu Ser His Arg Leu Trp Gln Leu Asp Ser Ala Pro Val
               20                  25                  30
   Asp Pro Ser Ser Pro His Ser Tyr
           35                  40
<210> 127
   <211> 41
   <212> PRT
   <213> Mus musculus
<400> 127
   Arg Val Gly Cys Val Leu Gly Thr Cys Gln Val Gln Asn Leu Ser His
   1               5                   10                  15
   Arg Leu Trp Gln Leu Met Gly Pro Ala Gly Arg Gln Asp Ser Ala Pro
               20                  25                  30
   Val Asp Pro Ser Ser Pro His Ser Tyr
   35 40
<210> 128
   <211> 40
   <212> PRT
   <213> Mus musculus
<400> 128
   Val Gly Cys Val Leu Gly Thr Cys Gln Val Gln Asn Leu Ser His Arg
   1               5                   10                  15
   Leu Trp Gln Leu Met Gly Pro Ala Gly Arg Gln Asp Ser Ala Pro Val
               20                  25                  30
   Glu Pro Ser Ser Pro His Ser Tyr
           35                  40
<210> 129
   <211> 35
   <212> PRT
   <213> Mus musculus
<400> 129
   Val Gly Cys Val Leu Gly Thr Cys Gln Val Gln Asn Leu Ser His Arg
   1               5                   10                  15
   Leu Trp Gln Leu Arg Gln Asp Ser Ala Pro Val Glu Pro Ser Ser Pro
               20                  25                  30
   His Ser Tyr
           35
<210> 130
   <211> 39
   <212> PRT
   <213> Mus musculus
<400> 130
   Gly Cys Val Leu Gly Thr Cys Gln Val Gln Asn Leu Ser His Arg Leu
   1               5                   10                  15
   Trp Gln Leu Met Gly Pro Ala Gly Arg Gln Asp Ser Ala Pro Val Asp
               20                  25                  30
   Pro Ser Ser Pro His Ser Tyr
           35
<210> 131
   <211> 34
   <212> PRT
   <213> Mus musculus
<400> 131
   Gly Cys Asn Thr Ala Thr Cys Gln Val Gln Asn Leu Ser His Arg Leu
   1 5 10 15
   Trp Gln Leu Arg Gln Asp Ser Ala Pro Val Asp Pro Ser Ser Pro His
   20 25 30
   Ser Tyr
<210> 132
   <211> 34
   <212> PRT
   <213> Mus musculus
<400> 132
   Gly Cys Asn Thr Ala Thr Cys Gln Val Gln Asn Leu Ser His Arg Leu
   1 5 10 15
   Trp Gln Leu Arg Gln Asp Ser Ala Pro Val Glu Pro Ser Ser Pro His
   20 25 30
   Ser Tyr
<210> 133
   <211> 35
   <212> PRT
   <213> Mus musculus
<400> 133
   Gly Cys Ser Asn Leu Ser Thr Cys Gln Val Gln Asn Leu Ser His Arg
   1 5 10 15
   Leu Trp Gln Leu Arg Gln Asp Ser Ala Pro Val Glu Pro Ser Ser Pro
   20 25 30
   His Ser Tyr
   35
<210> 134
   <211> 35
   <212> PRT
   <213> Mus musculus
<400> 134
   Gly Cys Gly Asn Leu Ser Thr Cys Gln Val Gln Asn Leu Ser His Arg
   1 5 10 15
   Leu Trp Gln Leu Arg Gln Asp Ser Ala Pro Val Glu Pro Ser Ser Pro
   20 25 30
   His Ser Tyr
   35
<210> 135
   <211> 34
   <212> PRT
   <213> Mus musculus
<400> 135
   Gly Cys Val Leu Gly Thr Cys Gln Val Gln Asn Leu Ser His Arg Leu
   1               5                   10                  15
   Trp Gln Leu Arg Gln Glu Ser Ala Pro Val Glu Pro Ser Ser Pro His
               20                  25                  30
   Ser Tyr
<210> 136
   <211> 38
   <212> PRT
   <213> Mus musculus
<400> 136
   Cys Val Leu Gly Thr Cys Gln Val Gln Asn Leu Ser His Arg Leu Trp
   1               5                   10                  15
   Gln Leu Met Gly Pro Ala Gly Arg Gln Asp Ser Ala Pro Val Asp Pro
               20                  25                  30
   Ser Ser Pro His Ser Tyr
           35
<210> 137
   <211> 32
   <212> PRT
   <213> Mus musculus
<400> 137
   Gln Val Gln Asn Leu Ser His Arg Leu Trp Gln Leu Met Gly Pro Ala
   1               5                   10                  15
   Gly Arg Gln Asp Ser Ala Pro Val Asp Pro Ser Ser Pro His Ser Tyr
               20                  25                  30
<210> 138
   <211> 31
   <212> PRT
   <213> Mus musculus
<400> 138
   Val Gln Asn Leu Ser His Arg Leu Trp Gln Leu Met Gly Pro Ala Gly
   1               5                   10                  15
   Arg Gln Asp Ser Ala Pro Val Asp Pro Ser Ser Pro His Ser Tyr
               20                  25                  30
<210> 139
   <211> 30
   <212> PRT
   <213> Mus musculus
<400> 139
   Val Gln Asn Leu Ser His Arg Leu Gln Leu Met Gly Pro Ala Gly Arg
   1 5 10 15
   Gln Asp Ser Ala Pro Val Asp Pro Ser Ser Pro His Ser Tyr
   20 25 30
<210> 140
   <211> 30
   <212> PRT
   <213> Mus musculus
<400> 140
   Gly Thr Met Gln Val Gln Asn Leu Ser His Arg Leu Trp Gln Leu Arg
   1 5 10 15
   Gln Asp Ser Ala Pro Val Glu Pro Ser Ser Pro His Ser Tyr
   20 25 30
<210> 141
   <211> 8
   <212> PRT
   <213> Homo sapiens
<220>
   <221> MOD_RES
   <222> (2)
   <223> Tyr(SO3H)
<400> 141
<210> 142
   <211> 8
   <212> PRT
   <213> Homo sapiens
<400> 142
<210> 143
   <211> 6
   <212> PRT
   <213> Homo sapiens
<400> 143
   Met Gly Trp Met Asp Phe
   1 5
<210> 144
   <211> 5
   <212> PRT
   <213> Homo sapiens
<400> 144
   Gly Trp Met Asp Phe
   1 5
<210> 145
   <211> 4
   <212> PRT
   <213> Homo sapiens
<400> 145
   Trp Met Asp Phe
   1
<210> 146
   <211> 9
   <212> PRT
   <213> Homo sapiens
<220>
   <221> MOD_RES
   <222> (3)
   <223> Tyr(SO3H)
<400> 146
   Lys Asp Tyr Met Gly Trp Met Asp Phe
   1 5
<210> 147
   <211> 9
   <212> PRT
   <213> Homo sapiens
<400> 147
   Lys Asp Tyr Met Gly Trp Met Asp Phe
   1 5
<210> 148
   <211> 7
   <212> PRT
   <213> Homo sapiens
<400> 148
   Lys Met Gly Trp Met Asp Phe
   1 5
<210> 149
   <211> 6
   <212> PRT
   <213> Homo sapiens
<400> 149
   Lys Gly Trp Met Asp Phe
   1 5
<210> 150
   <211> 5
   <212> PRT
   <213> Homo sapiens
<400> 150
   Lys Trp Met Asp Phe
   1 5
<210> 151
   <211> 167
   <212> PRT
   <213> Homo sapiens
<400> 151
   Met His Trp Gly Thr Leu Cys Gly Phe Leu Trp Leu Trp Pro Tyr Leu
   1 5 10 15
   Phe Tyr Val Gln Ala Val Pro Ile Gln Lys Val Gln Asp Asp Thr Lys
   20 25 30
   Thr Leu Ile Lys Thr Ile Val Thr Arg Ile Asp Asp Ile Ser His Thr
   35 40 45
   Gln Ser Val Ser Ser Lys Gln Lys Val Thr Gly Leu Asp Phe Ile Pro
   50 55 60
   Gly Leu His Pro Ile Leu Thr Leu Ser Lys Met Asp Gln Thr Leu Ala
   65 70 75 80
   Val Tyr Gln Gln Ile Leu Thr Ser Met Pro Ser Arg Asn Val Ile Gln
   85 90 95
   Ile Ser Asn Asp Leu Glu Asn Leu Arg Asp Leu Leu His Val Leu Ala
   100 105 110
   Phe Ser Lys Ser Cys His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Cys
   165
<210> 152
   <211> 167
   <212> PRT
   <213> Homo sapiens
<400> 152
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Glu Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Met Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Cys His Leu Pro Trp Ala Ser Gly Leu Glu Thr Leu
           115                 120                 125
   Asp Ser Leu Gly Gly Val Leu Glu Ala Ser Gly Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 153
   <211> 167
   <212> PRT
   <213> Homo sapiens
<400> 153
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Ala
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Met Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Cys His Leu Pro Trp Ala Ser Gly Leu Glu Thr Leu
           115                 120                 125
   Asp Ser Leu Gly Gly Val Leu Glu Ala Ser Gly Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 154
   <211> 167
   <212> PRT
   <213> Homo sapiens
<400> 154
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Glu Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Met Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Cys His Leu Pro Trp Ala Ser Gly Leu Glu Thr Leu
           115                 120                 125
   Asp Ser Leu Gly Gly Val Leu Glu Ala Ser Gly Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 155
   <211> 166
   <212> PRT
   <213> Homo sapiens
<400> 155
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Ser Val Ser Ser Lys Gln Lys Val Thr Gly Leu Asp Phe Ile Pro Gly
       50                  55                  60
   Leu His Pro Ile Leu Thr Leu Ser Lys Met Asp Gln Thr Leu Ala Val
   65                  70                  75                  80
   Tyr Gln Gln Ile Leu Thr Ser Met Pro Ser Arg Asn Val Ile Gln Ile
                   85                  90                  95
   Ser Asn Asp Leu Glu Asn Leu Arg Asp Leu Leu His Val Leu Ala Phe
               100                 105                 110
   Ser Lys Ser Cys His Leu Pro Trp Ala Ser Gly Leu Glu Thr Leu Asp
           115                 120                 125
   Ser Leu Gly Gly Val Leu Glu Ala Ser Gly Tyr Ser Thr Glu Val Val
       130                 135                 140
   Ala Leu Ser Arg Leu Gln Gly Ser Leu Gln Asp Met Leu Trp Gln Leu
   145                 150                 155                 160
   Asp Leu Ser Pro Gly Cys
                   165
<210> 156
   <211> 167
   <212> PRT
   <213> Homo sapiens
<400> 156
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Ala Asp Gln Thr Leu Ala
   65                  70                  75                      80
   Val Tyr Gln Gln Ile Leu Thr Ser Met Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Cys His Leu Pro Trp Ala Ser Gly Leu Glu Thr Leu
           115                 120                 125
   Asp Ser Leu Gly Gly Val Leu Glu Ala Ser Gly Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 157
   <211> 167
   <212> PRT
   <213> Homo sapiens
<400> 157
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Leu Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Cys His Leu Pro Trp Ala Ser Gly Leu Glu Thr Leu
           115                 120                 125
   Asp Ser Leu Gly Gly Val Leu Glu Ala Ser Gly Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 158
   <211> 167
   <212> PRT
   <213> Homo sapiens
<400> 158
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Met Pro Ser Arg Asp Val Ile Gln
   85 90 95
   Ile Ser Asn Asp Leu Glu Asn Leu Arg Asp Leu Leu His Val Leu Ala
   100 105 110
   Phe Ser Lys Ser Cys His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Cys
   165
<210> 159
   <211> 167
   <212> PRT
   <213> Homo sapiens
<400> 159
   Met His Trp Gly Thr Leu Cys Gly Phe Leu Trp Leu Trp Pro Tyr Leu
   1 5 10 15
   Phe Tyr Val Gln Ala Val Pro Ile Gln Lys Val Gln Asp Asp Thr Lys
   20 25 30
   Thr Leu Ile Lys Thr Ile Val Thr Arg Ile Asn Asp Ile Ser His Thr
   35 40 45
   Gln Ser Val Ser Ser Lys Gln Lys Val Thr Gly Leu Asp Phe Ile Pro
   50 55 60
   Gly Leu His Pro Ile Leu Thr Leu Ser Lys Met Asp Gln Thr Leu Ala
   65 70 75 80
   Val Tyr Gln Gln Ile Leu Thr Ser Met Pro Ser Arg Glu Val Ile Gln
   85 90 95
   Ile Ser Asn Asp Leu Glu Asn Leu Arg Asp Leu Leu His Val Leu Ala
   100 105 110
   Phe Ser Lys Ser Cys His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Cys
   165
<210> 160
   <211> 167
   <212> PRT
   <213> Homo sapiens
<400> 160
   Met His Trp Gly Thr Leu Cys Gly Phe Leu Trp Leu Trp Pro Tyr Leu
   1 5 10 15
   Phe Tyr Val Gln Ala Val Pro Ile Gln Lys Val Gln Asp Asp Thr Lys
   20 25 30
   Thr Leu Ile Lys Thr Ile Val Thr Arg Ile Asn Asp Ile Ser His Thr
   35 40 45
   Gln Ser Val Ser Ser Lys Gln Lys Val Thr Gly Leu Asp Phe Ile Pro
   50 55 60
   Gly Leu His Pro Ile Leu Thr Leu Ser Lys Met Asp Gln Thr Leu Ala
   65 70 75 80
   Val Tyr Gln Gln Ile Leu Thr Ser Met Pro Ser Arg Asn Val Ile Gln
   85 90 95
   Ile Ala Asn Asp Leu Glu Asn Leu Arg Asp Leu Leu His Val Leu Ala
   100 105 110
   Phe Ser Lys Ser Cys His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Cys
   165
<210> 161
   <211> 167
   <212> PRT
   <213> Homo sapiens
<400> 161
   Met His Trp Gly Thr Leu Cys Gly Phe Leu Trp Leu Trp Pro Tyr Leu
   1 5 10 15
   Phe Tyr Val Gln Ala Val Pro Ile Gln Lys Val Gln Asp Asp Thr Lys
   20 25 30
   Thr Leu Ile Lys Thr Ile Val Thr Arg Ile Asn Asp Ile Ser His Thr
   35 40 45
   Gln Ser Val Ser Ser Lys Gln Lys Val Thr Gly Leu Asp Phe Ile Pro
   50 55 60
   Gly Leu His Pro Ile Leu Thr Leu Ser Lys Met Asp Gln Thr Leu Ala
   65 70 75 80
   Val Tyr Gln Gln Ile Leu Thr Ser Met Pro Ser Arg Asn Val Ile Gln
   85 90 95
   Ile Ser Asn Asp Leu Glu Asn Leu Arg Asp Leu Leu His Val Leu Ala
   100 105 110
   Phe Ser Lys Ser Ser His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Cys
   165
<210> 162
   <211> 167
   <212> PRT
   <213> Homo sapiens
<400> 162
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Met Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Cys His Leu Pro Trp Ala Ser Gly Leu Glu Thr Leu
           115                 120                 125
   Asp Ser Leu Gly Gly Val Leu Glu Ala Ser Leu Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 163
   <211> 167
   <212> PRT
   <213> Homo sapiens
<400> 163
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Met Pro Ser Arg Asn Val Ile Gln
                   85                  90                  95
   Ile Ser Asn Asp Leu Glu Asn Leu Arg Asp Leu Leu His Val Leu Ala
   100 105 110
   Phe Ser Lys Ser Cys His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Ser
   165
<210> 164
   <211> 167
   <212> PRT
   <213> Homo sapiens
<400> 164
   Met His Trp Gly Thr Leu Cys Gly Phe Leu Trp Leu Trp Pro Tyr Leu
   1 5 10 15
   Phe Tyr Val Gln Ala Val Pro Ile Gln Lys Val Gln Asp Asp Thr Lys
   20 25 30
   Thr Leu Ile Lys Thr Ile Val Thr Arg Ile Asp Asp Ile Ser His Thr
   35 40 45
   Glu Ser Val Ser Ser Lys Gln Lys Val Thr Gly Leu Asp Phe Ile Pro
   50 55 60
   Gly Leu His Pro Ile Leu Thr Leu Ser Lys Met Asp Gln Thr Leu Ala
   65 70 75 80
   Val Tyr Gln Gln Ile Leu Thr Ser Met Pro Ser Arg Asn Val Ile Gln
   85 90 95
   Ile Ser Asn Asp Leu Glu Asn Leu Arg Asp Leu Leu His Val Leu Ala
   100 105 110
   Phe Ser Lys Ser Cys His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Cys
   165
<210> 165
   <211> 167
   <212> PRT
   <213> Homo sapiens
<400> 165
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asp Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Ala Asp Gln Thr Leu Ala
   65                  70                  75                  80
   Val Tyr Gln Gln Ile Leu Thr Ser Met Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Cys His Leu Pro Trp Ala Ser Gly Leu Glu Thr Leu
           115                 120                 125
   Asp Ser Leu Gly Gly Val Leu Glu Ala Ser Gly Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 166
   <211> 167
   <212> PRT
   <213> Homo sapiens
<400> 166
   Met His Trp Gly Thr Leu Cys Gly Phe Leu Trp Leu Trp Pro Tyr Leu
   1               5                   10                  15
   Phe Tyr Val Gln Ala Val Pro Ile Gln Lys Val Gln Asp Asp Thr Lys
               20                  25                  30
   Thr Leu Ile Lys Thr Ile Val Thr Arg Ile Asn Asp Ile Ser His Thr
           35                  40                  45
   Gln Ser Val Ser Ser Lys Gln Lys Val Thr Gly Leu Asp Phe Ile Pro
       50                  55                  60
   Gly Leu His Pro Ile Leu Thr Leu Ser Lys Met Asp Gln Thr Leu Ala
   65                  70                  75                  80
   Val Tyr Gln Gln Ile Leu Thr Ser Leu Pro Ser Arg Asn Val Ile Gln
                   85                  90                  95
   Ile Ser Asn Asp Leu Glu Asn Leu Arg Asp Leu Leu His Val Leu Ala
               100                 105                 110
   Phe Ser Lys Ser Ser His Leu Pro Trp Ala Ser Gly Leu Glu Thr Leu
           115                 120                 125
   Asp Ser Leu Gly Gly Val Leu Glu Ala Ser Gly Tyr Ser Thr Glu Val
       130                 135                 140
   Val Ala Leu Ser Arg Leu Gln Gly Ser Leu Gln Asp Met Leu Trp Gln
   145                 150                 155                 160
   Leu Asp Leu Ser Pro Gly Cys
                   165
<210> 167
   <211> 167
   <212> PRT
   <213> Homo sapiens
<400> 167
   Met His Trp Gly Thr Leu Cys Gly Phe Leu Trp Leu Trp Pro Tyr Leu
   1 5 10 15
   Phe Tyr Val Gln Ala Val Pro Ile Gln Lys Val Gln Asp Asp Thr Lys
   20 25 30
   Thr Leu Ile Lys Thr Ile Val Thr Arg Ile Asn Asp Ile Ser His Thr
   35 40 45
   Gln Ser Val Ser Ser Lys Gln Lys Val Thr Gly Leu Asp Phe Ile Pro
   50 55 60
   Gly Leu His Pro Ile Leu Thr Leu Ser Lys Met Asp Gln Thr Leu Ala
   65 70 75 80
   Val Tyr Gln Gln Ile Leu Thr Ser Met Pro Ser Arg Glu Val Ile Gln
   85 90 95
   Ile Ser Asn Asp Leu Glu Asn Leu Arg Asp Leu Leu His Val Leu Ala
   100 105 110
   Phe Ser Lys Ser Cys His Leu Pro Trp Ala Ser Gly Leu Glu Thr Leu
   115 120 125
   Asp Ser Leu Gly Gly Val Leu Glu Ala Ser Gly Tyr Ser Thr Glu Val
   130 135 140
   Val Ala Leu Ser Arg Leu Gln Gly Ser Leu Gln Asp Met Leu Trp Gln
   145 150 155 160
   Leu Asp Leu Ser Pro Gly Ser
   165
<210> 168
   <211> 31
   <212> PRT
   <213> Homo sapiens
<400> 168
   His Ala Gln Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 169
   <211> 31
   <212> PRT
   <213> Homo sapiens
<220>
   <221> MOD_RES
   <222> (3)
   <223> D-Gln
<400> 169
   His Ala Gln Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 170
   <211> 31
   <212> PRT
   <213> Homo sapiens
<400> 170
   His Ala Glu Gly Thr Phe Thr Ser Asp Thr Ser Lys Tyr Leu Glu Gly
   1 5 10 15
   Gln Ala Ala Leu Glu Phe Ile Ala Trp Leu Val Lys Gly Arg Gly
   20 25 30
<210> 171
   <211> 31
   <212> PRT
   <213> Homo sapiens
<400> 171
   His Ala Glu Gly Thr Phe Thr Ser Asp Val Ser Lys Tyr Leu Glu Gly
   1 5 10 15
   Gln Ala Ala Leu Glu Phe Ile Ala Trp Leu Val Lys Gly Arg Gly
   20 25 30
<210> 172
   <211> 30
   <212> PRT
   <213> Homo sapiens
<400> 172
   His Gly Glu Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg
   20 25 30
<210> 173
   <211> 31
   <212> PRT
   <213> Homo sapiens
<400> 173
   His Ala Gln Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 174
   <211> 31
   <212> PRT
   <213> Homo sapiens
<220>
   <221> MOD_RES
   <222> (3)
   <223> D-Gln
<400> 174
   His Ala Gln Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 175
   <211> 31
   <212> PRT
   <213> Homo sapiens
<220>
   <221> MOD_RES
   <222> (3)
   <223> acetyl-Lys
<400> 175
   His Ala Lys Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 176
   <211> 31
   <212> PRT
   <213> Homo sapiens
<400> 176
   His Ala Thr Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 177
   <211> 31
   <212> PRT
   <213> Homo sapiens
<220>
   <221> MOD_RES
   <222> (3)
   <223> D-Thr
<400> 177
   His Ala Thr Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 178
   <211> 31
   <212> PRT
   <213> Homo sapiens
<400> 178
   His Ala Asn Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 179
   <211> 31
   <212> PRT
   <213> Homo sapiens
<220>
   <221> MOD_RES
   <222> (3)
   <223> D-Asn
<400> 179
   His Ala Asn Gly Thr Phe Thr Ser Asp Val Ser Ser Tyr Leu Glu Gly
   1 5 10 15
   Gln Ala Ala Leu Glu Phe Ile Ala Trp Leu Val Lys Gly Arg Gly
   20 25 30
<210> 180
   <211> 31
   <212> PRT
   <213> Homo sapiens
<400> 180
   His Ala Glu Gly Thr Phe Thr Ser Asp Val Ser Ser Tyr Leu Glu Ser
   1 5 10 15
   Arg Arg Ala Gln Glu Phe Ile Ala Trp Leu Val Lys Gly Arg Gly
   20 25 30
<210> 181
   <211> 31
   <212> PRT
   <213> Homo sapiens
<400> 181
   His Ala Glu Gly Thr Phe Thr Ser Asp Thr Ser Lys Tyr Leu Glu Gly
   1               5                   10                  15
   Gln Ala Ala Leu Glu Phe Ile Ala Trp Leu Val Lys Gly Arg Gly
               20                  25                  30
<210> 182
   <211> 31
   <212> PRT
   <213> Homo sapiens
<400> 182
   His Ala Glu Gly Thr Phe Thr Ser Asp Val Ser Lys Tyr Leu Glu Gly
   1               5                   10                  15
   Gln Ala Ala Leu Glu Phe Ile Ala Trp Leu Val Lys Gly Arg Gly
               20                  25                  30
<210> 183
   <211> 31
   <212> PRT
   <213> Homo sapiens
<400> 183
   His Ala Glu Gly Thr Phe Thr Ser Asp Val Ser Ser Tyr Leu Glu Gly
   1               5                   10                  15
   Arg Ala Ala Leu Glu Phe Ile Ala Trp Leu Val Lys Gly Arg Gly
               20                  25                  30
<210> 184
   <211> 31
   <212> PRT
   <213> Homo sapiens
<400> 184
   His Ala Glu Gly Thr Phe Thr Ser Asp Val Ser Ser Tyr Leu Glu Gly
   1               5                   10                  15
   Gln Arg Ala Leu Glu Phe Ile Ala Trp Leu Val Lys Gly Arg Gly
               20                  25                  30
<210> 185
   <211> 39
   <212> PRT
   <213> Heloderma suspectum
<400> 185
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 186
   <211> 39
   <212> PRT
   <213> Heloderma suspectum
<400> 186
   His Gly Glu Gly Ala Phe Thr Ser Asp Leu Ser Lys Gln Leu Glu Glu
   1 5 10 15
   Glu Ala Val Arg Leu Phe Ile Glu Phe Leu Lys Asn Gly Gly Pro Ser
   20 25 30
   Ser Gly Ala Pro Pro Pro Ser
   35
<210> 187
   <211> 39
   <212> PRT
   <213> Heloderma suspectum
<400> 187
   His Gly Glu Gly Thr Phe Thr Ser Asp Leu Ser Lys Gln Leu Glu Glu
   1 5 10 15
   Glu Ala Val Arg Leu Ala Ile Glu Phe Leu Lys Asn Gly Gly Pro Ser
   20 25 30
   Ser Gly Ala Pro Pro Pro Ser
   35
<210> 188
   <211> 37
   <212> PRT
   <213> Xenopus laevis
<220>
   <221> MOD_RES
   <222> (37)
   <223> Ser-OH
<400> 188
   His Ala Glu Gly Thr Phe Thr Ser Asp Val Thr Gln Gln Leu Asp Glu
   1               5                   10                  15
   Lys Ala Ala Lys Glu Phe Ile Asp Trp Leu Ile Asn Gly Gly Pro Ser
               20                  25                  30
   Lys Glu Ile Ile Ser
           35
<210> 189
   <211> 37
   <212> PRT
   <213> Homo sapiens
<400> 189
   Lys Cys Asn Thr Ala Thr Cys Ala Thr Gln Arg Leu Ala Asn Phe Leu
   1               5                   10                  15
   Val His Ser Ser Asn Asn Phe Gly Ala Ile Leu Pro Ser Thr Asn Val
               20                  25                  30
   Gly Ser Asn Thr Tyr
           35
<210> 190
   <211> 28
   <212> PRT
   <213> Heloderma suspectum
<400> 190
   His Gly Glu Gly Thr Phe Thr Ser Asp Leu Ser Lys Gln Leu Glu Glu
   1               5                   10                  15
   Glu Ala Val Arg Leu Phe Ile Glu Trp Leu Lys Asn
               20                  25
<210> 191
   <211> 36
   <212> PRT
   <213> Homo sapiens
<400> 191
   Lys Cys Asn Thr Ala Thr Cys Ala Thr Gln Arg Leu Ala Asn Phe Leu
   1               5                   10                  15
   Val His Arg Tyr Tyr Ala Ser Leu Arg His Tyr Leu Asn Leu Val Thr
               20                  25                  30
   Arg Gln Arg Tyr
           35
<210> 192
   <211> 36
   <212> PRT
   <213> Homo sapiens
<400> 192
   Tyr Pro Leu Lys Pro Glu Ala Pro Gly Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 193
   <211> 36
   <212> PRT
   <213> Homo sapiens
<400> 193
   Tyr Pro Val Lys Pro Glu Ala Pro Gly Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 194
   <211> 36
   <212> PRT
   <213> Homo sapiens
<400> 194
   Tyr Pro Ile Arg Pro Glu Ala Pro Gly Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 195
   <211> 36
   <212> PRT
   <213> Homo sapiens
<400> 195
   Tyr Pro Ile Gln Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Val Thr
   20 25 30
   Arg Gln Arg Tyr
   35
<210> 196
   <211> 36
   <212> PRT
   <213> Homo sapiens
<400> 196
   Tyr Pro Ile Asn Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Val Thr
   20 25 30
   Arg Gln Arg Tyr
   35
<210> 197
   <211> 36
   <212> PRT
   <213> Homo sapiens
<400> 197
   Tyr Pro Ile Lys Pro Glu Ala Pro Gly Glu Asp Ala Ser Pro Glu Glu
   1 5 10 15
   Leu Asn Arg Tyr Tyr Ala Ser Leu Lys His Tyr Leu Asn Leu Val Thr
   20 25 30
   Arg Gln Arg Tyr
   35
<210> 198
   <211> 36
   <212> PRT
   <213> Homo sapiens
<400> 198
   Tyr Pro Ile Lys Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Val Thr
   20 25 30
   Arg Pro Arg Tyr
   35
<210> 199
   <211> 36
   <212> PRT
   <213> Homo sapiens
<400> 199
   Tyr Pro Ile Lys Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Val Thr
   20 25 30
   Arg His Arg Tyr
   35
<210> 200
   <211> 36
   <212> PRT
   <213> Homo sapiens
<400> 200
   Tyr Pro Ile Lys Pro Glu Ala Pro Gly Glu Asp Ala Pro Ala Glu Glu
   1 5 10 15
   Leu Asn Arg Tyr Tyr Ala Ser Leu Arg His Tyr Leu Asn Leu Val Thr
   20 25 30
   Arg Gln Arg Tyr
   35
<210> 201
   <211> 36
   <212> PRT
   <213> Homo sapiens
<400> 201
   Tyr Pro Ile Lys Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Leu Thr
   20 25 30
   Arg Pro Arg Tyr
   35
<210> 202
   <211> 35
   <212> PRT
   <213> Homo sapiens
<400> 202
   Tyr Pro Ile Pro Glu Ala Pro Gly Glu Asp Ala Ser Pro Glu Glu Leu
   1               5                   10                  15
   Asn Arg Tyr Tyr Ala Ser Leu Arg His Tyr Leu Asn Leu Val Thr Arg
               20                  25                  30
   Gln Arg Tyr
           35
<210> 203
   <211> 43
   <212> PRT
   <213> Artificial sequence
<220>
   <223> Description of Artificial Sequence: Chimeric human and salmon construct
<220>
   <221> MOD_RES
   <222> (2)
   <223> Phe(CH2so3)
<400> 203
   Asp Phe Met Gly Trp Met Asp Phe Gly Lys Arg Lys Cys Asn Thr Ala
   1               5                   10                  15
   Thr Cys Ala Thr Gln Arg Leu Ala Asn Glu Leu Val Arg Leu Gln Thr
               20                  25                  30
   Tyr Pro Arg Thr Asn Val Gly Ser Asn Thr Tyr
           35                  40
<210> 204
   <211> 31
   <212> PRT
   <213> Homo sapiens
<400> 204
   His Ala Glu Gly Thr Phe Thr Ser Asp Val Ser Ser Thr Leu Glu Gly
   1               5                   10                  15
   Gln Ala Ala Leu Glu Phe Ile Ala Trp Leu Val Lys Gly Arg Gly
               20                  25                  30
<210> 205
   <211> 29
   <212> PRT
   <213> Homo sapiens
<400> 205
   Glu Gly Thr Phe Thr Ser Asp Val Ser Ser Thr Leu Glu Gly Gln Ala
   1               5                   10                  15
   Ala Leu Glu Phe Ile Ala Trp Leu Val Lys Gly Arg Gly
               20                  25
<210> 206
   <211> 28
   <212> PRT
   <213> Homo sapiens
<400> 206
   Glu Gly Thr Phe Thr Ser Asp Val Ser Ser Thr Leu Glu Gly Gln Ala
   1 5 10 15
   Ala Leu Glu Phe Ile Ala Trp Leu Val Lys Gly Arg
   20 25
<210> 207
   <211> 31
   <212> PRT
   <213> Heloderma suspectum
<400> 207
   Asp Leu Ser Lys Gln Met Glu Glu Glu Ala Val Arg Leu Phe Ile Glu
   1 5 10 15
   Trp Leu Lys Asn Gly Gly Pro Ser Ser Gly Ala Pro Pro Pro Ser
   20 25 30
<210> 208
   <211> 4
   <212> PRT
   <213> Homo sapiens
<400> 208
   Trp Met Asp Phe
   1
<210> 209
   <211> 5
   <212> PRT
   <213> Homo sapiens
<400> 209
   Gly Trp Met Asp Phe
   1 5
<210> 210
   <211> 36
   <212> PRT
   <213> Homo sapiens
<400> 210
   Lys Cys Asn Thr Ala Thr Cys Ala Thr Gln Arg Leu Ala Asn Phe Leu
   1 5 10 15
   Val His Ser Ser Asn Asn Phe Gly Ala Ile Leu Ser Ser Thr Asn Val
   20 25 30
   Gly Ser Asn Thr
   35
<210> 211
   <211> 35
   <212> PRT
   <213> Homo sapiens
<400> 211
   Lys Cys Asn Thr Ala Thr Cys Ala Thr Gln Arg Leu Ala Asn Phe Leu
   1 5 10 15
   Val His Ser Ser Asn Asn Phe Gly Ala Ile Leu Ser Ser Thr Asn Val
   20 25 30
   Gly Ser Asn
   35
<210> 212
   <211> 20
   <212> PRT
   <213> Homo sapiens
<400> 212
   Lys Cys Asn Thr Ala Thr Cys Ala Thr Gln Arg Leu Ala Asn Phe Leu
   1 5 10 15
   Val His Ser Ser
   20
<210> 213
   <211> 18
   <212> PRT
   <213> Homo sapiens
<400> 213
   Lys Cys Asn Thr Ala Thr Cys Ala Thr Gln Arg Leu Ala Asn Phe Leu
   1 5 10 15
   Val His
<210> 214
   <211> 17
   <212> PRT
   <213> Homo sapiens
<400> 214
   Lys Cys Asn Thr Ala Thr Cys Ala Thr Gln Arg Leu Ala Asn Phe Leu
    1               5                   10                  15
   Val
<210> 215
   <211> 16
   <212> PRT
   <213> Homo sapiens
<400> 215
   Lys Cys Asn Thr Ala Thr Cys Ala Thr Gln Arg Leu Ala Asn Phe Leu
    1               5                   10                  15
<210> 216
   <211> 15
   <212> PRT
   <213> Homo sapiens
<400> 216
   Lys Cys Asn Thr Ala Thr Cys Ala Thr Gln Arg Leu Ala Asn Phe
    1               5                   10                  15
<210> 217
   <211> 7
   <212> PRT
   <213> Homo sapiens
<400> 217
   Lys Cys Asn Thr Ala Thr Cys
    1               5
<210> 218
   <211> 25
   <212> PRT
   <213> Homo sapiens
<400> 218
   Met Leu Gly Thr Tyr Thr Gln Asp Phe Asn Lys Phe His Thr Phe Pro
    1               5                   10                  15
   Gln Thr Ala Ile Gly Val Gly Ala Pro
               20                  25
<210> 219
   <211> 20
   <212> PRT
   <213> Homo sapiens
<400> 219
   Met Leu Gly Thr Tyr Thr Gln Asp Phe Asn Lys Phe His Thr Phe Pro
   1 5 10 15
   Gln Thr Ala Ile
   20
<210> 220
   <211> 19
   <212> PRT
   <213> Homo sapiens
<400> 220
   Met Leu Gly Thr Tyr Thr Gln Asp Phe Asn Lys Phe His Thr Phe Pro
   1 5 10 15
   Gln Thr Ala
<210> 221
   <211> 3
   <212> PRT
   <213> Homo sapiens
<400> 221
   Met Leu Gly
   1
<210> 222
   <211> 9
   <212> PRT
   <213> Homo sapiens
<400> 222
   Lys Phe His Thr Phe Pro Gln Thr Ala
   1 5
<210> 223
   <211> 10
   <212> PRT
   <213> Homo sapiens
<400> 223
   Lys Phe His Thr Phe Pro Gln Thr Ala Ile
   1 5 10
<210> 224
   <211> 10
   <212> PRT
   <213> Homo sapiens
<400> 224
   Gln Asn Leu Ser His Arg Leu Trp Gln Leu
   1 5 10
<210> 225
   <211> 146
   <212> PRT
   <213> Homo sapiens
<400> 225
   Val Pro Ile Gln Lys Val Gln Asp Asp Thr Lys Thr Leu Ile Lys Thr
   1 5 10 15
   Ile Val Thr Arg Ile Asn Asp Ile Ser His Thr Gln Ser Val Ser Ser
   20 25 30
   Lys Gln Lys Val Thr Gly Leu Asp Phe Ile Pro Gly Leu His Pro Ile
   35 40 45
   Leu Thr Leu Ser Lys Met Asp Gln Thr Leu Ala Val Tyr Gln Gln Ile
   50 55 60
   Leu Thr Ser Met Pro Ser Arg Asn Val Ile Gln Ile Ser Asn Asp Leu
   65 70 75 80
   Glu Asn Leu Arg Asp Leu Leu His Val Leu Ala Phe Ser Lys Ser Cys
   85 90 95
   His Leu Pro Trp Ala Ser Gly Leu Glu Thr Leu Asp Ser Leu Gly Gly
   100 105 110
   Val Leu Glu Ala Ser Gly Tyr Ser Thr Glu Val Val Ala Leu Ser Arg
   115 120 125
   Leu Gln Gly Ser Leu Gln Asp Met Leu Trp Gln Leu Asp Leu Ser Pro
   130 135 140
   Gly Cys
   145
<210> 226
   <211> 18
   <212> PRT
   <213> Homo sapiens
<400> 226
   Lys Val Thr Gly Leu Asp Phe Ile Pro Gly Leu His Pro Ile Leu Thr
   1 5 10 15
   Leu Ser
<210> 227
   <211> 35
   <212> PRT
   <213> Homo sapiens
<400> 227
   Tyr Pro Ile Lys Pro Glu Ala Pro Gly Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg
           35
<210> 228
   <211> 30
   <212> PRT
   <213> Homo sapiens
<400> 228
   Tyr Pro Ile Lys Pro Glu Ala Pro Gly Glu Asp Ala Ser Pro Glu Glu
   1               5                   10                  15
   Leu Asn Arg Tyr Tyr Ala Ser Leu Arg His Tyr Leu Asn Leu
               20                  25                  30
<210> 229
   <211> 25
   <212> PRT
   <213> Homo sapiens
<400> 229
   Tyr Pro Ile Lys Pro Glu Ala Pro Gly Glu Asp Ala Ser Pro Glu Glu
   1               5                   10                  15
   Leu Asn Arg Tyr Tyr Ala Ser Leu Arg
               20                  25
<210> 230
   <211> 15
   <212> PRT
   <213> Homo sapiens
<400> 230
   Tyr Pro Ile Lys Pro Glu Ala Pro Gly Glu Asp Ala Ser Pro Glu
   1               5                   10                  15
<210> 231
   <211> 10
   <212> PRT
   <213> Homo sapiens
<400> 231
   Tyr Pro Ile Lys Pro Glu Ala Pro Gly Glu
   1               5                   10
<210> 232
   <211> 35
   <212> PRT
   <213> Homo sapiens
<400> 232
   Pro Ile Lys Pro Glu Ala Pro Gly Glu Asp Ala Ser Pro Glu Glu Leu
   1               5                   10                  15
   Asn Arg Tyr Tyr Ala Ser Leu Arg His Tyr Leu Asn Leu Val Thr Arg
               20                  25                  30
   Gln Arg Tyr
           35
<210> 233
   <211> 33
   <212> PRT
   <213> Homo sapiens
<400> 233
   Lys Pro Glu Ala Pro Gly Glu Asp Ala Ser Pro Glu Glu Leu Asn Arg
   1               5                   10                  15
   Tyr Tyr Ala Ser Leu Arg His Tyr Leu Asn Leu Val Thr Arg Gln Arg
               20                  25                  30
   Tyr
<210> 234
   <211> 32
   <212> PRT
   <213> Homo sapiens
<400> 234
   Pro Glu Ala Pro Gly Glu Asp Ala Ser Pro Glu Glu Leu Asn Arg Tyr
   1               5                   10                  15
   Tyr Ala Ser Leu Arg His Tyr Leu Asn Leu Val Thr Arg Gln Arg Tyr
               20                  25                  30
<210> 235
   <211> 29
   <212> PRT
   <213> Homo sapiens
<400> 235
   His Ala Glu Gly Thr Phe Thr Ser Asp Val Ser Ser Thr Leu Glu Gly
   1               5                   10                  15
   Gln Ala Ala Leu Glu Phe Ile Ala Trp Leu Val Lys Gly
                   20                  25
<210> 236
   <211> 27
   <212> PRT
   <213> Heloderma suspectum
<400> 236
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys
               20                  25
<210> 237
   <211> 28
   <212> PRT
   <213> Heloderma suspectum
<400> 237
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn
               20                  25
<210> 238
   <211> 29
   <212> PRT
   <213> Heloderma suspectum
<400> 238
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly
               20                  25
<210> 239
   <211> 30
   <212> PRT
   <213> Heloderma suspectum
<400> 239
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly
               20                  25                  30
<210> 240
   <211> 28
   <212> PRT
   <213> Heloderma suspectum
<400> 240
   His Gly Glu Gly Ala Phe Thr Ser Asp Leu Ser Lys Gln Leu Glu Glu
   1 5 10 15
   Glu Ala Val Arg Leu Phe Ile Glu Phe Leu Lys Asn
   20 25
<210> 241
   <211> 27
   <212> PRT
   <213> Heloderma suspectum
<400> 241
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys
   20 25
<210> 242
   <211> 6
   <212> PRT
   <213> Homo sapiens
<400> 242
   Val Gly Ser Asn Thr Tyr
   1 5
<210> 243
   <211> 5
   <212> PRT
   <213> Homo sapiens
<400> 243
   Gly Ser Asn Thr Tyr
   1 5
<210> 244
   <211> 4
   <212> PRT
   <213> Homo sapiens
<400> 244
   Ser Asn Thr Tyr
   1
<210> 245
   <211> 6
   <212> PRT
   <213> Homo sapiens
<400> 245
   Ile Ser Pro Gln Gly Tyr
   1 5
<210> 246
   <211> 5
   <212> PRT
   <213> Homo sapiens
<400> 246
   Ser Pro Gln Gly Tyr
   1 5
<210> 247
   <211> 4
   <212> PRT
   <213> Homo sapiens
<400> 247
   Pro Gln Gly Tyr
   1
<210> 248
   <211> 6
   <212> PRT
   <213> Homo sapiens
<400> 248
   Ile Gly Val Gly Ala Pro
   1 5
<210> 249
   <211> 5
   <212> PRT
   <213> Homo sapiens
<400> 249
   Gly Val Gly Ala Pro
   1 5
<210> 250
   <211> 4
   <212> PRT
   <213> Homo sapiens
<400> 250
   Val Gly Ala Pro
   1
<210> 251
   <211> 6
   <212> PRT
   <213> Homo sapiens
<400> 251
   Val Gly Ser Lys Ala Phe
   1               5
<210> 252
   <211> 5
   <212> PRT
   <213> Homo sapiens
<400> 252
   Gly Ser Lys Ala Phe
   1               5
<210> 253
   <211> 4
   <212> PRT
   <213> Homo sapiens
<400> 253
   Ser Lys Ala Phe
   1
<210> 254
   <211> 6
   <212> PRT
   <213> Homo sapiens
<400> 254
   Ser Ser Pro His Ser Tyr
   1               5
<210> 255
   <211> 5
   <212> PRT
   <213> Homo sapiens
<400> 255
   Ser Pro His Ser Tyr
   1               5
<210> 256
   <211> 4
   <212> PRT
   <213> Homo sapiens
<400> 256
   Pro His Ser Tyr
   1
<210> 257
   <211> 12
   <212> PRT
   <213> Homo sapiens
<400> 257
   Arg His Tyr Leu Asn Leu Val Thr Arg Gln Arg Tyr
   1 5 10
<210> 258
   <211> 11
   <212> PRT
   <213> Homo sapiens
<400> 258
   His Tyr Leu Asn Leu Val Thr Arg Gln Arg Tyr
   1 5 10
<210> 259
   <211> 10
   <212> PRT
   <213> Homo sapiens
<400> 259
   Tyr Leu Asn Leu Val Thr Arg Gln Arg Tyr
   1 5 10
<210> 260
   <211> 9
   <212> PRT
   <213> Homo sapiens
<400> 260
   Leu Asn Leu Val Thr Arg Gln Arg Tyr
   1 5
<210> 261
   <211> 8
   <212> PRT
   <213> Homo sapiens
<400> 261
   Asn Leu Val Thr Arg Gln Arg Tyr
   1 5
<210> 262
   <211> 7
   <212> PRT
   <213> Homo sapiens
<400> 262
   Leu Val Thr Arg Gln Arg Tyr
   1 5
<210> 263
   <211> 6
   <212> PRT
   <213> Homo sapiens
<400> 263
   Val Thr Arg Gln Arg Tyr
   1 5
<210> 264
   <211> 5
   <212> PRT
   <213> Homo sapiens
<400> 264
   Thr Arg Gln Arg Tyr
   1 5
<210> 265
   <211> 11
   <212> PRT
   <213> Homo sapiens
<400> 265
   Arg His Tyr Leu Asn Leu Val Thr Arg Gln Arg
   1 5 10
<210> 266
   <211> 10
   <212> PRT
   <213> Homo sapiens
<400> 266
   His Tyr Leu Asn Leu Val Thr Arg Gln Arg
   1 5 10
<210> 267
   <211> 9
   <212> PRT
   <213> Homo sapiens
<400> 267
   Tyr Leu Asn Leu Val Thr Arg Gln Arg
   1 5
<210> 268
   <211> 8
   <212> PRT
   <213> Homo sapiens
<400> 268
   Leu Asn Leu Val Thr Arg Gln Arg
   1 5
<210> 269
   <211> 7
   <212> PRT
   <213> Homo sapiens
<400> 269
   Asn Leu Val Thr Arg Gln Arg
   1 5
<210> 270
   <211> 6
   <212> PRT
   <213> Homo sapiens
<400> 270
   Leu Val Thr Arg Gln Arg
   1 5
<210> 271
   <211> 5
   <212> PRT
   <213> Homo sapiens
<400> 271
   Val Thr Arg Gln Arg
   1 5
<210> 272
   <211> 4
   <212> PRT
   <213> Homo sapiens
<400> 272
   Thr Arg Gln Arg
   1
<210> 273
   <211> 9
   <212> PRT
   <213> Xenopus sp.
<400> 273
   Gly Lys Pro Lys Lys Ile Arg Tyr Ser
   1 5
<210> 274
   <211> 8
   <212> PRT
   <213> Xenopus sp.
<400> 274
   Lys Pro Lys Lys Ile Arg Tyr Ser
   1 5
<210> 275
   <211> 8
   <212> PRT
   <213> Xenopus sp.
<400> 275
   Gly Trp Leu Ile Lys Gly Arg Pro
   1 5
<210> 276
   <211> 7
   <212> PRT
   <213> Xenopus sp.
<400> 276
   Trp Leu Ile Lys Gly Arg Pro
   1 5
<210> 277
   <211> 9
   <212> PRT
   <213> Heloderma suspectum
<400> 277
   Pro Ser Ser Gly Ala Pro Pro Pro Ser
   1               5
<210> 278
   <211> 8
   <212> PRT
   <213> Heloderma suspectum
<400> 278
   Ser Ser Gly Ala Pro Pro Pro Ser
   1               5
<210> 279
   <211> 7
   <212> PRT
   <213> Heloderma suspectum
<400> 279
   Ser Gly Ala Pro Pro Pro Ser
   1               5
<210> 280
   <211> 6
   <212> PRT
   <213> Heloderma suspectum
<400> 280
   Gly Ala Pro Pro Pro Ser
   1               5
<210> 281
   <211> 5
   <212> PRT
   <213> Heloderma suspectum
<400> 281
   Ala Pro Pro Pro Ser
   1               5
<210> 282
   <211> 4
   <212> PRT
   <213> Heloderma suspectum
<400> 282
   Pro Pro Pro Ser
   1
<210> 283
   <211> 30
   <212> PRT
   <213> Xenopus sp.
<400> 283
   Thr Asn Asp Val Thr Glu Tyr Leu Glu Glu Lys Ala Ala Lys Glu Phe
   1 5 10 15
   Ile Glu Trp Leu Ile Lys Gly Lys Pro Lys Lys Ile Arg Tyr
   20 25 30
<210> 284
   <211> 28
   <212> PRT
   <213> Heloderma suspectum
<400> 284
   His Gly Glu Gly Thr Phe Thr Ser Asp Leu Ser Lys Gln Leu Glu Glu
   1 5 10 15
   Glu Ala Val Arg Leu Phe Ile Glu Phe Leu Lys Asn
   20 25
<210> 285
   <211> 7
   <212> PRT
   <213> Homo sapiens
<400> 285
   Lys Ala Asn Thr Ala Thr Ala
   1 5
<210> 286
   <211> 20
   <212> PRT
   <213> Oncorhynchus sp.
<400> 286
   Val Leu Gly Arg Leu Ser Glu Glu Leu His Arg Leu Gln Thr Tyr Pro
   1 5 10 15
   Arg Thr Asn Thr
   20
<210> 287
   <211> 8
   <212> PRT
   <213> Homo sapiens
<400> 287
   Asp Phe Met Gly Trp Met Asp Phe
   1 5
<210> 288
   <211> 20
   <212> PRT
   <213> Oncorhynchus sp.
<400> 288
   Val Leu Gly Lys Leu Ser Glu Glu Leu His Lys Leu Gln Thr Tyr Pro
   1               5                   10                  15
   Arg Thr Asn Thr
               20
<210> 289
   <211> 20
   <212> PRT
   <213> artificial
<220>
   <223> Chimeric human and salmon construct
<400> 289
   Val Leu Gly Arg Leu Ser Gln Glu Leu His Arg Leu Gln Thr Tyr Pro
   1               5                   10                  15
   Arg Thr Asn Thr
               20
<210> 290
   <211> 36
   <212> PRT
   <213> homo sapiens
<400> 290
   Ala Pro Leu Glu Pro Val Tyr Pro Gly Asp Asn Ala Thr Pro Glu Gln
   1               5                   10                  15
   Met Ala Gln Tyr Ala Ala Asp Leu Arg Arg Tyr Ile Asn Met Leu Thr
               20                  25                  30
   Arg Pro Arg Tyr
           35
<210> 291
   <211> 38
   <212> PRT
   <213> mus musculus
<220>
   <221> misc_feature
   <223> C-term amidated
<400> 291
   Val Ile Leu Ser Leu Asp Val Pro Ile Gly Leu Leu Arg Ile Leu Leu
   1               5                   10                  15
   Glu Gln Ala Arg Tyr Lys Ala Ala Arg Asn Gln Ala Ala Thr Asn Ala
               20                  25                  30
   Gln Ile Leu Ala His Val
           35
<210> 292
   <211> 24
   <212> PRT
   <213> Homo sapiens
<400> 292
   Trp Ser Pro Gly Ala Arg Asn Gln Gly Gly Gly Ala Arg Ala Leu Leu
   1               5                   10                  15
   Leu Leu Leu Ala Glu Arg Phe Pro
               20
<210> 293
   <211> 18
   <212> PRT
   <213> Homo sapiens
<220>
   <221> MISC_FEATURE
   <223> C-term amidated
<400> 293
   Thr Gln Ser Gln Arg Glu Arg Ala Glu Gln Asn Arg Ile Ile Phe Asp
   1               5                   10                  15
   Ser Val
<210> 294
   <211> 40
   <212> PRT
   <213> Homo sapiens
<220>
   <221> misc_feature
   <223> C-term amidated
<400> 294
   Asp Asn Pro Ser Leu Ser Ile Asp Leu Thr Phe His Leu Leu Arg Thr
   1 5 10 15
   Leu Leu Glu Leu Ala Arg Thr Gln Ser Gln Arg Glu Arg Ala Glu Gln
   20 25 30
   Asn Arg Ile Ile Phe Asp Ser Val
   35 40
<210> 295
   <211> 20
   <212> PRT
   <213> Homo sapiens
<400> 295
   Ser Phe His Tyr Leu Arg Ser Arg Asp Ala Ser Ser Gly Glu Glu Glu
   1 5 10 15
   Glu Gly Lys Glu
   20
<210> 296
   <211> 13
   <212> PRT
   <213> Homo sapiens
<400> 296
   Ala Gln Ala Ala Ala Asn Ala His Leu Met Ala Gln Ile
   1 5 10
<210> 297
   <211> 21
   <212> PRT
   <213> Homo sapiens
<400> 297
   Asp Asn Pro Ser Leu Ser Ile Asp Leu Thr Phe His Leu Leu Arg Thr
   1 5 10 15
   Leu Leu Glu Leu Ala
   20
<210> 298
   <211> 26
   <212> PRT
   <213> Homo sapiens
<400> 298
   Thr Lys Phe Thr Leu Ser Leu Asp Val Pro Thr Asn Ile Met Asn Leu
   1               5                   10                  15
   Leu Phe Asn Ile Ala Lys Ala Lys Asn Leu
               20                  25
<210> 299
   <211> 38
   <212> PRT
   <213> Homo sapiens
<220>
   <221> MISC_FEATURE
   <223> C-term amidated
<400> 299
   Phe Thr Leu Ser Leu Asp Val Pro Thr Asn Ile Met Asn Leu Leu Phe
   1               5                   10                  15
   Asn Ile Ala Lys Ala Lys Asn Leu Arg Ala Gln Ala Ala Ala Asn Ala
               20                  25                  30
   His Leu Met Ala Gln Ile
           35
<210> 300
   <211> 38
   <212> PRT
   <213> Homo sapiens
<220>
   <221> MISC_FEATURE
   <223> C-term amidated
<400> 300
   Phe Leu Phe His Tyr Ser Lys Thr Gln Lys Leu Gly Lys Ser Asn Val
   1               5                   10                  15
   Val Glu Glu Leu Gln Ser Pro Phe Ala Ser Gln Ser Arg Gly Tyr Phe
               20                  25                  30
   Leu Phe Arg Pro Arg Asn
           35
<210> 301
   <211> 28
   <212> PRT
   <213> Homo sapiens
<400> 301
   Ser Leu Arg Arg Ser Ser Cys Phe Gly Gly Arg Met Asp Arg Ile Gly
   1 5 10 15
   Ala Gln Ser Gly Leu Gly Cys Asn Ser Phe Arg Tyr
   20 25
<210> 302
   <211> 32
   <212> PRT
   <213> rat
<400> 302
   Asn Ser Lys Met Ala His Ser Ser Ser Cys Phe Gly Gln Lys Ile Asp
   1 5 10 15
   Arg Ile Gly Ala Val Ser Arg Leu Gly Cys Asp Gly Leu Arg Leu Phe
   20 25 30
<210> 303
   <211> 32
   <212> PRT
   <213> Homo sapiens
<400> 303
   Ser Pro Lys Met Val Gln Gly Ser Gly Cys Phe Gly Arg Lys Met Asp
   1 5 10 15
   Arg Ile Ser Ser Ser Ser Gly Leu Gly Cys Lys Val Leu Arg Arg His
   20 25 30
<210> 304
   <211> 22
   <212> PRT
   <213> porcine
<400> 304
   Gly Leu Ser Lys Gly Cys Phe Gly Leu Lys Leu Asp Arg Ile Gly Ser
   1 5 10 15
   Met Ser Gly Leu Gly Cys
   20
<210> 305
   <211> 8
   <212> PRT
   <213> porcine
<220>
   <221> MISC_FEATURE
   <223> C-term amidated
<400> 305
   Tyr Phe Leu Phe Arg Pro Arg Asn
   1 5
<210> 306
   <211> 23
   <212> PRT
   <213> rat
<220>
   <221> MISC_FEATURE
   <223> C-term amidated
<400> 306
   Tyr Lys Val Asn Glu Tyr Gln Gly Pro Val Ala Pro Ser Gly Gly Phe
   1 5 10 15
   Phe Leu Phe Arg Pro Arg Asn
   20
<210> 307
   <211> 9
   <212> PRT
   <213> Homo sapiens
<220>
   <221> MISC_FEATURE
   <223> C-term amidated
<400> 307
   Gly Tyr Phe Leu Phe Arg Pro Arg Asn
   1 5
<210> 308
   <211> 25
   <212> PRT
   <213> Homo sapiens
<220>
   <221> MISC_FEATURE
   <223> C-term amidated
<400> 308
   Phe Arg Val Asp Glu Glu Phe Gln Ser Pro Phe Ala Ser Gln Ser Arg
   1 5 10 15
   Gly Tyr Phe Leu Phe Arg Pro Arg Asn
   20 25
<210> 309
   <211> 36
   <212> PRT
   <213> Artificial
<220>
   <223> Hybrid of human CCK and human PYY
<220>
   <221> MISC_FEATURE
   <223> C-term amidated
<220>
   <221> MISC_FEATURE
   <223> N-terminal (head) to N-terminal (head) linked
<220>
   <221> MOD_RES
   <222> (1)..(1)
   <223> C-term amidated
<220>
   <221> MOD_RES
   <222> (7)..(7)
   <223> Sulfated Tyrosine
<220>
   <221> MOD_RES
   <222> (9)..(9)
   <223> Succinoyl-Cys
<400> 309
   Phe Asp Met Trp Gly Met Tyr Asp Cys Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 310
   <211> 36
   <212> PRT
   <213> Artificial
<220>
   <223> Hybrid of human CCK and human PYY
<220>
   <221> MISC_FEATURE
   <223> C-term amidated
<220>
   <221> MISC_FEATURE
   <223> N-terminal (head) to N-terminal (head) linked
<220>
   <221> MOD_RES
   <222> (1)..(1)
   <223> C-term amidated
<220>
   <221> MOD_RES
   <222> (7)..(7)
   <223> Sulfated Tyrosine
<220>
   <221> MOD_RES
   <222> (9)..(9)
   <223> Bis-Cys(N-Acetyl)
<400> 310
   Phe Asp Met Trp Gly Met Tyr Asp Cys Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 311
   <211> 36
   <212> PRT
   <213> Artificial
<220>
   <223> Hybrid of human CCK and human PYY
<220>
   <221> MISC_FEATURE
   <223> C-term amidated
<220>
   <221> MISC_FEATURE
   <223> N-terminal (head) to N-terminal (head) linked
<220>
   <221> MOD_RES
   <222> (1)..(1)
   <223> C-term amidated
<220>
   <221> MOD_RES
   <222> (7)..(7)
   <223> para(CH2SO3)Phe
<220>
   <221> MOD_RES
   <222> (9)..(9)
   <223> Gly-Aminoxymethylcarbonyl
<400> 311
   Phe Asp Met Trp Gly Met Phe Asp Gly Glu Asp Ala Ser Pro Glu Glu
   1               5                   10                  15
   Leu Asn Arg Tyr Tyr Ala Ser Leu Arg His Tyr Leu Asn Leu Val Thr
               20                  25                  30
   Arg Gln Arg Tyr
           35
<210> 312
   <211> 63
   <212> PRT
   <213> Artificial
<220>
   <223> Hybrid lizard, human and salmon construct
<400> 312
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Gly Lys
               20                  25                  30
   Cys Asn Thr Ala Thr Cys Val Leu Gly Arg Leu Ser Gln Glu Leu His
           35                  40                  45
   Arg Leu Gln Thr Tyr Pro Arg Thr Asn Thr Gly Ser Asn Thr Tyr
       50                  55                  60
<210> 313
   <211> 62
   <212> PRT
   <213> Artificial
<220>
   <223> Hybrid lizard, human and salmon construct
<400> 313
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Gly Cys
   20 25 30
   Asn Thr Ala Thr Cys Val Leu Gly Arg Leu Ser Gln Glu Leu His Arg
   35 40 45
   Leu Gln Thr Tyr Pro Arg Thr Asn Thr Gly Ser Asn Thr Tyr
   50 55 60
<210> 314
   <211> 25
   <212> PRT
   <213> porcine
<400> 314
   Phe Lys Val Asp Glu Glu Phe Gln Gly Pro Ile Val Ser Gln Asn Arg
   1 5 10 15
   Arg Tyr Phe Leu Phe Arg Pro Arg Asn
   20 25
<210> 315
   <211> 33
   <212> PRT
   <213> Homo sapiens
<400> 315
   Ile Leu Gln Arg Gly Ser Gly Thr Ala Ala Val Asp Phe Thr Lys Lys
   1 5 10 15
   Asp His Thr Ala Thr Trp Gly Arg Pro Phe Phe Leu Phe Arg Pro Arg
   20 25 30
   Asn
<210> 316
   <211> 36
   <212> PRT
   <213> Homo sapiens
<400> 316
   Leu Val Gln Pro Arg Gly Ser Arg Asn Gly Pro Gly Pro Trp Gln Gly
   1 5 10 15
   Gly Arg Arg Lys Phe Arg Arg Gln Arg Pro Arg Leu Ser His Lys Gly
   20 25 30
   Pro Met Pro Phe
   35
<210> 317
   <211> 14
   <212> PRT
   <213> Homo sapiens
<400> 317
   Pro Glu Arg Pro Arg Leu Ser His Lys Gly Pro Met Pro Phe
   1 5 10
<210> 318
   <211> 31
   <212> PRT
   <213> Homo sapiens
<220>
   <221> MISC_FEATURE
   <223> C-term amidated
<400> 318
   Ser Arg Thr His Arg His Ser Met Glu Ile Arg Thr Pro Asp Ile Asn
   1 5 10 15
   Pro Ala Trp Tyr Ala Ser Arg Gly Ile Arg Pro Val Gly Arg Phe
   20 25 30
<210> 319
   <211> 20
   <212> PRT
   <213> Homo sapiens
<220>
   <221> MISC_FEATURE
   <223> C-term amidated
<400> 319
   Thr Pro Asp Ile Asn Pro Ala Trp Tyr Ala Ser Arg Gly Ile Arg Pro
   1 5 10 15
   Val Gly Arg Phe
   20
<210> 320
   <211> 34
   <212> PRT
   <213> Homo sapiens
<220>
   <221> MISC_FEATURE
   <223> C-term amidated
<400> 320
   Gln Leu Gly Pro Gln Gly Pro Pro His Leu Val Ala Asp Pro Ser Lys
   1               5                   10                  15
   Lys Gln Gly Pro Trp Leu Glu Glu Glu Glu Glu Ala Tyr Gly Trp Met
               20                  25                  30
   Asp Phe
<210> 321
   <211> 18
   <212> PRT
   <213> Homo sapiens
<220>
   <221> MISC_FEATURE
   <223> C-term amidated
<220>
   <221> MOD_RES
   <222> (1)..(1)
   <223> pyroGlutamine
<400> 321
   Pro Glu Gly Pro Trp Leu Glu Glu Glu Glu Glu Ala Tyr Gly Trp Met
   1               5                   10                  15
   Asp Phe
<210> 322
   <211> 5
   <212> PRT
   <213> Homo sapiens
<220>
   <221> MISC_FEATURE
   <223> C-term amidated
<220>
   <221> MOD_RES
   <222> (1)..(1)
   <223> bAla
<400> 322
   Ala Trp Met Asp Phe
   1               5
<210> 323
   <211> 33
   <212> PRT
   <213> Homo sapiens
<220>
   <221> MISC_FEATURE
   <223> C-term amidated
<400> 323
   Lys Ala Pro Ser Gly Arg Met Ser Ile Val Lys Asn Leu Gln Asn Leu
   1               5                   10                  15
   Asp Pro Ser His Arg Ile Ser Asp Arg Asp Tyr Met Gly Trp Met Asp
               20                  25                  30
   Phe
<210> 324
   <211> 29
   <212> PRT
   <213> Homo sapiens
<400> 324
   Gln Glu Gly Ala Pro Pro Gln Gln Ser Ala Arg Arg Asp Arg Met Pro
   1               5                   10                  15
   Cys Arg Asn Phe Phe Trp Lys Thr Phe Ser Ser Cys Lys
               20                  25
<210> 325
   <211> 17
   <212> PRT
   <213> Homo sapiens
<400> 325
   Asp Arg Met Pro Cys Arg Asn Phe Phe Trp Lys Thr Phe Ser Ser Cys
   1               5                   10                  15
   Lys
<210> 326
   <211> 28
   <212> PRT
   <213> Homo sapiens
<400> 326
   Ser Ala Asn Ser Asn Pro Ala Met Ala Pro Arg Glu Arg Lys Ala Gly
   1               5                   10                  15
   Cys Lys Asn Phe Phe Trp Lys Thr Phe Thr Ser Cys
               20                  25
<210> 327
   <211> 14
   <212> PRT
   <213> Homo sapiens
<400> 327
   Ala Gly Cys Lys Asn Phe Phe Trp Lys Thr Phe Thr Ser Cys
   1 5 10
<210> 328
   <211> 27
   <212> PRT
   <213> Homo sapiens
<220>
   <221> MISC_FEATURE
   <223> C-term amidated
<400> 328
   Val Pro Leu Pro Ala Gly Gly Gly Thr Val Leu Thr Lys Met Tyr Pro
   1 5 10 15
   Arg Gly Asn His Trp Ala Val Gly His Leu Met
   20 25
<210> 329
   <211> 10
   <212> PRT
   <213> Homo sapiens
<220>
   <221> MISC_FEATURE
   <223> C-term amidated
<400> 329
   Gly Asn His Trp Ala Val Gly His Leu Met
   1 5 10
<210> 330
   <211> 30
   <212> PRT
   <213> Homo sapiens
<220>
   <221> MISC_FEATURE
   <223> C-term amidated
<400> 330
   Leu Ser Trp Asp Leu Pro Glu Pro Arg Ser Arg Ala Ser Lys Ile Arg
   1               5                   10                  15
   Val His Ser Arg Gly Asn Leu Trp Ala Thr Gly His Phe Met
               20                  25                  30
<210> 331
   <211> 10
   <212> PRT
   <213> Homo sapiens
<220>
   <221> MISC_FEATURE
   <223> C-term amidated
<400> 331
   Gly Asn Leu Trp Ala Thr Gly His Phe Met
   1               5                   10
<210> 332
   <211> 9
   <212> PRT
   <213> Homo sapiens
<220>
   <221> MISC_FEATURE
   <223> C-term amidated
<400> 332
   Pro Phe Phe Leu Phe Arg Pro Arg Asn
   1               5
<210> 333
   <211> 21
   <212> PRT
   <213> Homo sapiens
<400> 333
   Lys Ile Pro Tyr Ile Leu Lys Arg Gln Leu Tyr Glu Asn Lys Pro Arg
   1               5                   10                  15
   Arg Pro Tyr Ile Leu
               20
<210> 334
   <211> 13
   <212> PRT
   <213> Homo sapiens
<400> 334
   Gln Leu Tyr Glu Asn Lys Pro Arg Arg Pro Tyr Ile Leu
   1 5 10
<210> 335
   <211> 54
   <212> PRT
   <213> Homo sapiens
<220>
   <221> MISC_FEATURE
   <223> C-term amidated
<400> 335
   Gly Thr Ser Leu Ser Pro Pro Pro Glu Ser Ser Gly Ser Pro Gln Gln
   1 5 10 15
   Pro Gly Leu Ser Ala Pro His Ser Arg Gln Ile Pro Ala Pro Gln Gly
   20 25 30
   Ala Val Leu Val Gln Arg Glu Lys Asp Leu Pro Asn Tyr Asn Trp Asn
   35 40 45
   Ser Phe Gly Leu Arg Phe
   50
<210> 336
   <211> 16
   <212> PRT
   <213> Homo sapiens
<220>
   <221> MISC_FEATURE
   <223> C-term amidated
<400> 336
   Glu Lys Asp Leu Pro Asn Tyr Asn Trp Asn Ser Phe Gly Leu Arg Phe
   1 5 10 15
<210> 337
   <211> 31
   <212> PRT
   <213> Homo sapiens
<220>
   <221> MISC_FEATURE
   <223> C-term amidated
<400> 337
   Ser Ala Gly Ala Thr Ala Asn Leu Pro Leu Arg Ser Gly Arg Asn Met
   1               5                   10                  15
   Glu Val Ser Leu Val Arg Arg Val Pro Asn Leu Pro Gln Arg Phe
               20                  25                  30
<210> 338
   <211> 8
   <212> PRT
   <213> Homo sapiens
<220>
   <221> MISC_FEATURE
   <223> C-term amidated
<400> 338
   Val Pro Asn Leu Pro Gln Arg Phe
   1               5
<210> 339
   <211> 32
   <212> PRT
   <213> Homo sapiens
<400> 339
   Tyr Gly Gly Phe Leu Arg Arg Ile Arg Pro Lys Leu Lys Trp Asp Asn
   1               5                   10                  15
   Gln Lys Arg Tyr Gly Gly Phe Leu Arg Arg Gln Phe Lys Val Val Thr
               20                  25                  30
<210> 340
   <211> 13
   <212> PRT
   <213> Homo sapiens
<400> 340
   Tyr Gly Gly Phe Leu Arg Arg Gln Phe Lys Val Val Thr
   1               5                   10
<210> 341
   <211> 14
   <212> PRT
   <213> Homo sapiens
<220>
   <221> MISC_FEATURE
   <223> C-term amide
<400> 341
   Ser Leu Arg His Tyr Leu Asn Leu Val Thr Arg Gln Arg Tyr
   1               5                   10
<210> 342
   <211> 38
   <212> PRT
   <213> Homo sapiens
<400> 342
   Cys Ser Cys Ser Ser Leu Met Asp Lys Glu Cys Val Tyr Phe Cys His
   1               5                   10                  15
   Leu Asp Ile Ile Trp Val Asn Thr Pro Glu His Val Val Pro Tyr Gly
               20                  25                  30
   Leu Gly Ser Pro Arg Ser
           35
<210> 343
   <211> 21
   <212> PRT
   <213> Homo sapiens
<400> 343
   Cys Ser Cys Ser Ser Leu Met Asp Lys Glu Cys Val Tyr Phe Cys His
   1               5                   10                  15
   Leu Asp Ile Ile Trp
               20
<210> 344
   <211> 37
   <212> PRT
   <213> Homo sapiens
<400> 344
   Cys Ser Cys Ser Ser Trp Leu Asp Lys Glu Cys Val Tyr Phe Cys His
   1               5                   10                  15
   Leu Asp Ile Ile Trp Val Asn Thr Pro Glu Gln Thr Ala Pro Tyr Gly
               20                  25                  30
   Leu Gly Asn Pro Pro
           35
<210> 345
   <211> 21
   <212> PRT
   <213> Homo sapiens
<400> 345
   Cys Ser Cys Ser Ser Trp Leu Asp Lys Glu Cys Val Tyr Phe Cys His
   1 5 10 15
   Leu Asp Ile Ile Trp
   20
<210> 346
   <211> 41
   <212> PRT
   <213> Homo sapiens
<220>
   <221> MISC_FEATURE
   <223> C-term amidated
<400> 346
   Cys Thr Cys Phe Thr Tyr Lys Asp Lys Glu Cys Val Tyr Tyr Cys His
   1 5 10 15
   Leu Asp Ile Ile Trp Ile Asn Thr Pro Glu Gln Thr Val Pro Tyr Gly
   20 25 30
   Leu Ser Asn Tyr Arg Gly Ser Phe Arg
   35 40
<210> 347
   <211> 21
   <212> PRT
   <213> Homo sapiens
<400> 347
   Cys Thr Cys Phe Thr Tyr Lys Asp Lys Glu Cys Val Tyr Tyr Cys His
   1 5 10 15
   Leu Asp Ile Ile Trp
   20
<210> 348
   <211> 27
   <212> PRT
   <213> Homo sapiens
<400> 348
   Gly Ser Ser Phe Leu Ser Pro Glu His Gln Arg Val Gln Gln Arg Lys
   1 5 10 15
   Glu Ser Lys Lys Pro Pro Ala Lys Leu Gln Pro
   20 25
<210> 349
   <211> 10
   <212> PRT
   <213> Homo sapiens
<400> 349
   Gly Ser Ser Phe Leu Ser Pro Glu His Gln
   1               5                   10
<210> 350
   <211> 37
   <212> PRT
   <213> Homo sapiens
<400> 350
   His Ser Gln Gly Thr Phe Thr Ser Asp Tyr Ser Lys Tyr Leu Asp Ser
   1               5                   10                  15
   Arg Arg Ala Gln Asp Phe Val Gln Trp Leu Met Asn Thr Lys Arg Asn
               20                  25                  30
   Arg Asn Asn Ile Ala
           35
<210> 351
   <211> 29
   <212> PRT
   <213> Homo sapiens
<400> 351
   His Ser Gln Gly Thr Phe Thr Ser Asp Tyr Ser Lys Tyr Leu Asp Ser
   1               5                   10                  15
   Arg Arg Ala Gln Asp Phe Val Gln Trp Leu Met Asn Thr
               20                  25
<210> 352
   <211> 42
   <212> PRT
   <213> Homo sapiens
<400> 352
   Tyr Ala Glu Gly Thr Phe Ile Ser Asp Tyr Ser Ile Ala Met Asp Lys
   1               5                   10                  15
   Ile His Gln Gln Asp Phe Val Asn Trp Leu Leu Ala Gln Lys Gly Lys
               20                  25                  30
   Lys Asn Asp Trp Lys His Asn Ile Thr Gln
           35                  40
<210> 353
   <211> 30
   <212> PRT
   <213> Homo sapiens
<220>
   <221> MISC_FEATURE
   <223> C-term amidated
<400> 353
   Tyr Ala Glu Gly Thr Phe Ile Ser Asp Tyr Ser Ile Ala Met Asp Lys
   1               5                   10                  15
   Ile His Gln Gln Asp Phe Val Asn Trp Leu Leu Ala Gln Lys
               20                  25                  30
<210> 354
   <211> 30
   <212> PRT
   <213> Homo sapiens
<400> 354
   Trp Tyr Lys His Val Ala Ser Pro Arg Tyr His Thr Val Gly Arg Ala
   1               5                   10                  15
   Ala Gly Leu Leu Met Gly Leu Arg Arg Ser Pro Tyr Leu Trp
               20                  25                  30
<210> 355
   <211> 23
   <212> PRT
   <213> Homo sapiens
<400> 355
   Trp Tyr Lys His Val Ala Ser Pro Arg Tyr His Thr Val Gly Arg Ala
   1               5                   10                  15
   Ala Gly Leu Leu Met Gly Leu
               20
<210> 356
   <211> 38
   <212> PRT
   <213> Homo sapiens
<220>
   <221> MISC_FEATURE
   <223> C-term amidated
<400> 356
   His Ser Asp Gly Ile Phe Thr Asp Ser Tyr Ser Arg Tyr Arg Lys Gln
   1 5 10 15
   Met Ala Val Lys Lys Tyr Leu Ala Ala Val Leu Gly Lys Arg Tyr Lys
   20 25 30
   Gln Arg Val Lys Asn Lys
   35
<210> 357
   <211> 27
   <212> PRT
   <213> Homo sapiens
<220>
   <221> MISC_FEATURE
   <223> C-term amidated
<400> 357
   His Ser Asp Gly Ile Phe Thr Asp Ser Tyr Ser Arg Tyr Arg Lys Gln
   1 5 10 15
   Met Ala Val Lys Lys Tyr Leu Ala Ala Val Leu
   20 25
<210> 358
   <211> 42
   <212> PRT
   <213> Homo sapiens
<400> 358
   His Ala Asp Gly Val Phe Thr Ser Asp Phe Ser Lys Leu Leu Gly Gln
   1 5 10 15
   Leu Ser Ala Lys Lys Tyr Leu Glu Ser Leu Met Gly Lys Arg Val Ser
   20 25 30
   Ser Asn Ile Ser Glu Asp Pro Val Pro Val
   35 40
<210> 359
   <211> 27
   <212> PRT
   <213> Homo sapiens
<220>
   <221> MISC_FEATURE
   <223> C-term amidated
<400> 359
   His Ala Asp Gly Val Phe Thr Ser Asp Phe Ser Lys Leu Leu Gly Gln
   1 5 10 15
   Leu Ser Ala Lys Lys Tyr Leu Glu Ser Leu Met
   20 25
<210> 360
   <211> 44
   <212> PRT
   <213> Homo sapiens
<220>
   <221> MISC_FEATURE
   <223> C-term amidated
<400> 360
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser Arg Gln Gln Gly
   20 25 30
   Glu Ser Asn Gln Glu Arg Gly Ala Arg Ala Arg Leu
   35 40
<210> 361
   <211> 28
   <212> PRT
   <213> Homo sapiens
<400> 361
   Tyr Ala Asp Ala Ile Phe Thr Asn Ser Tyr Arg Lys Val Leu Gly Gln
   1 5 10 15
   Leu Ser Ala Arg Lys Leu Leu Gln Asp Ile Met Ser
   20 25
<210> 362
   <211> 84
   <212> PRT
   <213> Homo sapiens
<400> 362
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val Ala Leu Gly Ala Pro Leu Ala Pro Arg Asp Ala Gly Ser
   35 40 45
   Gln Arg Pro Arg Lys Lys Glu Asp Asn Val Leu Val Glu Ser His Glu
   50 55 60
   Lys Ser Leu Gly Glu Ala Asp Lys Ala Asp Val Asn Val Leu Thr Lys
   65 70 75 80
   Ala Lys Ser Gln
<210> 363
   <211> 37
   <212> PRT
   <213> Homo sapiens
<400> 363
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe Val Ala Leu
   35
<210> 364
   <211> 34
   <212> PRT
   <213> Homo sapiens
<400> 364
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe
<210> 365
   <211> 93
   <212> PRT
   <213> Homo sapiens
<220>
   <221> MISC_FEATURE
   <223> C-term amidated
<400> 365
   Ala Val Ser Glu His Gln Leu Leu His Asp Lys Gly Lys Ser Ile Gln
   1               5                   10                  15
   Asp Leu Arg Arg Arg Phe Phe Leu His His Leu Ile Ala Glu Ile His
               20                  25                  30
   Thr Ala Glu Ile Arg Ala Thr Ser Glu Val Ser Pro Asn Ser Lys Pro
           35                  40                  45
   Ser Pro Asn Thr Lys Asn His Pro Val Arg Gly Ser Asp Asp Glu Gly
       50                  55                  60
   Arg Tyr Leu Thr Gln Glu Thr Asn Lys Val Glu Thr Tyr Lys Glu Gln
   65                  70                  75                  80
   Pro Leu Lys Thr Pro Gly Lys Lys Lys Lys Gly Lys Pro
                   85                  90
<210> 366
   <211> 36
   <212> PRT
   <213> Homo sapiens
<400> 366
   Ala Val Ser Glu His Gln Leu Leu His Asp Lys Gly Lys Ser Ile Gln
   1               5                   10                  15
   Asp Leu Arg Arg Arg Phe Phe Leu His His Leu Ile Ala Glu Ile His
               20                  25                  30
   Thr Ala Glu Ile
           35
<210> 367
   <211> 26
   <212> PRT
   <213> Homo sapiens
<400> 367
   Tyr Val Met Gly His Phe Arg Trp Asp Arg Phe Gly Arg Arg Asn Ser
   1               5                   10                  15
   Ser Ser Ser Gly Ser Ser Gly Ala Gly Gln
               20                  25
<210> 368
   <211> 11
   <212> PRT
   <213> Homo sapiens
<220>
   <221> MISC_FEATURE
   <223> C-term amidated
<400> 368
   Tyr Val Met Gly His Phe Arg Trp Asp Arg Phe
   1               5                   10
<210> 369
   <211> 39
   <212> PRT
   <213> Homo sapiens
<400> 369
   Ser Tyr Ser Met Glu His Phe Arg Trp Gly Lys Pro Val Gly Lys Lys
   1               5                   10                  15
   Arg Arg Pro Val Lys Val Tyr Pro Asn Gly Ala Glu Asp Glu Ser Ala
               20                  25                  30
   Glu Ala Phe Pro Leu Glu Phe
           35
<210> 370
   <211> 13
   <212> PRT
   <213> Homo sapiens
<220>
   <221> MISC_FEATURE
   <223> C-term amidated
<400> 370
   Ser Tyr Ser Met Glu His Phe Arg Trp Gly Lys Pro Val
   1               5                   10
<210> 371
   <211> 31
   <212> PRT
   <213> Homo sapiens
<400> 371
   Tyr Gly Gly Phe Met Thr Ser Glu Lys Ser Gln Thr Pro Leu Val Thr
   1               5                   10                  15
   Leu Phe Lys Asn Ala Ile Ile Lys Asn Ala Tyr Lys Lys Gly Glu
               20                  25                  30
<210> 372
   <211> 27
   <212> PRT
   <213> Homo sapiens
<400> 372
   Tyr Gly Gly Phe Met Thr Ser Glu Lys Ser Gln Thr Pro Leu Val Thr
   1               5                   10                  15
   Leu Phe Lys Asn Ala Ile Ile Lys Asn Ala Tyr
               20                  25
<210> 373
   <211> 17
   <212> PRT
   <213> Homo sapiens
<400> 373
   Tyr Gly Gly Phe Met Thr Ser Glu Lys Ser Gln Thr Pro Leu Val Thr
   1               5                   10                  15
   Leu
<210> 374
   <211> 16
   <212> PRT
   <213> Homo sapiens
<400> 374
   Tyr Gly Gly Phe Met Thr Ser Glu Lys Ser Gln Thr Pro Leu Val Thr
   1               5                   10                  15
<210> 375
   <211> 151
   <212> PRT
   <213> Homo sapiens
<400> 375
<210> 376
   <211> 134
   <212> PRT
   <213> Homo sapiens
<400> 376
   Met Asp Pro Gln Thr Ala Pro Ser Arg Ala Leu Leu Leu Leu Leu Phe
   1               5                   10                  15
   Leu His Leu Ala Phe Leu Gly Gly Arg Ser His Pro Leu Gly Ser Pro
               20                  25                  30
   Gly Ser Ala Ser Asp Leu Glu Thr Ser Gly Leu Gln Glu Gln Arg Asn
           35                  40                  45
   His Leu Gln Gly Lys Leu Ser Glu Leu Gln Val Glu Gln Thr Ser Leu
       50                  55                  60
   Glu Pro Leu Gln Glu Ser Pro Arg Pro Thr Gly Val Trp Lys Ser Arg
   65                  70                  75                  80
   Glu Val Ala Thr Glu Gly Ile Arg Gly His Arg Lys Met Val Leu Tyr
                   85                  90                  95
   Thr Leu Arg Ala Pro Arg Ser Pro Lys Met Val Gln Gly Ser Gly Cys
               100                 105                 110
   Phe Gly Arg Lys Met Asp Arg Ile Ser Ser Ser Ser Gly Leu Gly Cys
           115                 120                 125
   Lys Val Leu Arg Arg His
       130
<210> 377
   <211> 126
   <212> PRT
   <213> Homo sapiens
<400> 377
   Met His Leu Ser Gln Leu Leu Ala Cys Ala Leu Leu Leu Thr Leu Leu
   1               5                   10                  15
   Ser Leu Arg Pro Ser Glu Ala Lys Pro Gly Ala Pro Pro Lys Val Pro
               20                  25                  30
   Arg Thr Pro Pro Ala Glu Glu Leu Ala Glu Pro Gln Ala Ala Gly Gly
           35                  40                  45
   Gly Gln Lys Lys Gly Asp Lys Ala Pro Gly Gly Gly Gly Ala Asn Leu
       50                  55                  60
   Lys Gly Asp Arg Ser Arg Leu Leu Arg Asp Leu Arg Val Asp Thr Lys
   65                  70                  75                  80
   Ser Arg Ala Ala Trp Ala Arg Leu Leu Gln Glu His Pro Asn Ala Arg
                   85                  90                  95
   Lys Tyr Lys Gly Ala Asn Lys Lys Gly Leu Ser Lys Gly Cys Phe Gly
               100                 105                 110
   Leu Lys Leu Asp Arg Ile Gly Ser Met Ser Gly Leu Gly Cys
           115                 120                 125
<210> 378
   <211> 9
   <212> PRT
   <213> Lizard
<400> 378
   His Ser Glu Gly Thr Phe Thr Ser Asp
   1               5
<210> 379
   <211> 59
   <212> PRT
   <213> Artificial
<220>
   <223> Hybrid lizard, human and salmon construct
<400> 379
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Lys Cys Asn Thr Ala
               20                  25                  30
   Thr Cys Val Leu Gly Arg Leu Ser Glu Glu Leu His Arg Leu Gln Thr
           35                  40                  45
   Tyr Pro Arg Thr Asn Thr Gly Ser Asn Thr Tyr
       50                  55
<210> 380
   <211> 61
   <212> PRT
   <213> Artificial
<220>
   <223> Hybrid lizard, human and salmon construct
<220>
   <221> MOD_RES
   <222> (29)..(29)
   <223> 12-Ado
<400> 380
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Lys Cys Asn
               20                  25                  30
   Thr Ala Thr Cys Val Leu Gly Arg Leu Ser Glu Glu Leu His Arg Leu
           35                  40                  45
   Gln Thr Tyr Pro Arg Thr Asn Thr Gly Ser Asn Thr Tyr
       50                  55                  60
<210> 381
   <211> 60
   <212> PRT
   <213> Artificial
<220>
   <223> Hybrid lizard, human and salmon construct
<220>
   <221> MOD_RES
   <222> (29)..(29).
   <223> 12-Ado
<400> 381
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Cys Asn Thr
               20                  25                  30
   Ala Thr Cys Val Leu Gly Arg Leu Ser Glu Glu Leu His Arg Leu Gln
           35                  40                  45
   Thr Tyr Pro Arg Thr Asn Thr Gly Ser Asn Thr Tyr
       50                  55                  60
<210> 382
   <211> 61
   <212> PRT
   <213> Artificial
<220>
   <223> Hybrid lizard, human and salmon construct
<220>
   <221> MOD_RES
   <222> (29)..(29)
   <223> 3,6-dioxactoanoyl
<400> 382
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Lys Cys Asn
               20                  25                  30
   Thr Ala Thr Cys Val Leu Gly Arg Leu Ser Glu Glu Leu His Arg Leu
           35                  40                  45
   Gln Thr Tyr Pro Arg Thr Asn Thr Gly Ser Asn Thr Tyr
       50                  55                  60
<210> 383
   <211> 60
   <212> PRT
   <213> Artificial
<220>
   <223> Hybrid lizard, human and salmon construct
<220>
   <221> MOD_RES
   <222> (29)..(29)
   <223> 3,6-dioxactoanoyl
<400> 383
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Cys Asn Thr
               20                  25                  30
   Ala Thr Cys Val Leu Gly Arg Leu Ser Glu Glu Leu His Arg Leu Gln
           35                  40                  45
   Thr Tyr Pro Arg Thr Asn Thr Gly Ser Asn Thr Tyr
       50                  55                  60
<210> 384
   <211> 61
   <212> PRT
   <213> Artificial
<220>
   <223> Hybrid lizard, human and salmon construct
<220>
   <221> MOD_RES
   <222> (29)..(29)
   <223> 5-Apa
<400> 384
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Lys Cys Asn
               20                  25                  30
   Thr Ala Thr Cys Val Leu Gly Arg Leu Ser Glu Glu Leu His Arg Leu
           35                  40                  45
   Gln Thr Tyr Pro Arg Thr Asn Thr Gly Ser Asn Thr Tyr
       50                  55                  60
<210> 385
   <211> 60
   <212> PRT
   <213> Artificial
<220>
   <223> Hybrid lizard, human and salmon construct
<220>
   <221> MOD_RES
   <222> (29)..(29)
   <223> 5-Apa
<400> 385
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Cys Asn Thr
               20                  25                  30
   Ala Thr Cys Val Leu Gly Arg Leu Ser Glu Glu Leu His Arg Leu Gln
           35                  40                  45
   Thr Tyr Pro Arg Thr Asn Thr Gly Ser Asn Thr Tyr
       50                  55                  60
<210> 386
   <211> 62
   <212> PRT
   <213> Artificial
<220>
   <223> Hybrid lizard, human and salmon construct
<220>
   <221> MOD_RES
   <222> (29)..(30)
   <223> bAla
<400> 386
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Lys Cys
               20                  25                  30
   Asn Thr Ala Thr Cys Val Leu Gly Arg Leu Ser Glu Glu Leu His Arg
           35                  40                  45
   Leu Gln Thr Tyr Pro Arg Thr Asn Thr Gly Ser Asn Thr Tyr
       50                  55                  60
<210> 387
   <211> 61
   <212> PRT
   <213> Artificial
<220>
   <223> Hybrid lizard, human and salmon construct
<220>
   <221> MOD_RES
   <222> (29)..(30)
   <223> bAla
<400> 387
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Cys Asn
               20                  25                  30
   Thr Ala Thr Cys Val Leu Gly Arg Leu Ser Glu Glu Leu His Arg Leu
           35                  40                  45
   Gln Thr Tyr Pro Arg Thr Asn Thr Gly Ser Asn Thr Tyr
       50                  55                  60
<210> 388
   <211> 61
   <212> PRT
   <213> Artificial
<220>
   <223> Hybrid lizard, human and salmon construct
<220>
   <221> MOD_RES
   <222> (29)..(29)
   <223> 4,7,10-trioxa-13-tridecanamine succinimidyl
<400> 388
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Lys Cys Asn
               20                  25                  30
   Thr Ala Thr Cys Val Leu Gly Arg Leu Ser Glu Glu Leu His Arg Leu
           35                  40                  45
   Gln Thr Tyr Pro Arg Thr Asn Thr Gly Ser Asn Thr Tyr
       50                  55                  60
<210> 389
   <211> 60
   <212> PRT
   <213> Artificial
<220>
   <223> Hybrid lizard, human and salmon construct
<220>
   <221> MOD_RES
   <222> (29)..(29)
   <223> 4,7,10-trioxa-13-tridecanamine succinimidyl
<400> 389
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Cys Asn Thr
               20                  25                  30
   Ala Thr Cys Val Leu Gly Arg Leu Ser Glu Glu Leu His Arg Leu Gln
           35                  40                  45
   Thr Tyr Pro Arg Thr Asn Thr Gly Ser Asn Thr Tyr
       50                  55                  60
<210> 390
   <211> 43
   <212> PRT
   <213> Artificial
<220>
   <223> Hybrid human and salmon construct
<220>
   <221> MOD_RES
   <222> (2)..(2)
   <223> Tyr(SO3)
<400> 390
   Asp Tyr Met Gly Trp Met Asp Phe Gly Lys Arg Lys Cys Asn Thr Ala
   1               5                   10                  15
   Thr Cys Ala Thr Gln Arg Leu Ala Asn Glu Leu Val Arg Leu Gln Thr
               20                  25                  30
   Tyr Pro Arg Thr Asn Val Gly Ser Asn Thr Tyr
           35                  40
<210> 391
   <211> 68
   <212> PRT
   <213> Artificial
<220>
   <223> Hybird lizard human construct
<220>
   <221> MISC_FEATURE
   <223> C-term amidated
<220>
   <221> MOD_RES
   <222> (29)..(30)
   <223> bAla
<400> 391
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Phe Leu
               20                  25                  30
   Phe His Tyr Ser Lys Thr Gln Lys Leu Gly Lys Ser Asn Val Val Glu
           35                  40                  45
   Glu Leu Gln Ser Pro Phe Ala Ser Gln Ser Arg Gly Tyr Phe Leu Phe
       50                  55                  60
   Arg Pro Arg Asn
   65
<210> 392
   <211> 55
   <212> PRT
   <213> Artificial
<220>
   <223> Hybrid lizard and human construct
<220>
   <221> MISC_FEATURE
   <223> C-term amidated
<220>
   <221> MOD_RES
   <222> (29)..(30)
   <223> bAla
<400> 392
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Phe Arg
               20                  25                  30
   Val Asp Glu Glu Phe Gln Ser Pro Phe Ala Ser Gln Ser Arg Gly Tyr
           35                  40                  45
   Phe Leu Phe Arg Pro Arg Asn
       50                  55
<210> 393
   <211> 39
   <212> PRT
   <213> Artificial
<220>
   <223> Hybrid lizard and human construct
<220>
   <221> MISC_FEATURE
   <223> C-term amidated
<220>
   <221> MOD_RES
   <222> (29)..(30)
   <223> bAla
<400> 393
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Gly Tyr
               20                  25                  30
   Phe Leu Phe Arg Pro Arg Asn
           35
<210> 394
   <211> 62
   <212> PRT
   <213> Artificial
<220>
   <223> Hybrid lizard and human construct
<220>
   <221> MOD_RES
   <222> (29)..(30)
   <223> bAla
<400> 394
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Ser Pro
               20                  25                  30
   Lys Met Val Gln Gly Ser Gly Cys Phe Gly Arg Lys Met Asp Arg Ile
           35                  40                  45
   Ser Ser Ser Ser Gly Leu Gly Cys Lys Val Leu Arg Arg His
       50                  55                  60
<210> 395
   <211> 73
   <212> PRT
   <213> Artificial
<220>
   <223> Hybrid lizard and human construct
<220>
   <221> MOD_RES
   <222> (40)..(41)
   <223> bAla
<400> 395
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser Xaa Xaa Ser Pro Lys Met Val Gln Gly
   35 40 45
   Ser Gly Cys Phe Gly Arg Lys Met Asp Arg Ile Ser Ser Ser Ser Gly
   50 55 60
   Leu Gly Cys Lys Val Leu Arg Arg His
   65 70
<210> 396
   <211> 32
   <212> PRT
   <213> Artificial
<220>
   <223> Hybrid salmon and human construct
<220>
   <221> MISC_FEATURE
   <223> C-term amidated
<400> 396
   Lys Cys Asn Thr Ala Thr Cys Val Leu Gly Arg Leu Ser Gln Glu Leu
   1 5 10 15
   His Arg Leu Gln Thr Tyr Pro Arg Thr Asn Thr Gly Ser Asn Thr Tyr
   20 25 30
<210> 397
   <211> 39
   <212> PRT
   <213> Artificial
<220>
   <223> Hybrid human and salmon construct
<220>
   <221> MOD_RES
   <222> (4)..(4)
   <223> d-Leucine
<400> 397
   Ser Ser Ser Leu Pro Gln Thr Lys Cys Asn Thr Ala Thr Cys Val Leu
   1 5 10 15
   Gly Arg Leu Ser Gln Glu Leu His Arg Leu Gln Thr Tyr Pro Arg Thr
   20 25 30
   Asn Thr Gly Ser Asn Thr Tyr
   35
<210> 398
   <211> 43
   <212> PRT
   <213> Artificial
<220>
   <223> Hybrid human and salmon construct
<220>
   <221> MOD_RES
   <222> (2)..(2)
   <223> para(CH2so3)Phe
<400> 398
   Asp Phe Met Gly Trp Met Asp Phe Gly Lys Arg Lys Cys Asn Thr Ala
   1 5 10 15
   Thr Cys Ala Thr Gln Arg Leu Ala Asn Glu Leu Val Arg Leu Gln Thr
   20 25 30
   Tyr Pro Arg Thr Asn Val Gly Ser Asn Thr Tyr
   35 40
<210> 399
   <211> 41
   <212> PRT
   <213> Homo sapiens
<400> 399
   Ile Val Leu Ser Leu Asp Val Pro Ile Gly Leu Leu Gln Ile Leu Leu
   1 5 10 15
   Glu Gln Ala Arg Ala Arg Ala Ala Arg Glu Gln Ala Thr Thr Asn Ala
   20 25 30
   Arg Ile Leu Ala Arg Val Gly His Cys
   35 40
<210> 400
   <211> 6
   <212> PRT
   <213> Homo sapiens
<400> 400
   Met Val Pro Ile Gln Lys
   1 5
<210> 401
   <211> 6
   <212> PRT
   <213> Homo sapiens
<400> 401
   Val Gln Asp Asp Thr Lys
   1 5
<210> 402
   <211> 4
   <212> PRT
   <213> Homo sapiens
<400> 402
   Thr Leu Ile Lys
   1
<210> 403
   <211> 5
   <212> PRT
   <213> Homo sapiens
<400> 403
   Thr Ile Val Thr Arg
   1 5
<210> 404
   <211> 13
   <212> PRT
   <213> Homo sapiens
<400> 404
   Ile Asn Asp Ile Ser His Thr Gln Ser Val Ser Ser Lys
   1 5 10
<210> 405
   <211> 18
   <212> PRT
   <213> Homo sapiens
<400> 405
   Val Thr Gly Leu Asp Phe Ile Pro Gly Leu His Pro Ile Leu Thr Leu
   1               5                   10                  15
   Ser Lys
<210> 406
   <211> 13
   <212> PRT
   <213> Homo sapiens
<400> 406
   Asn Val Ile Gln Ile Ser Asn Asp Leu Glu Asn Leu Arg
   1               5                   10
<210> 407
   <211> 10
   <212> PRT
   <213> Homo sapiens
<400> 407
   Asp Leu Leu His Val Leu Ala Phe Ser Lys
   1               5                   10
<210> 408
   <211> 34
   <212> PRT
   <213> Homo sapiens
<400> 408
   Ser Cys His Leu Pro Trp Ala Ser Gly Leu Glu Thr Leu Asp Ser Leu
   1               5                   10                  15
   Gly Gly Val Leu Glu Ala Ser Gly Tyr Ser Thr Glu Val Val Ala Leu
               20                  25                  30
   Ser Arg
<210> 409
   <211> 18
   <212> PRT
   <213> Homo sapiens
<400> 409
   Leu Gln Gly Ser Leu Gln Asp Met Leu Trp Gln Leu Asp Leu Ser Pro
   1               5                   10                  15
   Gly Cys
<210> 410
   <211> 9
   <212> PRT
   <213> Artificial
<220>
   <223> genus
<220>
   <221> MISC_FEATURE
   <222> (1)..()
   <223> xaa can be contiguous mouse or human leptin sequence
<220>
   <221> MISC_FEATURE
   <222> (4)..()
   <223> xaa can be any amino acid
<220>
   <221> MISC_FEATURE
   <222> (7)..()
   <223> xaa can be any amino acid
<220>
   <221> MISC_FEATURE
   <222> (8)..()
   <223> xaa is any amino acid
<220>
   <221> MISC_FEATURE
   <222> (9)..()
   <223> xaa can be contigous mouse or human leptin sequence
<400> 410
   Xaa Ser Cys Xaa Leu Pro Xaa Xaa Xaa
   1               5
<210> 411
   <211> 7
   <212> PRT
   <213> Mus musculus
<400> 411
   Ser Cys Ser Leu Pro Gln Thr
   1               5
<210> 412
   <211> 7
   <212> PRT
   <213> Homo sapiens
<400> 412
   Ser Cys His Leu Pro Trp Ala
   1               5
<210> 413
   <211> 52
   <212> PRT
   <213> Artificial
<220>
   <223> synthetic
<220>
   <221> MISC_FEATURE
   <222> (6)..()
   <223> Xaa is Trp or Gln
<220>
   <221> MISC_FEATURE
   <222> (36)..()
   <223> xaa is Gln or Glu
<220>
   <221> MISC_FEATURE
   <222> (40)..()
   <223> xaa is Gln or Glu
<220>
   <221> MISC_FEATURE
   <222> (42)..()
   <223> xaa is Ile, Leu, Met or methionine sulfoxide
<220>
   <221> MISC_FEATURE
   <222> (44)..()
   <223> xaa is Trp or Gln
<220>
   <221> MISC_FEATURE
   <222> (45)..()
   <223> xaa is Gln or Glu
<400> 413
   Ser Cys His Leu Pro Xaa Ala Ser Gly Leu Glu Thr Leu Asp Ser Leu
   1               5                   10                  15
   Gly Gly Val Leu Glu Ala Ser Gly Tyr Ser Thr Glu Val Val Ala Leu
               20                  25                  30
   Ser Arg Leu Xaa Gly Ser Leu Xaa Asp Xaa Leu Xaa Xaa Leu Asp Leu
           35                  40                  45
   Ser Pro Gly Cys
       50
<210> 414
   <211> 146
   <212> PRT
   <213> Mus musculus
<220>
   <221> MISC_FEATURE
   <222> (28)..()
   <223> xaa is Gln or absent
<400> 414
   Val Pro Ile Gln Lys Val Gln Asp Asp Thr Lys Thr Leu Ile Lys Thr
   1               5                   10                  15
   Ile Val Thr Arg Ile Asn Asp Ile Ser His Thr Xaa Ser Val Ser Ser
               20                  25                  30
   Lys Gln Lys Val Thr Gly Leu Asp Phe Ile Pro Gly Leu His Pro Ile
           35                  40                  45
   Leu Thr Leu Ser Lys Met Asp Gln Thr Leu Ala Val Tyr Gln Gln Ile
       50                  55                  60
   Leu Thr Ser Met Pro Ser Arg Asn Val Ile Gln Ile Ser Asn Asp Leu
   65                  70                  75                  80
   Glu Asn Leu Arg Asp Leu Leu His Val Leu Ala Phe Ser Lys Ser Cys
                   85                  90                  95
   His Leu Pro Gln Ala Ser Gly Leu Glu Thr Leu Glu Ser Leu Gly Gly
               100                 105                 110
   Val Leu Glu Ala Ser Gly Tyr Ser Thr Glu Val Val Ala Leu Ser Arg
           115                 120                 125
   Leu Gln Gly Ser Leu Gln Asp Met Leu Gln Gln Leu Asp Leu Ser Pro
       130                 135                 140
   Gly Cys
   145
<210> 415
   <211> 146
   <212> PRT
   <213> porcine
<400> 415
   Val Pro Ile Trp Arg Val Gln Asp Asp Thr Lys Thr Leu Ile Lys Thr
   1               5                   10                  15
   Ile Val Thr Arg Ile Ser Asp Ile Ser His Met Gln Ser Val Ser Ser
               20                  25                  30
   Lys Gln Arg Val Thr Gly Leu Asp Phe Ile Pro Gly Leu His Pro Val
           35                  40                  45
   Leu Ser Leu Ser Lys Met Asp Gln Thr Leu Ala Ile Tyr Gln Gln Ile
       50                  55                  60
   Leu Thr Ser Leu Pro Ser Arg Asn Val Ile Gln Ile Ser Asn Asp Leu
   65                  70                  75                  80
   Glu Asn Leu Arg Asp Leu Leu His Leu Leu Ala Ser Ser Lys Ser Cys
                   85                  90                  95
   Pro Leu Pro Gln Ala Arg Ala Leu Glu Thr Leu Glu Ser Leu Gly Gly
               100                 105                 110
   Val Leu Glu Ala Ser Leu Tyr Ser Thr Glu Val Val Ala Leu Ser Arg
           115                 120                 125
   Leu Gln Gly Ala Leu Gln Asp Met Leu Arg Gln Leu Asp Leu Ser Pro
       130                 135                 140
   Gly Cys
   145
<210> 416
   <211> 146
   <212> PRT
   <213> bovine
<220>
   <221> MISC_FEATURE
   <222> (28)..()
   <223> xaa is Gln or absent
<400> 416
   Val Pro Ile Cys Lys Val Gln Asp Asp Thr Lys Thr Leu Ile Lys Thr
   1 5 10 15
   Ile Val Thr Arg Ile Asn Asp Ile Ser His Thr Xaa Ser Val Ser Ser
   20 25 30
   Lys Gln Arg Val Thr Gly Leu Asp Phe Ile Pro Gly Leu His Pro Leu
   35 40 45
   Leu Ser Leu Ser Lys Met Asp Gln Thr Leu Ala Ile Tyr Gln Gln Ile
   50 55 60
   Leu Thr Ser Leu Pro Ser Arg Asn Val Val Gln Ile Ser Asn Asp Leu
   65 70 75 80
   Glu Asn Leu Arg Asp Leu Leu His Leu Leu Ala Ala Ser Lys Ser Cys
   85 90 95
   Pro Leu Pro Gln Val Arg Ala Leu Glu Ser Leu Glu Ser Leu Gly Val
   100 105 110
   Val Leu Glu Ala Ser Leu Tyr Ser Thr Glu Val Val Ala Leu Ser Arg
   115 120 125
   Leu Gln Gly Ser Leu Gln Asp Met Leu Arg Gln Leu Asp Leu Ser Pro
   130 135 140
   Gly Cys
   145
<210> 417
   <211> 146
   <212> PRT
   <213> Homo sapiens
<220>
   <221> MISC_FEATURE
   <222> (27)..()
   <223> xaa is Thr or Ala
<220>
   <221> MISC_FEATURE
   <222> (28)..()
   <223> Xaa is Gln or absent
<400> 417
   Val Pro Ile Gln Lys Val Gln Asp Asp Thr Lys Thr Leu Ile Lys Thr
   1               5                   10                  15
   Ile Val Thr Arg Ile Asn Asp Ile Ser His Xaa Xaa Ser Val Ser Ser
               20                  25                  30
   Lys Gln Lys Val Thr Gly Leu Asp Phe Ile Pro Gly Leu His Pro Ile
           35                  40                  45
   Leu Thr Leu Ser Lys Met Asp Gln Thr Leu Ala Val Tyr Gln Gln Ile
       50                  55                  60
   Leu Thr Ser Met Pro Ser Arg Asn Val Ile Gln Ile Ser Asn Asp Leu
   65                  70                  75                  80
   Glu Asn Leu Arg Asp Leu Leu His Val Leu Ala Phe Ser Lys Ser Cys
                   85                  90                  95
   His Leu Pro Trp Ala Ser Gly Leu Glu Thr Leu Asp Ser Leu Gly Gly
               100                 105                 110
   Val Leu Glu Ala Ser Gly Tyr Ser Thr Glu Val Val Ala Leu Ser Arg
           115                 120                 125
   Leu Gln Gly Ser Leu Gln Asp Met Leu Trp Gln Leu Asp Leu Ser Pro
       130                 135                 140
   Gly Cys
   145
<210> 418
   <211> 146
   <212> PRT
   <213> rhesus
<400> 418
   Val Pro Ile Gln Lys Val Gln Ser Asp Thr Lys Thr Leu Ile Lys Thr
   1               5                   10                  15
   Ile Val Thr Arg Ile Asn Asp Ile Ser His Thr Gln Ser Val Ser Ser
               20                  25                  30
   Lys Gln Arg Val Thr Gly Leu Asp Phe Ile Pro Gly Leu His Pro Val
           35                  40                  45
   Leu Thr Leu Ser Gln Met Asp Gln Thr Leu Ala Ile Tyr Gln Gln Ile
       50                  55                  60
   Leu Ile Asn Leu Pro Ser Arg Asn Val Ile Gln Ile Ser Asn Asp Leu
   65 70 75 80
   Glu Asn Leu Arg Asp Leu Leu His Leu Leu Ala Phe Ser Lys Ser Cys
   85 90 95
   His Leu Pro Leu Ala Ser Gly Leu Glu Thr Leu Glu Ser Leu Gly Asp
   100 105 110
   Val Leu Glu Ala Ser Leu Tyr Ser Thr Glu Val Val Ala Leu Ser Arg
   115 120 125
   Leu Gln Gly Ser Leu Gln Asp Met Leu Trp Gln Leu Asp Leu Ser Pro
   130 135 140
   Gly Cys
   145
<210> 419
   <211> 146
   <212> PRT
   <213> Rattus norvegicus
<400> 419
   Val Pro Ile His Lys Val Gln Asp Asp Thr Lys Thr Leu Ile Lys Thr
   1 5 10 15
   Ile Val Thr Arg Ile Asn Asp Ile Ser His Thr Gln Ser Val Ser Ala
   20 25 30
   Arg Gln Arg Val Thr Gly Leu Asp Phe Ile Pro Gly Leu His Pro Ile
   35 40 45
   Leu Ser Leu Ser Lys Met Asp Gln Thr Leu Ala Val Tyr Gln Gln Ile
   50 55 60
   Leu Thr Ser Leu Pro Ser Gln Asn Val Leu Gln Ile Ala His Asp Leu
   65 70 75 80
   Glu Asn Leu Arg Asp Leu Leu His Leu Leu Ala Phe Ser Lys Ser Cys
   85 90 95
   Ser Leu Pro Gln Thr Arg Gly Leu Gln Lys Pro Glu Ser Leu Asp Gly
   100 105 110
   Val Leu Glu Ala Ser Leu Tyr Ser Thr Glu Val Val Ala Leu Ser Arg
   115 120 125
   Leu Gln Gly Ser Leu Gln Asp Ile Leu Gln Gln Leu Asp Leu Ser Pro
   130 135 140
   Glu Cys
   145
<210> 420
   <211> 146
   <212> PRT
   <213> Artificial
<220>
   <223> leptin genus
<220>
   <221> MISC_FEATURE
   <222> (22)..()
   <223> xaa is Asn, Asp or Glu
<220>
   <221> MISC_FEATURE
   <222> (27)..()
   <223> Xaa is Thr or Ala
<220>
   <221> MISC_FEATURE
   <222> (28)..()
   <223> Xaa is Gln, Glu, or absent
<220>
   <221> MISC_FEATURE
   <222> (54)..()
   <223> xaa is Met or Ala
<220>
   <221> MISC_FEATURE
   <222> (68)..()
   <223> Xaa is Met or Leu
<220>
   <221> MISC_FEATURE
   <222> (72)..()
   <223> xaa is Asn, Asp or Glu
<220>
   <221> MISC_FEATURE
   <222> (77)..()
   <223> xaa is Ser or Ala
<220>
   <221> MISC_FEATURE
   <222> (118)..()
   <223> xaa is Gly or Leu
<400> 420
   Val Pro Ile Gln Lys Val Gln Asp Asp Thr Lys Thr Leu Ile Lys Thr
   1               5                   10                  15
   Ile Val Thr Arg Ile Xaa Asp Ile Ser His Xaa Xaa Ser Val Ser Ser
               20                  25                  30
   Lys Gln Lys Val Thr Gly Leu Asp Phe Ile Pro Gly Leu His Pro Ile
           35                  40                  45
   Leu Thr Leu Ser Lys Xaa Asp Gln Thr Leu Ala Val Tyr Gln Gln Ile
       50                  55                  60
   Leu Thr Ser Xaa Pro Ser Arg Xaa Val Ile Gln Ile Xaa Asn Asp Leu
   65                  70                  75                  80
   Glu Asn Leu Arg Asp Leu Leu His Val Leu Ala Phe Ser Lys Ser Cys
                   85                  90                  95
   His Leu Pro Trp Ala Ser Gly Leu Glu Thr Leu Asp Ser Leu Gly Gly
               100                 105                 110
   Val Leu Glu Ala Ser Xaa Tyr Ser Thr Glu Val Val Ala Leu Ser Arg
           115                 120                 125
   Leu Gln Gly Ser Leu Gln Asp Met Leu Trp Gln Leu Asp Leu Ser Pro
       130                 135                 140
   Gly Cys
   145
<210> 421
   <211> 146
   <212> PRT
   <213> Artificial
<220>
   <223> leptin genus
<220>
   <221> MISC_FEATURE
   <222> (27)..()
   <223> Xaa is Thr or Ala
<220>
   <221> MISC_FEATURE
   <222> (77)..()
   <223> xaa is ser or Ala
<220>
   <221> MISC_FEATURE
   <222> (118)..()
   <223> Xaa is Gly or Leu
<400> 421
   Val Pro Ile Gln Lys Val Gln Asp Asp Thr Lys Thr Leu Ile Lys Thr
   1               5                   10                  15
   Ile Val Thr Arg Ile Asn Asp Ile Ser His Xaa Gln Ser Val Ser Ser
               20                  25                  30
   Lys Gln Lys Val Thr Gly Leu Asp Phe Ile Pro Gly Leu His Pro Ile
           35                  40                  45
   Leu Thr Leu Ser Lys Met Asp Gln Thr Leu Ala Val Tyr Gln Gln Ile
       50                  55                  60
   Leu Thr Ser Met Pro Ser Arg Asn Val Ile Gln Ile Xaa Asn Asp Leu
   65                  70                  75                  80
   Glu Asn Leu Arg Asp Leu Leu His Val Leu Ala Phe Ser Lys Ser Cys
                   85                  90                  95
   His Leu Pro Trp Ala Ser Gly Leu Glu Thr Leu Asp Ser Leu Gly Gly
               100                 105                 110
   Val Leu Glu Ala Ser Xaa Tyr Ser Thr Glu Val Val Ala Leu Ser Arg
           115                 120                 125
   Leu Gln Gly Ser Leu Gln Asp Met Leu Trp Gln Leu Asp Leu Ser Pro
       130                 135                 140
   Gly Cys
   145
<210> 422
   <211> 105
   <212> PRT
   <213> Artificial
<220>
   <223> leptin genus
<220>
   <221> MISC_FEATURE
   <222> (13)..()
   <223> xaa is Ile, Leu, Met or methionine sulfoxide
<220>
   <221> MISC_FEATURE
   <222> (15)..()
   <223> Xaa is Gln or Glu
<220>
   <221> MISC_FEATURE
   <222> (21)..()
   <223> Xaa is Gln or Glu
<220>
   <221> MISC_FEATURE
   <222> (22)..()
   <223> Xaa is Gln or Glu
<220>
   <221> MISC_FEATURE
   <222> (27)..()
   <223> xaa is Ile, Leu, Met or methionine sulfoxide
<220>
   <221> MISC_FEATURE
   <222> (31)..()
   <223> Xaa is Asn, Asp or Gln
<220>
   <221> MISC_FEATURE
   <222> (34)..()
   <223> xaa is Gln or Glun
<220>
   <221> MISC_FEATURE
   <222> (37)..()
   <223> xaa is Asn, Asp or Gln
<220>
   <221> MISC_FEATURE
   <222> (41)..()
   <223> xaa is Asn, Asp or Gln
<220>
   <221> MISC_FEATURE
   <222> (59) .. ()
   <223> Xaa is Trp or Gln
<220>
   <221> MISC_FEATURE
   <222> (89)..()
   <223> Xaa is Gln or Glu
<220>
   <221> MISC_FEATURE
   <222> (93)..()
   <223> Xaa is Gln or Glu
<220>
   <221> MISC_FEATURE
   <222> (95) .. ()
   <223> xaa is Ile, Leu, Met or methionine sulfoxide
<220>
   <221> MISC_FEATURE
   <222> (97)..()
   <223> xaa is Trp or Gln
<220>
   <221> MISC_FEATURE
   <222> (98) .. ()
   <223> Xaa is Gln or Glu
<400> 422
   Ile Pro Gly Leu His Pro Ile Leu Thr Leu Ser Lys Xaa Asp Xaa Thr
   1               5                   10                  15
   Leu Ala Val Tyr Xaa Xaa Ile Leu Thr Ser Xaa Pro Ser Arg Xaa Val
               20                  25                  30
   Ile Xaa Ile Ser Xaa Asp Leu Glu Xaa Leu Arg Asp Leu Leu His Val
           35                  40                  45
   Leu Ala Phe Ser Lys Ser Cys His Leu Pro Xaa Ala Ser Gly Leu Glu
       50                  55                  60
   Thr Leu Asp Ser Leu Gly Gly Val Leu Glu Ala Ser Gly Tyr Ser Thr
   65                  70                  75                  80
   Glu Val Val Ala Leu Ser Arg Leu Xaa Gly Ser Leu Xaa Asp Xaa Leu
                   85                  90                  95
   Xaa Xaa Leu Asp Leu Ser Pro Gly Cys
               100                 105
<210> 423
   <211> 36
   <212> PRT
   <213> Artificial
<220>
   <223> PPF genus
<220>
   <221> MISC_FEATURE
   <222> (1)..()
   <223> xaa is Tyr or absent
<220>
   <221> MISC_FEATURE
   <222> (2)..()
   <223> xaa is Ile, Pro, or absent
<220>
   <221> MISC_FEATURE
   <222> (3)..()
   <223> xaa is Ile, Bolton-Hunter-modified Lys, Lys, val, or Pro
<220>
   <221> MISC_FEATURE
   <222> (4)..()
   <223> xaa is Lys, Bolton-Hunter-modified Lys, Ala, Ser, or Arg
<220>
   <221> MISC_FEATURE
   <222> (7) .. ()
   <223> xaa is Ala, Gly, or His
<220>
   <221> MISC_FEATURE
   <222> (9) .. ()
   <223> xaa is Gly or Ala
<220>
   <221> MISC_FEATURE
   <222> (12) .. ()
   <223> xaa is Ala or Pro
<220>
   <221> MISC_FEATURE
   <222> (13) .. ()
   <223> Xaa is Ser or Pro
<220>
   <221> MISC_FEATURE
   <222> (14)..()
   <223> xaa is Pro, Ala, or Ser
<220>
   <221> MISC_FEATURE
   <222> (16)..()
   <223> xaa is Glu or Asp
<220>
   <221> MISC_FEATURE
   <222> (17)..()
   <223> Xaa is Leu or Ile
<220>
   <221> MISC_FEATURE
   <222> (18) .. ()
   <223> Xaa is Asn or Ala
<220>
   <221> MISC_FEATURE
   <222> (19) .. ()
   <223> Xaa is Arg, Lys, Bolton-Hunter-modified Lys, Gln, or N(Me)Ala
<220>
   <221> MISC_FEATURE
   <222> (21)..()
   <223> Xaa is Tyr, Ala, Phe, Lys or Bolton-Hunter-modified Lys
<220>
   <221> MISC_FEATURE
   <222> (22)..()
   <223> Xaa is Ala or Ser
<220>
   <221> MISC_FEATURE
   <222> (23) .. ()
   <223> Xaa is Ser, Ala, or Asp
<220>
   <221> MISC_FEATURE
   <222> (25) .. ()
   <223> Xaa is Arg, Lys or Bolton-Hunter-modified Lys
<220>
   <221> MISC_FEATURE
   <222> (26) .. ()
   <223> Xaa is His, Ala, or Arg
<220>
   <221> MISC_FEATURE
   <222> (28)..()
   <223> Xaa is Leu or Ile
<220>
   <221> MISC_FEATURE
   <222> (30) .. ()
   <223> Xaa is Leu or Met
<220>
   <221> MISC_FEATURE
   <222> (31) .. ()
   <223> Xaa is val, Ile, or Leu
<220>
   <221> MISC_FEATURE
   <222> (35)..()
   <223> xaa is Lys, Bolton-Hunter-modified Lys, or Arg
<220>
   <221> MISC_FEATURE
   <222> (36) .. ()
   <223> Xaa is Tyr, Trp, or Phe
<400> 423
   Xaa Xaa Xaa Xaa Pro Glu Xaa Pro Xaa Glu Asp Xaa Xaa Xaa Glu Xaa
   1               5                   10                  15
   Xaa Xaa Xaa Tyr Xaa Xaa Xaa Leu Xaa Xaa Tyr Xaa Asn Xaa Xaa Thr
               20                  25                  30
   Arg Gln Xaa Xaa
           35
<210> 424
   <211> 36
   <212> PRT
   <213> Artificial
<220>
   <223> PPF genus
<220>
   <221> MISC_FEATURE
   <222> (1) .. ()
   <223> Xaa is Tyr or absent
<220>
   <221> MISC_FEATURE
   <222> (2) .. ()
   <223> Xaa is Ile, Pro, or absent
<220>
   <221> MISC_FEATURE
   <222> (4) .. ()
   <223> Xaa is Lys, Bolton-Hunter-modified Lys, Ala, Ser, or Arg
<220>
   <221> MISC_FEATURE
   <222> (6) .. ()
   <223> Xaa is Glu, Gln, Ala, Asn, Asp, or val
<220>
   <221> MISC_FEATURE
   <222> (9) .. ()
   <223> xaa is Gly or Ala
<220>
   <221> MISC_FEATURE
   <222> (10) .. ()
   <223> xaa is Glu, Ala, Asp, Asn, Gln, Gly, Pro, or Aib
<220>
   <221> MISC_FEATURE
   <222> (11)..()
   <223> Xaa is Glu, Ala, Asp, Asn, Gln, Gly, Pro, or Aib
<220>
   <221> MISC_FEATURE
   <222> (12)..()
   <223> xaa is Ala or Pro
<220>
   <221> MISC_FEATURE
   <222> (13)..()
   <223> Xaa is Ser or Pro
<220>
   <221> MISC_FEATURE
   <222> (14)..()
   <223> Xaa is Pro, Ala, or Ser
<220>
   <221> MISC_FEATURE
   <222> (15) .. ()
   <223> xaa is Glu, Ala, Asp, Asn, Gln, Gly, Pro, or Aib
<220>
   <221> MISC_FEATURE
   <222> (16)..()
   <223> xaa is Glu or Asp
<220>
   <221> MISC_FEATURE
   <222> (17) .. ()
   <223> xaa is Leu or Ile
<220>
   <221> MISC_FEATURE
   <222> (19) .. ()
   <223> xaa is Arg, Lys, BH-modified Lys, Gln, or N(Me)Ala
<220>
   <221> MISC_FEATURE
   <222> (21)..()
   <223> xaa is Tyr, Ala, Phe, Lys, or BH-modified Lys
<220>
   <221> MISC_FEATURE
   <222> (22) .. ()
   <223> xaa is Ala or Ser
<220>
   <221> MISC_FEATURE
   <222> (23)..()
   <223> xaa is Ser, Ala, or Asp
<220>
   <221> MISC_FEATURE
   <222> (25)..()
   <223> xaa is Arg, Lys or Bolton-Hunter-modified Lys
<220>
   <221> MISC_FEATURE
   <222> (26)..()
   <223> xaa is His, Ala, or Arg
<220>
   <221> MISC_FEATURE
   <222> (27)..()
   <223> Xaa is Tyr or Phe
<220>
   <221> MISC_FEATURE
   <222> (28)..()
   <223> xaa is Leu or Ile
<220>
   <221> MISC_FEATURE
   <222> (29)..()
   <223> xaa is Asn or Gln
<220>
   <221> MISC_FEATURE
   <222> (30)..()
   <223> Xaa is Leu or Met
<220>
   <221> MISC_FEATURE
   <222> (31)..()
   <223> Xaa is val, Ile, or Leu
<400> 424
   Xaa Xaa Pro Xaa Pro Xaa His Pro Xaa Xaa Xaa Xaa Xaa Xaa Xaa Xaa
   1               5                   10                  15
   Xaa Ala Xaa Tyr Xaa Xaa Xaa Leu Xaa Xaa Xaa Xaa Xaa Xaa Xaa Thr
               20                  25                  30
   Arg Gln Arg Tyr
           35
<210> 425
   <211> 34
   <212> PRT
   <213> Artificial
<220>
   <223> PPY-NPY chimera
<400> 425
   Pro Lys Pro Glu His Pro Gly Glu Asp Ala Ser Pro Glu Glu Leu Ala
   1               5                   10                  15
   Arg Tyr Tyr Ala Ser Leu Arg Ala Tyr Ile Asn Leu Ile Thr Arg Gln
               20                  25                  30
   Arg Tyr
<210> 426
   <211> 59
   <212> PRT
   <213> Artificial
<220>
   <223> hybrid
<400> 426
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Lys Cys Asn Thr Ala
               20                  25                  30
   Thr Cys Val Leu Gly Arg Leu Ser Gln Glu Leu His Arg Leu Gln Thr
           35                  40                  45
   Tyr Pro Arg Thr Asn Thr Gly Ser Asn Thr Tyr
       50                  55
<210> 427
   <211> 36
   <212> PRT
   <213> Artificial
<220>
   <223> hybrid
<220>
   <221> MISC_FEATURE
   <222> (30)..(30)
   <223> sulfated (SO3H) tyrosine
<400> 427
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Asp Tyr Met Gly
               20                  25                  30
   Trp Met Asp Phe
           35
<210> 428
   <211> 36
   <212> PRT
   <213> Artificial
<220>
   <223> hybrid
<220>
   <221> MISC_FEATURE
   <222> (30)..(30)
   <223> sulfated (CH2SO3H) tyrosine
<400> 428
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Asp Tyr Met Gly
               20                  25                  30
   Trp Met Asp Phe
           35
<210> 429
   <211> 63
   <212> PRT
   <213> Artificial
<220>
   <223> hybrid
<400> 429
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Gly Lys
   20 25 30
   Cys Asn Thr Ala Thr Cys Val Leu Gly Arg Leu Ser Gln Glu Leu His
   35 40 45
   Arg Leu Gln Thr Tyr Pro Arg Thr Asn Thr Gly Ser Asn Thr Tyr
   50 55 60
<210> 430
   <211> 67
   <212> PRT
   <213> Artificial
<220>
   <223> hybrid
<220>
   <221> MISC_FEATURE
   <222> (29)..(30)
   <223> beta-Alanine beta-Alanine linker
<400> 430
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Xaa Xaa Lys Cys
   20 25 30
   Asn Thr Ala Thr Cys Ala Thr Gln Arg Leu Ala Asn Phe Leu Val Arg
   35 40 45
   Ser Ser Asn Asn Leu Gly Pro Val Leu Pro Pro Thr Asn Val Gly Ser
   50 55 60
   Asn Thr Tyr
   65
<210> 431
   <211> 62
   <212> PRT
   <213> Artificial
<220>
   <223> hybrid
<220>
   <221> MISC_FEATURE
   <222> (29)..(30)
   <223> beta-Alanine beta-Alanine linker
<400> 431
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Lys Cys
               20                  25                  30
   Asn Thr Ala Thr Cys Val Leu Gly Arg Leu Ser Gln Glu Leu His Arg
           35                  40                  45
   Leu Gln Thr Tyr Pro Arg Thr Asn Thr Gly Ser Asn Thr Tyr
       50                  55                  60
<210> 432
   <211> 31
   <212> PRT
   <213> Artificial
<220>
   <223> hybrid component
<400> 432
   Cys Asn Thr Ala Thr Cys Val Leu Gly Arg Leu Ser Gln Glu Leu His
   1               5                   10                  15
   Arg Leu Gln Thr Tyr Pro Arg Thr Asn Thr Gly Ser Asn Thr Tyr
               20                  25                  30
<210> 433
   <211> 35
   <212> PRT
   <213> Artificial
<220>
   <223> hybrid component
<220>
   <221> MISC_FEATURE
   <222> (1)..(1)
   <223> xaa is succinimide beta-Alanine
<400> 433
   Xaa Ile Lys Pro Glu Ala Pro Gly Glu Asp Ala Ser Pro Glu Glu Leu
   1               5                   10                  15
   Asn Arg Tyr Tyr Ala Ser Leu Arg His Tyr Leu Asn Leu Val Thr Arg
               20                  25                  30
   Gln Arg Tyr
           35
<210> 434
   <211> 33
   <212> PRT
   <213> Artificial
<220>
   <223> hybrid component
<220>
   <221> MISC_FEATURE
   <222> (1) .. (1)
   <223> Xaa is thiopropyl beta-Alanine
<400> 434
   Xaa Lys Cys Asn Thr Ala Thr Cys Val Leu Gly Arg Leu Ser Gln Glu
   1               5                   10                  15
   Leu His Arg Leu Gln Thr Tyr Pro Arg Thr Asn Thr Gly Ser Asn Thr
               20                  25                  30
   Tyr
<210> 435
   <211> 35
   <212> PRT
   <213> Artificial
<220>
   <223> hybrid component
<220>
   <221> MISC_FEATURE
   <222> (1)..(1)
   <223> xaa is maleimide butyl beta-Alanine
<400> 435
   Xaa Ile Lys Pro Glu Ala Pro Gly Glu Asp Ala Ser Pro Glu Glu Leu
   1               5                   10                  15
   Asn Arg Tyr Tyr Ala Ser Leu Arg His Tyr Leu Asn Leu Val Thr Arg
               20                  25                  30
   Gln Arg Tyr
           35
<210> 436
   <211> 36
   <212> PRT
   <213> Artificial
<220>
   <223> hybrid component
<400> 436
   Cys Asn Thr Ala Thr Cys Ala Thr Gln Arg Leu Ala Asn Phe Leu Val
   1 5 10 15
   Arg Ser Ser Asn Asn Leu Gly Pro Val Leu Pro Pro Thr Asn Val Gly
   20 25 30
   Ser Asn Thr Tyr
   35
<210> 437
   <211> 9
   <212> PRT
   <213> Artificial
<220>
   <223> hybrid component
<220>
   <221> MISC_FEATURE
   <222> (1)..(1)
   <223> Xaa is maleimide butyl beta-Alanine
<220>
   <221> MISC_FEATURE
   <222> (3)..(3)
   <223> sulfated (CH2SO3H) phenylalanine
<400> 437
   Xaa Asp Phe Met Gly Trp Met Asp Phe
   1 5
<210> 438
   <211> 43
   <212> PRT
   <213> Artificial
<220>
   <223> hybrid
<220>
   <221> MISC_FEATURE
   <222> (2)..(2)
   <223> sulfated (para-CH2SO3) phenylalanine
<400> 438
   Asp Phe Met Gly Trp Met Asp Phe Gly Lys Arg Lys Cys Asn Thr Ala
   1               5                   10                  15
   Thr Cys Ala Thr Gln Arg Leu Ala Asn Glu Leu Val Arg Leu Gln Thr
               20                  25                  30
   Tyr Pro Arg Thr Asn Val Gly Ser Asn Thr Tyr
           35                  40
<210> 439
   <211> 9
   <212> PRT
   <213> Artificial
<220>
   <223> hybrid component
<220>
   <221> MISC_FEATURE
   <222> (1)..(1)
   <223> xaa is succinimide beta-ALanine
<220>
   <221> MISC_FEATURE
   <222> (3)..(3)
   <223> sulfated (CH2SO3H) phenylalanine
<400> 439
   Xaa Asp Phe Met Gly Trp Met Asp Phe
   1               5
<210> 440
   <211> 16
   <212> PRT
   <213> Artificial
<220>
   <223> hybird component
<220>
   <221> MISC_FEATURE
   <222> (1)..(1)
   <223> N-terminal acetyl
<220>
   <221> MISC_FEATURE
   <222> (14)..(15)
   <223> beta-Alanine beta-Alanine
<400> 440
   Ser Tyr Ser Met Glu His Phe Arg Trp Gly Lys Pro Val Xaa Xaa Lys
   1               5                   10                  15
<210> 441
   <211> 32
   <212> PRT
   <213> Artificial
<220>
   <223> hybrid component
<220>
   <221> MISC_FEATURE
   <222> (29)..(30)
   <223> beta-Alanine beta-Alanine linker
<400> 441
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Xaa Xaa Lys Cys
               20                  25                  30
<210> 442
   <211> 47
   <212> PRT
   <213> Artificial
<220>
   <223> hybrid
<220>
   <221> MISC_FEATURE
   <222> (1) .. ()
   <223> N-terminal acetyl
<220>
   <221> MISC_FEATURE
   <222> (14)..(15)
   <223> beta-Alanine beta-Alanine
<400> 442
   Ser Tyr Ser Met Glu His Phe Arg Trp Gly Lys Pro Val Xaa Xaa Lys
   1               5                   10                  15
   Cys Asn Thr Ala Thr Cys Val Leu Gly Arg Leu Ser Gln Glu Leu His
               20                  25                  30
   Arg Leu Gln Thr Tyr Pro Arg Thr Asn Thr Gly Ser Asn Thr Tyr
           35                  40                  45
<210> 443
   <211> 49
   <212> PRT
   <213> Artificial
<220>
   <223> hybrid
<220>
   <221> MISC_FEATURE
   <222> (1)..()
   <223> N-terminal acetyl
<220>
   <221> MISC_FEATURE
   <222> (14)..(15)
   <223> beta-Alanine beta-Alanine
<400> 443
   Ser Tyr Ser Met Glu His Phe Arg Trp Gly Lys Pro Val Xaa Xaa Ile
   1               5                   10                  15
   Lys Pro Glu Ala Pro Gly Glu Asp Ala Ser Pro Glu Glu Leu Asn Arg
               20                  25                  30
   Tyr Tyr Ala Ser Leu Arg His Tyr Leu Asn Leu Val Thr Arg Gln Arg
           35                  40                  45
   Tyr
<210> 444
   <211> 39
   <212> PRT
   <213> Artificial
<220>
   <223> hybrid component
<220>
   <221> MISC_FEATURE
   <222> (1)..()
   <223> thiopropyl beta-Alanine
<400> 444
   Xaa Phe Leu Phe His Tyr Ser Lys Thr Gln Lys Leu Gly Lys Ser Asn
   1               5                   10                  15
   Val Val Glu Glu Leu Gln Ser Pro Phe Ala Ser Gln Ser Arg Gly Tyr
               20                  25                  30
   Phe Leu Phe Arg Pro Arg Asn
           35
<210> 445
   <211> 39
   <212> PRT
   <213> Artificial
<220>
   <223> hybrid component
<220>
   <221> MISC_FEATURE
   <222> (1)..()
   <223> succinimide beta-Alanine
<400> 445
   Xaa Phe Leu Phe His Tyr Ser Lys Thr Gln Lys Leu Gly Lys Ser Asn
   1               5                   10                  15
   Val Val Glu Glu Leu Gln Ser Pro Phe Ala Ser Gln Ser Arg Gly Tyr
               20                  25                  30
   Phe Leu Phe Arg Pro Arg Asn
           35
<210> 446
   <211> 34
   <212> PRT
   <213> Artificial
<220>
   <223> hybrid component
<220>
   <221> MISC_FEATURE
   <222> (1)..()
   <223> N-terminal succinimide
<400> 446
   Pro Lys Pro Glu His Pro Gly Glu Asp Ala Ser Pro Glu Glu Leu Ala
   1               5                   10                  15
   Arg Tyr Tyr Ala Ser Leu Arg Ala Tyr Ile Asn Leu Ile Thr Arg Gln
               20                  25                  30
   Arg Tyr
<210> 447
   <211> 35
   <212> PRT
   <213> Artificial
<220>
   <223> hybrid component
<220>
   <221> MISC_FEATURE
   <222> (1)..()
   <223> succinimide beta-Alanine
<400> 447
   Xaa Pro Lys Pro Glu His Pro Gly Glu Asp Ala Ser Pro Glu Glu Leu
   1               5                   10                  15
   Ala Arg Tyr Tyr Ala Ser Leu Arg Ala Tyr Ile Asn Leu Ile Thr Arg
               20                  25                  30
   Gln Arg Tyr
           35
<210> 448
   <211> 41
   <212> PRT
   <213> Artificial
<220>
   <223> hybrid component
<400> 448
   Asp Pro Pro Pro Pro Pro Pro Ile Lys Pro Glu Ala Pro Gly Glu Asp
   1               5                   10                  15
   Ala Ser Pro Glu Glu Leu Asn Arg Tyr Tyr Ala Ser Leu Arg His Tyr
               20                  25                  30
   Leu Asn Leu Val Thr Arg Gln Arg Tyr
           35                  40
<210> 449
   <211> 37
   <212> PRT
   <213> Artificial
<220>
   <223> hybrid component
<220>
   <221> MISC_FEATURE
   <222> (2)..()
   <223> xaa is mini-PEG3 linker
<400> 449
   Asp Xaa Gly Ile Lys Pro Glu Ala Pro Gly Glu Asp Ala Ser Pro Glu
   1               5                   10                  15
   Glu Leu Asn Arg Tyr Tyr Ala Ser Leu Arg His Tyr Leu Asn Leu Val
               20                  25                  30
   Thr Arg Gln Arg Tyr
           35
<210> 450
   <211> 9
   <212> PRT
   <213> Artificial
<220>
   <223> hybrid component
<220>
   <221> MISC_FEATURE
   <222> (1)..()
   <223> xaa is thiopropyl beta-Alanine
<220>
   <221> MISC_FEATURE
   <222> (3)..()
   <223> sulfated (CH2SO3H) phenylalanine
<400> 450
   Xaa Asp Phe Met Gly Trp Met Asp Phe
   1               5
<210> 451
   <211> 9
   <212> PRT
   <213> Artificial
<220>
   <223> hybrid component
<220>
   <221> MISC_FEATURE
   <222> (1)..()
   <223> xaa is thiopropyl beta-Alanine
<400> 451
   Xaa Asp Tyr Met Gly Trp Met Asp Phe
   1               5
<210> 452
   <211> 35
   <212> PRT
   <213> Artificial
<220>
   <223> hybrid component
<220>
   <221> MISC_FEATURE
   <222> (1)..()
   <223> maleimide butyl beta-Alanine
<220>
   <221> MISC_FEATURE
   <222> (1)..()
   <223> xaa is maleimide butyl beta-Alanine
<400> 452
   Xaa Pro Lys Pro Glu His Pro Gly Glu Asp Ala Ser Pro Glu Glu Leu
   1 5 10 15
   Ala Arg Tyr Tyr Ala Ser Leu Arg Ala Tyr Ile Asn Leu Ile Thr Arg
   20 25 30
   Gln Arg Tyr
   35
<210> 453
   <211> 37
   <212> PRT
   <213> Artificial
<220>
   <223> hybrid component
<400> 453
   Cys Gly Gly Ile Lys Pro Glu Ala Pro Gly Glu Asp Ala Ser Pro Glu
   1 5 10 15
   Glu Leu Asn Arg Tyr Tyr Ala Ser Leu Arg His Tyr Leu Asn Leu Val
   20 25 30
   Thr Arg Gln Arg Tyr
   35
<210> 454
   <211> 37
   <212> PRT
   <213> Artificial
<220>
   <223> hybrid component
<220>
   <221> MISC_FEATURE
   <222> (2)..()
   <223> xaa is mini-PEG linker
<400> 454
   Asp Xaa Gly Ile Lys Pro Glu Ala Pro Gly Glu Asp Ala Ser Pro Glu
   1 5 10 15
   Glu Leu Asn Arg Tyr Tyr Ala Ser Leu Arg His Tyr Leu Asn Leu Val
   20 25 30
   Thr Arg Gln Arg Tyr
   35
<210> 455
   <211> 35
   <212> PRT
   <213> Artificial
<220>
   <223> hybrid component
<220>
   <221> MISC_FEATURE
   <222> (2)..()
   <223> Xaa is beta-Alanine
<400> 455
   Cys Xaa Ile Lys Pro Glu Ala Pro Gly Glu Asp Ala Ser Pro Glu Glu
   1               5                   10                  15
   Asn Arg Tyr Tyr Ala Ser Leu Arg His Tyr Leu Asn Leu Val Thr Arg
               20                  25                  30
   Gln Arg Tyr
           35
<210> 456
   <211> 36
   <212> PRT
   <213> Artificial
<220>
   <223> hybrid component
<220>
   <221> MISC_FEATURE
   <222> (2)..()
   <223> Xaa is beta-Alanine
<400> 456
   Gly Xaa Pro Lys Pro Glu His Pro Gly Glu Asp Ala Ser Pro Glu Glu
   1               5                   10                  15
   Leu Ala Arg Tyr Tyr Ala Ser Leu Arg Ala Tyr Ile Asn Leu Ile Thr
               20                  25                  30
   Arg Gln Arg Tyr
           35
<210> 457
   <211> 36
   <212> PRT
   <213> Homo sapiens
<400> 457
   Tyr Pro Ser Lys Pro Asp Asn Pro Gly Glu Asp Ala Pro Ala Glu Asp
   1               5                   10                  15
   Met Ala Arg Tyr Tyr Ser Ala Leu Arg His Tyr Ile Asn Leu Ile Thr
               20                  25                  30
   Arg Gln Arg Tyr
           35